# EUROPEAN PATENT APPLICATION

(11) **EP 2 949 660 A1**
(43) Date of publication of application: **02.12.2015**
(21) Application number: 15162372.5
(22) Date of filing: 28.12.2005
(51) Int. Cl.: C07K 14/34, C07K 14/345, C12P 13/14

(54) **L-GLUTAMIC ACID-PRODUCING MICROORGANISM AND A METHOD FOR PRODUCING L-GLUTAMIC ACID**

(30) Priority: 28.12.2004 JP 2004378615; 28.12.2004 JP 2004378604; 09.09.2005 JP 2005262087
(62) Divisional of application: 05824547.3
(71) Applicant: Ajinomoto Co., Inc., Tokyo 104-8315 (JP)
(72) Inventor: Nakamura, Jun, Kanagawa 210-8681 (JP); Hirano, Seiko, Kanagawa 210-8681 (JP); Ito, Hisao, Kanagawa 210-8681 (JP)
(74) Representative: Hoffmann Eitle

(57) **Abstract**

A coryneform bacterium that is modified by using a yggB gene so that L-glutamic acid-producing ability is enhanced as compared to a non-modified strains is cultured in a medium to cause accumulation of L-glutamic acid in the medium or bacterial cells, and L-glutamic acid is collected from the medium or cells.

SEQUENCE LISTING

<110>  Ajinomoto Co., Inc.

<120>  L-GLUTAMIC ACID-PRODUCING MICROORGANISM AND A METHOD FOR PRODUCING
L-GLUTAMIC ACID

<130>  C401-C5216

<150>  JP2004-378615
<151>  2004-12-28

<150>  JP2004-378604
<151>  2004-12-28

<150>  JP2005-262087
<151>  2005-09-09


<160>  91


<170>  PatentIn version 3.1

<210>  1
<211>  33
<212>  DNA
<213>  Artificial sequence

<220>
<223>  odhA primer

<400>  1
ggggatccat cggtatgcca caccgtggtc gcc                                    33


<210>  2
<211>  33
<212>  DNA
<213>  Artificial sequence

<220>
<223>  odhA primer

<400>  2
ggggatccac gactgcttct gcatcttcgt tgg                                    33


<210>  3
<211>  17
<212>  DNA
<213>  Artificial sequence

<220>
<223>  M1 primer

<400>  3
gtaaaacgac ggccagt                                                      17


<210>  4
<211>  25
<212>  DNA

<213>  Artificial sequence

<220>
<223>  odhA primer

<400>  4
cagaaggaat catcggtgag caggc                                          25


<210>  5
<211>  3481
<212>  DNA
<213>  Corynebacterium glutamicum

<220>
<221>  CDS
<222>  (1437)..(3035)
<223>  yggB

<400>  5
gatcttgccg atttcggcag gatcaatgtc ggcgtgaatg atcttggcat caggtgcgaa      60
agtgtcaacg tcaccggtga cgcggtcatc aaagcgggag ccgatagcaa tcagcaggtc     120
gctgcgctgc agtgcaccaa cagcggacac agtgccatgc atgcctggca tacccatgtg     180
cagctcgtgg gactctggga aggttcccag cgccatcaat gtggtgacaa ctggaatgcc     240
ggtgtgctca gcgaacgcac gaagctcttc gtgggcatca gccttgataa cgccgccgcc     300
aacgtaaagg acaggcttct tagactcacc gatcagtttg acagcctgct caatctgtcg     360
agcatgcggt gttgaaactg ggcggtagcc tggcaggtcg atctttggtg gccagacgaa     420
atccaattca gcgttctgaa catccttggg gatatccact agaacaggac cagggcgacc     480
agtaatcgcg aggtggaatg cctcagccaa tgcctgtgga atgtcgttgg ggttggtgac     540
catgaagttg tgcttggtca ctggcatggt gatgccgcgg atatcggctt cctggaaagc     600
atcggtaccc agcaggctac ttccgacctg gccggtgatg gcaaccatgg gaacggagtc     660
caagtttgca tcagcgattg gggtaaccaa gttggttgcg cctgggccag aggttgcaat     720
gcagacgcca acgcgtccag taacctgcgc gtagccggtt gctgcgtggc ctgcgccctg     780
ctcgtggcgc actaggacgt ggcgcacctt tgtggaggaa tagagcgggt catacaccgg     840
tagcaccgca ccaccaggaa taccgaacac gatgtcggcg ttaagctcct cgagcgatcg     900
aacaattgcc tgtgcacctg tcatccgctc aggggcggcg gatcgaccac ggcttgcaac     960
cgtggcggga gtgggctgtt gagaagctgc cacattcacg actttctggc tcctttacta    1020
aataaggatt ttcacaggac ccgtccaagc caagccgatt tcaactcagc ctaaagacaa    1080
agccctcatt taaaattgtt ccgacgcgga tgcgtgtgca cgcagtgcga cagatgtctg    1140
ttgcaaagtt ggctacttgg gtcataacca acaagaaagc cctcgttcca acactgtggt    1200
gagtgttgtc gagggcgctt gacgagacga cttggaaggc cgttacggca ggcgccgcgc    1260
ggttactact acaagtcgaa taatggtcat ggtgtgtcat gctacacaca tcgagtttcc    1320
aattccacaa cgcacgaaaa ttcccacccc caaaactccc ccacttcggt taaggaatca    1380
ggattctcac aaagttcagg caggctcccg ctacttttca gcgctaatct tggctc atg    1439
                                                              Met
                                                              1
att tta ggc gta ccc att caa tat ttg ctc tat tca ttg tgg aat tgg      1487
Ile Leu Gly Val Pro Ile Gln Tyr Leu Leu Tyr Ser Leu Trp Asn Trp
            5                   10                  15
att gtc gat acc ggt ttt gat gta gca att atc ctg gtc ttg gcg ttt      1535
Ile Val Asp Thr Gly Phe Asp Val Ala Ile Ile Leu Val Leu Ala Phe
        20                  25                  30
ttg att cca cgt atc ggc cga ctg gcc atg cgt att atc aag cag cga      1583
Leu Ile Pro Arg Ile Gly Arg Leu Ala Met Arg Ile Ile Lys Gln Arg
    35                  40                  45
gtg gag tct gca gcc gat gcg gac acc act aag aac cag ctc gcg ttc      1631
Val Glu Ser Ala Ala Asp Ala Asp Thr Thr Lys Asn Gln Leu Ala Phe
50                  55                  60                  65
gct ggc gtt ggc gtt tat atc gcg caa att gtg gcg ttt ttc atg ctt      1679
Ala Gly Val Gly Val Tyr Ile Ala Gln Ile Val Ala Phe Phe Met Leu
                70                  75                  80
gcc gtc tcc gcg atg cag gct ttt ggt ttc tct ctc gcg ggc gct gcg      1727
Ala Val Ser Ala Met Gln Ala Phe Gly Phe Ser Leu Ala Gly Ala Ala

            85                  90                  95
att ccg gca acc att gcg tca gct gcc att ggt ctt ggt gcg cag tcg 1775
Ile Pro Ala Thr Ile Ala Ser Ala Ala Ile Gly Leu Gly Ala Gln Ser
        100                 105                 110
att gtt gcg gac ttc ttg gcc gga ttt ttc atc ctg acg gaa aag caa 1823
Ile Val Ala Asp Phe Leu Ala Gly Phe Phe Ile Leu Thr Glu Lys Gln
    115                 120                 125
ttc ggc gtg ggt gac tgg gtg cgc ttt gag ggc aac ggc atc gtt gtt 1871
Phe Gly Val Gly Asp Trp Val Arg Phe Glu Gly Asn Gly Ile Val Val
130                 135                 140                 145
gaa ggc acc gtc att gag atc acc atg cgc gcg acc aaa att cgc acg 1919
Glu Gly Thr Val Ile Glu Ile Thr Met Arg Ala Thr Lys Ile Arg Thr
                150                 155                 160
att gca caa gag acc gtg atc atc ccg aac tcc acg gcg aaa gtg tgc 1967
Ile Ala Gln Glu Thr Val Ile Ile Pro Asn Ser Thr Ala Lys Val Cys
            165                 170                 175
atc aac aat tct aat aac tgg tcg cgt gcg gtt gtc gtt att ccg atc 2015
Ile Asn Asn Ser Asn Asn Trp Ser Arg Ala Val Val Val Ile Pro Ile
        180                 185                 190
ccc atg ttg ggt tct gaa aac atc aca gat gtc atc gcg cgc tct gaa 2063
Pro Met Leu Gly Ser Glu Asn Ile Thr Asp Val Ile Ala Arg Ser Glu
    195                 200                 205
gct gcg act cgt cgc gca ctt ggc cag gag aaa atc gca ccg gaa atc 2111
Ala Ala Thr Arg Arg Ala Leu Gly Gln Glu Lys Ile Ala Pro Glu Ile
210                 215                 220                 225
ctc ggt gaa ctc gat gtg cac cca gcc acg gaa gtc aca ccg cca acg 2159
Leu Gly Glu Leu Asp Val His Pro Ala Thr Glu Val Thr Pro Pro Thr
                230                 235                 240
gtg gtc ggc atg ccg tgg atg gtc acc atg cgt ttc ctc gtg caa gtc 2207
Val Val Gly Met Pro Trp Met Val Thr Met Arg Phe Leu Val Gln Val
            245                 250                 255
acc gcc ggc aat caa tgg ctg gtc gaa cgc gcc atc cgc aca gaa atc 2255
Thr Ala Gly Asn Gln Trp Leu Val Glu Arg Ala Ile Arg Thr Glu Ile
        260                 265                 270
atc aac gaa ttc tgg gaa gaa tac ggc agc gca acc act aca tcg gga 2303
Ile Asn Glu Phe Trp Glu Glu Tyr Gly Ser Ala Thr Thr Thr Ser Gly
    275                 280                 285
acc ctc att gat tcc tta cac gtt gag cat gaa gag cca aag acc tcg 2351
Thr Leu Ile Asp Ser Leu His Val Glu His Glu Glu Pro Lys Thr Ser
290                 295                 300                 305
ctt atc gac gcc tcc ccc cag gct ctt aag gaa ccg aag ccg gag gct 2399
Leu Ile Asp Ala Ser Pro Gln Ala Leu Lys Glu Pro Lys Pro Glu Ala
                310                 315                 320
gcg gcg acg gtt gca tcg cta gct gca tcg tct aac gac gat gca gac 2447
Ala Ala Thr Val Ala Ser Leu Ala Ala Ser Ser Asn Asp Asp Ala Asp
            325                 330                 335
aat gca gac gcc tcg gcg atc aat gca ggc aat cca gag aag gaa ctt 2495
Asn Ala Asp Ala Ser Ala Ile Asn Ala Gly Asn Pro Glu Lys Glu Leu
        340                 345                 350
gat tcc gat gtg ctg gaa caa gaa ctc tcc agc gaa gaa ccg gaa gaa 2543
Asp Ser Asp Val Leu Glu Gln Glu Leu Ser Ser Glu Glu Pro Glu Glu
    355                 360                 365
aca gca aaa cca gat cac tct ctc cga ggc ttc ttc cgc act gat tac 2591
Thr Ala Lys Pro Asp His Ser Leu Arg Gly Phe Phe Arg Thr Asp Tyr
370                 375                 380                 385
tac cca aat cgg tgg cag aag atc ctg tcg ttt ggc gga cgt gtc cgc 2639
Tyr Pro Asn Arg Trp Gln Lys Ile Leu Ser Phe Gly Gly Arg Val Arg
                390                 395                 400
atg agc act tcc ctg ttg ttg ggt gcg ctg ctc ttg ctg tca cta ttt 2687
Met Ser Thr Ser Leu Leu Leu Gly Ala Leu Leu Leu Leu Ser Leu Phe
            405                 410                 415
aag gtc atg act gtg gaa cca agt gag aat tgg caa aac tcc agt gga 2735
Lys Val Met Thr Val Glu Pro Ser Glu Asn Trp Gln Asn Ser Ser Gly

420                 425                 430
tgg ctg tca cca agc act gcc acc tca act gcg gtg acc acc tcc gaa      2783
Trp Leu Ser Pro Ser Thr Ala Thr Ser Thr Ala Val Thr Thr Ser Glu
    435                 440                 445
act tcc gcg cca gca agc acg cct tcg atg aca gtg ccc act acg gtg      2831
Thr Ser Ala Pro Ala Ser Thr Pro Ser Met Thr Val Pro Thr Thr Val
450                 455                 460                 465
gag gag acc cca acg atg gaa tct agc gtc gaa acg cag cag gaa acc      2879
Glu Glu Thr Pro Thr Met Glu Ser Ser Val Glu Thr Gln Gln Glu Thr

                470                 475                 480
tca acc cct gca acc gca acg ccc cag cga gcc gac acc atc gaa ccg      2927
Ser Thr Pro Ala Thr Ala Thr Pro Gln Arg Ala Asp Thr Ile Glu Pro
            485                 490                 495
acc gag gaa gcc acg tcg cag gag gaa acg act gca tcg cag acg cag      2975
Thr Glu Glu Ala Thr Ser Gln Glu Glu Thr Thr Ala Ser Gln Thr Gln
        500                 505                 510
tct cca gca gtg gaa gca cca acc gcg gtc caa gaa aca gtt gcg ccg      3023
Ser Pro Ala Val Glu Ala Pro Thr Ala Val Gln Glu Thr Val Ala Pro
    515                 520                 525
acg tcc acc cct taggacgctg attacagacg tgtcccattt ctttactact          3075
Thr Ser Thr Pro
530
attggaaatt atgagttcag acgcagaaaa ggcatccgtg gagctttccg aaaaatttca    3135
cccagaacgc acccatattt tgggcgccgt tgtttttggc ctgatctcat tattagtcat    3195
cggcgcagcc cctcagtacc tgttttggct gctcgcgctc cctgtcatct tcggttactg    3255
ggttctaaaa tcatccacga tcgttgatga acagggcatc accgcaaact acgccttcaa    3315
gggcaaaaag gttgtggcct gggaagacct cgcaggaatc ggattcaagg gtgcccgcac    3375
tttcgctcgc accacctccg atgcagaagt caccctcccc ggcgtcacct tcaactccct    3435
tccccgcctt gaagctgctt cccacggccg catccccgat gcgatc                   3481


<210>  6
<211>  533
<212>  PRT
<213>  Corynebacterium glutamicum

<400>  6
Met Ile Leu Gly Val Pro Ile Gln Tyr Leu Leu Tyr Ser Leu Trp Asn
1               5                   10                  15
Trp Ile Val Asp Thr Gly Phe Asp Val Ala Ile Ile Leu Val Leu Ala
            20                  25                  30
Phe Leu Ile Pro Arg Ile Gly Arg Leu Ala Met Arg Ile Ile Lys Gln
        35                  40                  45
Arg Val Glu Ser Ala Ala Asp Ala Asp Thr Thr Lys Asn Gln Leu Ala
    50                  55                  60
Phe Ala Gly Val Gly Val Tyr Ile Ala Gln Ile Val Ala Phe Phe Met
65                  70                  75                  80
Leu Ala Val Ser Ala Met Gln Ala Phe Gly Phe Ser Leu Ala Gly Ala
                85                  90                  95
Ala Ile Pro Ala Thr Ile Ala Ser Ala Ala Ile Gly Leu Gly Ala Gln
            100                 105                 110
Ser Ile Val Ala Asp Phe Leu Ala Gly Phe Phe Ile Leu Thr Glu Lys
        115                 120                 125
Gln Phe Gly Val Gly Asp Trp Val Arg Phe Glu Gly Asn Gly Ile Val
    130                 135                 140
Val Glu Gly Thr Val Ile Glu Ile Thr Met Arg Ala Thr Lys Ile Arg
145                 150                 155                 160
Thr Ile Ala Gln Glu Thr Val Ile Ile Pro Asn Ser Thr Ala Lys Val
                165                 170                 175
Cys Ile Asn Asn Ser Asn Asn Trp Ser Arg Ala Val Val Val Ile Pro
            180                 185                 190
Ile Pro Met Leu Gly Ser Glu Asn Ile Thr Asp Val Ile Ala Arg Ser

195                 200                 205
Glu Ala Ala Thr Arg Arg Ala Leu Gly Gln Glu Lys Ile Ala Pro Glu
    210                 215                 220
Ile Leu Gly Glu Leu Asp Val His Pro Ala Thr Glu Val Thr Pro Pro
225                 230                 235                 240
Thr Val Val Gly Met Pro Trp Met Val Thr Met Arg Phe Leu Val Gln
                245                 250                 255
Val Thr Ala Gly Asn Gln Trp Leu Val Glu Arg Ala Ile Arg Thr Glu
            260                 265                 270
Ile Ile Asn Glu Phe Trp Glu Glu Tyr Gly Ser Ala Thr Thr Thr Ser
        275                 280                 285
Gly Thr Leu Ile Asp Ser Leu His Val Glu His Glu Glu Pro Lys Thr
    290                 295                 300
Ser Leu Ile Asp Ala Ser Pro Gln Ala Leu Lys Glu Pro Lys Pro Glu
305                 310                 315                 320
Ala Ala Ala Thr Val Ala Ser Leu Ala Ala Ser Ser Asn Asp Asp Ala
                325                 330                 335
Asp Asn Ala Asp Ala Ser Ala Ile Asn Ala Gly Asn Pro Glu Lys Glu
            340                 345                 350
Leu Asp Ser Asp Val Leu Glu Gln Glu Leu Ser Ser Glu Glu Pro Glu
        355                 360                 365
Glu Thr Ala Lys Pro Asp His Ser Leu Arg Gly Phe Phe Arg Thr Asp
    370                 375                 380
Tyr Tyr Pro Asn Arg Trp Gln Lys Ile Leu Ser Phe Gly Gly Arg Val
385                 390                 395                 400
Arg Met Ser Thr Ser Leu Leu Leu Gly Ala Leu Leu Leu Leu Ser Leu
                405                 410                 415
Phe Lys Val Met Thr Val Glu Pro Ser Glu Asn Trp Gln Asn Ser Ser
            420                 425                 430
Gly Trp Leu Ser Pro Ser Thr Ala Thr Ser Thr Ala Val Thr Thr Ser
        435                 440                 445
Glu Thr Ser Ala Pro Ala Ser Thr Pro Ser Met Thr Val Pro Thr Thr
    450                 455                 460
Val Glu Glu Thr Pro Thr Met Glu Ser Ser Val Glu Thr Gln Gln Glu
465                 470                 475                 480
Thr Ser Thr Pro Ala Thr Ala Thr Pro Gln Arg Ala Asp Thr Ile Glu
                485                 490                 495
Pro Thr Glu Glu Ala Thr Ser Gln Glu Glu Thr Thr Ala Ser Gln Thr
            500                 505                 510
Gln Ser Pro Ala Val Glu Ala Pro Thr Ala Val Gln Glu Thr Val Ala
        515                 520                 525
Pro Thr Ser Thr Pro
    530

<210>  7
<211>  4934
<212>  DNA
<213>  Corynebacterium glutamicum

<220>
<221>  CDS
<222>  (1437)..(2705)
<223>  2A-1

<400>  7
gatcttgccg atttcggcag gatcaatgtc ggcgtgaatg atcttggcat caggtgcgaa      60
agtgtcaacg tcaccggtga cgcggtcatc aaagcgggag ccgatagcaa tcagcaggtc     120
gctgcgctgc agtgcaccaa cagcggacac agtgccatgc atgcctggca tacccatgtg     180
cagctcgtgg gactctggga aggttcccag cgccatcaat gtggtgacaa ctggaatgcc     240
ggtgtgctca gcgaacgcac gaagctcttc gtgggcatca gccttgataa cgccgccgcc     300
aacgtaaagg acaggcttct tagactcacc gatcagtttg acagcctgct caatctgtcg     360
agcatgcggt gttgaaactg ggcggtagcc tggcaggtcg atctttggtg gccagacgaa     420

atccaattca gcgttctgaa catccttggg gatatccact agaacaggac cagggcgacc 480
agtaatcgcg aggtggaatg cctcagccaa tgcctgtgga atgtcgttgg ggttggtgac 540
catgaagttg tgcttggtca ctggcatggt gatgccgcgg atatcggctt cctggaaagc 600
atcggtaccc agcaggctac ttccgacctg gccggtgatg gcaaccatgg gaacggagtc 660
caagtttgca tcagcgattg gggtaaccaa gttggttgcg cctgggccag aggttgcaat 720
gcagacgcca acgcgtccag taacctgcgc gtagccggtt gctgcgtggc ctgcgccctg 780
ctcgtggcgc actaggacgt ggcgcacctt tgtggaggaa tagagcgggt catacaccgg 840
tagcaccgca ccaccaggaa taccgaacac gatgtcggcg ttaagctcct cgagcgatcg 900
aacaattgcc tgtgcacctg tcatccgctc aggggcggcg gatcgaccac ggcttgcaac 960
cgtggcggga gtgggctgtt gagaagctgc cacattcacg actttctggc tcctttacta 1020
aataaggatt ttcacaggac ccgtccaagc caagccgatt tcaactcagc ctaaagacaa 1080
agccctcatt taaaattgtt ccgacgcgga tgcgtgtgca cgcagtgcga cagatgtctg 1140

ttgcaaagtt ggctacttgg gtcataacca acaagaaagc cctcgttcca acactgtggt 1200
gagtgttgtc gagggcgctt gacgagacga cttggaaggc cgttacggca ggcgccgcgc 1260
ggttactact acaagtcgaa taatggtcat ggtgtgtcat gctacacaca tcgagtttcc 1320
aattccacaa cgcacgaaaa ttcccacccc caaaactccc ccacttcggt taaggaatca 1380
ggattctcac aaagttcagg caggctcccg ctacttttca gcgctaatct tggctc atg 1439
Met
1
att tta ggc gta ccc att caa tat ttg ctc tat tca ttg tgg aat tgg 1487
Ile Leu Gly Val Pro Ile Gln Tyr Leu Leu Tyr Ser Leu Trp Asn Trp
            5                   10                  15
att gtc gat acc ggt ttt gat gta gca att atc ctg gtc ttg gcg ttt 1535
Ile Val Asp Thr Gly Phe Asp Val Ala Ile Ile Leu Val Leu Ala Phe
        20                  25                  30
ttg att cca cgt atc ggc cga ctg gcc atg cgt att atc aag cag cga 1583
Leu Ile Pro Arg Ile Gly Arg Leu Ala Met Arg Ile Ile Lys Gln Arg
    35                  40                  45
gtg gag tct gca gcc gat gcg gac acc act aag aac cag ctc gcg ttc 1631
Val Glu Ser Ala Ala Asp Ala Asp Thr Thr Lys Asn Gln Leu Ala Phe
50                  55                  60                  65
gct ggc gtt ggc gtt tat atc gcg caa att gtg gcg ttt ttc atg ctt 1679
Ala Gly Val Gly Val Tyr Ile Ala Gln Ile Val Ala Phe Phe Met Leu
                70                  75                  80
gcc gtc tcc gcg atg cag gct ttt ggt ttc tct ctc gcg ggc gct gcg 1727
Ala Val Ser Ala Met Gln Ala Phe Gly Phe Ser Leu Ala Gly Ala Ala
            85                  90                  95
att ccg gca acc att gcg tca gct gcc att ggt ctt ggt gcg cag tcg 1775
Ile Pro Ala Thr Ile Ala Ser Ala Ala Ile Gly Leu Gly Ala Gln Ser
        100                 105                 110
att gtt gcg gac ttc ttg gcc gga ttt ttc atc ctg acg gaa aag caa 1823
Ile Val Ala Asp Phe Leu Ala Gly Phe Phe Ile Leu Thr Glu Lys Gln
    115                 120                 125
ttc ggc gtg ggt gac tgg gtg cgc ttt gag ggc aac ggc atc gtt gtt 1871
Phe Gly Val Gly Asp Trp Val Arg Phe Glu Gly Asn Gly Ile Val Val
130                 135                 140                 145
gaa ggc acc gtc att gag atc acc atg cgc gcg acc aaa att cgc acg 1919
Glu Gly Thr Val Ile Glu Ile Thr Met Arg Ala Thr Lys Ile Arg Thr
                150                 155                 160
att gca caa gag acc gtg atc atc ccg aac tcc acg gcg aaa gtg tgc 1967
Ile Ala Gln Glu Thr Val Ile Ile Pro Asn Ser Thr Ala Lys Val Cys
            165                 170                 175
atc aac aat tct aat aac tgg tcg cgt gcg gtt gtc gtt att ccg atc 2015
Ile Asn Asn Ser Asn Asn Trp Ser Arg Ala Val Val Val Ile Pro Ile
        180                 185                 190
ccc atg ttg ggt tct gaa aac atc aca gat gtc atc gcg cgc tct gaa 2063
Pro Met Leu Gly Ser Glu Asn Ile Thr Asp Val Ile Ala Arg Ser Glu
    195                 200                 205
gct gcg act cgt cgc gca ctt ggc cag gag aaa atc gca ccg gaa atc 2111
Ala Ala Thr Arg Arg Ala Leu Gly Gln Glu Lys Ile Ala Pro Glu Ile
210                 215                 220                 225
ctc ggt gaa ctc gat gtg cac cca gcc acg gaa gtc aca ccg cca acg 2159

Leu Gly Glu Leu Asp Val His Pro Ala Thr Glu Val Thr Pro Pro Thr
            230                 235                 240
gtg gtc ggc atg ccg tgg atg gtc acc atg cgt ttc ctc gtg caa gtc     2207
Val Val Gly Met Pro Trp Met Val Thr Met Arg Phe Leu Val Gln Val
        245                 250                 255
acc gcc ggc aat caa tgg ctg gtc gaa cgc gcc atc cgc aca gaa atc     2255
Thr Ala Gly Asn Gln Trp Leu Val Glu Arg Ala Ile Arg Thr Glu Ile
        260                 265                 270
atc aac gaa ttc tgg gaa gaa tac ggc agc gca acc act aca tcg gga     2303
Ile Asn Glu Phe Trp Glu Glu Tyr Gly Ser Ala Thr Thr Thr Ser Gly
    275                 280                 285
acc ctc att gat tcc tta cac gtt gag cat gaa gag cca aag acc tcg     2351
Thr Leu Ile Asp Ser Leu His Val Glu His Glu Glu Pro Lys Thr Ser
290                 295                 300                 305
ctt atc gac gcc tcc ccc cag gct ctt aag gaa ccg aag ccg gag gct     2399
Leu Ile Asp Ala Ser Pro Gln Ala Leu Lys Glu Pro Lys Pro Glu Ala
                310                 315                 320
gcg gcg acg gtt gca tcg cta gct gca tcg tct aac gac gat gca gac     2447
Ala Ala Thr Val Ala Ser Leu Ala Ala Ser Ser Asn Asp Asp Ala Asp
            325                 330                 335
aat gca gac gcc tcg gcg atc aat gca ggc aat cca gag aag gaa ctt     2495
Asn Ala Asp Ala Ser Ala Ile Asn Ala Gly Asn Pro Glu Lys Glu Leu
        340                 345                 350
gat tcc gat gtg ctg gaa caa gaa ctc tcc agc gaa gaa ccg gaa gaa     2543
Asp Ser Asp Val Leu Glu Gln Glu Leu Ser Ser Glu Glu Pro Glu Glu
    355                 360                 365
aca gca aaa cca gat cac tct ctc cga ggc ttc ttc cgc act gat tac     2591
Thr Ala Lys Pro Asp His Ser Leu Arg Gly Phe Phe Arg Thr Asp Tyr
370                 375                 380                 385
tac cca aat cgg tgg cag aag atc ctg tcg ttt ggc gga cgt gtc cgc     2639
Tyr Pro Asn Arg Trp Gln Lys Ile Leu Ser Phe Gly Gly Arg Val Arg
                390                 395                 400
atg agc act tcc ctg ttg ttg ggt gcg ctg ctc ttg ctg tca cta ttt     2687
Met Ser Thr Ser Leu Leu Leu Gly Ala Leu Leu Leu Leu Ser Leu Phe
            405                 410                 415
aag ggg ctc ttc ctg ttt tagagtgcat tgatcttatg gaccaactgc            2735
Lys Gly Leu Phe Leu Phe
        420
cctgaatgga taaggcaccg cagaatgtag tggttcaaat tacggaaacc tagagcaatc   2795
ccacgcaaat gctccaaccg tccgttgatc gcttcgaccg gaccgttgga gacaccaaca   2855
tcgaaatacg ccaacacatc accaagtcgt ttaaacaaac tacgacccaa ctgcgcgagt   2915
tccttattcg gccccttcaa cacccgaagc tgatcaataa tggtccgcat tttcttcttc   2975
gcttcacgct tattacccat ctgataacaa tcaataatcg cctgatacgc aagccacgca   3035
agctttaaca ccccgtagtc tttgtcatac gcccacaact gctccaagct ttcttgctga   3095
cgaggactca accacttgtg cgtggtcaac aaggtcttcc ggtttttata caacggatcc   3155
tggcttaaac cacgacgctg gtatttctcc cgctggaggc gttgccggca ggcggtgagc   3215
ttgtcaccag caagccgcac aacatggaat ggatccatca cgcgacgagc agaaggaatg   3275
agttctttac ttgctgtggc gtagccttgg aacccatcca tggacacgat ccgtatctga   3335
ttgcggaact gttcaccgcg ggaaccaagc caggaccgta aagcatcagc actacgacct   3395
gggacgacat ctaataaccg ggcaggacac cgtgagtcat accgatgccc ggtcatatcg   3455
acaatcacgg tgacaaaccc atcaccatgc ttagccctat tatgtgacca cttatgctca   3515
tccaccccaa tgacatacac tccatcaaga tggtgaggat cgttatagac cagctcacgg   3575
cacatatcga gggctagttg gcaggttaaa tcccacccta gcccaagtgc tttcgcggtt   3635
gcgtgaacac tcatccggtc aatagcaagg cgttgcaaaa tccagcgggt gacccggtgg   3695
gtgacctttt taccgtggtc agcgcagctt agttctgctt ggaaatactt ttgcttacat   3755
gtcgggttgg tgcagcggta gcgaggtaga cggataaaca gtttggtggg aaacccgacg   3815

atgggtaaat caatgagcat ccggtgggtg tgatgacgaa acaccccagg ttgggagcat   3875
tctggcaggt tggaggtata gtcgagtgcg tctgcttcga tcagggtgta atcacctgca   3935
tcggaagcgc cggtgatggt gagtcctagt tccgcagtgc ggcagatggt gtcagcgatg   3995
atgttgccgg tagacttcat gggtagagcc ttttgttggt gtttggttag cttagatacc   4055
taaaccttaa ccctgacaaa aggctcgttt attttcgggt ctacaccgct agcccaggtt   4115
ctgtgatgta ccccaaaacc ggaagggcct catgactgtg gaaccaagtg agaattggca   4175

aaactccagt ggatggctgt caccaagcac tgccacctca actgcggtga ccacctccga 4235
aacttccgcg ccagcaagca cgccttcgat gacagtgccc actacggtgg aggagacccc 4295
aacgatggaa tctagcgtcg aaacgcagca ggaaacctca acccctgcaa ccgcaacgcc 4355
ccagcgagcc gacaccatcg aaccgaccga ggaagccacg tcgcaggagg aaacgactgc 4415
atcgcagacg cagtctccag cagtggaagc accaaccgcg gtccaagaaa cagttgcgcc 4475
gacgtccacc ccttaggacg ctgattacag acgtgtccca tttctttact actattggaa 4535
attatgagtt cagacgcaga aaaggcatcc gtggagcttt ccgaaaaatt tcacccagaa 4595
cgcacccata ttttgggcgc cgttgttttt ggcctgatct cattattagt catcggcgca 4655
gcccctcagt acctgttttg gctgctcgcg ctccctgtca tcttcggtta ctgggttcta 4715
aaatcatcca cgatcgttga tgaacagggc atcaccgcaa actacgcctt caagggcaaa 4775
aaggttgtgg cctgggaaga cctcgcagga atcggattca agggtgcccg cactttcgct 4835
cgcaccacct ccgatgcaga agtcaccctc cccggcgtca ccttcaactc ccttccccgc 4895
cttgaagctg cttcccacgg ccgcatcccc gatgcgatc 4934

<210> 8
<211> 423
<212> PRT
<213> Corynebacterium glutamicum

<400> 8
   Met Ile Leu Gly Val Pro Ile Gln Tyr Leu Leu Tyr Ser Leu Trp Asn
   1 5 10 15
   Trp Ile Val Asp Thr Gly Phe Asp Val Ala Ile Ile Leu Val Leu Ala
   20 25 30
   Phe Leu Ile Pro Arg Ile Gly Arg Leu Ala Met Arg Ile Ile Lys Gln
   35 40 45
   Arg Val Glu Ser Ala Ala Asp Ala Asp Thr Thr Lys Asn Gln Leu Ala
   50 55 60
   Phe Ala Gly Val Gly Val Tyr Ile Ala Gln Ile Val Ala Phe Phe Met
   65 70 75 80
   Leu Ala Val Ser Ala Met Gln Ala Phe Gly Phe Ser Leu Ala Gly Ala
   85 90 95
   Ala Ile Pro Ala Thr Ile Ala Ser Ala Ala Ile Gly Leu Gly Ala Gln
   100 105 110
   Ser Ile Val Ala Asp Phe Leu Ala Gly Phe Phe Ile Leu Thr Glu Lys
   115 120 125
   Gln Phe Gly Val Gly Asp Trp Val Arg Phe Glu Gly Asn Gly Ile Val
   130 135 140
   Val Glu Gly Thr Val Ile Glu Ile Thr Met Arg Ala Thr Lys Ile Arg
   145 150 155 160
   Thr Ile Ala Gln Glu Thr Val Ile Ile Pro Asn Ser Thr Ala Lys Val
   165 170 175
   Cys Ile Asn Asn Ser Asn Asn Trp Ser Arg Ala Val Val Val Ile Pro
   180 185 190
   Ile Pro Met Leu Gly Ser Glu Asn Ile Thr Asp Val Ile Ala Arg Ser
   195 200 205
   Glu Ala Ala Thr Arg Arg Ala Leu Gly Gln Glu Lys Ile Ala Pro Glu
   210 215 220
   Ile Leu Gly Glu Leu Asp Val His Pro Ala Thr Glu Val Thr Pro Pro
   225 230 235 240
   Thr Val Val Gly Met Pro Trp Met Val Thr Met Arg Phe Leu Val Gln
   245 250 255
   Val Thr Ala Gly Asn Gln Trp Leu Val Glu Arg Ala Ile Arg Thr Glu
   260 265 270
   Ile Ile Asn Glu Phe Trp Glu Glu Tyr Gly Ser Ala Thr Thr Thr Ser
   275 280 285
   Gly Thr Leu Ile Asp Ser Leu His Val Glu His Glu Glu Pro Lys Thr
   290 295 300
   Ser Leu Ile Asp Ala Ser Pro Gln Ala Leu Lys Glu Pro Lys Pro Glu
   305 310 315 320
   Ala Ala Ala Thr Val Ala Ser Leu Ala Ala Ser Ser Asn Asp Asp Ala
   325 330 335
   Asp Asn Ala Asp Ala Ser Ala Ile Asn Ala Gly Asn Pro Glu Lys Glu
               340                 345                 350
   Leu Asp Ser Asp Val Leu Glu Gln Glu Leu Ser Ser Glu Glu Pro Glu
           355                 360                 365
   Glu Thr Ala Lys Pro Asp His Ser Leu Arg Gly Phe Phe Arg Thr Asp
       370                 375                 380
   Tyr Tyr Pro Asn Arg Trp Gln Lys Ile Leu Ser Phe Gly Gly Arg Val
   385                 390                 395                 400
   Arg Met Ser Thr Ser Leu Leu Leu Gly Ala Leu Leu Leu Leu Ser Leu
                   405                 410                 415
   Phe Lys Gly Leu Phe Leu Phe
               420

<210>  9
<211>  28
<212>  DNA
<213>  Artificial sequence

<220>
<223>  primer

<400>  9
gggagctcga ctttctggct cctttact                                          28

<210>  10
<211>  28
<212>  DNA
<213>  Artificial sequence

<220>
<223>  primer

<400>  10
gggagctcgc cgatgactaa taatgaga                                          28

<210>  11
<211>  2014
<212>  DNA
<213>  Bacillus subtilis

<220>
<221>  CDS
<222>  (464)..(1885)
<223>  sacB

<400>  11
gatccttttt aacccatcac atatacctgc cgttcactat tatttagtga aatgagatat      60
tatgatattt tctgaattgt gattaaaaag gcaactttat gcccatgcaa cagaaactat     120
aaaaaataca gagaatgaaa agaaacagat agatttttta gttctttagg cccgtagtct     180
gcaaatcctt ttatgatttt ctatcaaaca aaagaggaaa atagaccagt tgcaatccaa     240
acgagagtct aatagaatga ggtcgaaaag taaatcgcgc gggtttgtta ctgataaagc     300
aggcaagacc taaaatgtgt aaagggcaaa gtgtatactt tggcgtcacc ccttacatat     360
tttaggtctt tttttattgt gcgtaactaa cttgccatct tcaaacagga gggctggaag     420
aagcagaccg ctaacacagt acataaaaaa ggagacatga acg atg aac atc aaa       475
                                                Met Asn Ile Lys
                                                1
aag ttt gca aaa caa gca aca gta tta acc ttt act acc gca ctg ctg       523
Lys Phe Ala Lys Gln Ala Thr Val Leu Thr Phe Thr Thr Ala Leu Leu
5                   10                  15                  20
gca gga ggc gca act caa gcg ttt gcg aaa gaa acg aac caa aag cca       571

Ala Gly Gly Ala Thr Gln Ala Phe Ala Lys Glu Thr Asn Gln Lys Pro
            25                  30                  35
tat aag gaa aca tac ggc att tcc cat att aca cgc cat gat atg ctg      619
Tyr Lys Glu Thr Tyr Gly Ile Ser His Ile Thr Arg His Asp Met Leu
        40                  45                  50
caa atc cct gaa cag caa aaa aat gaa aaa tat caa gtt cct gaa ttc      667
Gln Ile Pro Glu Gln Gln Lys Asn Glu Lys Tyr Gln Val Pro Glu Phe
    55                  60                  65
gat tcg tcc aca att aaa aat atc tct tct gca aaa ggc ctg gac gtt      715
Asp Ser Ser Thr Ile Lys Asn Ile Ser Ser Ala Lys Gly Leu Asp Val
    70                  75                  80
tgg gac agc tgg cca tta caa aac gct gac ggc act gtc gca aac tat      763
Trp Asp Ser Trp Pro Leu Gln Asn Ala Asp Gly Thr Val Ala Asn Tyr
85                  90                  95                  100
cac ggc tac cac atc gtc ttt gca tta gcc gga gat cct aaa aat gcg      811
His Gly Tyr His Ile Val Phe Ala Leu Ala Gly Asp Pro Lys Asn Ala
                105                 110                 115
gat gac aca tcg att tac atg ttc tat caa aaa gtc ggc gaa act tct      859
Asp Asp Thr Ser Ile Tyr Met Phe Tyr Gln Lys Val Gly Glu Thr Ser
            120                 125                 130
att gac agc tgg aaa aac gct ggc cgc gtc ttt aaa gac agc gac aaa      907
Ile Asp Ser Trp Lys Asn Ala Gly Arg Val Phe Lys Asp Ser Asp Lys
        135                 140                 145
ttc gat gca aat gat tct atc cta aaa gac caa aca caa gaa tgg tca      955
Phe Asp Ala Asn Asp Ser Ile Leu Lys Asp Gln Thr Gln Glu Trp Ser
    150                 155                 160
ggt tca gcc aca ttt aca tct gac gga aaa atc cgt tta ttc tac act      1003
Gly Ser Ala Thr Phe Thr Ser Asp Gly Lys Ile Arg Leu Phe Tyr Thr
165                 170                 175                 180
gat ttc tcc ggt aaa cat tac ggc aaa caa aca ctg aca act gca caa      1051
Asp Phe Ser Gly Lys His Tyr Gly Lys Gln Thr Leu Thr Thr Ala Gln
                185                 190                 195
gtt aac gta tca gca tca gac agc tct ttg aac atc aac ggt gta gag      1099
Val Asn Val Ser Ala Ser Asp Ser Ser Leu Asn Ile Asn Gly Val Glu
            200                 205                 210
gat tat aaa tca atc ttt gac ggt gac gga aaa acg tat caa aat gta      1147
Asp Tyr Lys Ser Ile Phe Asp Gly Asp Gly Lys Thr Tyr Gln Asn Val
        215                 220                 225
cag cag ttc atc gat gaa ggc aac tac agc tca ggc gac aac cat acg      1195
Gln Gln Phe Ile Asp Glu Gly Asn Tyr Ser Ser Gly Asp Asn His Thr
    230                 235                 240
ctg aga gat cct cac tac gta gaa gat aaa ggc cac aaa tac tta gta      1243
Leu Arg Asp Pro His Tyr Val Glu Asp Lys Gly His Lys Tyr Leu Val
245                 250                 255                 260
ttt gaa gca aac act gga act gaa gat ggc tac caa ggc gaa gaa tct      1291
Phe Glu Ala Asn Thr Gly Thr Glu Asp Gly Tyr Gln Gly Glu Glu Ser
                265                 270                 275
tta ttt aac aaa gca tac tat ggc aaa agc aca tca ttc ttc cgt caa      1339
Leu Phe Asn Lys Ala Tyr Tyr Gly Lys Ser Thr Ser Phe Phe Arg Gln
            280                 285                 290
gaa agt caa aaa ctt ctg caa agc gat aaa aaa cgc acg gct gag tta      1387
Glu Ser Gln Lys Leu Leu Gln Ser Asp Lys Lys Arg Thr Ala Glu Leu
        295                 300                 305
gca aac ggc gct ctc ggt atg att gag cta aac gat gat tac aca ctg      1435
Ala Asn Gly Ala Leu Gly Met Ile Glu Leu Asn Asp Asp Tyr Thr Leu
    310                 315                 320
aaa aaa gtg atg aaa ccg ctg att gca tct aac aca gta aca gat gaa      1483
Lys Lys Val Met Lys Pro Leu Ile Ala Ser Asn Thr Val Thr Asp Glu
325                 330                 335                 340
att gaa cgc gcg aac gtc ttt aaa atg aac ggc aaa tgg tac ctg ttc      1531
Ile Glu Arg Ala Asn Val Phe Lys Met Asn Gly Lys Trp Tyr Leu Phe
                345                 350                 355
act gac tcc cgc gga tca aaa atg acg att gac ggc att acg tct aac      1579

Thr Asp Ser Arg Gly Ser Lys Met Thr Ile Asp Gly Ile Thr Ser Asn
            360                 365                 370
gat att tac atg ctt ggt tat gtt tct aat tct tta act ggc cca tac      1627
Asp Ile Tyr Met Leu Gly Tyr Val Ser Asn Ser Leu Thr Gly Pro Tyr
        375                 380                 385
aag ccg ctg aac aaa act ggc ctt gtg tta aaa atg gat ctt gat cct      1675
Lys Pro Leu Asn Lys Thr Gly Leu Val Leu Lys Met Asp Leu Asp Pro
    390                 395                 400
aac gat gta acc ttt act tac tca cac ttc gct gta cct caa gcg aaa      1723
Asn Asp Val Thr Phe Thr Tyr Ser His Phe Ala Val Pro Gln Ala Lys
405                 410                 415                 420
gga aac aat gtc gtg att aca agc tat atg aca aac aga gga ttc tac      1771
Gly Asn Asn Val Val Ile Thr Ser Tyr Met Thr Asn Arg Gly Phe Tyr
                425                 430                 435
gca gac aaa caa tca acg ttt gcg cca agc ttc ctg ctg aac atc aaa      1819
Ala Asp Lys Gln Ser Thr Phe Ala Pro Ser Phe Leu Leu Asn Ile Lys
            440                 445                 450
ggc aag aaa aca tct gtt gtc aaa gac agc atc ctt gaa caa gga caa      1867
Gly Lys Lys Thr Ser Val Val Lys Asp Ser Ile Leu Glu Gln Gly Gln
        455                 460                 465
tta aca gtt aac aaa taa aaacgcaaaa gaaaatgccg atatcctatt             1915
Leu Thr Val Asn Lys
    470
ggcattttct tttatttctt atcaacataa aggtgaatcc catatgaact atataaaagc    1975
aggcaaatgg ctaaccgtat tcctaacctt ttgaagatc                           2014


<210>  12
<211>  473
<212>  PRT
<213>  Bacillus subtilis

<400>  12
Met Asn Ile Lys Lys Phe Ala Lys Gln Ala Thr Val Leu Thr Phe Thr
1               5                   10                  15
Thr Ala Leu Leu Ala Gly Gly Ala Thr Gln Ala Phe Ala Lys Glu Thr
            20                  25                  30
Asn Gln Lys Pro Tyr Lys Glu Thr Tyr Gly Ile Ser His Ile Thr Arg
        35                  40                  45
His Asp Met Leu Gln Ile Pro Glu Gln Gln Lys Asn Glu Lys Tyr Gln
    50                  55                  60
Val Pro Glu Phe Asp Ser Ser Thr Ile Lys Asn Ile Ser Ser Ala Lys
65                  70                  75                  80
Gly Leu Asp Val Trp Asp Ser Trp Pro Leu Gln Asn Ala Asp Gly Thr
                85                  90                  95
Val Ala Asn Tyr His Gly Tyr His Ile Val Phe Ala Leu Ala Gly Asp
            100                 105                 110
Pro Lys Asn Ala Asp Asp Thr Ser Ile Tyr Met Phe Tyr Gln Lys Val
        115                 120                 125
Gly Glu Thr Ser Ile Asp Ser Trp Lys Asn Ala Gly Arg Val Phe Lys
    130                 135                 140
Asp Ser Asp Lys Phe Asp Ala Asn Asp Ser Ile Leu Lys Asp Gln Thr
145                 150                 155                 160
Gln Glu Trp Ser Gly Ser Ala Thr Phe Thr Ser Asp Gly Lys Ile Arg
                165                 170                 175
Leu Phe Tyr Thr Asp Phe Ser Gly Lys His Tyr Gly Lys Gln Thr Leu
            180                 185                 190
Thr Thr Ala Gln Val Asn Val Ser Ala Ser Asp Ser Ser Leu Asn Ile
        195                 200                 205
Asn Gly Val Glu Asp Tyr Lys Ser Ile Phe Asp Gly Asp Gly Lys Thr
    210                 215                 220
Tyr Gln Asn Val Gln Gln Phe Ile Asp Glu Gly Asn Tyr Ser Ser Gly
225                 230                 235                 240

Asp Asn His Thr Leu Arg Asp Pro His Tyr Val Glu Asp Lys Gly His
                245                 250                 255
Lys Tyr Leu Val Phe Glu Ala Asn Thr Gly Thr Glu Asp Gly Tyr Gln
            260                 265                 270
Gly Glu Glu Ser Leu Phe Asn Lys Ala Tyr Tyr Gly Lys Ser Thr Ser
        275                 280                 285
Phe Phe Arg Gln Glu Ser Gln Lys Leu Leu Gln Ser Asp Lys Lys Arg
    290                 295                 300
Thr Ala Glu Leu Ala Asn Gly Ala Leu Gly Met Ile Glu Leu Asn Asp
305                 310                 315                 320
Asp Tyr Thr Leu Lys Lys Val Met Lys Pro Leu Ile Ala Ser Asn Thr
                325                 330                 335
Val Thr Asp Glu Ile Glu Arg Ala Asn Val Phe Lys Met Asn Gly Lys
            340                 345                 350
Trp Tyr Leu Phe Thr Asp Ser Arg Gly Ser Lys Met Thr Ile Asp Gly
        355                 360                 365
Ile Thr Ser Asn Asp Ile Tyr Met Leu Gly Tyr Val Ser Asn Ser Leu
    370                 375                 380
Thr Gly Pro Tyr Lys Pro Leu Asn Lys Thr Gly Leu Val Leu Lys Met
385                 390                 395                 400
Asp Leu Asp Pro Asn Asp Val Thr Phe Thr Tyr Ser His Phe Ala Val
                405                 410                 415
Pro Gln Ala Lys Gly Asn Asn Val Val Ile Thr Ser Tyr Met Thr Asn
            420                 425                 430
Arg Gly Phe Tyr Ala Asp Lys Gln Ser Thr Phe Ala Pro Ser Phe Leu
        435                 440                 445
Leu Asn Ile Lys Gly Lys Lys Thr Ser Val Val Lys Asp Ser Ile Leu
    450                 455                 460
Glu Gln Gly Gln Leu Thr Val Asn Lys
465                 470


<210>  13
<211>  24
<212>  DNA
<213>  Artificial sequence



<220>
<223>  sacB primer

<400>  13
cgggatcctt tttaacccat caca                                          24


<210>  14
<211>  29
<212>  DNA
<213>  Artificial sequence

<220>
<223>  sacB primer

<400>  14
gaagatcttc aaaaggttag gaatacggt                                     29


<210>  15
<211>  23
<212>  DNA
<213>  Artificial sequence

<220>

<223>  sacB primer

<400>  15
ccttttgaag atcgaccagt tgg                                              23


<210>  16
<211>  44
<212>  DNA
<213>  Artificial sequence

<220>
<223>  sacB primer

<400>  16
tacctggaat gctgttttcc cagggatcgc agtggtgagt aacc                       44


<210>  17
<211>  28
<212>  DNA
<213>  Artificial sequence

<220>
<223>  sacB primer

<400>  17
cctgggaaaa cagcattcca ggtattag                                         28


<210>  18
<211>  23
<212>  DNA
<213>  Artificial sequence

<220>
<223>  sacB primer

<400>  18
tgcaggtcga ctctagagga tcc                                              23


<210>  19
<211>  3490
<212>  DNA
<213>  Corynebacterium glutamicum

<220>
<221>  CDS
<222>  (1437)..(3044)
<223>  A1

<400>  19
gatcttgccg atttcggcag gatcaatgtc ggcgtgaatg atcttggcat caggtgcgaa      60
agtgtcaacg tcaccggtga cgcggtcatc aaagcgggag ccgatagcaa tcagcaggtc     120
gctgcgctgc agtgcaccaa cagcggacac agtgccatgc atgcctggca tacccatgtg     180
cagctcgtgg gactctggga aggttcccag cgccatcaat gtggtgacaa ctggaatgcc     240
ggtgtgctca gcgaacgcac gaagctcttc gtgggcatca gccttgataa cgccgccgcc     300
aacgtaaagg acaggcttct tagactcacc gatcagtttg acagcctgct caatctgtcg     360
agcatgcggt gttgaaactg ggcggtagcc tggcaggtcg atctttggtg gccagacgaa     420
atccaattca gcgttctgaa catccttggg gatatccact agaacaggac cagggcgacc     480
agtaatcgcg aggtggaatg cctcagccaa tgcctgtgga atgtcgttgg ggttggtgac     540
catgaagttg tgcttggtca ctggcatggt gatgccgcgg atatcggctt cctggaaagc     600

atcggtaccc agcaggctac ttccgacctg gccggtgatg gcaaccatgg gaacggagtc 660
caagtttgca tcagcgattg gggtaaccaa gttggttgcg cctgggccag aggttgcaat 720
gcagacgcca acgcgtccag taacctgcgc gtagccggtt gctgcgtggc ctgcgccctg 780
ctcgtggcgc actaggacgt ggcgcacctt tgtggaggaa tagagcgggt catacaccgg 840
tagcaccgca ccaccaggaa taccgaacac gatgtcggcg ttaagctcct cgagcgatcg 900
aacaattgcc tgtgcacctg tcatccgctc aggggcggcg gatcgaccac ggcttgcaac 960
cgtggcggga gtgggctgtt gagaagctgc cacattcacg actttctggc tcctttacta 1020
aataaggatt ttcacaggac ccgtccaagc caagccgatt tcaactcagc ctaaagacaa 1080
agccctcatt taaaattgtt ccgacgcgga tgcgtgtgca cgcagtgcga cagatgtctg 1140
ttgcaaagtt ggctacttgg gtcataacca acaagaaagc cctcgttcca acactgtggt 1200
gagtgttgtc gagggcgctt gacgagacga cttggaaggc cgttacggca ggcgccgcgc 1260
ggttactact acaagtcgaa taatggtcat ggtgtgtcat gctacacaca tcgagtttcc 1320
aattccacaa cgcacgaaaa ttcccacccc caaaactccc ccacttcggt taaggaatca 1380
ggattctcac aaagttcagg caggctcccg ctacttttca gcgctaatct tggctc atg 1439
Met
1
att tta ggc gta ccc att caa tat ttg ctc tat tca ttg tgt tca ttg 1487
Ile Leu Gly Val Pro Ile Gln Tyr Leu Leu Tyr Ser Leu Cys Ser Leu
            5                   10                  15
tgg aat tgg att gtc gat acc ggt ttt gat gta gca att atc ctg gtc 1535
Trp Asn Trp Ile Val Asp Thr Gly Phe Asp Val Ala Ile Ile Leu Val
        20                  25                  30
ttg gcg ttt ttg att cca cgt atc ggc cga ctg gcc atg cgt att atc 1583
Leu Ala Phe Leu Ile Pro Arg Ile Gly Arg Leu Ala Met Arg Ile Ile
    35                  40                  45
aag cag cga gtg gag tct gca gcc gat gcg gac acc act aag aac cag 1631
Lys Gln Arg Val Glu Ser Ala Ala Asp Ala Asp Thr Thr Lys Asn Gln
50                  55                  60                  65
ctc gcg ttc gct ggc gtt ggc gtt tat atc gcg caa att gtg gcg ttt 1679
Leu Ala Phe Ala Gly Val Gly Val Tyr Ile Ala Gln Ile Val Ala Phe
                70                  75                  80
ttc atg ctt gcc gtc tcc gcg atg cag gct ttt ggt ttc tct ctc gcg 1727
Phe Met Leu Ala Val Ser Ala Met Gln Ala Phe Gly Phe Ser Leu Ala
            85                  90                  95
ggc gct gcg att ccg gca acc att gcg tca gct gcc att ggt ctt ggt 1775
Gly Ala Ala Ile Pro Ala Thr Ile Ala Ser Ala Ala Ile Gly Leu Gly
        100                 105                 110
gcg cag tcg att gtt gcg gac ttc ttg gcc gga ttt ttc atc ctg acg 1823
Ala Gln Ser Ile Val Ala Asp Phe Leu Ala Gly Phe Phe Ile Leu Thr
    115                 120                 125
gaa aag caa ttc ggc gtg ggt gac tgg gtg cgc ttt gag ggc aac ggc 1871
Glu Lys Gln Phe Gly Val Gly Asp Trp Val Arg Phe Glu Gly Asn Gly
130                 135                 140                 145
atc gtt gtt gaa ggc acc gtc att gag atc acc atg cgc gcg acc aaa 1919
Ile Val Val Glu Gly Thr Val Ile Glu Ile Thr Met Arg Ala Thr Lys
                150                 155                 160
att cgc acg att gca caa gag acc gtg atc atc ccg aac tcc acg gcg 1967
Ile Arg Thr Ile Ala Gln Glu Thr Val Ile Ile Pro Asn Ser Thr Ala
            165                 170                 175
aaa gtg tgc atc aac aat tct aat aac tgg tcg cgt gcg gtt gtc gtt 2015
Lys Val Cys Ile Asn Asn Ser Asn Asn Trp Ser Arg Ala Val Val Val
        180                 185                 190
att ccg atc ccc atg ttg ggt tct gaa aac atc aca gat gtc atc gcg 2063
Ile Pro Ile Pro Met Leu Gly Ser Glu Asn Ile Thr Asp Val Ile Ala
    195                 200                 205
cgc tct gaa gct gcg act cgt cgc gca ctt ggc cag gag aaa atc gca 2111
Arg Ser Glu Ala Ala Thr Arg Arg Ala Leu Gly Gln Glu Lys Ile Ala
210                 215                 220                 225
ccg gaa atc ctc ggt gaa ctc gat gtg cac cca gcc acg gaa gtc aca 2159
Pro Glu Ile Leu Gly Glu Leu Asp Val His Pro Ala Thr Glu Val Thr
                230                 235                 240
ccg cca acg gtg gtc ggc atg ccg tgg atg gtc acc atg cgt ttc ctc 2207
Pro Pro Thr Val Val Gly Met Pro Trp Met Val Thr Met Arg Phe Leu

245                 250                 255
gtg caa gtc acc gcc ggc aat caa tgg ctg gtc gaa cgc gcc atc cgc      2255
Val Gln Val Thr Ala Gly Asn Gln Trp Leu Val Glu Arg Ala Ile Arg
        260                 265                 270
aca gaa atc atc aac gaa ttc tgg gaa gaa tac ggc agc gca acc act      2303
Thr Glu Ile Ile Asn Glu Phe Trp Glu Glu Tyr Gly Ser Ala Thr Thr
    275                 280                 285
aca tcg gga acc ctc att gat tcc tta cac gtt gag cat gaa gag cca      2351
Thr Ser Gly Thr Leu Ile Asp Ser Leu His Val Glu His Glu Glu Pro
290                 295                 300                 305
aag acc tcg ctt atc gac gcc tcc ccc cag gct ctt aag gaa ccg aag      2399
Lys Thr Ser Leu Ile Asp Ala Ser Pro Gln Ala Leu Lys Glu Pro Lys
                310                 315                 320
ccg gag gct gcg gcg acg gtt gca tcg cta gct gca tcg tct aac gac      2447
Pro Glu Ala Ala Ala Thr Val Ala Ser Leu Ala Ala Ser Ser Asn Asp
            325                 330                 335
gat gca gac aat gca gac gcc tcg gcg atc aat gca ggc aat cca gag      2495
Asp Ala Asp Asn Ala Asp Ala Ser Ala Ile Asn Ala Gly Asn Pro Glu
        340                 345                 350
aag gaa ctt gat tcc gat gtg ctg gaa caa gaa ctc tcc agc gaa gaa      2543
Lys Glu Leu Asp Ser Asp Val Leu Glu Gln Glu Leu Ser Ser Glu Glu
    355                 360                 365
ccg gaa gaa aca gca aaa cca gat cac tct ctc cga ggc ttc ttc cgc      2591
Pro Glu Glu Thr Ala Lys Pro Asp His Ser Leu Arg Gly Phe Phe Arg
370                 375                 380                 385
act gat tac tac cca aat cgg tgg cag aag atc ctg tcg ttt ggc gga      2639
Thr Asp Tyr Tyr Pro Asn Arg Trp Gln Lys Ile Leu Ser Phe Gly Gly
                390                 395                 400
cgt gtc cgc atg agc act tcc ctg ttg ttg ggt gcg ctg ctc ttg ctg      2687
Arg Val Arg Met Ser Thr Ser Leu Leu Leu Gly Ala Leu Leu Leu Leu
            405                 410                 415
tca cta ttt aag gtc atg act gtg gaa cca agt gag aat tgg caa aac      2735
Ser Leu Phe Lys Val Met Thr Val Glu Pro Ser Glu Asn Trp Gln Asn
        420                 425                 430
tcc agt gga tgg ctg tca cca agc act gcc acc tca act gcg gtg acc      2783
Ser Ser Gly Trp Leu Ser Pro Ser Thr Ala Thr Ser Thr Ala Val Thr
    435                 440                 445
acc tcc gaa act tcc gcg cca gca agc acg cct tcg atg aca gtg ccc      2831
Thr Ser Glu Thr Ser Ala Pro Ala Ser Thr Pro Ser Met Thr Val Pro
450                 455                 460                 465
act acg gtg gag gag acc cca acg atg gaa tct agc gtc gaa acg cag      2879
Thr Thr Val Glu Glu Thr Pro Thr Met Glu Ser Ser Val Glu Thr Gln
                470                 475                 480
cag gaa acc tca acc cct gca acc gca acg ccc cag cga gcc gac acc      2927
Gln Glu Thr Ser Thr Pro Ala Thr Ala Thr Pro Gln Arg Ala Asp Thr
            485                 490                 495
atc gaa ccg acc gag gaa gcc acg tcg cag gag gaa acg act gca tcg      2975
Ile Glu Pro Thr Glu Glu Ala Thr Ser Gln Glu Glu Thr Thr Ala Ser
        500                 505                 510
cag acg cag tct cca gca gtg gaa gca cca acc gcg gtc caa gaa aca      3023
Gln Thr Gln Ser Pro Ala Val Glu Ala Pro Thr Ala Val Gln Glu Thr
    515                 520                 525
gtt gcg ccg acg tcc acc cct taggacgctg attacagacg tgtcccattt      3074
Val Ala Pro Thr Ser Thr Pro
530                 535
ctttactact attggaaatt atgagttcag acgcagaaaa ggcatccgtg gagctttccg      3134
aaaaatttca cccagaacgc acccatattt tgggcgccgt tgtttttggc ctgatctcat      3194
tattagtcat cggcgcagcc cctcagtacc tgttttggct gctcgcgctc cctgtcatct      3254
tcggttactg ggttctaaaa tcatccacga tcgttgatga acagggcatc accgcaaact      3314
acgccttcaa gggcaaaaag gttgtggcct gggaagacct cgcaggaatc ggattcaagg      3374
gtgcccgcac tttcgctcgc accacctccg atgcagaagt caccctcccc ggcgtcacct      3434
tcaactccct tccccgcctt gaagctgctt cccacggccg catccccgat gcgatc      3490

<210>  20
<211>  536
<212>  PRT
<213>  Corynebacterium glutamicum

<400>  20
Met Ile Leu Gly Val Pro Ile Gln Tyr Leu Leu Tyr Ser Leu Cys Ser
1               5                   10                  15
Leu Trp Asn Trp Ile Val Asp Thr Gly Phe Asp Val Ala Ile Ile Leu
            20                  25                  30
Val Leu Ala Phe Leu Ile Pro Arg Ile Gly Arg Leu Ala Met Arg Ile
        35                  40                  45
Ile Lys Gln Arg Val Glu Ser Ala Ala Asp Ala Asp Thr Thr Lys Asn
    50                  55                  60
Gln Leu Ala Phe Ala Gly Val Gly Val Tyr Ile Ala Gln Ile Val Ala
65                  70                  75                  80
Phe Phe Met Leu Ala Val Ser Ala Met Gln Ala Phe Gly Phe Ser Leu
                85                  90                  95
Ala Gly Ala Ala Ile Pro Ala Thr Ile Ala Ser Ala Ala Ile Gly Leu
            100                 105                 110
Gly Ala Gln Ser Ile Val Ala Asp Phe Leu Ala Gly Phe Phe Ile Leu
        115                 120                 125
Thr Glu Lys Gln Phe Gly Val Gly Asp Trp Val Arg Phe Glu Gly Asn
    130                 135                 140
Gly Ile Val Val Glu Gly Thr Val Ile Glu Ile Thr Met Arg Ala Thr
145                 150                 155                 160
Lys Ile Arg Thr Ile Ala Gln Glu Thr Val Ile Ile Pro Asn Ser Thr
                165                 170                 175
Ala Lys Val Cys Ile Asn Asn Ser Asn Asn Trp Ser Arg Ala Val Val
            180                 185                 190
Val Ile Pro Ile Pro Met Leu Gly Ser Glu Asn Ile Thr Asp Val Ile
        195                 200                 205
Ala Arg Ser Glu Ala Ala Thr Arg Arg Ala Leu Gly Gln Glu Lys Ile
    210                 215                 220
Ala Pro Glu Ile Leu Gly Glu Leu Asp Val His Pro Ala Thr Glu Val
225                 230                 235                 240
Thr Pro Pro Thr Val Val Gly Met Pro Trp Met Val Thr Met Arg Phe
                245                 250                 255
Leu Val Gln Val Thr Ala Gly Asn Gln Trp Leu Val Glu Arg Ala Ile
            260                 265                 270
Arg Thr Glu Ile Ile Asn Glu Phe Trp Glu Glu Tyr Gly Ser Ala Thr
        275                 280                 285
Thr Thr Ser Gly Thr Leu Ile Asp Ser Leu His Val Glu His Glu Glu
    290                 295                 300
Pro Lys Thr Ser Leu Ile Asp Ala Ser Pro Gln Ala Leu Lys Glu Pro
305                 310                 315                 320
Lys Pro Glu Ala Ala Ala Thr Val Ala Ser Leu Ala Ala Ser Ser Asn
                325                 330                 335
Asp Asp Ala Asp Asn Ala Asp Ala Ser Ala Ile Asn Ala Gly Asn Pro
            340                 345                 350
Glu Lys Glu Leu Asp Ser Asp Val Leu Glu Gln Glu Leu Ser Ser Glu
        355                 360                 365
Glu Pro Glu Glu Thr Ala Lys Pro Asp His Ser Leu Arg Gly Phe Phe
    370                 375                 380
Arg Thr Asp Tyr Tyr Pro Asn Arg Trp Gln Lys Ile Leu Ser Phe Gly
385                 390                 395                 400
Gly Arg Val Arg Met Ser Thr Ser Leu Leu Leu Gly Ala Leu Leu Leu
                405                 410                 415
Leu Ser Leu Phe Lys Val Met Thr Val Glu Pro Ser Glu Asn Trp Gln
            420                 425                 430
Asn Ser Ser Gly Trp Leu Ser Pro Ser Thr Ala Thr Ser Thr Ala Val
        435                 440                 445

Thr Thr Ser Glu Thr Ser Ala Pro Ala Ser Thr Pro Ser Met Thr Val
    450                 455                 460
Pro Thr Thr Val Glu Glu Thr Pro Thr Met Glu Ser Ser Val Glu Thr
465                 470                 475                 480
Gln Gln Glu Thr Ser Thr Pro Ala Thr Ala Thr Pro Gln Arg Ala Asp
                485                 490                 495
Thr Ile Glu Pro Thr Glu Glu Ala Thr Ser Gln Glu Glu Thr Thr Ala
            500                 505                 510
Ser Gln Thr Gln Ser Pro Ala Val Glu Ala Pro Thr Ala Val Gln Glu
        515                 520                 525
Thr Val Ala Pro Thr Ser Thr Pro
    530                 535

<210>  21
<211>  3481
<212>  DNA
<213>  Corynebacterium glutamicum

<220>
<221>  CDS
<222>  (1437)..(3035)
<223>  19

<400>  21
gatcttgccg atttcggcag gatcaatgtc ggcgtgaatg atcttggcat caggtgcgaa      60
agtgtcaacg tcaccggtga cgcggtcatc aaagcgggag ccgatagcaa tcagcaggtc     120
gctgcgctgc agtgcaccaa cagcggacac agtgccatgc atgcctggca tacccatgtg     180
cagctcgtgg gactctggga aggttcccag cgccatcaat gtggtgacaa ctggaatgcc     240
ggtgtgctca gcgaacgcac gaagctcttc gtgggcatca gccttgataa cgccgccgcc     300
aacgtaaagg acaggcttct tagactcacc gatcagtttg acagcctgct caatctgtcg     360
agcatgcggt gttgaaactg ggcggtagcc tggcaggtcg atctttggtg gccagacgaa     420
atccaattca gcgttctgaa catccttggg gatatccact agaacaggac cagggcgacc      480
agtaatcgcg aggtggaatg cctcagccaa tgcctgtgga atgtcgttgg ggttggtgac     540
catgaagttg tgcttggtca ctggcatggt gatgccgcgg atatcggctt cctggaaagc     600
atcggtaccc agcaggctac ttccgacctg gccggtgatg gcaaccatgg gaacggagtc     660
caagtttgca tcagcgattg gggtaaccaa gttggttgcg cctgggccag aggttgcaat     720
gcagacgcca acgcgtccag taacctgcgc gtagccggtt gctgcgtggc ctgcgccctg     780
ctcgtggcgc actaggacgt ggcgcacctt tgtggaggaa tagagcgggt catacaccgg     840
tagcaccgca ccaccaggaa taccgaacac gatgtcggcg ttaagctcct cgagcgatcg     900
aacaattgcc tgtgcacctg tcatccgctc aggggcggcg gatcgaccac ggcttgcaac     960
cgtggcggga gtgggctgtt gagaagctgc cacattcacg actttctggc tcctttacta    1020
aataaggatt ttcacaggac ccgtccaagc caagccgatt tcaactcagc ctaaagacaa    1080
agccctcatt taaaattgtt ccgacgcgga tgcgtgtgca cgcagtgcga cagatgtctg    1140
ttgcaaagtt ggctacttgg gtcataacca acaagaaagc cctcgttcca acactgtggt    1200
gagtgttgtc gagggcgctt gacgagacga cttggaaggc cgttacggca ggcgccgcgc    1260
ggttactact acaagtcgaa taatggtcat ggtgtgtcat gctacacaca tcgagtttcc    1320
aattccacaa cgcacgaaaa ttcccacccc caaaactccc ccacttcggt taaggaatca    1380
ggattctcac aaagttcagg caggctcccg ctacttttca gcgctaatct tggctc atg    1439
                                                              Met
                                                              1
att tta ggc gta ccc att caa tat ttg ctc tat tca ttg tgg aat tgg      1487
Ile Leu Gly Val Pro Ile Gln Tyr Leu Leu Tyr Ser Leu Trp Asn Trp
            5                   10                  15
att gtc gat acc ggt ttt gat gta gca att atc ctg gtc ttg gcg ttt      1535
Ile Val Asp Thr Gly Phe Asp Val Ala Ile Ile Leu Val Leu Ala Phe
        20                  25                  30
ttg att cca cgt atc ggc cga ctg gcc atg cgt att atc aag cag cga      1583
Leu Ile Pro Arg Ile Gly Arg Leu Ala Met Arg Ile Ile Lys Gln Arg
    35                  40                  45
gtg gag tct gca gcc gat gcg gac acc act aag aac cag ctc gcg ttc      1631
Val Glu Ser Ala Ala Asp Ala Asp Thr Thr Lys Asn Gln Leu Ala Phe
50                  55                  60                  65

gct ggc gtt ggc gtt tat atc gcg caa att gtg gcg ttt ttc atg ctt     1679
Ala Gly Val Gly Val Tyr Ile Ala Gln Ile Val Ala Phe Phe Met Leu
                70                  75                  80
gcc gtc tcc gcg atg cag gct ttt ggt ttc tct ctc gcg ggc gct gcg     1727
Ala Val Ser Ala Met Gln Ala Phe Gly Phe Ser Leu Ala Gly Ala Ala
            85                  90                  95
att ccg aca acc att gcg tca gct gcc att ggt ctt ggt gcg cag tcg     1775
Ile Pro Thr Thr Ile Ala Ser Ala Ala Ile Gly Leu Gly Ala Gln Ser
        100                 105                 110
att gtt gcg gac ttc ttg gcc gga ttt ttc atc ctg acg gaa aag caa     1823
Ile Val Ala Asp Phe Leu Ala Gly Phe Phe Ile Leu Thr Glu Lys Gln
    115                 120                 125
ttc ggc gtg ggt gac tgg gtg cgc ttt gag ggc aac ggc atc gtt gtt     1871
Phe Gly Val Gly Asp Trp Val Arg Phe Glu Gly Asn Gly Ile Val Val
130                 135                 140                 145
gaa ggc acc gtc att gag atc acc atg cgc gcg acc aaa att cgc acg     1919
Glu Gly Thr Val Ile Glu Ile Thr Met Arg Ala Thr Lys Ile Arg Thr
                150                 155                 160
att gca caa gag acc gtg atc atc ccg aac tcc acg gcg aaa gtg tgc     1967
Ile Ala Gln Glu Thr Val Ile Ile Pro Asn Ser Thr Ala Lys Val Cys
            165                 170                 175
atc aac aat tct aat aac tgg tcg cgt gcg gtt gtc gtt att ccg atc     2015
Ile Asn Asn Ser Asn Asn Trp Ser Arg Ala Val Val Val Ile Pro Ile
        180                 185                 190
ccc atg ttg ggt tct gaa aac atc aca gat gtc atc gcg cgc tct gaa     2063
Pro Met Leu Gly Ser Glu Asn Ile Thr Asp Val Ile Ala Arg Ser Glu
    195                 200                 205
gct gcg act cgt cgc gca ctt ggc cag gag aaa atc gca ccg gaa atc     2111
Ala Ala Thr Arg Arg Ala Leu Gly Gln Glu Lys Ile Ala Pro Glu Ile
210                 215                 220                 225
ctc ggt gaa ctc gat gtg cac cca gcc acg gaa gtc aca ccg cca acg     2159
Leu Gly Glu Leu Asp Val His Pro Ala Thr Glu Val Thr Pro Pro Thr
                230                 235                 240
gtg gtc ggc atg ccg tgg atg gtc acc atg cgt ttc ctc gtg caa gtc     2207
Val Val Gly Met Pro Trp Met Val Thr Met Arg Phe Leu Val Gln Val
            245                 250                 255
acc gcc ggc aat caa tgg ctg gtc gaa cgc gcc atc cgc aca gaa atc     2255
Thr Ala Gly Asn Gln Trp Leu Val Glu Arg Ala Ile Arg Thr Glu Ile
        260                 265                 270
atc aac gaa ttc tgg gaa gaa tac ggc agc gca acc act aca tcg gga     2303
Ile Asn Glu Phe Trp Glu Glu Tyr Gly Ser Ala Thr Thr Thr Ser Gly
    275                 280                 285
acc ctc att gat tcc tta cac gtt gag cat gaa gag cca aag acc tcg     2351
Thr Leu Ile Asp Ser Leu His Val Glu His Glu Glu Pro Lys Thr Ser
290                 295                 300                 305
ctt atc gac gcc tcc ccc cag gct ctt aag gaa ccg aag ccg gag gct     2399
Leu Ile Asp Ala Ser Pro Gln Ala Leu Lys Glu Pro Lys Pro Glu Ala
                310                 315                 320
gcg gcg acg gtt gca tcg cta gct gca tcg tct aac gac gat gca gac     2447
Ala Ala Thr Val Ala Ser Leu Ala Ala Ser Ser Asn Asp Asp Ala Asp
            325                 330                 335
aat gca gac gcc tcg gcg atc aat gca ggc aat cca gag aag gaa ctt     2495
Asn Ala Asp Ala Ser Ala Ile Asn Ala Gly Asn Pro Glu Lys Glu Leu
        340                 345                 350
gat tcc gat gtg ctg gaa caa gaa ctc tcc agc gaa gaa ccg gaa gaa     2543
Asp Ser Asp Val Leu Glu Gln Glu Leu Ser Ser Glu Glu Pro Glu Glu
    355                 360                 365
aca gca aaa cca gat cac tct ctc cga ggc ttc ttc cgc act gat tac     2591
Thr Ala Lys Pro Asp His Ser Leu Arg Gly Phe Phe Arg Thr Asp Tyr
370                 375                 380                 385
tac cca aat cgg tgg cag aag atc ctg tcg ttt ggc gga cgt gtc cgc     2639
Tyr Pro Asn Arg Trp Gln Lys Ile Leu Ser Phe Gly Gly Arg Val Arg
                390                 395                 400

atg agc act tcc ctg ttg ttg ggt gcg ctg ctc ttg ctg tca cta ttt     2687
Met Ser Thr Ser Leu Leu Leu Gly Ala Leu Leu Leu Leu Ser Leu Phe
            405                 410                 415
aag gtc atg act gtg gaa cca agt gag aat tgg caa aac tcc agt gga     2735
Lys Val Met Thr Val Glu Pro Ser Glu Asn Trp Gln Asn Ser Ser Gly
        420                 425                 430
tgg ctg tca cca agc act gcc acc tca act gcg gtg acc acc tcc gaa     2783
Trp Leu Ser Pro Ser Thr Ala Thr Ser Thr Ala Val Thr Thr Ser Glu
    435                 440                 445
act tcc gcg cca gca agc acg cct tcg atg aca gtg ccc act acg gtg     2831
Thr Ser Ala Pro Ala Ser Thr Pro Ser Met Thr Val Pro Thr Thr Val
450                 455                 460                 465
gag gag acc cca acg atg gaa tct agc gtc gaa acg cag cag gaa acc     2879
Glu Glu Thr Pro Thr Met Glu Ser Ser Val Glu Thr Gln Gln Glu Thr
                470                 475                 480
tca acc cct gca acc gca acg ccc cag cga gcc gac acc atc gaa ccg     2927
Ser Thr Pro Ala Thr Ala Thr Pro Gln Arg Ala Asp Thr Ile Glu Pro
            485                 490                 495
acc gag gaa gcc acg tcg cag gag gaa acg act gca tcg cag acg cag     2975
Thr Glu Glu Ala Thr Ser Gln Glu Glu Thr Thr Ala Ser Gln Thr Gln
        500                 505                 510
tct cca gca gtg gaa gca cca acc gcg gtc caa gaa aca gtt gcg ccg     3023
Ser Pro Ala Val Glu Ala Pro Thr Ala Val Gln Glu Thr Val Ala Pro
    515                 520                 525
acg tcc acc cct taggacgctg attacagacg tgtcccattt ctttactact     3075
Thr Ser Thr Pro
530
attggaaatt atgagttcag acgcagaaaa ggcatccgtg gagctttccg aaaaatttca     3135
cccagaacgc acccatattt tgggcgccgt tgtttttggc ctgatctcat tattagtcat     3195
cggcgcagcc cctcagtacc tgttttggct gctcgcgctc cctgtcatct tcggttactg     3255
ggttctaaaa tcatccacga tcgttgatga acagggcatc accgcaaact acgccttcaa     3315
gggcaaaaag gttgtggcct gggaagacct cgcaggaatc ggattcaagg gtgcccgcac     3375
tttcgctcgc accacctccg atgcagaagt caccctcccc ggcgtcacct tcaactccct     3435
tccccgcctt gaagctgctt cccacggccg catccccgat gcgatc     3481

<210>  22
<211>  533
<212>  PRT
<213>  Corynebacterium glutamicum

<400>  22
Met Ile Leu Gly Val Pro Ile Gln Tyr Leu Leu Tyr Ser Leu Trp Asn
1               5                   10                  15
Trp Ile Val Asp Thr Gly Phe Asp Val Ala Ile Ile Leu Val Leu Ala
            20                  25                  30
Phe Leu Ile Pro Arg Ile Gly Arg Leu Ala Met Arg Ile Ile Lys Gln
        35                  40                  45
Arg Val Glu Ser Ala Ala Asp Ala Asp Thr Thr Lys Asn Gln Leu Ala
    50                  55                  60
Phe Ala Gly Val Gly Val Tyr Ile Ala Gln Ile Val Ala Phe Phe Met
65                  70                  75                  80
Leu Ala Val Ser Ala Met Gln Ala Phe Gly Phe Ser Leu Ala Gly Ala
                85                  90                  95
Ala Ile Pro Thr Thr Ile Ala Ser Ala Ala Ile Gly Leu Gly Ala Gln
            100                 105                 110
Ser Ile Val Ala Asp Phe Leu Ala Gly Phe Phe Ile Leu Thr Glu Lys
        115                 120                 125
Gln Phe Gly Val Gly Asp Trp Val Arg Phe Glu Gly Asn Gly Ile Val
    130                 135                 140
Val Glu Gly Thr Val Ile Glu Ile Thr Met Arg Ala Thr Lys Ile Arg
145                 150                 155                 160

Thr Ile Ala Gln Glu Thr Val Ile Ile Pro Asn Ser Thr Ala Lys Val
                165                 170                 175
Cys Ile Asn Asn Ser Asn Asn Trp Ser Arg Ala Val Val Val Ile Pro
            180                 185                 190
Ile Pro Met Leu Gly Ser Glu Asn Ile Thr Asp Val Ile Ala Arg Ser
        195                 200                 205
Glu Ala Ala Thr Arg Arg Ala Leu Gly Gln Glu Lys Ile Ala Pro Glu
    210                 215                 220

Ile Leu Gly Glu Leu Asp Val His Pro Ala Thr Glu Val Thr Pro Pro
225                 230                 235                 240
Thr Val Val Gly Met Pro Trp Met Val Thr Met Arg Phe Leu Val Gln
                245                 250                 255
Val Thr Ala Gly Asn Gln Trp Leu Val Glu Arg Ala Ile Arg Thr Glu
            260                 265                 270
Ile Ile Asn Glu Phe Trp Glu Glu Tyr Gly Ser Ala Thr Thr Thr Ser
        275                 280                 285
Gly Thr Leu Ile Asp Ser Leu His Val Glu His Glu Glu Pro Lys Thr
    290                 295                 300
Ser Leu Ile Asp Ala Ser Pro Gln Ala Leu Lys Glu Pro Lys Pro Glu
305                 310                 315                 320
Ala Ala Ala Thr Val Ala Ser Leu Ala Ala Ser Ser Asn Asp Asp Ala
                325                 330                 335
Asp Asn Ala Asp Ala Ser Ala Ile Asn Ala Gly Asn Pro Glu Lys Glu
            340                 345                 350
Leu Asp Ser Asp Val Leu Glu Gln Glu Leu Ser Ser Glu Glu Pro Glu
        355                 360                 365
Glu Thr Ala Lys Pro Asp His Ser Leu Arg Gly Phe Phe Arg Thr Asp
    370                 375                 380
Tyr Tyr Pro Asn Arg Trp Gln Lys Ile Leu Ser Phe Gly Gly Arg Val
385                 390                 395                 400
Arg Met Ser Thr Ser Leu Leu Leu Gly Ala Leu Leu Leu Leu Ser Leu

                405                 410                 415
Phe Lys Val Met Thr Val Glu Pro Ser Glu Asn Trp Gln Asn Ser Ser
            420                 425                 430
Gly Trp Leu Ser Pro Ser Thr Ala Thr Ser Thr Ala Val Thr Thr Ser
        435                 440                 445
Glu Thr Ser Ala Pro Ala Ser Thr Pro Ser Met Thr Val Pro Thr Thr
    450                 455                 460
Val Glu Glu Thr Pro Thr Met Glu Ser Ser Val Glu Thr Gln Gln Glu
465                 470                 475                 480
Thr Ser Thr Pro Ala Thr Ala Thr Pro Gln Arg Ala Asp Thr Ile Glu
                485                 490                 495
Pro Thr Glu Glu Ala Thr Ser Gln Glu Glu Thr Thr Ala Ser Gln Thr
            500                 505                 510
Gln Ser Pro Ala Val Glu Ala Pro Thr Ala Val Gln Glu Thr Val Ala
        515                 520                 525
Pro Thr Ser Thr Pro
    530


<210>  23
<211>  3481
<212>  DNA
<213>  Corynebacterium glutamicum

<220>
<221>  CDS
<222>  (1437)..(3035)
<223>  L30

<400>  23

gatcttgccg atttcggcag gatcaatgtc ggcgtgaatg atcttggcat caggtgcgaa 60
agtgtcaacg tcaccggtga cgcggtcatc aaagcgggag ccgatagcaa tcagcaggtc 120
gctgcgctgc agtgcaccaa cagcggacac agtgccatgc atgcctggca tacccatgtg 180
cagctcgtgg gactctggga aggttcccag cgccatcaat gtggtgacaa ctggaatgcc 240
ggtgtgctca gcgaacgcac gaagctcttc gtgggcatca gccttgataa cgccgccgcc 300
aacgtaaagg acaggcttct tagactcacc gatcagtttg acagcctgct caatctgtcg 360
agcatgcggt gttgaaactg ggcggtagcc tggcaggtcg atctttggtg gccagacgaa 420
atccaattca gcgttctgaa catccttggg gatatccact agaacaggac cagggcgacc 480
agtaatcgcg aggtggaatg cctcagccaa tgcctgtgga atgtcgttgg ggttggtgac 540
catgaagttg tgcttggtca ctggcatggt gatgccgcgg atatcggctt cctggaaagc 600
atcggtaccc agcaggctac ttccgacctg gccggtgatg gcaaccatgg gaacggagtc 660
caagtttgca tcagcgattg gggtaaccaa gttggttgcg cctgggccag aggttgcaat 720
gcagacgcca acgcgtccag taacctgcgc gtagccggtt gctgcgtggc ctgcgccctg 780
ctcgtggcgc actaggacgt ggcgcacctt tgtggaggaa tagagcgggt catacaccgg 840
tagcaccgca ccaccaggaa taccgaacac gatgtcggcg ttaagctcct cgagcgatcg 900
aacaattgcc tgtgcacctg tcatccgctc aggggcggcg gatcgaccac ggcttgcaac 960
cgtggcggga gtgggctgtt gagaagctgc cacattcacg actttctggc tcctttacta 1020
aataaggatt ttcacaggac ccgtccaagc caagccgatt tcaactcagc ctaaagacaa 1080
agccctcatt taaaattgtt ccgacgcgga tgcgtgtgca cgcagtgcga cagatgtctg 1140
ttgcaaagtt ggctacttgg gtcataacca acaagaaagc cctcgttcca acactgtggt 1200
gagtgttgtc gagggcgctt gacgagacga cttggaaggc cgttacggca ggcgccgcgc 1260
ggttactact acaagtcgaa taatggtcat ggtgtgtcat gctacacaca tcgagtttcc 1320
aattccacaa cgcacgaaaa ttcccacccc caaaactccc ccacttcggt taaggaatca 1380
ggattctcac aaagttcagg caggctcccg ctacttttca gcgctaatct tggctc atg 1439
Met
1
att tta ggc gta ccc att caa tat ttg ctc tat tca ttg tgg aat tgg 1487
Ile Leu Gly Val Pro Ile Gln Tyr Leu Leu Tyr Ser Leu Trp Asn Trp
            5                   10                  15
att gtc gat acc ggt ttt gat gta gca att atc ctg gtc ttg gcg ttt 1535
Ile Val Asp Thr Gly Phe Asp Val Ala Ile Ile Leu Val Leu Ala Phe
        20                  25                  30
ttg att cca cgt atc ggc cga ctg gcc atg cgt att atc aag cag cga 1583
Leu Ile Pro Arg Ile Gly Arg Leu Ala Met Arg Ile Ile Lys Gln Arg
    35                  40                  45
gtg gag tct gca gcc gat gcg gac acc act aag aac cag ctc gcg ttc 1631
Val Glu Ser Ala Ala Asp Ala Asp Thr Thr Lys Asn Gln Leu Ala Phe
50                  55                  60                  65
gct ggc gtt ggc gtt tat atc gcg caa att gtg gcg ttt ttc atg ctt 1679
Ala Gly Val Gly Val Tyr Ile Ala Gln Ile Val Ala Phe Phe Met Leu
                70                  75                  80
gcc gtc tcc gcg atg cag gct ttt ggt ttc tct ctc gcg ggc gct gcg 1727
Ala Val Ser Ala Met Gln Ala Phe Gly Phe Ser Leu Ala Gly Ala Ala
            85                  90                  95
att ccg gca acc att gcg tca gct gcc att ggt ctt ggt gtg cag tcg 1775
Ile Pro Ala Thr Ile Ala Ser Ala Ala Ile Gly Leu Gly Val Gln Ser
        100                 105                 110
att gtt gcg gac ttc ttg gcc gga ttt ttc atc ctg acg gaa aag caa 1823
Ile Val Ala Asp Phe Leu Ala Gly Phe Phe Ile Leu Thr Glu Lys Gln
    115                 120                 125
ttc ggc gtg ggt gac tgg gtg cgc ttt gag ggc aac ggc atc gtt gtt 1871
Phe Gly Val Gly Asp Trp Val Arg Phe Glu Gly Asn Gly Ile Val Val
130                 135                 140                 145
gaa ggc acc gtc att gag atc acc atg cgc gcg acc aaa att cgc acg 1919
Glu Gly Thr Val Ile Glu Ile Thr Met Arg Ala Thr Lys Ile Arg Thr
                150                 155                 160
att gca caa gag acc gtg atc atc ccg aac tcc acg gcg aaa gtg tgc 1967
Ile Ala Gln Glu Thr Val Ile Ile Pro Asn Ser Thr Ala Lys Val Cys
            165                 170                 175
atc aac aat tct aat aac tgg tcg cgt gcg gtt gtc gtt att ccg atc 2015
Ile Asn Asn Ser Asn Asn Trp Ser Arg Ala Val Val Val Ile Pro Ile
        180                 185                 190
ccc atg ttg ggt tct gaa aac atc aca gat gtc atc gcg cgc tct gaa 2063

Pro Met Leu Gly Ser Glu Asn Ile Thr Asp Val Ile Ala Arg Ser Glu
    195                 200                 205

gct gcg act cgt cgc gca ctt ggc cag gag aaa atc gca ccg gaa atc      2111
Ala Ala Thr Arg Arg Ala Leu Gly Gln Glu Lys Ile Ala Pro Glu Ile
210                 215                 220                 225

ctc ggt gaa ctc gat gtg cac cca gcc acg gaa gtc aca ccg cca acg      2159
Leu Gly Glu Leu Asp Val His Pro Ala Thr Glu Val Thr Pro Pro Thr
                230                 235                 240

gtg gtc ggc atg ccg tgg atg gtc acc atg cgt ttc ctc gtg caa gtc      2207
Val Val Gly Met Pro Trp Met Val Thr Met Arg Phe Leu Val Gln Val
            245                 250                 255

acc gcc ggc aat caa tgg ctg gtc gaa cgc gcc atc cgc aca gaa atc      2255
Thr Ala Gly Asn Gln Trp Leu Val Glu Arg Ala Ile Arg Thr Glu Ile
        260                 265                 270

atc aac gaa ttc tgg gaa gaa tac ggc agc gca acc act aca tcg gga      2303
Ile Asn Glu Phe Trp Glu Glu Tyr Gly Ser Ala Thr Thr Thr Ser Gly
    275                 280                 285

acc ctc att gat tcc tta cac gtt gag cat gaa gag cca aag acc tcg      2351
Thr Leu Ile Asp Ser Leu His Val Glu His Glu Glu Pro Lys Thr Ser
290                 295                 300                 305

ctt atc gac gcc tcc ccc cag gct ctt aag gaa ccg aag ccg gag gct      2399
Leu Ile Asp Ala Ser Pro Gln Ala Leu Lys Glu Pro Lys Pro Glu Ala
                310                 315                 320

gcg gcg acg gtt gca tcg cta gct gca tcg tct aac gac gat gca gac      2447
Ala Ala Thr Val Ala Ser Leu Ala Ala Ser Ser Asn Asp Asp Ala Asp
            325                 330                 335

aat gca gac gcc tcg gcg atc aat gca ggc aat cca gag aag gaa ctt      2495
Asn Ala Asp Ala Ser Ala Ile Asn Ala Gly Asn Pro Glu Lys Glu Leu
        340                 345                 350

gat tcc gat gtg ctg gaa caa gaa ctc tcc agc gaa gaa ccg gaa gaa      2543
Asp Ser Asp Val Leu Glu Gln Glu Leu Ser Ser Glu Glu Pro Glu Glu
    355                 360                 365

aca gca aaa cca gat cac tct ctc cga ggc ttc ttc cgc act gat tac      2591
Thr Ala Lys Pro Asp His Ser Leu Arg Gly Phe Phe Arg Thr Asp Tyr
370                 375                 380                 385

tac cca aat cgg tgg cag aag atc ctg tcg ttt ggc gga cgt gtc cgc      2639
Tyr Pro Asn Arg Trp Gln Lys Ile Leu Ser Phe Gly Gly Arg Val Arg
                390                 395                 400

atg agc act tcc ctg ttg ttg ggt gcg ctg ctc ttg ctg tca cta ttt      2687
Met Ser Thr Ser Leu Leu Leu Gly Ala Leu Leu Leu Leu Ser Leu Phe
            405                 410                 415

aag gtc atg act gtg gaa cca agt gag aat tgg caa aac tcc agt gga      2735
Lys Val Met Thr Val Glu Pro Ser Glu Asn Trp Gln Asn Ser Ser Gly
        420                 425                 430

tgg ctg tca cca agc act gcc acc tca act gcg gtg acc acc tcc gaa      2783
Trp Leu Ser Pro Ser Thr Ala Thr Ser Thr Ala Val Thr Thr Ser Glu
    435                 440                 445

act tcc gcg cca gca agc acg cct tcg atg aca gtg ccc act acg gtg      2831
Thr Ser Ala Pro Ala Ser Thr Pro Ser Met Thr Val Pro Thr Thr Val
450                 455                 460                 465

gag gag acc cca acg atg gaa tct agc gtc gaa acg cag cag gaa acc      2879
Glu Glu Thr Pro Thr Met Glu Ser Ser Val Glu Thr Gln Gln Glu Thr
                470                 475                 480

tca acc cct gca acc gca acg ccc cag cga gcc gac acc atc gaa ccg      2927
Ser Thr Pro Ala Thr Ala Thr Pro Gln Arg Ala Asp Thr Ile Glu Pro
            485                 490                 495

acc gag gaa gcc acg tcg cag gag gaa acg act gca tcg cag acg cag      2975
Thr Glu Glu Ala Thr Ser Gln Glu Glu Thr Thr Ala Ser Gln Thr Gln
        500                 505                 510

tct cca gca gtg gaa gca cca acc gcg gtc caa gaa aca gtt gcg ccg      3023
Ser Pro Ala Val Glu Ala Pro Thr Ala Val Gln Glu Thr Val Ala Pro
    515                 520                 525

acg tcc acc cct taggacgctg attacagacg tgtcccattt ctttactact 3075
Thr Ser Thr Pro
530
attggaaatt atgagttcag acgcagaaaa ggcatccgtg gagctttccg aaaaatttca 3135
cccagaacgc acccatattt tgggcgccgt tgtttttggc ctgatctcat tattagtcat 3195
cggcgcagcc cctcagtacc tgttttggct gctcgcgctc cctgtcatct tcggttactg 3255
ggttctaaaa tcatccacga tcgttgatga acagggcatc accgcaaact acgccttcaa 3315
gggcaaaaag gttgtggcct gggaagacct cgcaggaatc ggattcaagg gtgcccgcac 3375
tttcgctcgc accacctccg atgcagaagt caccctcccc ggcgtcacct tcaactccct 3435
tccccgcctt gaagctgctt cccacggccg catccccgat gcgatc 3481

<210> 24
<211> 533
<212> PRT
<213> Corynebacterium glutamicum

<400> 24
Met Ile Leu Gly Val Pro Ile Gln Tyr Leu Leu Tyr Ser Leu Trp Asn
1               5                   10                  15
Trp Ile Val Asp Thr Gly Phe Asp Val Ala Ile Ile Leu Val Leu Ala
            20                  25                  30
Phe Leu Ile Pro Arg Ile Gly Arg Leu Ala Met Arg Ile Ile Lys Gln
        35                  40                  45
Arg Val Glu Ser Ala Ala Asp Ala Asp Thr Thr Lys Asn Gln Leu Ala
    50                  55                  60
Phe Ala Gly Val Gly Val Tyr Ile Ala Gln Ile Val Ala Phe Phe Met
65                  70                  75                  80
Leu Ala Val Ser Ala Met Gln Ala Phe Gly Phe Ser Leu Ala Gly Ala
                85                  90                  95
Ala Ile Pro Ala Thr Ile Ala Ser Ala Ala Ile Gly Leu Gly Val Gln
            100                 105                 110
Ser Ile Val Ala Asp Phe Leu Ala Gly Phe Phe Ile Leu Thr Glu Lys
        115                 120                 125
Gln Phe Gly Val Gly Asp Trp Val Arg Phe Glu Gly Asn Gly Ile Val
    130                 135                 140
Val Glu Gly Thr Val Ile Glu Ile Thr Met Arg Ala Thr Lys Ile Arg
145                 150                 155                 160
Thr Ile Ala Gln Glu Thr Val Ile Ile Pro Asn Ser Thr Ala Lys Val
                165                 170                 175
Cys Ile Asn Asn Ser Asn Asn Trp Ser Arg Ala Val Val Val Ile Pro
            180                 185                 190
Ile Pro Met Leu Gly Ser Glu Asn Ile Thr Asp Val Ile Ala Arg Ser
        195                 200                 205
Glu Ala Ala Thr Arg Arg Ala Leu Gly Gln Glu Lys Ile Ala Pro Glu
    210                 215                 220
Ile Leu Gly Glu Leu Asp Val His Pro Ala Thr Glu Val Thr Pro Pro
225                 230                 235                 240
Thr Val Val Gly Met Pro Trp Met Val Thr Met Arg Phe Leu Val Gln
                245                 250                 255
Val Thr Ala Gly Asn Gln Trp Leu Val Glu Arg Ala Ile Arg Thr Glu
            260                 265                 270
Ile Ile Asn Glu Phe Trp Glu Glu Tyr Gly Ser Ala Thr Thr Thr Ser
        275                 280                 285
Gly Thr Leu Ile Asp Ser Leu His Val Glu His Glu Glu Pro Lys Thr
    290                 295                 300
Ser Leu Ile Asp Ala Ser Pro Gln Ala Leu Lys Glu Pro Lys Pro Glu
305                 310                 315                 320
Ala Ala Ala Thr Val Ala Ser Leu Ala Ala Ser Ser Asn Asp Asp Ala
                325                 330                 335
Asp Asn Ala Asp Ala Ser Ala Ile Asn Ala Gly Asn Pro Glu Lys Glu
            340                 345                 350
Leu Asp Ser Asp Val Leu Glu Gln Glu Leu Ser Ser Glu Glu Pro Glu

355                 360                 365
Glu Thr Ala Lys Pro Asp His Ser Leu Arg Gly Phe Phe Arg Thr Asp
    370                 375                 380
Tyr Tyr Pro Asn Arg Trp Gln Lys Ile Leu Ser Phe Gly Gly Arg Val
385                 390                 395                 400
Arg Met Ser Thr Ser Leu Leu Leu Gly Ala Leu Leu Leu Leu Ser Leu
                405                 410                 415
Phe Lys Val Met Thr Val Glu Pro Ser Glu Asn Trp Gln Asn Ser Ser
            420                 425                 430
Gly Trp Leu Ser Pro Ser Thr Ala Thr Ser Thr Ala Val Thr Thr Ser
        435                 440                 445
Glu Thr Ser Ala Pro Ala Ser Thr Pro Ser Met Thr Val Pro Thr Thr
    450                 455                 460
Val Glu Glu Thr Pro Thr Met Glu Ser Ser Val Glu Thr Gln Gln Glu
465                 470                 475                 480
Thr Ser Thr Pro Ala Thr Ala Thr Pro Gln Arg Ala Asp Thr Ile Glu
                485                 490                 495
Pro Thr Glu Glu Ala Thr Ser Gln Glu Glu Thr Thr Ala Ser Gln Thr
            500                 505                 510
Gln Ser Pro Ala Val Glu Ala Pro Thr Ala Val Gln Glu Thr Val Ala
        515                 520                 525
Pro Thr Ser Thr Pro
    530

<210>  25
<211>  23
<212>  PRT
<213>  Corynebacterium glutamicum

<400>  25
Met Ile Leu Gly Val Pro Ile Gln Tyr Leu Leu Tyr Ser Leu Trp Asn
1               5                   10                  15
Trp Ile Val Asp Thr Gly Phe
            20

<210>  26
<211>  23
<212>  PRT
<213>  Corynebacterium glutamicum

<400>  26
Val Ala Ile Ile Leu Val Leu Ala Phe Leu Ile Pro Arg Ile Gly Arg
1               5                   10                  15
Leu Ala Met Arg Ile Ile Lys
            20

<210>  27
<211>  23
<212>  PRT
<213>  Corynebacterium glutamicum

<400>  27
Gln Leu Ala Phe Ala Gly Val Gly Val Tyr Ile Ala Gln Ile Val Ala
1               5                   10                  15
Phe Phe Met Leu Ala Val Ser
            20

<210>  28
<211>  23

<212>  PRT
<213>  Corynebacterium glutamicum

<400>  28
Met Gln Ala Phe Gly Phe Ser Leu Ala Gly Ala Ala Ile Pro Ala Thr
1               5                   10                  15
Ile Ala Ser Ala Ala Ile Gly
            20


<210>  29
<211>  23
<212>  PRT
<213>  Corynebacterium glutamicum

<400>  29
Gly Ala Gln Ser Ile Val Ala Asp Phe Leu Ala Gly Phe Phe Ile Leu
1               5                   10                  15
Thr Glu Lys Gln Phe Gly Val
            20


<210>  30
<211>  20
<212>  DNA
<213>  Artificial sequence

<220>
<223>  primer

<400>  30

ggctgttgag aagctgccac                                                   20


<210>  31
<211>  40
<212>  DNA
<213>  Artificial sequence

<220>
<223>  primer

<400>  31

ccaattccac aatgaacaca atgaatagag caaatattga                             40



<210>  32
<211>  40
<212>  DNA
<213>  Artificial sequence

<220>
<223>  primer

<400>  32
tcaatatttg ctctattcat tgtgttcatt gtggaattgg                             40


<210>  33
<211>  20

<212>  DNA
<213>  Artificial sequence

<220>
<223>  primer

<400>  33
ttcggttcct taagagcctg                                                    20


<210>  34
<211>  28
<212>  DNA
<213>  Artificial sequence

<220>
<223>  primer

<400>  34
gggagctcca cggcatgccg accaccgt                                           28


<210>  35
<211>  23
<212>  DNA
<213>  Artificial sequence

<220>
<223>  primer

<400>  35
gacgcaatgg ttgtcggaat cgc                                                23


<210>  36
<211>  23
<212>  DNA
<213>  Artificial sequence

<220>
<223>  primer

<400>  36
gcgattccga caaccattgc gtc                                                23


<210>  37
<211>  29
<212>  DNA
<213>  Artificial sequence

<220>
<223>  primer

<400>  37
ccgcaacaat cgactgcaca ccaagacca                                          29


<210>  38
<211>  29
<212>  DNA
<213>  Artificial sequence

<220>
<223>  primer

<400>  38
tggtcttggt gtgcagtcga ttgttgcgg                                          29


<210>  39
<211>  28

<212>  DNA

<213>  Artificial sequence

<220>
<223>  primer

<400>  39
gggagctcac ctttgtggag gaatagag                                           28


<210>  40
<211>  46
<212>  DNA
<213>  Artificial sequence

<220>
<223>  primer

<400>  40
gggacacgtc tgtaatcagc gtcctagagc caagattagc gctgaa                       46


<210>  41
<211>  46
<212>  DNA
<213>  Artificial sequence

<220>
<223>  primer

<400>  41
ttcagcgcta atcttggctc taggacgctg attacagacg tgtccc                       46


<210>  42
<211>  28
<212>  DNA
<213>  Artificial sequence

<220>
<223>  primer

<400>  42
gggagctcag tactcttcct tggacatc                                           28


<210>  43
<211>  4400
<212>  DNA
<213>  Corynebacterium glutamicum

<220>

<221>  CDS

<222>  (443)..(4213)
<223>  odhA

<400>  43
gtcgacaagc aaaatcgaag cggcagcacg ccgcgtcgga gccttaaacg ccatcgccgc      60
catccctgat ggtttcaacc atcaagtcgg tgaacgcggg cgcaacctgt catccggaca     120
gcgccaactg atcgcgctgg cgcgcgccga acttatcgag ccttccatca tgcttctcga     180
cgaagccacc tccaccctcg accccgccac cgaagccgtt atcctcaacg cctccgatcg     240
agtcactaag ggacgcacca gcatcatcgt cgcgcaccgc ttggcaaccg ctaaaagggc     300
cgaccgtatt cttgttgttg aacaaggacg tatcattgag gacggatctc acgacgcgtt     360
gttgtctgct aacggcacct acgcccgcat gtggcattta atggcctgac acgttatttt     420
taggagaact gtcaacaaat ta atg cta caa ctg ggg ctt agg cat aat cag      472
                         Met Leu Gln Leu Gly Leu Arg His Asn Gln
                         1               5                   10
cca acg acc aac gtt aca gtg gat aaa aca aag ctc aat aaa ccc tca      520
Pro Thr Thr Asn Val Thr Val Asp Lys Thr Lys Leu Asn Lys Pro Ser
                15                  20                  25
aga agc aag gaa aag agg cga gta cct gcc gtg agc agc gct agt act      568
Arg Ser Lys Glu Lys Arg Arg Val Pro Ala Val Ser Ser Ala Ser Thr
            30                  35                  40
ttc ggc cag aat gcg tgg ctg gta gac gag atg ttc cag cag ttc cag      616
Phe Gly Gln Asn Ala Trp Leu Val Asp Glu Met Phe Gln Gln Phe Gln
        45                  50                  55
aag gac ccc aag tcc gtg gac aag gaa tgg aga gaa ctc ttt gag gcg      664
Lys Asp Pro Lys Ser Val Asp Lys Glu Trp Arg Glu Leu Phe Glu Ala
    60                  65                  70
cag ggg gga cca aat act acc ccc gct aca aca gaa gca cag cct tca      712
Gln Gly Gly Pro Asn Thr Thr Pro Ala Thr Thr Glu Ala Gln Pro Ser
75                  80                  85                  90
gcg ccc aag gag tct gcg aaa cca gca cca aag gct gcc cct gca gcc      760
Ala Pro Lys Glu Ser Ala Lys Pro Ala Pro Lys Ala Ala Pro Ala Ala
                95                  100                 105
aag gca gca ccg cgc gta gaa acc aag ccg gcc gac aag acc gcc cct      808
Lys Ala Ala Pro Arg Val Glu Thr Lys Pro Ala Asp Lys Thr Ala Pro
            110                 115                 120
aag gcc aag gag tcc tca gtg cca cag caa cct aag ctt ccg gag cca      856
Lys Ala Lys Glu Ser Ser Val Pro Gln Gln Pro Lys Leu Pro Glu Pro
        125                 130                 135
gga caa acc cca atc agg ggt att ttc aag tcc atc gcg aag aac atg      904
Gly Gln Thr Pro Ile Arg Gly Ile Phe Lys Ser Ile Ala Lys Asn Met
    140                 145                 150
gat atc tcc ctg gaa atc cca acc gca acc tcg gtt cgc gat atg cca      952
Asp Ile Ser Leu Glu Ile Pro Thr Ala Thr Ser Val Arg Asp Met Pro
155                 160                 165                 170
gct cgc ctc atg ttc gaa aac cgc gcg atg gtc aac gat cag ctc aag     1000
Ala Arg Leu Met Phe Glu Asn Arg Ala Met Val Asn Asp Gln Leu Lys
                175                 180                 185
cgc acc cgc ggt ggc aag atc tcc ttc acc cac atc att ggc tac gcc     1048
Arg Thr Arg Gly Gly Lys Ile Ser Phe Thr His Ile Ile Gly Tyr Ala
            190                 195                 200
atg gtg aag gca gtc atg gct cac ccg gac atg aac aac tcc tac gac     1096
Met Val Lys Ala Val Met Ala His Pro Asp Met Asn Asn Ser Tyr Asp
        205                 210                 215
gtc atc gac ggc aag cca acc ctg atc gtg cct gag cac atc aac ctg     1144
Val Ile Asp Gly Lys Pro Thr Leu Ile Val Pro Glu His Ile Asn Leu
    220                 225                 230
ggc ctt gct atc gac ctt cct cag aag gac ggc tcc cgc gca ctt gtc     1192
Gly Leu Ala Ile Asp Leu Pro Gln Lys Asp Gly Ser Arg Ala Leu Val
235                 240                 245                 250
gta gca gcc atc aag gaa acc gag aag atg aac ttc tcc gag ttc ctc     1240
Val Ala Ala Ile Lys Glu Thr Glu Lys Met Asn Phe Ser Glu Phe Leu

255                 260                 265
gca gcc tac gaa gac atc gtg gca cgc tcc cgc aag ggc aag ctc acc      1288
Ala Ala Tyr Glu Asp Ile Val Ala Arg Ser Arg Lys Gly Lys Leu Thr
        270                 275                 280
atg gat gac tac cag ggc gtt acc gtt tcc ttg acc aac cca ggt ggc      1336
Met Asp Asp Tyr Gln Gly Val Thr Val Ser Leu Thr Asn Pro Gly Gly
    285                 290                 295
atc ggt acc cgc cac tct gtt cca cgt cta acc aag ggc cag ggc acc      1384
Ile Gly Thr Arg His Ser Val Pro Arg Leu Thr Lys Gly Gln Gly Thr
    300                 305                 310
atc atc ggt gtc ggt tcc atg gat tac cca gca gag ttc cag ggc gct      1432
Ile Ile Gly Val Gly Ser Met Asp Tyr Pro Ala Glu Phe Gln Gly Ala
315                 320                 325                 330
tca gaa gac cgc ctt gca gag ctc ggc gtt ggc aaa ctt gtc acc atc      1480
Ser Glu Asp Arg Leu Ala Glu Leu Gly Val Gly Lys Leu Val Thr Ile
                335                 340                 345
acc tcc acc tac gat cac cgc gtg atc cag ggt gct gtg tcc ggt gaa      1528
Thr Ser Thr Tyr Asp His Arg Val Ile Gln Gly Ala Val Ser Gly Glu
            350                 355                 360
ttc ctg cgc acc atg tct cgc ctg ctc acc gat gat tcc ttc tgg gat      1576
Phe Leu Arg Thr Met Ser Arg Leu Leu Thr Asp Asp Ser Phe Trp Asp
        365                 370                 375
gag atc ttc gac gca atg aac gtt cct tac acc cca atg cgt tgg gca      1624
Glu Ile Phe Asp Ala Met Asn Val Pro Tyr Thr Pro Met Arg Trp Ala
    380                 385                 390
cag gac gtt cca aac acc ggt gtt gat aag aac acc cgc gtc atg cag      1672
Gln Asp Val Pro Asn Thr Gly Val Asp Lys Asn Thr Arg Val Met Gln
395                 400                 405                 410
ctc att gag gca tac cgc tcc cgt gga cac ctc atc gct gac acc aac      1720
Leu Ile Glu Ala Tyr Arg Ser Arg Gly His Leu Ile Ala Asp Thr Asn
                415                 420                 425
cca ctt tca tgg gtt cag cct ggc atg cca gtt cca gac cac cgc gac      1768
Pro Leu Ser Trp Val Gln Pro Gly Met Pro Val Pro Asp His Arg Asp
            430                 435                 440
ctc gac atc gag acc cac aac ctg acc atc tgg gat ctg gac cgt acc      1816
Leu Asp Ile Glu Thr His Asn Leu Thr Ile Trp Asp Leu Asp Arg Thr
        445                 450                 455
ttc aac gtc ggt ggc ttc ggc ggc aag gag acc atg acc ctg cgc gag      1864
Phe Asn Val Gly Gly Phe Gly Gly Lys Glu Thr Met Thr Leu Arg Glu
    460                 465                 470
gta ctg tcc cgc ctc cgc gct gcg tac acc ctc aag gtc ggc tcc gaa      1912
Val Leu Ser Arg Leu Arg Ala Ala Tyr Thr Leu Lys Val Gly Ser Glu
475                 480                 485                 490
tac acc cac atc ctg gac cgc gac gag cgc acc tgg ctg cag gac cgc      1960
Tyr Thr His Ile Leu Asp Arg Asp Glu Arg Thr Trp Leu Gln Asp Arg
                495                 500                 505
ctc gag gcc gga atg cca aag cca acc cag gca gag cag aag tac atc      2008
Leu Glu Ala Gly Met Pro Lys Pro Thr Gln Ala Glu Gln Lys Tyr Ile
            510                 515                 520
ctg cag aag ctg aac gcc gcg gag gct ttc gag aac ttc ctg cag acc      2056
Leu Gln Lys Leu Asn Ala Ala Glu Ala Phe Glu Asn Phe Leu Gln Thr
        525                 530                 535
aag tac gtc ggc cag aag cgc ttc tcc ctc gaa ggt gca gaa gca ctt      2104
Lys Tyr Val Gly Gln Lys Arg Phe Ser Leu Glu Gly Ala Glu Ala Leu
    540                 545                 550
atc cca ctg atg gac tcc gcc atc gac acc gcc gca ggc caa ggc ctc      2152
Ile Pro Leu Met Asp Ser Ala Ile Asp Thr Ala Ala Gly Gln Gly Leu
555                 560                 565                 570
gac gaa gtt gtc atc ggt atg cca cac cgt ggt cgc ctc aac gtg ctg      2200
Asp Glu Val Val Ile Gly Met Pro His Arg Gly Arg Leu Asn Val Leu
                575                 580                 585
ttc aac atc gtg ggc aag cca ctg gca tcc atc ttc aac gag ttt gaa      2248
Phe Asn Ile Val Gly Lys Pro Leu Ala Ser Ile Phe Asn Glu Phe Glu

            590                 595                 600
ggc caa atg gag cag ggc cag atc ggt ggc tcc ggt gac gtg aag tac 2296
Gly Gln Met Glu Gln Gly Gln Ile Gly Gly Ser Gly Asp Val Lys Tyr
        605                 610                 615
cac ctc ggt tcc gaa ggc cag cac ctg cag atg ttc ggc gac ggc gag 2344
His Leu Gly Ser Glu Gly Gln His Leu Gln Met Phe Gly Asp Gly Glu
    620                 625                 630
atc aag gtc tcc ctg act gct aac ccg tcc cac ctg gaa gct gtt aac 2392
Ile Lys Val Ser Leu Thr Ala Asn Pro Ser His Leu Glu Ala Val Asn
635                 640                 645                 650
cca gtg atg gaa ggt atc gtc cgc gca aag cag gac tac ctg gac aag 2440
Pro Val Met Glu Gly Ile Val Arg Ala Lys Gln Asp Tyr Leu Asp Lys
                655                 660                 665
ggc gta gac ggc aag act gtt gtg cca ctg ctg ctc cac ggt gac gct 2488
Gly Val Asp Gly Lys Thr Val Val Pro Leu Leu Leu His Gly Asp Ala
            670                 675                 680
gca ttc gca ggc ctg ggc atc gtg cca gaa acc atc aac ctg gct aag 2536
Ala Phe Ala Gly Leu Gly Ile Val Pro Glu Thr Ile Asn Leu Ala Lys
        685                 690                 695
ctg cgt ggc tac gac gtc ggc ggc acc atc cac atc gtg gtg aac aac 2584
Leu Arg Gly Tyr Asp Val Gly Gly Thr Ile His Ile Val Val Asn Asn
    700                 705                 710
cag atc ggc ttc acc acc acc cca gac tcc agc cgc tcc atg cac tac 2632
Gln Ile Gly Phe Thr Thr Thr Pro Asp Ser Ser Arg Ser Met His Tyr
715                 720                 725                 730
gca acc gac tac gcc aag gca ttc ggc tgc cca gtc ttc cac gtc aac 2680
Ala Thr Asp Tyr Ala Lys Ala Phe Gly Cys Pro Val Phe His Val Asn
                735                 740                 745
ggc gac gac cca gag gca gtt gtc tgg gtt ggc cag ctg gcc acc gag 2728
Gly Asp Asp Pro Glu Ala Val Val Trp Val Gly Gln Leu Ala Thr Glu
            750                 755                 760
tac cgt cgt cgc ttc ggc aag gac gtc ttc atc gac ctc gtc tgc tac 2776
Tyr Arg Arg Arg Phe Gly Lys Asp Val Phe Ile Asp Leu Val Cys Tyr
        765                 770                 775
cgc ctc cgc ggc cac aac gaa gct gat gat cct tcc atg acc cag cca 2824
Arg Leu Arg Gly His Asn Glu Ala Asp Asp Pro Ser Met Thr Gln Pro
    780                 785                 790
aag atg tat gag ctc atc acc ggc cgc gag acc gtt cgt gct cag tac 2872
Lys Met Tyr Glu Leu Ile Thr Gly Arg Glu Thr Val Arg Ala Gln Tyr
795                 800                 805                 810
acc gaa gac ctg ctc gga cgt gga gac ctc tcc aac gaa gat gca gaa 2920
Thr Glu Asp Leu Leu Gly Arg Gly Asp Leu Ser Asn Glu Asp Ala Glu
                815                 820                 825
gca gtc gtc cgc gac ttc cac gac cag atg gaa tct gtg ttc aac gaa 2968
Ala Val Val Arg Asp Phe His Asp Gln Met Glu Ser Val Phe Asn Glu
            830                 835                 840
gtc aag gaa ggc ggc aag aag cag gct gag gca cag acc ggc atc acc 3016
Val Lys Glu Gly Gly Lys Lys Gln Ala Glu Ala Gln Thr Gly Ile Thr
        845                 850                 855
ggc tcc cag aag ctt cca cac ggc ctt gag acc aac atc tcc cgt gaa 3064
Gly Ser Gln Lys Leu Pro His Gly Leu Glu Thr Asn Ile Ser Arg Glu
    860                 865                 870
gag ctc ctg gaa ctg gga cag gct ttc gcc aac acc cca gaa ggc ttc 3112
Glu Leu Leu Glu Leu Gly Gln Ala Phe Ala Asn Thr Pro Glu Gly Phe
875                 880                 885                 890
aac tac cac cca cgt gtg gct ccc gtt gct aag aag cgc gtc tcc tct 3160
Asn Tyr His Pro Arg Val Ala Pro Val Ala Lys Lys Arg Val Ser Ser
                895                 900                 905
gtc acc gaa ggt ggc atc gac tgg gca tgg ggc gag ctc ctc gcc ttc 3208
Val Thr Glu Gly Gly Ile Asp Trp Ala Trp Gly Glu Leu Leu Ala Phe
            910                 915                 920
ggt tcc ctg gct aac tcc ggc cgc ttg gtt cgc ctt gca ggt gaa gat 3256
Gly Ser Leu Ala Asn Ser Gly Arg Leu Val Arg Leu Ala Gly Glu Asp

925                 930                 935
tcc cgc cgc ggt acc ttc acc cag cgc cac gca gtt gcc atc gac cca     3304
Ser Arg Arg Gly Thr Phe Thr Gln Arg His Ala Val Ala Ile Asp Pro
    940                 945                 950
gcg acc gct gaa gag ttc aac cca ctc cac gag ctt gca cag tcc aag     3352
Ala Thr Ala Glu Glu Phe Asn Pro Leu His Glu Leu Ala Gln Ser Lys
955                 960                 965                 970
ggc aac aac ggt aag ttc ctg gtc tac aac tcc gca ctg acc gag tac     3400
Gly Asn Asn Gly Lys Phe Leu Val Tyr Asn Ser Ala Leu Thr Glu Tyr
                975                 980                 985
gca ggc atg ggc ttc gag tac ggc tac tcc gta gga aac gaa  gac tcc    3448
Ala Gly Met Gly Phe Glu Tyr Gly Tyr Ser Val Gly Asn Glu  Asp Ser
            990                 995                 1000
atc gtt gca  tgg gaa gca cag ttc  ggc gac ttc gcc aac  ggc gct     3493
Ile Val Ala  Trp Glu Ala Gln Phe  Gly Asp Phe Ala Asn  Gly Ala
        1005                1010                1015
cag acc atc  atc gat gag tac gtc  tcc tca ggc gaa gct  aag tgg     3538
Gln Thr Ile  Ile Asp Glu Tyr Val  Ser Ser Gly Glu Ala  Lys Trp
        1020                1025                1030
ggc cag acc  tcc aag ctg atc ctt  ctg ctg cct cac ggc  tac gaa     3583
Gly Gln Thr  Ser Lys Leu Ile Leu  Leu Leu Pro His Gly  Tyr Glu
        1035                1040                1045
ggc cag ggc  cca gac cac tct tcc  gca cgt atc gag cgc  ttc ctg     3628
Gly Gln Gly  Pro Asp His Ser Ser  Ala Arg Ile Glu Arg  Phe Leu
        1050                1055                1060
cag ctg tgc  gct gag ggt tcc atg  act gtt gct cag cca  tcc acc     3673
Gln Leu Cys  Ala Glu Gly Ser Met  Thr Val Ala Gln Pro  Ser Thr
        1065                1070                1075
cca gca aac  cac ttc cac cta ctg  cgt cgt cac gct ctg  tcc gac     3718
Pro Ala Asn  His Phe His Leu Leu  Arg Arg His Ala Leu  Ser Asp
        1080                1085                1090
ctg aag cgt  cca ctg gtt atc ttc  acc ccg aag tcc atg  ctg cgt     3763
Leu Lys Arg  Pro Leu Val Ile Phe  Thr Pro Lys Ser Met  Leu Arg
        1095                1100                1105
aac aag gct  gct gcc tcc gca cca  gaa gac ttc act gag  gtc acc     3808
Asn Lys Ala  Ala Ala Ser Ala Pro  Glu Asp Phe Thr Glu  Val Thr
        1110                1115                1120
aag ttc cag  tcc gtg atc aac gat  cca aac gtt gca gat  gca gcc     3853
Lys Phe Gln  Ser Val Ile Asn Asp  Pro Asn Val Ala Asp  Ala Ala
        1125                1130                1135
aag gtg aag  aag gtc atg ctg gtc  tcc ggc aag ctg tac  tac gaa     3898
Lys Val Lys  Lys Val Met Leu Val  Ser Gly Lys Leu Tyr  Tyr Glu
        1140                1145                1150
ttg gca aag  cgc aag gag aag gac  gga cgc gac gac atc  gcg atc     3943
Leu Ala Lys  Arg Lys Glu Lys Asp  Gly Arg Asp Asp Ile  Ala Ile
        1155                1160                1165
gtt cgt atc  gaa atg ctc cac cca  att ccg ttc aac cgc  atc tcc     3988
Val Arg Ile  Glu Met Leu His Pro  Ile Pro Phe Asn Arg  Ile Ser
        1170                1175                1180
gag gct ctt  gcc ggc tac cct aac  gct gag gaa gtc ctc  ttc gtt     4033
Glu Ala Leu  Ala Gly Tyr Pro Asn  Ala Glu Glu Val Leu  Phe Val
        1185                1190                1195
cag gat gag  cca gca aac cag ggc  cca tgg ccg ttc tac  cag gag     4078
Gln Asp Glu  Pro Ala Asn Gln Gly  Pro Trp Pro Phe Tyr  Gln Glu
        1200                1205                1210
cac ctc cca  gag ctg atc ccg aac  atg cca aag atg cgc  cgc gtt     4123
His Leu Pro  Glu Leu Ile Pro Asn  Met Pro Lys Met Arg  Arg Val
        1215                1220                1225
tcc cgc cgc  gct cag tcc tcc acc  gca act ggt gtt gcc  aag gtg     4168
Ser Arg Arg  Ala Gln Ser Ser Thr  Ala Thr Gly Val Ala  Lys Val
        1230                1235                1240
cac cag ctg  gag gag aag cag ctt  atc gac gag gct ttc  gag gct     4213
His Gln Leu  Glu Glu Lys Gln Leu  Ile Asp Glu Ala Phe  Glu Ala

1245                1250                1255
taagtcttta tagtcctgca ctagcctaga agggccttat gccgtgtgaa tcacacagca   4273
tacggccctt tttgctgccg tggttgccta aggtggaagg tatgaaacga atctgtgcgg   4333
tcgcgatctc ttcagtactt ttgttaagtg gctgctcctc cacttccaca acgcagctcg   4393
agggatt                                                             4400

<210>  44
<211>  1257
<212>  PRT
<213>  Corynebacterium glutamicum

<400>  44
Met Leu Gln Leu Gly Leu Arg His Asn Gln Pro Thr Thr Asn Val Thr
1               5                   10                  15
Val Asp Lys Thr Lys Leu Asn Lys Pro Ser Arg Ser Lys Glu Lys Arg
            20                  25                  30
Arg Val Pro Ala Val Ser Ser Ala Ser Thr Phe Gly Gln Asn Ala Trp
        35                  40                  45
Leu Val Asp Glu Met Phe Gln Gln Phe Gln Lys Asp Pro Lys Ser Val
    50                  55                  60
Asp Lys Glu Trp Arg Glu Leu Phe Glu Ala Gln Gly Gly Pro Asn Thr
65                  70                  75                  80
Thr Pro Ala Thr Thr Glu Ala Gln Pro Ser Ala Pro Lys Glu Ser Ala
                85                  90                  95
Lys Pro Ala Pro Lys Ala Ala Pro Ala Ala Lys Ala Ala Pro Arg Val
            100                 105                 110
Glu Thr Lys Pro Ala Asp Lys Thr Ala Pro Lys Ala Lys Glu Ser Ser
        115                 120                 125
Val Pro Gln Gln Pro Lys Leu Pro Glu Pro Gly Gln Thr Pro Ile Arg
    130                 135                 140
Gly Ile Phe Lys Ser Ile Ala Lys Asn Met Asp Ile Ser Leu Glu Ile
145                 150                 155                 160
Pro Thr Ala Thr Ser Val Arg Asp Met Pro Ala Arg Leu Met Phe Glu
                165                 170                 175
Asn Arg Ala Met Val Asn Asp Gln Leu Lys Arg Thr Arg Gly Gly Lys
            180                 185                 190
Ile Ser Phe Thr His Ile Ile Gly Tyr Ala Met Val Lys Ala Val Met
        195                 200                 205
Ala His Pro Asp Met Asn Asn Ser Tyr Asp Val Ile Asp Gly Lys Pro
    210                 215                 220
Thr Leu Ile Val Pro Glu His Ile Asn Leu Gly Leu Ala Ile Asp Leu
225                 230                 235                 240
Pro Gln Lys Asp Gly Ser Arg Ala Leu Val Val Ala Ala Ile Lys Glu
                245                 250                 255
Thr Glu Lys Met Asn Phe Ser Glu Phe Leu Ala Ala Tyr Glu Asp Ile
            260                 265                 270
Val Ala Arg Ser Arg Lys Gly Lys Leu Thr Met Asp Asp Tyr Gln Gly
        275                 280                 285
Val Thr Val Ser Leu Thr Asn Pro Gly Gly Ile Gly Thr Arg His Ser
    290                 295                 300
Val Pro Arg Leu Thr Lys Gly Gln Gly Thr Ile Ile Gly Val Gly Ser
305                 310                 315                 320
Met Asp Tyr Pro Ala Glu Phe Gln Gly Ala Ser Glu Asp Arg Leu Ala
                325                 330                 335
Glu Leu Gly Val Gly Lys Leu Val Thr Ile Thr Ser Thr Tyr Asp His
            340                 345                 350
Arg Val Ile Gln Gly Ala Val Ser Gly Glu Phe Leu Arg Thr Met Ser
        355                 360                 365
Arg Leu Leu Thr Asp Asp Ser Phe Trp Asp Glu Ile Phe Asp Ala Met
    370                 375                 380
Asn Val Pro Tyr Thr Pro Met Arg Trp Ala Gln Asp Val Pro Asn Thr
385                 390                 395                 400

Gly Val Asp Lys Asn Thr Arg Val Met Gln Leu Ile Glu Ala Tyr Arg
                405                 410                 415
Ser Arg Gly His Leu Ile Ala Asp Thr Asn Pro Leu Ser Trp Val Gln
            420                 425                 430
Pro Gly Met Pro Val Pro Asp His Arg Asp Leu Asp Ile Glu Thr His
        435                 440                 445
Asn Leu Thr Ile Trp Asp Leu Asp Arg Thr Phe Asn Val Gly Gly Phe
    450                 455                 460
Gly Gly Lys Glu Thr Met Thr Leu Arg Glu Val Leu Ser Arg Leu Arg
465                 470                 475                 480
Ala Ala Tyr Thr Leu Lys Val Gly Ser Glu Tyr Thr His Ile Leu Asp
                485                 490                 495
Arg Asp Glu Arg Thr Trp Leu Gln Asp Arg Leu Glu Ala Gly Met Pro
            500                 505                 510
Lys Pro Thr Gln Ala Glu Gln Lys Tyr Ile Leu Gln Lys Leu Asn Ala
        515                 520                 525
Ala Glu Ala Phe Glu Asn Phe Leu Gln Thr Lys Tyr Val Gly Gln Lys
    530                 535                 540
Arg Phe Ser Leu Glu Gly Ala Glu Ala Leu Ile Pro Leu Met Asp Ser
545                 550                 555                 560
Ala Ile Asp Thr Ala Ala Gly Gln Gly Leu Asp Glu Val Val Ile Gly
                565                 570                 575
Met Pro His Arg Gly Arg Leu Asn Val Leu Phe Asn Ile Val Gly Lys
            580                 585                 590
Pro Leu Ala Ser Ile Phe Asn Glu Phe Glu Gly Gln Met Glu Gln Gly
        595                 600                 605
Gln Ile Gly Gly Ser Gly Asp Val Lys Tyr His Leu Gly Ser Glu Gly
    610                 615                 620
Gln His Leu Gln Met Phe Gly Asp Gly Glu Ile Lys Val Ser Leu Thr
625                 630                 635                 640
Ala Asn Pro Ser His Leu Glu Ala Val Asn Pro Val Met Glu Gly Ile
                645                 650                 655
Val Arg Ala Lys Gln Asp Tyr Leu Asp Lys Gly Val Asp Gly Lys Thr
            660                 665                 670
Val Val Pro Leu Leu Leu His Gly Asp Ala Ala Phe Ala Gly Leu Gly
        675                 680                 685
Ile Val Pro Glu Thr Ile Asn Leu Ala Lys Leu Arg Gly Tyr Asp Val
    690                 695                 700
Gly Gly Thr Ile His Ile Val Val Asn Asn Gln Ile Gly Phe Thr Thr
705                 710                 715                 720
Thr Pro Asp Ser Ser Arg Ser Met His Tyr Ala Thr Asp Tyr Ala Lys
                725                 730                 735
Ala Phe Gly Cys Pro Val Phe His Val Asn Gly Asp Asp Pro Glu Ala
            740                 745                 750
Val Val Trp Val Gly Gln Leu Ala Thr Glu Tyr Arg Arg Arg Phe Gly
        755                 760                 765
Lys Asp Val Phe Ile Asp Leu Val Cys Tyr Arg Leu Arg Gly His Asn
    770                 775                 780
Glu Ala Asp Asp Pro Ser Met Thr Gln Pro Lys Met Tyr Glu Leu Ile
785                 790                 795                 800
Thr Gly Arg Glu Thr Val Arg Ala Gln Tyr Thr Glu Asp Leu Leu Gly
                805                 810                 815
Arg Gly Asp Leu Ser Asn Glu Asp Ala Glu Ala Val Val Arg Asp Phe
            820                 825                 830
His Asp Gln Met Glu Ser Val Phe Asn Glu Val Lys Glu Gly Gly Lys
        835                 840                 845
Lys Gln Ala Glu Ala Gln Thr Gly Ile Thr Gly Ser Gln Lys Leu Pro
    850                 855                 860
His Gly Leu Glu Thr Asn Ile Ser Arg Glu Glu Leu Leu Glu Leu Gly
865                 870                 875                 880
Gln Ala Phe Ala Asn Thr Pro Glu Gly Phe Asn Tyr His Pro Arg Val
                885                 890                 895
Ala Pro Val Ala Lys Lys Arg Val Ser Ser Val Thr Glu Gly Gly Ile

900                 905                 910
Asp Trp Ala Trp Gly Glu Leu Leu Ala Phe Gly Ser Leu Ala Asn Ser
        915                 920                 925
Gly Arg Leu Val Arg Leu Ala Gly Glu Asp Ser Arg Arg Gly Thr Phe
    930                 935                 940
Thr Gln Arg His Ala Val Ala Ile Asp Pro Ala Thr Ala Glu Glu Phe
945                 950                 955                 960
Asn Pro Leu His Glu Leu Ala Gln Ser Lys Gly Asn Asn Gly Lys Phe
                965                 970                 975
Leu Val Tyr Asn Ser Ala Leu Thr Glu Tyr Ala Gly Met Gly Phe Glu
            980                 985                 990
Tyr Gly Tyr Ser Val Gly Asn Glu  Asp Ser Ile Val Ala  Trp Glu Ala
        995                 1000                1005
Gln Phe  Gly Asp Phe Ala Asn  Gly Ala Gln Thr Ile  Ile Asp Glu
    1010                1015                1020
Tyr Val  Ser Ser Gly Glu Ala  Lys Trp Gly Gln Thr  Ser Lys Leu
    1025                1030                1035
Ile Leu  Leu Leu Pro His Gly  Tyr Glu Gly Gln Gly  Pro Asp His
    1040                1045                1050
Ser Ser  Ala Arg Ile Glu Arg  Phe Leu Gln Leu Cys  Ala Glu Gly
    1055                1060                1065
Ser Met  Thr Val Ala Gln Pro  Ser Thr Pro Ala Asn  His Phe His
    1070                1075                1080
Leu Leu  Arg Arg His Ala Leu  Ser Asp Leu Lys Arg  Pro Leu Val
    1085                1090                1095
Ile Phe  Thr Pro Lys Ser Met  Leu Arg Asn Lys Ala  Ala Ala Ser
    1100                1105                1110
Ala Pro  Glu Asp Phe Thr Glu  Val Thr Lys Phe Gln  Ser Val Ile
    1115                1120                1125
Asn Asp  Pro Asn Val Ala Asp  Ala Ala Lys Val Lys  Lys Val Met
    1130                1135                1140
Leu Val  Ser Gly Lys Leu Tyr  Tyr Glu Leu Ala Lys  Arg Lys Glu
    1145                1150                1155
Lys Asp  Gly Arg Asp Asp Ile  Ala Ile Val Arg Ile  Glu Met Leu
    1160                1165                1170
His Pro  Ile Pro Phe Asn Arg  Ile Ser Glu Ala Leu  Ala Gly Tyr
    1175                1180                1185
Pro Asn  Ala Glu Glu Val Leu  Phe Val Gln Asp Glu  Pro Ala Asn
    1190                1195                1200
Gln Gly  Pro Trp Pro Phe Tyr  Gln Glu His Leu Pro  Glu Leu Ile
    1205                1210                1215
Pro Asn  Met Pro Lys Met Arg  Arg Val Ser Arg Arg  Ala Gln Ser
    1220                1225                1230
Ser Thr  Ala Thr Gly Val Ala  Lys Val His Gln Leu  Glu Glu Lys
    1235                1240                1245
Gln Leu  Ile Asp Glu Ala Phe  Glu Ala
    1250                1255

<210>  45
<211>  4389
<212>  DNA
<213>  Corynebacterium glutamicum

<220>
<221>  CDS
<222>  (443)..(2542)
<223>  sucA8

<400>  45
gtcgacaagc aaaatcgaag cggcagcacg ccgcgtcgga gccttaaacg ccatcgccgc      60
catccctgat ggtttcaatc atcaagtcgg tgaacgcggg cgcaacctgt catccggaca     120
gcgccaactg atcgcgctgg cgcgcgccga actcatcgag ccttccatca tgcttctcga     180

cgaagccacc tccaccctcg accccgccac cgaagccgtt atcctcaacg cctccgatcg 240
agtcactaag ggacgcacca gcatcatcgt cgcgcaccgc ttggcaaccg ctaaaagggc 300
cgaccgtatt cttgttgttg aacaaggacg tatcattgag gacggatctc acgacgcgtt 360
gttgtctgct aacggcacct acgcccgcat gtggcattta atggcctgac acgttatttt 420
taggagaact gtcaacaaat ta atg cta caa ctg ggg ctt agg cat aat cag 472
Met Leu Gln Leu Gly Leu Arg His Asn Gln
1               5                   10
cca acg acc aac gtt aca gtg gat aaa ata aag ctc aat aaa ccc tca 520
Pro Thr Thr Asn Val Thr Val Asp Lys Ile Lys Leu Asn Lys Pro Ser
                15                  20                  25
aga agc aag gaa aag agg cga gta cct gcc gtg agc agc gct agt act 568
Arg Ser Lys Glu Lys Arg Arg Val Pro Ala Val Ser Ser Ala Ser Thr
            30                  35                  40
ttc ggc cag aat gcg tgg ctg gta gac gag atg ttc cag cag ttc cag 616
Phe Gly Gln Asn Ala Trp Leu Val Asp Glu Met Phe Gln Gln Phe Gln
        45                  50                  55
aag gac ccc aag tcc gtg gac aag gaa tgg aga gaa ctc ttt gag gcg 664
Lys Asp Pro Lys Ser Val Asp Lys Glu Trp Arg Glu Leu Phe Glu Ala
    60                  65                  70
cag ggg gga cca aat gct acc ccc gct aca aca gaa gca cag cct tca 712
Gln Gly Gly Pro Asn Ala Thr Pro Ala Thr Thr Glu Ala Gln Pro Ser
75                  80                  85                  90
gcg ccc aag gag tct gcg aaa cca gca cca aag gct gcc cct gca gcc 760
Ala Pro Lys Glu Ser Ala Lys Pro Ala Pro Lys Ala Ala Pro Ala Ala
                95                  100                 105
aag gca gca ccg cgc gta gaa acc aag ccg gcc gcc aag acc gcc cct 808
Lys Ala Ala Pro Arg Val Glu Thr Lys Pro Ala Ala Lys Thr Ala Pro
            110                 115                 120
aag gcc aag gag tcc tca gtg cca cag caa cct aag ctt ccg gag cca 856
Lys Ala Lys Glu Ser Ser Val Pro Gln Gln Pro Lys Leu Pro Glu Pro
        125                 130                 135
gga caa acc cca atc agg ggt att ttc aag tcc atc gcg aag aac atg 904
Gly Gln Thr Pro Ile Arg Gly Ile Phe Lys Ser Ile Ala Lys Asn Met
    140                 145                 150
gat atc tcc ctg gaa atc cca acc gca acc tcg gtt cgc gat atg cca 952
Asp Ile Ser Leu Glu Ile Pro Thr Ala Thr Ser Val Arg Asp Met Pro
155                 160                 165                 170
gct cgc ctc atg ttc gaa aac cgc gcg atg gtc aac gat cag ctc aag 1000
Ala Arg Leu Met Phe Glu Asn Arg Ala Met Val Asn Asp Gln Leu Lys
                175                 180                 185
cgc acc cgc ggt ggc aag atc tcc ttc acc cac atc att ggc tac gcc 1048
Arg Thr Arg Gly Gly Lys Ile Ser Phe Thr His Ile Ile Gly Tyr Ala
            190                 195                 200
atg gtg aag gca gtc atg gct cac ccg gac atg aac aac tcc tac gac 1096
Met Val Lys Ala Val Met Ala His Pro Asp Met Asn Asn Ser Tyr Asp
        205                 210                 215
gtc atc gac ggc aag cca acc ctg atc gtg cct gag cac atc aac ctg 1144
Val Ile Asp Gly Lys Pro Thr Leu Ile Val Pro Glu His Ile Asn Leu
    220                 225                 230
ggc ctt gcc atc gac ctt cct cag aag gac ggc tcc cgc gca ctt gtc 1192
Gly Leu Ala Ile Asp Leu Pro Gln Lys Asp Gly Ser Arg Ala Leu Val
235                 240                 245                 250
gta gca gcc atc aag gaa acc gag aag atg aac ttc tcc gag ttc ctc 1240
Val Ala Ala Ile Lys Glu Thr Glu Lys Met Asn Phe Ser Glu Phe Leu
                255                 260                 265
gca gca tac gaa gac atc gtg aca cgc tcc cgc aag ggc aag ctc acc 1288
Ala Ala Tyr Glu Asp Ile Val Thr Arg Ser Arg Lys Gly Lys Leu Thr
            270                 275                 280
atg gat gac tac cag ggc gtt acc gtt tcc ttg acc aac cca ggt ggc 1336
Met Asp Asp Tyr Gln Gly Val Thr Val Ser Leu Thr Asn Pro Gly Gly
        285                 290                 295
atc ggt acc cgc cac tct gtc cca cgt ctg acc aag ggc cag ggc acc 1384
Ile Gly Thr Arg His Ser Val Pro Arg Leu Thr Lys Gly Gln Gly Thr

300                 305                 310
atc atc ggt gtc ggt tcc atg gat tac cca gca gag ttc cag ggc gct      1432
Ile Ile Gly Val Gly Ser Met Asp Tyr Pro Ala Glu Phe Gln Gly Ala
315                 320                 325                 330
tcc gaa gac cgc ctt gca gag ctc ggc gtt gga aag ctt gtc acc atc      1480
Ser Glu Asp Arg Leu Ala Glu Leu Gly Val Gly Lys Leu Val Thr Ile
                335                 340                 345
acc tcc acc tac gat cac cgc gtg atc cag ggt gct gtg tcc ggt gaa      1528
Thr Ser Thr Tyr Asp His Arg Val Ile Gln Gly Ala Val Ser Gly Glu
            350                 355                 360
ttc ctg cgt acc atg tct cgc ctg ctc acc gat gat tcc ttc tgg gat      1576
Phe Leu Arg Thr Met Ser Arg Leu Leu Thr Asp Asp Ser Phe Trp Asp
        365                 370                 375
gag atc ttc gac gca atg aac gtt cct tac acc cca atg cgt tgg gca      1624
Glu Ile Phe Asp Ala Met Asn Val Pro Tyr Thr Pro Met Arg Trp Ala
    380                 385                 390
cag gac gtt cca aac acc ggt gtt gat aag aac acc cgc gtc atg cag      1672
Gln Asp Val Pro Asn Thr Gly Val Asp Lys Asn Thr Arg Val Met Gln
395                 400                 405                 410
ctc att gag gca tac cgc tcc cgt gga cac ctc atc gct gac acc aac      1720
Leu Ile Glu Ala Tyr Arg Ser Arg Gly His Leu Ile Ala Asp Thr Asn
                415                 420                 425
cca ctt tca tgg gtt cag cct ggc atg cca gtt cca gac cac cgc gac      1768
Pro Leu Ser Trp Val Gln Pro Gly Met Pro Val Pro Asp His Arg Asp
            430                 435                 440
ctc gac atc gag acc cac agc ctg acc atc tgg gat ctg gac cgt acc      1816
Leu Asp Ile Glu Thr His Ser Leu Thr Ile Trp Asp Leu Asp Arg Thr
        445                 450                 455
ttc agc gtc ggt ggc ttc ggc ggc aag gag acc atg acc ctg cgc gag      1864
Phe Ser Val Gly Gly Phe Gly Gly Lys Glu Thr Met Thr Leu Arg Glu
    460                 465                 470
gta ctg tcc cgc ctg cgc gct gcc tac acc ttg aag gtc ggc tcc gaa      1912
Val Leu Ser Arg Leu Arg Ala Ala Tyr Thr Leu Lys Val Gly Ser Glu
475                 480                 485                 490
tac acc cac atc ctg gac cgc gac gag cgc acc tgg ctg cag gac cgc      1960
Tyr Thr His Ile Leu Asp Arg Asp Glu Arg Thr Trp Leu Gln Asp Arg
                495                 500                 505
ctc gaa gcc gga atg cca aag cca acc cag gca gag cag aag tac atc      2008
Leu Glu Ala Gly Met Pro Lys Pro Thr Gln Ala Glu Gln Lys Tyr Ile
            510                 515                 520
ctg cag aag ctg aac gcc gca gag gct ttc gag aac ttc ctg cag acc      2056
Leu Gln Lys Leu Asn Ala Ala Glu Ala Phe Glu Asn Phe Leu Gln Thr
        525                 530                 535
aag tac gtc ggc cag aag cgc ttc tcc ctc gaa ggt gca gaa gct ctc      2104
Lys Tyr Val Gly Gln Lys Arg Phe Ser Leu Glu Gly Ala Glu Ala Leu
    540                 545                 550
atc cca ctg atg gac tcc gcc atc gac acc gcc gca ggc cag ggc ctc      2152
Ile Pro Leu Met Asp Ser Ala Ile Asp Thr Ala Ala Gly Gln Gly Leu
555                 560                 565                 570
gac gaa gtt gtc atc ggt atg cca cac cgt ggt cgc ctc aac gtg ctg      2200
Asp Glu Val Val Ile Gly Met Pro His Arg Gly Arg Leu Asn Val Leu
                575                 580                 585
ttc aac atc gtg ggc aag cca ctg gca tcc atc ttc aac gag ttt gaa      2248
Phe Asn Ile Val Gly Lys Pro Leu Ala Ser Ile Phe Asn Glu Phe Glu
            590                 595                 600
ggc caa atg gag cag ggc cag atc ggt ggc tcc ggt gac gtg aag tac      2296
Gly Gln Met Glu Gln Gly Gln Ile Gly Gly Ser Gly Asp Val Lys Tyr
        605                 610                 615
cac ctc ggt tcc gaa ggc cag cac ctg cag atg ttc ggc gac ggc gag      2344
His Leu Gly Ser Glu Gly Gln His Leu Gln Met Phe Gly Asp Gly Glu
    620                 625                 630
atc aag gtc tcc ctg act gct aac ccg tcc cac ctg gaa gct gtt aac      2392
Ile Lys Val Ser Leu Thr Ala Asn Pro Ser His Leu Glu Ala Val Asn

635                 640                 645                 650
cca gtg atg gaa ggt atc gtc cgc gca aag cag gac tac ctg gac aag      2440
Pro Val Met Glu Gly Ile Val Arg Ala Lys Gln Asp Tyr Leu Asp Lys
                655                 660                 665
ggc gta gac ggc aag act gtt gtg cca ctg ctg ctc cac ggt gac gct      2488
Gly Val Asp Gly Lys Thr Val Val Pro Leu Leu Leu His Gly Asp Ala
            670                 675                 680
gca ttc gca ggc ctg ggc atc gtg cca gaa acc atc aac ctg gct aag      2536
Ala Phe Ala Gly Leu Gly Ile Val Pro Glu Thr Ile Asn Leu Ala Lys
        685                 690                 695
ctg cgt cgacgtcgga ggcaccatcc acatcgtggt gaacaaccag atcggcttca      2592
Leu Arg
    700
ccaccacccc agactccagc cgctccatgc actacgcaac cgactacgcc aaggcattcg      2652
gctgcccagt cttccacgtc aatggtgatg acccagaggc agttgtctgg gttggccagc      2712
tggcaaccga gtaccgtcgt cgcttcggca aggacgtctt catcgacctc gtttgctacc      2772
gcctccgcgg ccacaacgaa gctgatgatc cttccatgac ccagccaaag atgtatgagc      2832
tcatcaccgg ccgcgagacc gttcgtgctc agtacaccga agacctgctc ggacgtggag      2892
acctctccaa cgaagatgca gaagcagtcg tccgcgactt ccacgaccag atggaatctg      2952
tgttcaacga agtcaaggaa ggcggcaaga agcaggctga ggcacagacc ggcatcaccg      3012
gctcccagaa gcttccacac ggccttgaga ccaacatctc ccgtgaagag ctcctggaac      3072
tgggacaggc tttcgccaac accccagaag gcttcaacta ccacccacgt gtggctccag      3132
ttgctaagaa gcgcgtctcc tctgtcaccg aaggtggcat cgactgggca tggggcgagc      3192
tcctcgcctt cggttccctg gctaactccg gccgcttggt tcgccttgca ggtgaagatt      3252
cccgccgcgg taccttcacc cagcgccacg cagttgccat cgacccagcg accgctgaag      3312
agttcaaccc actccacgag cttgcacagt ccaagggcaa caacggtaag ttcctggtct      3372
acaactccgc actgaccgag tacgcaggca tgggcttcga gtacggctac tccgtaggaa      3432
acgaagactc cgtcgttgca tgggaagcac agttcggcga cttcgccaac ggcgctcaga      3492
ccatcatcga tgagtacgtc tcctcaggcg aagctaagtg gggccagacc tccaagctga      3552
tccttctgct gcctcacggc tacgaaggcc agggcccaga ccactcttcc gcacgtatcg      3612
agcgcttcct gcagctgtgc gctgagggtt ccatgactgt tgctcagcca tccaccccag      3672
caaaccactt ccacctgctg cgtcgtcacg ctctgtccga cctgaagcgt ccactggtta      3732
tcttcacccc gaagtccatg ctgcgtaaca aggctgctgc ctccgcacca gaagacttca      3792
ctgaggtcac caagttccaa tccgtgatcg acgatccaaa cgttgcagat gcagccaagg      3852
tgaagaaggt catgctggtc tccggcaagc tgtactacga attggcaaag cgcaaggaga      3912
aggacggacg cgacgacatc gcgatcgttc gtatcgaaat gctccaccca attccgttca      3972
accgcatctc cgaggctctt gccggctacc ctaacgctga ggaagtcctc ttcgttcagg      4032
atgagccagc aaaccagggc ccatggccgt tctaccagga gcacctccca gagctgatcc      4092
cgaacatgcc aaagatgcgc cgcgtttccc gccgcgctca gtcctccacc gcaactggtg      4152
ttgctaaggt gcaccagctg gaggagaagc agcttatcga cgaggctttc gaggcttaag      4212
tctttatagt cctgcactag cctagagggc cttatgcagt gtgaatcaca cagcataagg      4272
ccctttttgc tgccgtggtt gcctaaggtg gaaggcatga aacgaatctg tgcggtcacg      4332
atctcttcag tacttttgct aagtggctgc tcctccactt ccaccacgca gctcgag      4389

<210>  46
<211>  700
<212>  PRT
<213>  Corynebacterium glutamicum

<400>  46
Met Leu Gln Leu Gly Leu Arg His Asn Gln Pro Thr Thr Asn Val Thr
1               5                   10                  15
Val Asp Lys Ile Lys Leu Asn Lys Pro Ser Arg Ser Lys Glu Lys Arg
            20                  25                  30
Arg Val Pro Ala Val Ser Ser Ala Ser Thr Phe Gly Gln Asn Ala Trp
        35                  40                  45
Leu Val Asp Glu Met Phe Gln Gln Phe Gln Lys Asp Pro Lys Ser Val
    50                  55                  60
Asp Lys Glu Trp Arg Glu Leu Phe Glu Ala Gln Gly Gly Pro Asn Ala
65                  70                  75                  80
Thr Pro Ala Thr Thr Glu Ala Gln Pro Ser Ala Pro Lys Glu Ser Ala
                85                  90                  95
Lys Pro Ala Pro Lys Ala Ala Pro Ala Ala Lys Ala Ala Pro Arg Val

                100                 105                 110
Glu Thr Lys Pro Ala Ala Lys Thr Ala Pro Lys Ala Lys Glu Ser Ser
        115                 120                 125
Val Pro Gln Gln Pro Lys Leu Pro Glu Pro Gly Gln Thr Pro Ile Arg
    130                 135                 140
Gly Ile Phe Lys Ser Ile Ala Lys Asn Met Asp Ile Ser Leu Glu Ile
145                 150                 155                 160
Pro Thr Ala Thr Ser Val Arg Asp Met Pro Ala Arg Leu Met Phe Glu
                165                 170                 175
Asn Arg Ala Met Val Asn Asp Gln Leu Lys Arg Thr Arg Gly Gly Lys
            180                 185                 190
Ile Ser Phe Thr His Ile Ile Gly Tyr Ala Met Val Lys Ala Val Met
        195                 200                 205
Ala His Pro Asp Met Asn Asn Ser Tyr Asp Val Ile Asp Gly Lys Pro
    210                 215                 220
Thr Leu Ile Val Pro Glu His Ile Asn Leu Gly Leu Ala Ile Asp Leu
225                 230                 235                 240
Pro Gln Lys Asp Gly Ser Arg Ala Leu Val Val Ala Ala Ile Lys Glu
                245                 250                 255
Thr Glu Lys Met Asn Phe Ser Glu Phe Leu Ala Ala Tyr Glu Asp Ile
            260                 265                 270
Val Thr Arg Ser Arg Lys Gly Lys Leu Thr Met Asp Asp Tyr Gln Gly
        275                 280                 285
Val Thr Val Ser Leu Thr Asn Pro Gly Gly Ile Gly Thr Arg His Ser
    290                 295                 300
Val Pro Arg Leu Thr Lys Gly Gln Gly Thr Ile Ile Gly Val Gly Ser
305                 310                 315                 320
Met Asp Tyr Pro Ala Glu Phe Gln Gly Ala Ser Glu Asp Arg Leu Ala
                325                 330                 335
Glu Leu Gly Val Gly Lys Leu Val Thr Ile Thr Ser Thr Tyr Asp His
            340                 345                 350
Arg Val Ile Gln Gly Ala Val Ser Gly Glu Phe Leu Arg Thr Met Ser
        355                 360                 365
Arg Leu Leu Thr Asp Asp Ser Phe Trp Asp Glu Ile Phe Asp Ala Met
    370                 375                 380
Asn Val Pro Tyr Thr Pro Met Arg Trp Ala Gln Asp Val Pro Asn Thr
385                 390                 395                 400
Gly Val Asp Lys Asn Thr Arg Val Met Gln Leu Ile Glu Ala Tyr Arg
                405                 410                 415
Ser Arg Gly His Leu Ile Ala Asp Thr Asn Pro Leu Ser Trp Val Gln
            420                 425                 430
Pro Gly Met Pro Val Pro Asp His Arg Asp Leu Asp Ile Glu Thr His
        435                 440                 445
Ser Leu Thr Ile Trp Asp Leu Asp Arg Thr Phe Ser Val Gly Gly Phe
    450                 455                 460
Gly Gly Lys Glu Thr Met Thr Leu Arg Glu Val Leu Ser Arg Leu Arg
465                 470                 475                 480
Ala Ala Tyr Thr Leu Lys Val Gly Ser Glu Tyr Thr His Ile Leu Asp
                485                 490                 495
Arg Asp Glu Arg Thr Trp Leu Gln Asp Arg Leu Glu Ala Gly Met Pro
            500                 505                 510
Lys Pro Thr Gln Ala Glu Gln Lys Tyr Ile Leu Gln Lys Leu Asn Ala
        515                 520                 525
Ala Glu Ala Phe Glu Asn Phe Leu Gln Thr Lys Tyr Val Gly Gln Lys
    530                 535                 540
Arg Phe Ser Leu Glu Gly Ala Glu Ala Leu Ile Pro Leu Met Asp Ser
545                 550                 555                 560
Ala Ile Asp Thr Ala Ala Gly Gln Gly Leu Asp Glu Val Val Ile Gly
                565                 570                 575
Met Pro His Arg Gly Arg Leu Asn Val Leu Phe Asn Ile Val Gly Lys
            580                 585                 590
Pro Leu Ala Ser Ile Phe Asn Glu Phe Glu Gly Gln Met Glu Gln Gly

595                 600                 605
Gln Ile Gly Gly Ser Gly Asp Val Lys Tyr His Leu Gly Ser Glu Gly
    610                 615                 620
Gln His Leu Gln Met Phe Gly Asp Gly Glu Ile Lys Val Ser Leu Thr
625                 630                 635                 640
Ala Asn Pro Ser His Leu Glu Ala Val Asn Pro Val Met Glu Gly Ile
                645                 650                 655
Val Arg Ala Lys Gln Asp Tyr Leu Asp Lys Gly Val Asp Gly Lys Thr
            660                 665                 670
Val Val Pro Leu Leu Leu His Gly Asp Ala Ala Phe Ala Gly Leu Gly
        675                 680                 685
Ile Val Pro Glu Thr Ile Asn Leu Ala Lys Leu Arg
    690                 695                 700

<210>  47
<211>  4391
<212>  DNA
<213>  Corynebacterium glutamicum

<220>
<221>  CDS
<222>  (443)..(4210)
<223>  sucA801

<400>  47
gtcgacaagc aaaatcgaag cggcagcacg ccgcgtcgga gccttaaacg ccatcgccgc      60
catccctgat ggtttcaatc atcaagtcgg tgaacgcggg cgcaacctgt catccggaca     120
gcgccaactg atcgcgctgg cgcgcgccga actcatcgag ccttccatca tgcttctcga     180
cgaagccacc tccaccctcg accccgccac cgaagccgtt atcctcaacg cctccgatcg     240
agtcactaag ggacgcacca gcatcatcgt cgcgcaccgc ttggcaaccg ctaaaagggc     300
cgaccgtatt cttgttgttg aacaaggacg tatcattgag gacggatctc acgacgcgtt     360
gttgtctgct aacggcacct acgcccgcat gtggcattta atggcctgac acgttatttt     420
taggagaact gtcaacaaat ta atg cta caa ctg ggg ctt agg cat aat cag     472
                         Met Leu Gln Leu Gly Leu Arg His Asn Gln
                         1               5                   10
cca acg acc aac gtt aca gtg gat aaa ata aag ctc aat aaa ccc tca     520
Pro Thr Thr Asn Val Thr Val Asp Lys Ile Lys Leu Asn Lys Pro Ser
                15                  20                  25
aga agc aag gaa aag agg cga gta cct gcc gtg agc agc gct agt act     568
Arg Ser Lys Glu Lys Arg Arg Val Pro Ala Val Ser Ser Ala Ser Thr
            30                  35                  40
ttc ggc cag aat gcg tgg ctg gta gac gag atg ttc cag cag ttc cag     616
Phe Gly Gln Asn Ala Trp Leu Val Asp Glu Met Phe Gln Gln Phe Gln
        45                  50                  55
aag gac ccc aag tcc gtg gac aag gaa tgg aga gaa ctc ttt gag gcg     664
Lys Asp Pro Lys Ser Val Asp Lys Glu Trp Arg Glu Leu Phe Glu Ala
    60                  65                  70
cag ggg gga cca aat gct acc ccc gct aca aca gaa gca cag cct tca     712
Gln Gly Gly Pro Asn Ala Thr Pro Ala Thr Thr Glu Ala Gln Pro Ser
75                  80                  85                  90
gcg ccc aag gag tct gcg aaa cca gca cca aag gct gcc cct gca gcc     760
Ala Pro Lys Glu Ser Ala Lys Pro Ala Pro Lys Ala Ala Pro Ala Ala
                95                  100                 105
aag gca gca ccg cgc gta gaa acc aag ccg gcc gcc aag acc gcc cct     808
Lys Ala Ala Pro Arg Val Glu Thr Lys Pro Ala Ala Lys Thr Ala Pro
            110                 115                 120
aag gcc aag gag tcc tca gtg cca cag caa cct aag ctt ccg gag cca     856
Lys Ala Lys Glu Ser Ser Val Pro Gln Gln Pro Lys Leu Pro Glu Pro
        125                 130                 135
gga caa acc cca atc agg ggt att ttc aag tcc atc gcg aag aac atg     904
Gly Gln Thr Pro Ile Arg Gly Ile Phe Lys Ser Ile Ala Lys Asn Met
    140                 145                 150
gat atc tcc ctg gaa atc cca acc gca acc tcg gtt cgc gat atg cca     952

Asp Ile Ser Leu Glu Ile Pro Thr Ala Thr Ser Val Arg Asp Met Pro
155                 160                 165                 170
gct cgc ctc atg ttc gaa aac cgc gcg atg gtc aac gat cag ctc aag      1000
Ala Arg Leu Met Phe Glu Asn Arg Ala Met Val Asn Asp Gln Leu Lys
                175                 180                 185
cgc acc cgc ggt ggc aag atc tcc ttc acc cac atc att ggc tac gcc      1048
Arg Thr Arg Gly Gly Lys Ile Ser Phe Thr His Ile Ile Gly Tyr Ala
            190                 195                 200
atg gtg aag gca gtc atg gct cac ccg gac atg aac aac tcc tac gac      1096
Met Val Lys Ala Val Met Ala His Pro Asp Met Asn Asn Ser Tyr Asp
        205                 210                 215
gtc atc gac ggc aag cca acc ctg atc gtg cct gag cac atc aac ctg      1144
Val Ile Asp Gly Lys Pro Thr Leu Ile Val Pro Glu His Ile Asn Leu
    220                 225                 230
ggc ctt gcc atc gac ctt cct cag aag gac ggc tcc cgc gca ctt gtc      1192
Gly Leu Ala Ile Asp Leu Pro Gln Lys Asp Gly Ser Arg Ala Leu Val
235                 240                 245                 250
gta gca gcc atc aag gaa acc gag aag atg aac ttc tcc gag ttc ctc      1240
Val Ala Ala Ile Lys Glu Thr Glu Lys Met Asn Phe Ser Glu Phe Leu
                255                 260                 265
gca gca tac gaa gac atc gtg aca cgc tcc cgc aag ggc aag ctc acc      1288
Ala Ala Tyr Glu Asp Ile Val Thr Arg Ser Arg Lys Gly Lys Leu Thr
            270                 275                 280
atg gat gac tac cag ggc gtt acc gtt tcc ttg acc aac cca ggt ggc      1336
Met Asp Asp Tyr Gln Gly Val Thr Val Ser Leu Thr Asn Pro Gly Gly
        285                 290                 295
atc ggt acc cgc cac tct gtc cca cgt ctg acc aag ggc cag ggc acc      1384
Ile Gly Thr Arg His Ser Val Pro Arg Leu Thr Lys Gly Gln Gly Thr
    300                 305                 310
atc atc ggt gtc ggt tcc atg gat tac cca gca gag ttc cag ggc gct      1432
Ile Ile Gly Val Gly Ser Met Asp Tyr Pro Ala Glu Phe Gln Gly Ala
315                 320                 325                 330
tcc gaa gac cgc ctt gca gag ctc ggc gtt gga aag ctt gtc acc atc      1480
Ser Glu Asp Arg Leu Ala Glu Leu Gly Val Gly Lys Leu Val Thr Ile
                335                 340                 345
acc tcc acc tac gat cac cgc gtg atc cag ggt gct gtg tcc ggt gaa      1528
Thr Ser Thr Tyr Asp His Arg Val Ile Gln Gly Ala Val Ser Gly Glu
            350                 355                 360
ttc ctg cgt acc atg tct cgc ctg ctc acc gat gat tcc ttc tgg gat      1576
Phe Leu Arg Thr Met Ser Arg Leu Leu Thr Asp Asp Ser Phe Trp Asp
        365                 370                 375
gag atc ttc gac gca atg aac gtt cct tac acc cca atg cgt tgg gca      1624
Glu Ile Phe Asp Ala Met Asn Val Pro Tyr Thr Pro Met Arg Trp Ala
    380                 385                 390
cag gac gtt cca aac acc ggt gtt gat aag aac acc cgc gtc atg cag      1672
Gln Asp Val Pro Asn Thr Gly Val Asp Lys Asn Thr Arg Val Met Gln
395                 400                 405                 410
ctc att gag gca tac cgc tcc cgt gga cac ctc atc gct gac acc aac      1720
Leu Ile Glu Ala Tyr Arg Ser Arg Gly His Leu Ile Ala Asp Thr Asn
                415                 420                 425
cca ctt tca tgg gtt cag cct ggc atg cca gtt cca gac cac cgc gac      1768
Pro Leu Ser Trp Val Gln Pro Gly Met Pro Val Pro Asp His Arg Asp
            430                 435                 440
ctc gac atc gag acc cac agc ctg acc atc tgg gat ctg gac cgt acc      1816
Leu Asp Ile Glu Thr His Ser Leu Thr Ile Trp Asp Leu Asp Arg Thr
        445                 450                 455
ttc agc gtc ggt ggc ttc ggc ggc aag gag acc atg acc ctg cgc gag      1864
Phe Ser Val Gly Gly Phe Gly Gly Lys Glu Thr Met Thr Leu Arg Glu
    460                 465                 470
gta ctg tcc cgc ctg cgc gct gcc tac acc ttg aag gtc ggc tcc gaa      1912
Val Leu Ser Arg Leu Arg Ala Ala Tyr Thr Leu Lys Val Gly Ser Glu
475                 480                 485                 490
tac acc cac atc ctg gac cgc gac gag cgc acc tgg ctg cag gac cgc      1960

Tyr Thr His Ile Leu Asp Arg Asp Glu Arg Thr Trp Leu Gln Asp Arg
                495                 500                 505
ctc gaa gcc gga atg cca aag cca acc cag gca gag cag aag tac atc      2008
Leu Glu Ala Gly Met Pro Lys Pro Thr Gln Ala Glu Gln Lys Tyr Ile
            510                 515                 520
ctg cag aag ctg aac gcc gca gag gct ttc gag aac ttc ctg cag acc      2056
Leu Gln Lys Leu Asn Ala Ala Glu Ala Phe Glu Asn Phe Leu Gln Thr
        525                 530                 535
aag tac gtc ggc cag aag cgc ttc tcc ctc gaa ggt gca gaa gct ctc      2104
Lys Tyr Val Gly Gln Lys Arg Phe Ser Leu Glu Gly Ala Glu Ala Leu
    540                 545                 550
atc cca ctg atg gac tcc gcc atc gac acc gcc gca ggc cag ggc ctc      2152
Ile Pro Leu Met Asp Ser Ala Ile Asp Thr Ala Ala Gly Gln Gly Leu
555                 560                 565                 570
gac gaa gtt gtc atc ggt atg cca cac cgt ggt cgc ctc aac gtg ctg      2200
Asp Glu Val Val Ile Gly Met Pro His Arg Gly Arg Leu Asn Val Leu
                575                 580                 585
ttc aac atc gtg ggc aag cca ctg gca tcc atc ttc aac gag ttt gaa      2248
Phe Asn Ile Val Gly Lys Pro Leu Ala Ser Ile Phe Asn Glu Phe Glu
            590                 595                 600
ggc caa atg gag cag ggc cag atc ggt ggc tcc ggt gac gtg aag tac      2296
Gly Gln Met Glu Gln Gly Gln Ile Gly Gly Ser Gly Asp Val Lys Tyr
        605                 610                 615
cac ctc ggt tcc gaa ggc cag cac ctg cag atg ttc ggc gac ggc gag      2344
His Leu Gly Ser Glu Gly Gln His Leu Gln Met Phe Gly Asp Gly Glu
    620                 625                 630
atc aag gtc tcc ctg act gct aac ccg tcc cac ctg gaa gct gtt aac      2392
Ile Lys Val Ser Leu Thr Ala Asn Pro Ser His Leu Glu Ala Val Asn
635                 640                 645                 650
cca gtg atg gaa ggt atc gtc cgc gca aag cag gac tac ctg gac aag      2440
Pro Val Met Glu Gly Ile Val Arg Ala Lys Gln Asp Tyr Leu Asp Lys
                655                 660                 665
ggc gta gac ggc aag act gtt gtg cca ctg ctg ctc cac ggt gac gct      2488
Gly Val Asp Gly Lys Thr Val Val Pro Leu Leu Leu His Gly Asp Ala
            670                 675                 680
gca ttc gca ggc ctg ggc atc gtg cca gaa acc atc aac ctg gct aag      2536
Ala Phe Ala Gly Leu Gly Ile Val Pro Glu Thr Ile Asn Leu Ala Lys
        685                 690                 695
ctg cgt ctc gac gtc gga ggc acc atc cac atc gtg gtg aac aac cag      2584
Leu Arg Leu Asp Val Gly Gly Thr Ile His Ile Val Val Asn Asn Gln
    700                 705                 710
atc ggc ttc acc acc acc cca gac tcc agc cgc tcc atg cac tac gca      2632
Ile Gly Phe Thr Thr Thr Pro Asp Ser Ser Arg Ser Met His Tyr Ala
715                 720                 725                 730
acc gac tac gcc aag gca ttc ggc tgc cca gtc ttc cac gtc aat ggt      2680
Thr Asp Tyr Ala Lys Ala Phe Gly Cys Pro Val Phe His Val Asn Gly
                735                 740                 745
gat gac cca gag gca gtt gtc tgg gtt ggc cag ctg gca acc gag tac      2728
Asp Asp Pro Glu Ala Val Val Trp Val Gly Gln Leu Ala Thr Glu Tyr
            750                 755                 760
cgt cgt cgc ttc ggc aag gac gtc ttc atc gac ctc gtt tgc tac cgc      2776
Arg Arg Arg Phe Gly Lys Asp Val Phe Ile Asp Leu Val Cys Tyr Arg
        765                 770                 775
ctc cgc ggc cac aac gaa gct gat gat cct tcc atg acc cag cca aag      2824
Leu Arg Gly His Asn Glu Ala Asp Asp Pro Ser Met Thr Gln Pro Lys
    780                 785                 790
atg tat gag ctc atc acc ggc cgc gag acc gtt cgt gct cag tac acc      2872
Met Tyr Glu Leu Ile Thr Gly Arg Glu Thr Val Arg Ala Gln Tyr Thr
795                 800                 805                 810
gaa gac ctg ctc gga cgt gga gac ctc tcc aac gaa gat gca gaa gca      2920
Glu Asp Leu Leu Gly Arg Gly Asp Leu Ser Asn Glu Asp Ala Glu Ala
                815                 820                 825
gtc gtc cgc gac ttc cac gac cag atg gaa tct gtg ttc aac gaa gtc      2968

Val Val Arg Asp Phe His Asp Gln Met Glu Ser Val Phe Asn Glu Val
        830                 835                 840
aag gaa ggc ggc aag aag cag gct gag gca cag acc ggc atc acc ggc     3016
Lys Glu Gly Gly Lys Lys Gln Ala Glu Ala Gln Thr Gly Ile Thr Gly
    845                 850                 855
tcc cag aag ctt cca cac ggc ctt gag acc aac atc tcc cgt gaa gag     3064
Ser Gln Lys Leu Pro His Gly Leu Glu Thr Asn Ile Ser Arg Glu Glu
    860                 865                 870
ctc ctg gaa ctg gga cag gct ttc gcc aac acc cca gaa ggc ttc aac     3112
Leu Leu Glu Leu Gly Gln Ala Phe Ala Asn Thr Pro Glu Gly Phe Asn
875                 880                 885                 890
tac cac cca cgt gtg gct cca gtt gct aag aag cgc gtc tcc tct gtc     3160
Tyr His Pro Arg Val Ala Pro Val Ala Lys Lys Arg Val Ser Ser Val
                895                 900                 905
acc gaa ggt ggc atc gac tgg gca tgg ggc gag ctc ctc gcc ttc ggt     3208
Thr Glu Gly Gly Ile Asp Trp Ala Trp Gly Glu Leu Leu Ala Phe Gly
            910                 915                 920
tcc ctg gct aac tcc ggc cgc ttg gtt cgc ctt gca ggt gaa gat tcc     3256
Ser Leu Ala Asn Ser Gly Arg Leu Val Arg Leu Ala Gly Glu Asp Ser
        925                 930                 935
cgc cgc ggt acc ttc acc cag cgc cac gca gtt gcc atc gac cca gcg     3304
Arg Arg Gly Thr Phe Thr Gln Arg His Ala Val Ala Ile Asp Pro Ala
    940                 945                 950
acc gct gaa gag ttc aac cca ctc cac gag ctt gca cag tcc aag ggc     3352
Thr Ala Glu Glu Phe Asn Pro Leu His Glu Leu Ala Gln Ser Lys Gly
955                 960                 965                 970
aac aac ggt aag ttc ctg gtc tac aac tcc gca ctg acc gag tac gca     3400
Asn Asn Gly Lys Phe Leu Val Tyr Asn Ser Ala Leu Thr Glu Tyr Ala
                975                 980                 985
ggc atg ggc ttc gag tac ggc tac tcc gta gga aac gaa gac  tcc gtc     3448
Gly Met Gly Phe Glu Tyr Gly Tyr Ser Val Gly Asn Glu Asp  Ser Val
            990                 995                 1000
gtt gca tgg  gaa gca cag ttc ggc  gac ttc gcc aac ggc  gct cag     3493
Val Ala Trp  Glu Ala Gln Phe Gly  Asp Phe Ala Asn Gly  Ala Gln
        1005                 1010                 1015
acc atc atc  gat gag tac gtc tcc  tca ggc gaa gct aag  tgg ggc     3538
Thr Ile Ile  Asp Glu Tyr Val Ser  Ser Gly Glu Ala Lys  Trp Gly
        1020                 1025                 1030
cag acc tcc  aag ctg atc ctt ctg  ctg cct cac ggc tac  gaa ggc     3583
Gln Thr Ser  Lys Leu Ile Leu Leu  Leu Pro His Gly Tyr  Glu Gly
        1035                 1040                 1045
cag ggc cca  gac cac tct tcc gca  cgt atc gag cgc ttc  ctg cag     3628
Gln Gly Pro  Asp His Ser Ser Ala  Arg Ile Glu Arg Phe  Leu Gln
        1050                 1055                 1060
ctg tgc gct  gag ggt tcc atg act  gtt gct cag cca tcc  acc cca     3673
Leu Cys Ala  Glu Gly Ser Met Thr  Val Ala Gln Pro Ser  Thr Pro
        1065                 1070                 1075
gca aac cac  ttc cac ctg ctg cgt  cgt cac gct ctg tcc  gac ctg     3718
Ala Asn His  Phe His Leu Leu Arg  Arg His Ala Leu Ser  Asp Leu
        1080                 1085                 1090
aag cgt cca  ctg gtt atc ttc acc  ccg aag tcc atg ctg  cgt aac     3763
Lys Arg Pro  Leu Val Ile Phe Thr  Pro Lys Ser Met Leu  Arg Asn
        1095                 1100                 1105
aag gct gct  gcc tcc gca cca gaa  gac ttc act gag gtc  acc aag     3808
Lys Ala Ala  Ala Ser Ala Pro Glu  Asp Phe Thr Glu Val  Thr Lys
        1110                 1115                 1120
ttc caa tcc  gtg atc gac gat cca  aac gtt gca gat gca  gcc aag     3853
Phe Gln Ser  Val Ile Asp Asp Pro  Asn Val Ala Asp Ala  Ala Lys
        1125                 1130                 1135
gtg aag aag  gtc atg ctg gtc tcc  ggc aag ctg tac tac  gaa ttg     3898
Val Lys Lys  Val Met Leu Val Ser  Gly Lys Leu Tyr Tyr  Glu Leu
        1140                 1145                 1150
gca aag cgc  aag gag aag gac gga  cgc gac gac atc gcg  atc gtt     3943

Ala Lys Arg Lys Glu Lys Asp Gly Arg Asp Asp Ile Ala Ile Val
        1155                1160                1165

cgt atc gaa atg ctc cac cca att ccg ttc aac cgc atc tcc gag      3988
Arg Ile Glu Met Leu His Pro Ile Pro Phe Asn Arg Ile Ser Glu
        1170                1175                1180

gct ctt gcc ggc tac cct aac gct gag gaa gtc ctc ttc gtt cag      4033
Ala Leu Ala Gly Tyr Pro Asn Ala Glu Glu Val Leu Phe Val Gln
        1185                1190                1195

gat gag cca gca aac cag ggc cca tgg ccg ttc tac cag gag cac      4078
Asp Glu Pro Ala Asn Gln Gly Pro Trp Pro Phe Tyr Gln Glu His
        1200                1205                1210

ctc cca gag ctg atc ccg aac atg cca aag atg cgc cgc gtt tcc      4123
Leu Pro Glu Leu Ile Pro Asn Met Pro Lys Met Arg Arg Val Ser
        1215                1220                1225

cgc cgc gct cag tcc tcc acc gca act ggt gtt gct aag gtg cac      4168
Arg Arg Ala Gln Ser Ser Thr Ala Thr Gly Val Ala Lys Val His
        1230                1235                1240

cag ctg gag gag aag cag ctt atc gac gag gct ttc gag gct          4210
Gln Leu Glu Glu Lys Gln Leu Ile Asp Glu Ala Phe Glu Ala
        1245                1250                1255

taagtcttta tagtcctgca ctagcctaga gggccttatg cagtgtgaat cacacagcat      4270
aaggcccttt ttgctgccgt ggttgcctaa ggtggaaggc atgaaacgaa tctgtgcggt      4330
cacgatctct tcagtacttt tgctaagtgg ctgctcctcc acttccacca cgcagctcga      4390
g                                                                      4391

<210>  48
<211>  1256
<212>  PRT
<213>  Corynebacterium glutamicum

<400>  48
Met Leu Gln Leu Gly Leu Arg His Asn Gln Pro Thr Thr Asn Val Thr
1               5                   10                  15

Val Asp Lys Ile Lys Leu Asn Lys Pro Ser Arg Ser Lys Glu Lys Arg
            20                  25                  30

Arg Val Pro Ala Val Ser Ser Ala Ser Thr Phe Gly Gln Asn Ala Trp
        35                  40                  45

Leu Val Asp Glu Met Phe Gln Gln Phe Gln Lys Asp Pro Lys Ser Val
    50                  55                  60

Asp Lys Glu Trp Arg Glu Leu Phe Glu Ala Gln Gly Gly Pro Asn Ala
65                  70                  75                  80

Thr Pro Ala Thr Thr Glu Ala Gln Pro Ser Ala Pro Lys Glu Ser Ala
                85                  90                  95

Lys Pro Ala Pro Lys Ala Ala Pro Ala Ala Lys Ala Ala Pro Arg Val
            100                 105                 110

Glu Thr Lys Pro Ala Ala Lys Thr Ala Pro Lys Ala Lys Glu Ser Ser
        115                 120                 125

Val Pro Gln Gln Pro Lys Leu Pro Glu Pro Gly Gln Thr Pro Ile Arg
    130                 135                 140

Gly Ile Phe Lys Ser Ile Ala Lys Asn Met Asp Ile Ser Leu Glu Ile
145                 150                 155                 160

Pro Thr Ala Thr Ser Val Arg Asp Met Pro Ala Arg Leu Met Phe Glu
                165                 170                 175

Asn Arg Ala Met Val Asn Asp Gln Leu Lys Arg Thr Arg Gly Gly Lys
            180                 185                 190

Ile Ser Phe Thr His Ile Ile Gly Tyr Ala Met Val Lys Ala Val Met
        195                 200                 205

Ala His Pro Asp Met Asn Asn Ser Tyr Asp Val Ile Asp Gly Lys Pro
    210                 215                 220

Thr Leu Ile Val Pro Glu His Ile Asn Leu Gly Leu Ala Ile Asp Leu
225                 230                 235                 240

Pro Gln Lys Asp Gly Ser Arg Ala Leu Val Val Ala Ala Ile Lys Glu

                    245                     250                     255
Thr Glu Lys Met Asn Phe Ser Glu Phe Leu Ala Ala Tyr Glu Asp Ile
            260                     265                     270
Val Thr Arg Ser Arg Lys Gly Lys Leu Thr Met Asp Asp Tyr Gln Gly
        275                     280                     285
Val Thr Val Ser Leu Thr Asn Pro Gly Gly Ile Gly Thr Arg His Ser
    290                     295                     300
Val Pro Arg Leu Thr Lys Gly Gln Gly Thr Ile Ile Gly Val Gly Ser
305                     310                     315                     320
Met Asp Tyr Pro Ala Glu Phe Gln Gly Ala Ser Glu Asp Arg Leu Ala
                    325                     330                     335
Glu Leu Gly Val Gly Lys Leu Val Thr Ile Thr Ser Thr Tyr Asp His
            340                     345                     350
Arg Val Ile Gln Gly Ala Val Ser Gly Glu Phe Leu Arg Thr Met Ser
        355                     360                     365
Arg Leu Leu Thr Asp Asp Ser Phe Trp Asp Glu Ile Phe Asp Ala Met
    370                     375                     380
Asn Val Pro Tyr Thr Pro Met Arg Trp Ala Gln Asp Val Pro Asn Thr
385                     390                     395                     400
Gly Val Asp Lys Asn Thr Arg Val Met Gln Leu Ile Glu Ala Tyr Arg
                    405                     410                     415
Ser Arg Gly His Leu Ile Ala Asp Thr Asn Pro Leu Ser Trp Val Gln
            420                     425                     430
Pro Gly Met Pro Val Pro Asp His Arg Asp Leu Asp Ile Glu Thr His
        435                     440                     445
Ser Leu Thr Ile Trp Asp Leu Asp Arg Thr Phe Ser Val Gly Gly Phe
    450                     455                     460
Gly Gly Lys Glu Thr Met Thr Leu Arg Glu Val Leu Ser Arg Leu Arg
465                     470                     475                     480
Ala Ala Tyr Thr Leu Lys Val Gly Ser Glu Tyr Thr His Ile Leu Asp
                    485                     490                     495
Arg Asp Glu Arg Thr Trp Leu Gln Asp Arg Leu Glu Ala Gly Met Pro
            500                     505                     510
Lys Pro Thr Gln Ala Glu Gln Lys Tyr Ile Leu Gln Lys Leu Asn Ala
        515                     520                     525
Ala Glu Ala Phe Glu Asn Phe Leu Gln Thr Lys Tyr Val Gly Gln Lys
    530                     535                     540
Arg Phe Ser Leu Glu Gly Ala Glu Ala Leu Ile Pro Leu Met Asp Ser
545                     550                     555                     560
Ala Ile Asp Thr Ala Ala Gly Gln Gly Leu Asp Glu Val Val Ile Gly
                    565                     570                     575
Met Pro His Arg Gly Arg Leu Asn Val Leu Phe Asn Ile Val Gly Lys
            580                     585                     590
Pro Leu Ala Ser Ile Phe Asn Glu Phe Glu Gly Gln Met Glu Gln Gly
        595                     600                     605
Gln Ile Gly Gly Ser Gly Asp Val Lys Tyr His Leu Gly Ser Glu Gly
    610                     615                     620
Gln His Leu Gln Met Phe Gly Asp Gly Glu Ile Lys Val Ser Leu Thr
625                     630                     635                     640
Ala Asn Pro Ser His Leu Glu Ala Val Asn Pro Val Met Glu Gly Ile
                    645                     650                     655
Val Arg Ala Lys Gln Asp Tyr Leu Asp Lys Gly Val Asp Gly Lys Thr
            660                     665                     670
Val Val Pro Leu Leu Leu His Gly Asp Ala Ala Phe Ala Gly Leu Gly
        675                     680                     685
Ile Val Pro Glu Thr Ile Asn Leu Ala Lys Leu Arg Leu Asp Val Gly
    690                     695                     700
Gly Thr Ile His Ile Val Val Asn Asn Gln Ile Gly Phe Thr Thr Thr
705                     710                     715                     720
Pro Asp Ser Ser Arg Ser Met His Tyr Ala Thr Asp Tyr Ala Lys Ala
                    725                     730                     735
Phe Gly Cys Pro Val Phe His Val Asn Gly Asp Asp Pro Glu Ala Val
            740                     745                     750

Val Trp Val Gly Gln Leu Ala Thr Glu Tyr Arg Arg Arg Phe Gly Lys
        755                 760                 765
Asp Val Phe Ile Asp Leu Val Cys Tyr Arg Leu Arg Gly His Asn Glu
    770                 775                 780
Ala Asp Asp Pro Ser Met Thr Gln Pro Lys Met Tyr Glu Leu Ile Thr
785                 790                 795                 800
Gly Arg Glu Thr Val Arg Ala Gln Tyr Thr Glu Asp Leu Leu Gly Arg
                805                 810                 815
Gly Asp Leu Ser Asn Glu Asp Ala Glu Ala Val Val Arg Asp Phe His
            820                 825                 830
Asp Gln Met Glu Ser Val Phe Asn Glu Val Lys Glu Gly Gly Lys Lys
        835                 840                 845
Gln Ala Glu Ala Gln Thr Gly Ile Thr Gly Ser Gln Lys Leu Pro His
    850                 855                 860
Gly Leu Glu Thr Asn Ile Ser Arg Glu Glu Leu Leu Glu Leu Gly Gln
865                 870                 875                 880
Ala Phe Ala Asn Thr Pro Glu Gly Phe Asn Tyr His Pro Arg Val Ala
                885                 890                 895
Pro Val Ala Lys Lys Arg Val Ser Ser Val Thr Glu Gly Gly Ile Asp
            900                 905                 910
Trp Ala Trp Gly Glu Leu Leu Ala Phe Gly Ser Leu Ala Asn Ser Gly
        915                 920                 925
Arg Leu Val Arg Leu Ala Gly Glu Asp Ser Arg Arg Gly Thr Phe Thr
    930                 935                 940
Gln Arg His Ala Val Ala Ile Asp Pro Ala Thr Ala Glu Glu Phe Asn
945                 950                 955                 960
Pro Leu His Glu Leu Ala Gln Ser Lys Gly Asn Asn Gly Lys Phe Leu
                965                 970                 975
Val Tyr Asn Ser Ala Leu Thr Glu Tyr Ala Gly Met Gly Phe Glu Tyr
            980                 985                 990
Gly Tyr Ser Val Gly Asn Glu Asp  Ser Val Val Ala Trp  Glu Ala Gln
        995                 1000                1005
Phe Gly  Asp Phe Ala Asn Gly  Ala Gln Thr Ile Ile  Asp Glu Tyr
    1010                1015                1020
Val Ser  Ser Gly Glu Ala Lys  Trp Gly Gln Thr Ser  Lys Leu Ile
    1025                1030                1035
Leu Leu  Leu Pro His Gly Tyr  Glu Gly Gln Gly Pro  Asp His Ser
    1040                1045                1050
Ser Ala  Arg Ile Glu Arg Phe  Leu Gln Leu Cys Ala  Glu Gly Ser
    1055                1060                1065
Met Thr  Val Ala Gln Pro Ser  Thr Pro Ala Asn His  Phe His Leu
    1070                1075                1080
Leu Arg  Arg His Ala Leu Ser  Asp Leu Lys Arg Pro  Leu Val Ile
    1085                1090                1095
Phe Thr  Pro Lys Ser Met Leu  Arg Asn Lys Ala Ala  Ala Ser Ala
    1100                1105                1110
Pro Glu  Asp Phe Thr Glu Val  Thr Lys Phe Gln Ser  Val Ile Asp
    1115                1120                1125
Asp Pro  Asn Val Ala Asp Ala  Ala Lys Val Lys Lys  Val Met Leu
    1130                1135                1140
Val Ser  Gly Lys Leu Tyr Tyr  Glu Leu Ala Lys Arg  Lys Glu Lys

    1145                1150                1155
Asp Gly  Arg Asp Asp Ile Ala  Ile Val Arg Ile Glu  Met Leu His
    1160                1165                1170
Pro Ile  Pro Phe Asn Arg Ile  Ser Glu Ala Leu Ala  Gly Tyr Pro
    1175                1180                1185
Asn Ala  Glu Glu Val Leu Phe  Val Gln Asp Glu Pro  Ala Asn Gln
    1190                1195                1200
Gly Pro  Trp Pro Phe Tyr Gln  Glu His Leu Pro Glu  Leu Ile Pro
    1205                1210                1215
Asn Met  Pro Lys Met Arg Arg  Val Ser Arg Arg Ala  Gln Ser Ser
    1220                1225                1230

Thr Ala Thr Gly Val Ala Lys Val His Gln Leu Glu Glu Lys Gln
    1235                1240                1245
Leu Ile Asp Glu Ala Phe Glu Ala
    1250                1255

<210> 49
<211> 4391
<212> DNA
<213> Corynebacterium glutamicum

<220>
<221> CDS
<222> (443)..(4210)
<223> sucA805

<400> 49
gtcgacaagc aaaatcgaag cggcagcacg ccgcgtcgga gccttaaacg ccatcgccgc 60
catccctgat ggtttcaatc atcaagtcgg tgaacgcggg cgcaacctgt catccggaca 120
gcgccaactg atcgcgctgg cgcgcgccga actcatcgag ccttccatca tgcttctcga 180
cgaagccacc tccaccctcg accccgccac cgaagccgtt atcctcaacg cctccgatcg 240
agtcactaag ggacgcacca gcatcatcgt cgcgcaccgc ttggcaaccg ctaaaagggc 300
cgaccgtatt cttgttgttg aacaaggacg tatcattgag gacggatctc acgacgcgtt 360
gttgtctgct aacggcacct acgcccgcat gtggcattta atggcctgac acgttatttt 420
taggagaact gtcaacaaat ta atg cta caa ctg ggg ctt agg cat aat cag 472
Met Leu Gln Leu Gly Leu Arg His Asn Gln
1               5                   10
cca acg acc aac gtt aca gtg gat aaa ata aag ctc aat aaa ccc tca 520
Pro Thr Thr Asn Val Thr Val Asp Lys Ile Lys Leu Asn Lys Pro Ser
                15                  20                  25
aga agc aag gaa aag agg cga gta cct gcc gtg agc agc gct agt act 568
Arg Ser Lys Glu Lys Arg Arg Val Pro Ala Val Ser Ser Ala Ser Thr
            30                  35                  40
ttc ggc cag aat gcg tgg ctg gta gac gag atg ttc cag cag ttc cag 616
Phe Gly Gln Asn Ala Trp Leu Val Asp Glu Met Phe Gln Gln Phe Gln
        45                  50                  55
aag gac ccc aag tcc gtg gac aag gaa tgg aga gaa ctc ttt gag gcg 664
Lys Asp Pro Lys Ser Val Asp Lys Glu Trp Arg Glu Leu Phe Glu Ala
    60                  65                  70
cag ggg gga cca aat gct acc ccc gct aca aca gaa gca cag cct tca 712
Gln Gly Gly Pro Asn Ala Thr Pro Ala Thr Thr Glu Ala Gln Pro Ser
75                  80                  85                  90
gcg ccc aag gag tct gcg aaa cca gca cca aag gct gcc cct gca gcc 760
Ala Pro Lys Glu Ser Ala Lys Pro Ala Pro Lys Ala Ala Pro Ala Ala
                95                  100                 105
aag gca gca ccg cgc gta gaa acc aag ccg gcc gcc aag acc gcc cct 808
Lys Ala Ala Pro Arg Val Glu Thr Lys Pro Ala Ala Lys Thr Ala Pro
            110                 115                 120
aag gcc aag gag tcc tca gtg cca cag caa cct aag ctt ccg gag cca 856
Lys Ala Lys Glu Ser Ser Val Pro Gln Gln Pro Lys Leu Pro Glu Pro
        125                 130                 135
gga caa acc cca atc agg ggt att ttc aag tcc atc gcg aag aac atg 904
Gly Gln Thr Pro Ile Arg Gly Ile Phe Lys Ser Ile Ala Lys Asn Met
    140                 145                 150
gat atc tcc ctg gaa atc cca acc gca acc tcg gtt cgc gat atg cca 952
Asp Ile Ser Leu Glu Ile Pro Thr Ala Thr Ser Val Arg Asp Met Pro
155                 160                 165                 170
gct cgc ctc atg ttc gaa aac cgc gcg atg gtc aac gat cag ctc aag 1000
Ala Arg Leu Met Phe Glu Asn Arg Ala Met Val Asn Asp Gln Leu Lys
                175                 180                 185
cgc acc cgc ggt ggc aag atc tcc ttc acc cac atc att ggc tac gcc 1048
Arg Thr Arg Gly Gly Lys Ile Ser Phe Thr His Ile Ile Gly Tyr Ala
            190                 195                 200

atg gtg aag gca gtc atg gct cac ccg gac atg aac aac tcc tac gac      1096
Met Val Lys Ala Val Met Ala His Pro Asp Met Asn Asn Ser Tyr Asp
        205                 210                 215
gtc atc gac ggc aag cca acc ctg atc gtg cct gag cac atc aac ctg      1144
Val Ile Asp Gly Lys Pro Thr Leu Ile Val Pro Glu His Ile Asn Leu
    220                 225                 230
ggc ctt gcc atc gac ctt cct cag aag gac ggc tcc cgc gca ctt gtc      1192
Gly Leu Ala Ile Asp Leu Pro Gln Lys Asp Gly Ser Arg Ala Leu Val
235                 240                 245                 250
gta gca gcc atc aag gaa acc gag aag atg aac ttc tcc gag ttc ctc      1240
Val Ala Ala Ile Lys Glu Thr Glu Lys Met Asn Phe Ser Glu Phe Leu
                255                 260                 265
gca gca tac gaa gac atc gtg aca cgc tcc cgc aag ggc aag ctc acc      1288
Ala Ala Tyr Glu Asp Ile Val Thr Arg Ser Arg Lys Gly Lys Leu Thr
            270                 275                 280
atg gat gac tac cag ggc gtt acc gtt tcc ttg acc aac cca ggt ggc      1336
Met Asp Asp Tyr Gln Gly Val Thr Val Ser Leu Thr Asn Pro Gly Gly
        285                 290                 295
atc ggt acc cgc cac tct gtc cca cgt ctg acc aag ggc cag ggc acc      1384
Ile Gly Thr Arg His Ser Val Pro Arg Leu Thr Lys Gly Gln Gly Thr
    300                 305                 310
atc atc ggt gtc ggt tcc atg gat tac cca gca gag ttc cag ggc gct      1432
Ile Ile Gly Val Gly Ser Met Asp Tyr Pro Ala Glu Phe Gln Gly Ala
315                 320                 325                 330
tcc gaa gac cgc ctt gca gag ctc ggc gtt gga aag ctt gtc acc atc      1480
Ser Glu Asp Arg Leu Ala Glu Leu Gly Val Gly Lys Leu Val Thr Ile
                335                 340                 345
acc tcc acc tac gat cac cgc gtg atc cag ggt gct gtg tcc ggt gaa      1528
Thr Ser Thr Tyr Asp His Arg Val Ile Gln Gly Ala Val Ser Gly Glu
            350                 355                 360
ttc ctg cgt acc atg tct cgc ctg ctc acc gat gat tcc ttc tgg gat      1576
Phe Leu Arg Thr Met Ser Arg Leu Leu Thr Asp Asp Ser Phe Trp Asp
        365                 370                 375
gag atc ttc gac gca atg aac gtt cct tac acc cca atg cgt tgg gca      1624
Glu Ile Phe Asp Ala Met Asn Val Pro Tyr Thr Pro Met Arg Trp Ala
    380                 385                 390
cag gac gtt cca aac acc ggt gtt gat aag aac acc cgc gtc atg cag      1672
Gln Asp Val Pro Asn Thr Gly Val Asp Lys Asn Thr Arg Val Met Gln
395                 400                 405                 410
ctc att gag gca tac cgc tcc cgt gga cac ctc atc gct gac acc aac      1720
Leu Ile Glu Ala Tyr Arg Ser Arg Gly His Leu Ile Ala Asp Thr Asn
                415                 420                 425
cca ctt tca tgg gtt cag cct ggc atg cca gtt cca gac cac cgc gac      1768
Pro Leu Ser Trp Val Gln Pro Gly Met Pro Val Pro Asp His Arg Asp
            430                 435                 440
ctc gac atc gag acc cac agc ctg acc atc tgg gat ctg gac cgt acc      1816
Leu Asp Ile Glu Thr His Ser Leu Thr Ile Trp Asp Leu Asp Arg Thr
        445                 450                 455
ttc agc gtc ggt ggc ttc ggc ggc aag gag acc atg acc ctg cgc gag      1864
Phe Ser Val Gly Gly Phe Gly Gly Lys Glu Thr Met Thr Leu Arg Glu
    460                 465                 470
gta ctg tcc cgc ctg cgc gct gcc tac acc ttg aag gtc ggc tcc gaa      1912
Val Leu Ser Arg Leu Arg Ala Ala Tyr Thr Leu Lys Val Gly Ser Glu
475                 480                 485                 490
tac acc cac atc ctg gac cgc gac gag cgc acc tgg ctg cag gac cgc      1960
Tyr Thr His Ile Leu Asp Arg Asp Glu Arg Thr Trp Leu Gln Asp Arg
                495                 500                 505
ctc gaa gcc gga atg cca aag cca acc cag gca gag cag aag tac atc      2008
Leu Glu Ala Gly Met Pro Lys Pro Thr Gln Ala Glu Gln Lys Tyr Ile
            510                 515                 520
ctg cag aag ctg aac gcc gca gag gct ttc gag aac ttc ctg cag acc      2056
Leu Gln Lys Leu Asn Ala Ala Glu Ala Phe Glu Asn Phe Leu Gln Thr
        525                 530                 535

aag tac gtc ggc cag aag cgc ttc tcc ctc gaa ggt gca gaa gct ctc 2104
Lys Tyr Val Gly Gln Lys Arg Phe Ser Leu Glu Gly Ala Glu Ala Leu
    540                 545                 550
atc cca ctg atg gac tcc gcc atc gac acc gcc gca ggc cag ggc ctc 2152
Ile Pro Leu Met Asp Ser Ala Ile Asp Thr Ala Ala Gly Gln Gly Leu
555                 560                 565                 570
gac gaa gtt gtc atc ggt atg cca cac cgt ggt cgc ctc aac gtg ctg 2200
Asp Glu Val Val Ile Gly Met Pro His Arg Gly Arg Leu Asn Val Leu
                575                 580                 585
ttc aac atc gtg ggc aag cca ctg gca tcc atc ttc aac gag ttt gaa 2248
Phe Asn Ile Val Gly Lys Pro Leu Ala Ser Ile Phe Asn Glu Phe Glu
            590                 595                 600
ggc caa atg gag cag ggc cag atc ggt ggc tcc ggt gac gtg aag tac 2296
Gly Gln Met Glu Gln Gly Gln Ile Gly Gly Ser Gly Asp Val Lys Tyr
        605                 610                 615
cac ctc ggt tcc gaa ggc cag cac ctg cag atg ttc ggc gac ggc gag 2344
His Leu Gly Ser Glu Gly Gln His Leu Gln Met Phe Gly Asp Gly Glu
    620                 625                 630
atc aag gtc tcc ctg act gct aac ccg tcc cac ctg gaa gct gtt aac 2392
Ile Lys Val Ser Leu Thr Ala Asn Pro Ser His Leu Glu Ala Val Asn
635                 640                 645                 650
cca gtg atg gaa ggt atc gtc cgc gca aag cag gac tac ctg gac aag 2440
Pro Val Met Glu Gly Ile Val Arg Ala Lys Gln Asp Tyr Leu Asp Lys
                655                 660                 665
ggc gta gac ggc aag act gtt gtg cca ctg ctg ctc cac ggt gac gct 2488
Gly Val Asp Gly Lys Thr Val Val Pro Leu Leu Leu His Gly Asp Ala
            670                 675                 680
gca ttc gca ggc ctg ggc atc gtg cca gaa acc atc aac ctg gct aaa 2536
Ala Phe Ala Gly Leu Gly Ile Val Pro Glu Thr Ile Asn Leu Ala Lys
        685                 690                 695
agc tgc gtc gac gtc gga ggc acc atc cac atc gtg gtg aac aac cag 2584
Ser Cys Val Asp Val Gly Gly Thr Ile His Ile Val Val Asn Asn Gln
    700                 705                 710
atc ggc ttc acc acc acc cca gac tcc agc cgc tcc atg cac tac gca 2632
Ile Gly Phe Thr Thr Thr Pro Asp Ser Ser Arg Ser Met His Tyr Ala
715                 720                 725                 730
acc gac tac gcc aag gca ttc ggc tgc cca gtc ttc cac gtc aat ggt 2680
Thr Asp Tyr Ala Lys Ala Phe Gly Cys Pro Val Phe His Val Asn Gly
                735                 740                 745
gat gac cca gag gca gtt gtc tgg gtt ggc cag ctg gca acc gag tac 2728
Asp Asp Pro Glu Ala Val Val Trp Val Gly Gln Leu Ala Thr Glu Tyr
            750                 755                 760
cgt cgt cgc ttc ggc aag gac gtc ttc atc gac ctc gtt tgc tac cgc 2776
Arg Arg Arg Phe Gly Lys Asp Val Phe Ile Asp Leu Val Cys Tyr Arg
        765                 770                 775
ctc cgc ggc cac aac gaa gct gat gat cct tcc atg acc cag cca aag 2824
Leu Arg Gly His Asn Glu Ala Asp Asp Pro Ser Met Thr Gln Pro Lys
    780                 785                 790
atg tat gag ctc atc acc ggc cgc gag acc gtt cgt gct cag tac acc 2872
Met Tyr Glu Leu Ile Thr Gly Arg Glu Thr Val Arg Ala Gln Tyr Thr
795                 800                 805                 810
gaa gac ctg ctc gga cgt gga gac ctc tcc aac gaa gat gca gaa gca 2920
Glu Asp Leu Leu Gly Arg Gly Asp Leu Ser Asn Glu Asp Ala Glu Ala
                815                 820                 825
gtc gtc cgc gac ttc cac gac cag atg gaa tct gtg ttc aac gaa gtc 2968
Val Val Arg Asp Phe His Asp Gln Met Glu Ser Val Phe Asn Glu Val
            830                 835                 840
aag gaa ggc ggc aag aag cag gct gag gca cag acc ggc atc acc ggc 3016
Lys Glu Gly Gly Lys Lys Gln Ala Glu Ala Gln Thr Gly Ile Thr Gly
        845                 850                 855
tcc cag aag ctt cca cac ggc ctt gag acc aac atc tcc cgt gaa gag 3064
Ser Gln Lys Leu Pro His Gly Leu Glu Thr Asn Ile Ser Arg Glu Glu

860                 865                 870
ctc ctg gaa ctg gga cag gct ttc gcc aac acc cca gaa ggc ttc aac     3112
Leu Leu Glu Leu Gly Gln Ala Phe Ala Asn Thr Pro Glu Gly Phe Asn
875                 880                 885                 890
tac cac cca cgt gtg gct cca gtt gct aag aag cgc gtc tcc tct gtc     3160
Tyr His Pro Arg Val Ala Pro Val Ala Lys Lys Arg Val Ser Ser Val
                895                 900                 905
acc gaa ggt ggc atc gac tgg gca tgg ggc gag ctc ctc gcc ttc ggt     3208
Thr Glu Gly Gly Ile Asp Trp Ala Trp Gly Glu Leu Leu Ala Phe Gly
            910                 915                 920
tcc ctg gct aac tcc ggc cgc ttg gtt cgc ctt gca ggt gaa gat tcc     3256
Ser Leu Ala Asn Ser Gly Arg Leu Val Arg Leu Ala Gly Glu Asp Ser
        925                 930                 935
cgc cgc ggt acc ttc acc cag cgc cac gca gtt gcc atc gac cca gcg     3304
Arg Arg Gly Thr Phe Thr Gln Arg His Ala Val Ala Ile Asp Pro Ala
    940                 945                 950
acc gct gaa gag ttc aac cca ctc cac gag ctt gca cag tcc aag ggc     3352
Thr Ala Glu Glu Phe Asn Pro Leu His Glu Leu Ala Gln Ser Lys Gly
955                 960                 965                 970
aac aac ggt aag ttc ctg gtc tac aac tcc gca ctg acc gag tac gca     3400
Asn Asn Gly Lys Phe Leu Val Tyr Asn Ser Ala Leu Thr Glu Tyr Ala
                975                 980                 985
ggc atg ggc ttc gag tac ggc tac tcc gta gga aac gaa gac  tcc gtc     3448
Gly Met Gly Phe Glu Tyr Gly Tyr Ser Val Gly Asn Glu Asp  Ser Val
            990                 995                 1000
gtt gca tgg  gaa gca cag ttc ggc  gac ttc gcc aac ggc  gct cag     3493
Val Ala Trp  Glu Ala Gln Phe Gly  Asp Phe Ala Asn Gly  Ala Gln
        1005                1010                1015
acc atc atc  gat gag tac gtc tcc  tca ggc gaa gct aag  tgg ggc     3538
Thr Ile Ile  Asp Glu Tyr Val Ser  Ser Gly Glu Ala Lys  Trp Gly
        1020                1025                1030
cag acc tcc  aag ctg atc ctt ctg  ctg cct cac ggc tac  gaa ggc     3583
Gln Thr Ser  Lys Leu Ile Leu Leu  Leu Pro His Gly Tyr  Glu Gly
        1035                1040                1045
cag ggc cca  gac cac tct tcc gca  cgt atc gag cgc ttc  ctg cag     3628
Gln Gly Pro  Asp His Ser Ser Ala  Arg Ile Glu Arg Phe  Leu Gln
        1050                1055                1060
ctg tgc gct  gag ggt tcc atg act  gtt gct cag cca tcc  acc cca     3673
Leu Cys Ala  Glu Gly Ser Met Thr  Val Ala Gln Pro Ser  Thr Pro
        1065                1070                1075
gca aac cac  ttc cac ctg ctg cgt  cgt cac gct ctg tcc  gac ctg     3718
Ala Asn His  Phe His Leu Leu Arg  Arg His Ala Leu Ser  Asp Leu
        1080                1085                1090
aag cgt cca  ctg gtt atc ttc acc  ccg aag tcc atg ctg  cgt aac     3763
Lys Arg Pro  Leu Val Ile Phe Thr  Pro Lys Ser Met Leu  Arg Asn
        1095                1100                1105
aag gct gct  gcc tcc gca cca gaa  gac ttc act gag gtc  acc aag     3808
Lys Ala Ala  Ala Ser Ala Pro Glu  Asp Phe Thr Glu Val  Thr Lys
        1110                1115                1120
ttc caa tcc  gtg atc gac gat cca  aac gtt gca gat gca  gcc aag     3853
Phe Gln Ser  Val Ile Asp Asp Pro  Asn Val Ala Asp Ala  Ala Lys
        1125                1130                1135
gtg aag aag  gtc atg ctg gtc tcc  ggc aag ctg tac tac  gaa ttg     3898
Val Lys Lys  Val Met Leu Val Ser  Gly Lys Leu Tyr Tyr  Glu Leu
        1140                1145                1150
gca aag cgc  aag gag aag gac gga  cgc gac gac atc gcg  atc gtt     3943
Ala Lys Arg  Lys Glu Lys Asp Gly  Arg Asp Asp Ile Ala  Ile Val
        1155                1160                1165
cgt atc gaa  atg ctc cac cca att  ccg ttc aac cgc atc  tcc gag     3988
Arg Ile Glu  Met Leu His Pro Ile  Pro Phe Asn Arg Ile  Ser Glu
        1170                1175                1180
gct ctt gcc  ggc tac cct aac gct  gag gaa gtc ctc ttc  gtt cag     4033
Ala Leu Ala  Gly Tyr Pro Asn Ala  Glu Glu Val Leu Phe  Val Gln

        1185                1190                1195
gat gag cca  gca aac cag ggc cca  tgg ccg ttc tac cag  gag cac      4078
Asp Glu Pro  Ala Asn Gln Gly Pro  Trp Pro Phe Tyr Gln  Glu His
        1200                1205                1210
ctc cca gag  ctg atc ccg aac atg  cca aag atg cgc cgc  gtt tcc      4123
Leu Pro Glu  Leu Ile Pro Asn Met  Pro Lys Met Arg Arg  Val Ser
        1215                1220                1225
cgc cgc gct  cag tcc tcc acc gca  act ggt gtt gct aag  gtg cac      4168
Arg Arg Ala  Gln Ser Ser Thr Ala  Thr Gly Val Ala Lys  Val His
        1230                1235                1240
cag ctg gag  gag aag cag ctt atc  gac gag gct ttc gag  gct          4210
Gln Leu Glu  Glu Lys Gln Leu Ile  Asp Glu Ala Phe Glu  Ala
        1245                1250                1255
taagtcttta tagtcctgca ctagcctaga gggccttatg cagtgtgaat cacacagcat   4270
aaggcccttt ttgctgccgt ggttgcctaa ggtggaaggc atgaaacgaa tctgtgcggt   4330
cacgatctct tcagtacttt tgctaagtgg ctgctcctcc acttccacca cgcagctcga   4390
g                                                                   4391


<210>  50
<211>  1256
<212>  PRT
<213>  Corynebacterium glutamicum

<400>  50
Met Leu Gln Leu Gly Leu Arg His Asn Gln Pro Thr Thr Asn Val Thr
1               5                   10                  15
Val Asp Lys Ile Lys Leu Asn Lys Pro Ser Arg Ser Lys Glu Lys Arg
            20                  25                  30
Arg Val Pro Ala Val Ser Ser Ala Ser Thr Phe Gly Gln Asn Ala Trp
        35                  40                  45
Leu Val Asp Glu Met Phe Gln Gln Phe Gln Lys Asp Pro Lys Ser Val
    50                  55                  60
Asp Lys Glu Trp Arg Glu Leu Phe Glu Ala Gln Gly Gly Pro Asn Ala
65                  70                  75                  80
Thr Pro Ala Thr Thr Glu Ala Gln Pro Ser Ala Pro Lys Glu Ser Ala
                85                  90                  95
Lys Pro Ala Pro Lys Ala Ala Pro Ala Ala Lys Ala Ala Pro Arg Val
            100                 105                 110
Glu Thr Lys Pro Ala Ala Lys Thr Ala Pro Lys Ala Lys Glu Ser Ser
        115                 120                 125
Val Pro Gln Gln Pro Lys Leu Pro Glu Pro Gly Gln Thr Pro Ile Arg
    130                 135                 140
Gly Ile Phe Lys Ser Ile Ala Lys Asn Met Asp Ile Ser Leu Glu Ile
145                 150                 155                 160
Pro Thr Ala Thr Ser Val Arg Asp Met Pro Ala Arg Leu Met Phe Glu
                165                 170                 175
Asn Arg Ala Met Val Asn Asp Gln Leu Lys Arg Thr Arg Gly Gly Lys
            180                 185                 190
Ile Ser Phe Thr His Ile Ile Gly Tyr Ala Met Val Lys Ala Val Met
        195                 200                 205
Ala His Pro Asp Met Asn Asn Ser Tyr Asp Val Ile Asp Gly Lys Pro
    210                 215                 220
Thr Leu Ile Val Pro Glu His Ile Asn Leu Gly Leu Ala Ile Asp Leu
225                 230                 235                 240
Pro Gln Lys Asp Gly Ser Arg Ala Leu Val Val Ala Ala Ile Lys Glu
                245                 250                 255
Thr Glu Lys Met Asn Phe Ser Glu Phe Leu Ala Ala Tyr Glu Asp Ile
            260                 265                 270
Val Thr Arg Ser Arg Lys Gly Lys Leu Thr Met Asp Asp Tyr Gln Gly
        275                 280                 285
Val Thr Val Ser Leu Thr Asn Pro Gly Gly Ile Gly Thr Arg His Ser
    290                 295                 300

Val Pro Arg Leu Thr Lys Gly Gln Gly Thr Ile Ile Gly Val Gly Ser
305                 310                 315                 320
Met Asp Tyr Pro Ala Glu Phe Gln Gly Ala Ser Glu Asp Arg Leu Ala
                325                 330                 335
Glu Leu Gly Val Gly Lys Leu Val Thr Ile Thr Ser Thr Tyr Asp His
            340                 345                 350
Arg Val Ile Gln Gly Ala Val Ser Gly Glu Phe Leu Arg Thr Met Ser
        355                 360                 365
Arg Leu Leu Thr Asp Asp Ser Phe Trp Asp Glu Ile Phe Asp Ala Met
    370                 375                 380
Asn Val Pro Tyr Thr Pro Met Arg Trp Ala Gln Asp Val Pro Asn Thr
385                 390                 395                 400
Gly Val Asp Lys Asn Thr Arg Val Met Gln Leu Ile Glu Ala Tyr Arg
                405                 410                 415
Ser Arg Gly His Leu Ile Ala Asp Thr Asn Pro Leu Ser Trp Val Gln
            420                 425                 430
Pro Gly Met Pro Val Pro Asp His Arg Asp Leu Asp Ile Glu Thr His
        435                 440                 445
Ser Leu Thr Ile Trp Asp Leu Asp Arg Thr Phe Ser Val Gly Gly Phe
    450                 455                 460
Gly Gly Lys Glu Thr Met Thr Leu Arg Glu Val Leu Ser Arg Leu Arg
465                 470                 475                 480
Ala Ala Tyr Thr Leu Lys Val Gly Ser Glu Tyr Thr His Ile Leu Asp
                485                 490                 495
Arg Asp Glu Arg Thr Trp Leu Gln Asp Arg Leu Glu Ala Gly Met Pro
            500                 505                 510
Lys Pro Thr Gln Ala Glu Gln Lys Tyr Ile Leu Gln Lys Leu Asn Ala
        515                 520                 525
Ala Glu Ala Phe Glu Asn Phe Leu Gln Thr Lys Tyr Val Gly Gln Lys
    530                 535                 540
Arg Phe Ser Leu Glu Gly Ala Glu Ala Leu Ile Pro Leu Met Asp Ser
545                 550                 555                 560
Ala Ile Asp Thr Ala Ala Gly Gln Gly Leu Asp Glu Val Val Ile Gly
                565                 570                 575
Met Pro His Arg Gly Arg Leu Asn Val Leu Phe Asn Ile Val Gly Lys
            580                 585                 590
Pro Leu Ala Ser Ile Phe Asn Glu Phe Glu Gly Gln Met Glu Gln Gly
        595                 600                 605
Gln Ile Gly Gly Ser Gly Asp Val Lys Tyr His Leu Gly Ser Glu Gly
    610                 615                 620
Gln His Leu Gln Met Phe Gly Asp Gly Glu Ile Lys Val Ser Leu Thr
625                 630                 635                 640
Ala Asn Pro Ser His Leu Glu Ala Val Asn Pro Val Met Glu Gly Ile
                645                 650                 655
Val Arg Ala Lys Gln Asp Tyr Leu Asp Lys Gly Val Asp Gly Lys Thr
            660                 665                 670
Val Val Pro Leu Leu Leu His Gly Asp Ala Ala Phe Ala Gly Leu Gly
        675                 680                 685
Ile Val Pro Glu Thr Ile Asn Leu Ala Lys Ser Cys Val Asp Val Gly
    690                 695                 700
Gly Thr Ile His Ile Val Val Asn Asn Gln Ile Gly Phe Thr Thr Thr

705                 710                 715                 720
Pro Asp Ser Ser Arg Ser Met His Tyr Ala Thr Asp Tyr Ala Lys Ala
                725                 730                 735
Phe Gly Cys Pro Val Phe His Val Asn Gly Asp Asp Pro Glu Ala Val
            740                 745                 750
Val Trp Val Gly Gln Leu Ala Thr Glu Tyr Arg Arg Arg Phe Gly Lys
        755                 760                 765
Asp Val Phe Ile Asp Leu Val Cys Tyr Arg Leu Arg Gly His Asn Glu
    770                 775                 780
Ala Asp Asp Pro Ser Met Thr Gln Pro Lys Met Tyr Glu Leu Ile Thr
785                 790                 795                 800

Gly Arg Glu Thr Val Arg Ala Gln Tyr Thr Glu Asp Leu Leu Gly Arg
                805                 810                 815
Gly Asp Leu Ser Asn Glu Asp Ala Glu Ala Val Val Arg Asp Phe His
            820                 825                 830
Asp Gln Met Glu Ser Val Phe Asn Glu Val Lys Glu Gly Gly Lys Lys
        835                 840                 845
Gln Ala Glu Ala Gln Thr Gly Ile Thr Gly Ser Gln Lys Leu Pro His
    850                 855                 860
Gly Leu Glu Thr Asn Ile Ser Arg Glu Glu Leu Leu Glu Leu Gly Gln
865                 870                 875                 880
Ala Phe Ala Asn Thr Pro Glu Gly Phe Asn Tyr His Pro Arg Val Ala
                885                 890                 895
Pro Val Ala Lys Lys Arg Val Ser Ser Val Thr Glu Gly Gly Ile Asp
            900                 905                 910
Trp Ala Trp Gly Glu Leu Leu Ala Phe Gly Ser Leu Ala Asn Ser Gly
        915                 920                 925
Arg Leu Val Arg Leu Ala Gly Glu Asp Ser Arg Arg Gly Thr Phe Thr
    930                 935                 940
Gln Arg His Ala Val Ala Ile Asp Pro Ala Thr Ala Glu Glu Phe Asn
945                 950                 955                 960
Pro Leu His Glu Leu Ala Gln Ser Lys Gly Asn Asn Gly Lys Phe Leu
                965                 970                 975
Val Tyr Asn Ser Ala Leu Thr Glu Tyr Ala Gly Met Gly Phe Glu Tyr
            980                 985                 990
Gly Tyr Ser Val Gly Asn Glu Asp  Ser Val Val Ala Trp  Glu Ala Gln
        995                 1000                 1005
Phe Gly  Asp Phe Ala Asn Gly  Ala Gln Thr Ile Ile  Asp Glu Tyr
    1010                 1015                 1020
Val Ser  Ser Gly Glu Ala Lys  Trp Gly Gln Thr Ser  Lys Leu Ile
    1025                 1030                 1035
Leu Leu  Leu Pro His Gly Tyr  Glu Gly Gln Gly Pro  Asp His Ser
    1040                 1045                 1050
Ser Ala  Arg Ile Glu Arg Phe  Leu Gln Leu Cys Ala  Glu Gly Ser
    1055                 1060                 1065
Met Thr  Val Ala Gln Pro Ser  Thr Pro Ala Asn His  Phe His Leu
    1070                 1075                 1080
Leu Arg  Arg His Ala Leu Ser  Asp Leu Lys Arg Pro  Leu Val Ile
    1085                 1090                 1095
Phe Thr  Pro Lys Ser Met Leu  Arg Asn Lys Ala Ala  Ala Ser Ala
    1100                 1105                 1110
Pro Glu  Asp Phe Thr Glu Val  Thr Lys Phe Gln Ser  Val Ile Asp
    1115                 1120                 1125
Asp Pro  Asn Val Ala Asp Ala  Ala Lys Val Lys Lys  Val Met Leu
    1130                 1135                 1140
Val Ser  Gly Lys Leu Tyr Tyr  Glu Leu Ala Lys Arg  Lys Glu Lys
    1145                 1150                 1155
Asp Gly  Arg Asp Asp Ile Ala  Ile Val Arg Ile Glu  Met Leu His
    1160                 1165                 1170
Pro Ile  Pro Phe Asn Arg Ile  Ser Glu Ala Leu Ala  Gly Tyr Pro
    1175                 1180                 1185
Asn Ala  Glu Glu Val Leu Phe  Val Gln Asp Glu Pro  Ala Asn Gln
    1190                 1195                 1200
Gly Pro  Trp Pro Phe Tyr Gln  Glu His Leu Pro Glu  Leu Ile Pro
    1205                 1210                 1215
Asn Met  Pro Lys Met Arg Arg  Val Ser Arg Arg Ala  Gln Ser Ser
    1220                 1225                 1230
Thr Ala  Thr Gly Val Ala Lys  Val His Gln Leu Glu  Glu Lys Gln
    1235                 1240                 1245
Leu Ile  Asp Glu Ala Phe Glu  Ala
    1250                 1255

<210>  51

<211>  4391
<212>  DNA
<213>  Corynebacterium glutamicum

<220>
<221>  CDS
<222>  (443)..(4210)
<223>  sucA77

<400>  51
gtcgacaagc aaaatcgaag cggcagcacg ccgcgtcgga gccttaaacg ccatcgccgc      60
catccctgat ggtttcaatc atcaagtcgg tgaacgcggg cgcaacctgt catccggaca     120
gcgccaactg atcgcgctgg cgcgcgccga actcatcgag ccttccatca tgcttctcga     180
cgaagccacc tccaccctcg accccgccac cgaagccgtt atcctcaacg cctccgatcg     240
agtcactaag ggacgcacca gcatcatcgt cgcgcaccgc ttggcaaccg ctaaaagggc     300
cgaccgtatt cttgttgttg aacaaggacg tatcattgag gacggatctc acgacgcgtt     360
gttgtctgct aacggcacct acgcccgcat gtggcattta atggcctgac acgttatttt     420
taggagaact gtcaacaaat ta atg cta caa ctg ggg ctt agg cat aat cag      472
                         Met Leu Gln Leu Gly Leu Arg His Asn Gln
                         1               5                   10
cca acg acc aac gtt aca gtg gat aaa ata aag ctc aat aaa ccc tca      520
Pro Thr Thr Asn Val Thr Val Asp Lys Ile Lys Leu Asn Lys Pro Ser

                15                  20                  25
aga agc aag gaa aag agg cga gta cct gcc gtg agc agc gct agt act      568
Arg Ser Lys Glu Lys Arg Arg Val Pro Ala Val Ser Ser Ala Ser Thr
            30                  35                  40
ttc ggc cag aat gcg tgg ctg gta gac gag atg ttc cag cag ttc cag      616
Phe Gly Gln Asn Ala Trp Leu Val Asp Glu Met Phe Gln Gln Phe Gln
        45                  50                  55
aag gac ccc aag tcc gtg gac aag gaa tgg aga gaa ctc ttt gag gcg      664
Lys Asp Pro Lys Ser Val Asp Lys Glu Trp Arg Glu Leu Phe Glu Ala
    60                  65                  70
cag ggg gga cca aat gct acc ccc gct aca aca gaa gca cag cct tca      712
Gln Gly Gly Pro Asn Ala Thr Pro Ala Thr Thr Glu Ala Gln Pro Ser
75                  80                  85                  90
gcg ccc aag gag tct gcg aaa cca gca cca aag gct gcc cct gca gcc      760
Ala Pro Lys Glu Ser Ala Lys Pro Ala Pro Lys Ala Ala Pro Ala Ala
                95                  100                 105
aag gca gca ccg cgc gta gaa acc aag ccg gcc gcc aag acc gcc cct      808
Lys Ala Ala Pro Arg Val Glu Thr Lys Pro Ala Ala Lys Thr Ala Pro
            110                 115                 120
aag gcc aag gag tcc tca gtg cca cag caa cct aag ctt ccg gag cca      856
Lys Ala Lys Glu Ser Ser Val Pro Gln Gln Pro Lys Leu Pro Glu Pro
        125                 130                 135
gga caa acc cca atc agg ggt att ttc aag tcc atc gcg aag aac atg      904
Gly Gln Thr Pro Ile Arg Gly Ile Phe Lys Ser Ile Ala Lys Asn Met
    140                 145                 150
gat atc tcc ctg gaa atc cca acc gca acc tcg gtt cgc gat atg cca      952
Asp Ile Ser Leu Glu Ile Pro Thr Ala Thr Ser Val Arg Asp Met Pro
155                 160                 165                 170
gct cgc ctc atg ttc gaa aac cgc gcg atg gtc aac gat cag ctc aag     1000
Ala Arg Leu Met Phe Glu Asn Arg Ala Met Val Asn Asp Gln Leu Lys
                175                 180                 185
cgc acc cgc ggt ggc aag atc tcc ttc acc cac atc att ggc tac gcc     1048
Arg Thr Arg Gly Gly Lys Ile Ser Phe Thr His Ile Ile Gly Tyr Ala
            190                 195                 200
atg gtg aag gca gtc atg gct cac ccg gac atg aac aac tcc tac gac     1096
Met Val Lys Ala Val Met Ala His Pro Asp Met Asn Asn Ser Tyr Asp
        205                 210                 215
gtc atc gac ggc aag cca acc ctg atc gtg cct gag cac atc aac ctg     1144
Val Ile Asp Gly Lys Pro Thr Leu Ile Val Pro Glu His Ile Asn Leu
    220                 225                 230

ggc ctt gcc atc gac ctt cct cag aag gac ggc tcc cgc gca ctt gtc 1192
Gly Leu Ala Ile Asp Leu Pro Gln Lys Asp Gly Ser Arg Ala Leu Val
235                 240                 245                 250
gta gca gcc atc aag gaa acc gag aag atg aac ttc tcc gag ttc ctc 1240
Val Ala Ala Ile Lys Glu Thr Glu Lys Met Asn Phe Ser Glu Phe Leu
                255                 260                 265
gca gca tac gaa gac atc gtg aca cgc tcc cgc aag ggc aag ctc acc 1288
Ala Ala Tyr Glu Asp Ile Val Thr Arg Ser Arg Lys Gly Lys Leu Thr
            270                 275                 280
atg gat gac tac cag ggc gtt acc gtt tcc ttg acc aac cca ggt ggc 1336
Met Asp Asp Tyr Gln Gly Val Thr Val Ser Leu Thr Asn Pro Gly Gly
        285                 290                 295
atc ggt acc cgc cac tct gtc cca cgt ctg acc aag ggc cag ggc acc 1384
Ile Gly Thr Arg His Ser Val Pro Arg Leu Thr Lys Gly Gln Gly Thr
    300                 305                 310
atc atc ggt gtc ggt tcc atg gat tac cca gca gag ttc cag ggc gct 1432
Ile Ile Gly Val Gly Ser Met Asp Tyr Pro Ala Glu Phe Gln Gly Ala
315                 320                 325                 330
tcc gaa gac cgc ctt gca gag ctc ggc gtt gga aag ctt gtc acc atc 1480
Ser Glu Asp Arg Leu Ala Glu Leu Gly Val Gly Lys Leu Val Thr Ile
                335                 340                 345
acc tcc acc tac gat cac cgc gtg atc cag ggt gct gtg tcc ggt gaa 1528
Thr Ser Thr Tyr Asp His Arg Val Ile Gln Gly Ala Val Ser Gly Glu
            350                 355                 360
ttc ctg cgt acc atg tct cgc ctg ctc acc gat gat tcc ttc tgg gat 1576
Phe Leu Arg Thr Met Ser Arg Leu Leu Thr Asp Asp Ser Phe Trp Asp
        365                 370                 375
gag atc ttc gac gca atg aac gtt cct tac acc cca atg cgt tgg gca 1624
Glu Ile Phe Asp Ala Met Asn Val Pro Tyr Thr Pro Met Arg Trp Ala
    380                 385                 390
cag gac gtt cca aac acc ggt gtt gat aag aac acc cgc gtc atg cag 1672
Gln Asp Val Pro Asn Thr Gly Val Asp Lys Asn Thr Arg Val Met Gln
395                 400                 405                 410
ctc att gag gca tac cgc tcc cgt gga cac ctc atc gct gac acc aac 1720
Leu Ile Glu Ala Tyr Arg Ser Arg Gly His Leu Ile Ala Asp Thr Asn
                415                 420                 425
cca ctt tca tgg gtt cag cct ggc atg cca gtt cca gac cac cgc gac 1768
Pro Leu Ser Trp Val Gln Pro Gly Met Pro Val Pro Asp His Arg Asp
            430                 435                 440
ctc gac atc gag acc cac agc ctg acc atc tgg gat ctg gac cgt acc 1816
Leu Asp Ile Glu Thr His Ser Leu Thr Ile Trp Asp Leu Asp Arg Thr
        445                 450                 455
ttc agc gtc ggt ggc ttc ggc ggc aag gag acc atg acc ctg cgc gag 1864
Phe Ser Val Gly Gly Phe Gly Gly Lys Glu Thr Met Thr Leu Arg Glu
    460                 465                 470
gta ctg tcc cgc ctg cgc gct gcc tac acc ttg aag gtc ggc tcc gaa 1912
Val Leu Ser Arg Leu Arg Ala Ala Tyr Thr Leu Lys Val Gly Ser Glu
475                 480                 485                 490
tac acc cac atc ctg gac cgc gac gag cgc acc tgg ctg cag gac cgc 1960
Tyr Thr His Ile Leu Asp Arg Asp Glu Arg Thr Trp Leu Gln Asp Arg
                495                 500                 505
ctc gaa gcc gga atg cca aag cca acc cag gca gag cag aag tac atc 2008
Leu Glu Ala Gly Met Pro Lys Pro Thr Gln Ala Glu Gln Lys Tyr Ile
            510                 515                 520
ctg cag aag ctg aac gcc gca gag gct ttc gag aac ttc ctg cag acc 2056
Leu Gln Lys Leu Asn Ala Ala Glu Ala Phe Glu Asn Phe Leu Gln Thr
        525                 530                 535
aag tac gtc ggc cag aag cgc ttc tcc ctc gaa ggt gca gaa gct ctc 2104
Lys Tyr Val Gly Gln Lys Arg Phe Ser Leu Glu Gly Ala Glu Ala Leu
    540                 545                 550
atc cca ctg atg gac tcc gcc atc gac acc gcc gca ggc cag ggc ctc 2152
Ile Pro Leu Met Asp Ser Ala Ile Asp Thr Ala Ala Gly Gln Gly Leu
555                 560                 565                 570

gac gaa gtt gtc atc ggt atg cca cac cgt ggt cgc ctc aac gtg ctg 2200
Asp Glu Val Val Ile Gly Met Pro His Arg Gly Arg Leu Asn Val Leu
                575                 580                 585
ttc aac atc gtg ggc aag cca ctg gca tcc atc ttc aac gag ttt gaa 2248
Phe Asn Ile Val Gly Lys Pro Leu Ala Ser Ile Phe Asn Glu Phe Glu
            590                 595                 600
ggc caa atg gag cag ggc cag atc ggt ggc tcc ggt gac gtg aag tac 2296
Gly Gln Met Glu Gln Gly Gln Ile Gly Gly Ser Gly Asp Val Lys Tyr
        605                 610                 615
cac ctc ggt tcc gaa ggc cag cac ctg cag atg ttc ggc gac ggc gag 2344
His Leu Gly Ser Glu Gly Gln His Leu Gln Met Phe Gly Asp Gly Glu
    620                 625                 630
atc aag gtc tcc ctg act gct aac ccg tcc cac ctg gaa gct gtt aac 2392
Ile Lys Val Ser Leu Thr Ala Asn Pro Ser His Leu Glu Ala Val Asn
635                 640                 645                 650
cca gtg atg gaa ggt atc gtc cgc gca aag cag gac tac ctg gac aag 2440
Pro Val Met Glu Gly Ile Val Arg Ala Lys Gln Asp Tyr Leu Asp Lys
                655                 660                 665
ggc gta gac ggc aag act gtt gtg cca ctg ctg ctc cac ggt gac gct 2488
Gly Val Asp Gly Lys Thr Val Val Pro Leu Leu Leu His Gly Asp Ala
            670                 675                 680
gca ttc gca ggc ctg ggc atc gtg cca gaa acc atc aac ctg gct ata 2536
Ala Phe Ala Gly Leu Gly Ile Val Pro Glu Thr Ile Asn Leu Ala Ile
        685                 690                 695
agc tgc gtc gac gtc gga ggc acc atc cac atc gtg gtg aac aac cag 2584
Ser Cys Val Asp Val Gly Gly Thr Ile His Ile Val Val Asn Asn Gln
    700                 705                 710
atc ggc ttc acc acc acc cca gac tcc agc cgc tcc atg cac tac gca 2632
Ile Gly Phe Thr Thr Thr Pro Asp Ser Ser Arg Ser Met His Tyr Ala
715                 720                 725                 730
acc gac tac gcc aag gca ttc ggc tgc cca gtc ttc cac gtc aat ggt 2680
Thr Asp Tyr Ala Lys Ala Phe Gly Cys Pro Val Phe His Val Asn Gly
                735                 740                 745
gat gac cca gag gca gtt gtc tgg gtt ggc cag ctg gca acc gag tac 2728
Asp Asp Pro Glu Ala Val Val Trp Val Gly Gln Leu Ala Thr Glu Tyr
            750                 755                 760
cgt cgt cgc ttc ggc aag gac gtc ttc atc gac ctc gtt tgc tac cgc 2776
Arg Arg Arg Phe Gly Lys Asp Val Phe Ile Asp Leu Val Cys Tyr Arg
        765                 770                 775
ctc cgc ggc cac aac gaa gct gat gat cct tcc atg acc cag cca aag 2824
Leu Arg Gly His Asn Glu Ala Asp Asp Pro Ser Met Thr Gln Pro Lys
    780                 785                 790
atg tat gag ctc atc acc ggc cgc gag acc gtt cgt gct cag tac acc 2872
Met Tyr Glu Leu Ile Thr Gly Arg Glu Thr Val Arg Ala Gln Tyr Thr
795                 800                 805                 810
gaa gac ctg ctc gga cgt gga gac ctc tcc aac gaa gat gca gaa gca 2920
Glu Asp Leu Leu Gly Arg Gly Asp Leu Ser Asn Glu Asp Ala Glu Ala
                815                 820                 825
gtc gtc cgc gac ttc cac gac cag atg gaa tct gtg ttc aac gaa gtc 2968
Val Val Arg Asp Phe His Asp Gln Met Glu Ser Val Phe Asn Glu Val
            830                 835                 840
aag gaa ggc ggc aag aag cag gct gag gca cag acc ggc atc acc ggc 3016
Lys Glu Gly Gly Lys Lys Gln Ala Glu Ala Gln Thr Gly Ile Thr Gly
        845                 850                 855
tcc cag aag ctt cca cac ggc ctt gag acc aac atc tcc cgt gaa gag 3064
Ser Gln Lys Leu Pro His Gly Leu Glu Thr Asn Ile Ser Arg Glu Glu
    860                 865                 870
ctc ctg gaa ctg gga cag gct ttc gcc aac acc cca gaa ggc ttc aac 3112
Leu Leu Glu Leu Gly Gln Ala Phe Ala Asn Thr Pro Glu Gly Phe Asn
875                 880                 885                 890
tac cac cca cgt gtg gct cca gtt gct aag aag cgc gtc tcc tct gtc 3160
Tyr His Pro Arg Val Ala Pro Val Ala Lys Lys Arg Val Ser Ser Val

895                 900                 905
acc gaa ggt ggc atc gac tgg gca tgg ggc gag ctc ctc gcc ttc ggt     3208
Thr Glu Gly Gly Ile Asp Trp Ala Trp Gly Glu Leu Leu Ala Phe Gly
        910                 915                 920
tcc ctg gct aac tcc ggc cgc ttg gtt cgc ctt gca ggt gaa gat tcc     3256
Ser Leu Ala Asn Ser Gly Arg Leu Val Arg Leu Ala Gly Glu Asp Ser
    925                 930                 935
cgc cgc ggt acc ttc acc cag cgc cac gca gtt gcc atc gac cca gcg     3304
Arg Arg Gly Thr Phe Thr Gln Arg His Ala Val Ala Ile Asp Pro Ala
940                 945                 950
acc gct gaa gag ttc aac cca ctc cac gag ctt gca cag tcc aag ggc     3352
Thr Ala Glu Glu Phe Asn Pro Leu His Glu Leu Ala Gln Ser Lys Gly
955                 960                 965                 970
aac aac ggt aag ttc ctg gtc tac aac tcc gca ctg acc gag tac gca     3400
Asn Asn Gly Lys Phe Leu Val Tyr Asn Ser Ala Leu Thr Glu Tyr Ala
                975                 980                 985
ggc atg ggc ttc gag tac ggc tac tcc gta gga aac gaa gac  tcc gtc     3448
Gly Met Gly Phe Glu Tyr Gly Tyr Ser Val Gly Asn Glu Asp  Ser Val
            990                 995                 1000
gtt gca tgg  gaa gca cag ttc ggc  gac ttc gcc aac ggc  gct cag     3493
Val Ala Trp  Glu Ala Gln Phe Gly  Asp Phe Ala Asn Gly  Ala Gln
    1005                 1010                 1015
acc atc atc  gat gag tac gtc tcc  tca ggc gaa gct aag  tgg ggc     3538
Thr Ile Ile  Asp Glu Tyr Val Ser  Ser Gly Glu Ala Lys  Trp Gly
    1020                 1025                 1030
cag acc tcc  aag ctg atc ctt ctg  ctg cct cac ggc tac  gaa ggc     3583
Gln Thr Ser  Lys Leu Ile Leu Leu  Leu Pro His Gly Tyr  Glu Gly
    1035                 1040                 1045
cag ggc cca  gac cac tct tcc gca  cgt atc gag cgc ttc  ctg cag     3628
Gln Gly Pro  Asp His Ser Ser Ala  Arg Ile Glu Arg Phe  Leu Gln
    1050                 1055                 1060
ctg tgc gct  gag ggt tcc atg act  gtt gct cag cca tcc  acc cca     3673
Leu Cys Ala  Glu Gly Ser Met Thr  Val Ala Gln Pro Ser  Thr Pro
    1065                 1070                 1075
gca aac cac  ttc cac ctg ctg cgt  cgt cac gct ctg tcc  gac ctg     3718
Ala Asn His  Phe His Leu Leu Arg  Arg His Ala Leu Ser  Asp Leu
    1080                 1085                 1090
aag cgt cca  ctg gtt atc ttc acc  ccg aag tcc atg ctg  cgt aac     3763
Lys Arg Pro  Leu Val Ile Phe Thr  Pro Lys Ser Met Leu  Arg Asn
    1095                 1100                 1105
aag gct gct  gcc tcc gca cca gaa  gac ttc act gag gtc  acc aag     3808
Lys Ala Ala  Ala Ser Ala Pro Glu  Asp Phe Thr Glu Val  Thr Lys
    1110                 1115                 1120
ttc caa tcc  gtg atc gac gat cca  aac gtt gca gat gca  gcc aag     3853
Phe Gln Ser  Val Ile Asp Asp Pro  Asn Val Ala Asp Ala  Ala Lys
    1125                 1130                 1135
gtg aag aag  gtc atg ctg gtc tcc  ggc aag ctg tac tac  gaa ttg     3898
Val Lys Lys  Val Met Leu Val Ser  Gly Lys Leu Tyr Tyr  Glu Leu
    1140                 1145                 1150
gca aag cgc  aag gag aag gac gga  cgc gac gac atc gcg  atc gtt     3943
Ala Lys Arg  Lys Glu Lys Asp Gly  Arg Asp Asp Ile Ala  Ile Val
    1155                 1160                 1165
cgt atc gaa  atg ctc cac cca att  ccg ttc aac cgc atc  tcc gag     3988
Arg Ile Glu  Met Leu His Pro Ile  Pro Phe Asn Arg Ile  Ser Glu
    1170                 1175                 1180
gct ctt gcc  ggc tac cct aac gct  gag gaa gtc ctc ttc  gtt cag     4033
Ala Leu Ala  Gly Tyr Pro Asn Ala  Glu Glu Val Leu Phe  Val Gln
    1185                 1190                 1195
gat gag cca  gca aac cag ggc cca  tgg ccg ttc tac cag  gag cac     4078
Asp Glu Pro  Ala Asn Gln Gly Pro  Trp Pro Phe Tyr Gln  Glu His
    1200                 1205                 1210
ctc cca gag  ctg atc ccg aac atg  cca aag atg cgc cgc  gtt tcc     4123
Leu Pro Glu  Leu Ile Pro Asn Met  Pro Lys Met Arg Arg  Val Ser

1215                1220                1225
cgc cgc gct  cag tcc tcc acc gca  act ggt gtt gct aag  gtg cac      4168
Arg Arg Ala  Gln Ser Ser Thr Ala  Thr Gly Val Ala Lys  Val His
        1230                1235                1240
cag ctg gag  gag aag cag ctt atc  gac gag gct ttc gag  gct          4210
Gln Leu Glu  Glu Lys Gln Leu Ile  Asp Glu Ala Phe Glu  Ala
        1245                1250                1255
taagtcttta tagtcctgca ctagcctaga gggccttatg cagtgtgaat cacacagcat   4270
aaggcccttt ttgctgccgt ggttgcctaa ggtggaaggc atgaaacgaa tctgtgcggt   4330
cacgatctct tcagtacttt tgctaagtgg ctgctcctcc acttccacca cgcagctcga   4390
g                                                                   4391

<210>  52
<211>  1256
<212>  PRT
<213>  Corynebacterium glutamicum

<400>  52
Met Leu Gln Leu Gly Leu Arg His Asn Gln Pro Thr Thr Asn Val Thr
1               5                   10                  15
Val Asp Lys Ile Lys Leu Asn Lys Pro Ser Arg Ser Lys Glu Lys Arg
            20                  25                  30
Arg Val Pro Ala Val Ser Ser Ala Ser Thr Phe Gly Gln Asn Ala Trp
        35                  40                  45
Leu Val Asp Glu Met Phe Gln Gln Phe Gln Lys Asp Pro Lys Ser Val
    50                  55                  60
Asp Lys Glu Trp Arg Glu Leu Phe Glu Ala Gln Gly Gly Pro Asn Ala
65                  70                  75                  80
Thr Pro Ala Thr Thr Glu Ala Gln Pro Ser Ala Pro Lys Glu Ser Ala
                85                  90                  95
Lys Pro Ala Pro Lys Ala Ala Pro Ala Ala Lys Ala Ala Pro Arg Val
            100                 105                 110
Glu Thr Lys Pro Ala Ala Lys Thr Ala Pro Lys Ala Lys Glu Ser Ser
        115                 120                 125
Val Pro Gln Gln Pro Lys Leu Pro Glu Pro Gly Gln Thr Pro Ile Arg
    130                 135                 140
Gly Ile Phe Lys Ser Ile Ala Lys Asn Met Asp Ile Ser Leu Glu Ile
145                 150                 155                 160
Pro Thr Ala Thr Ser Val Arg Asp Met Pro Ala Arg Leu Met Phe Glu
                165                 170                 175
Asn Arg Ala Met Val Asn Asp Gln Leu Lys Arg Thr Arg Gly Gly Lys
            180                 185                 190
Ile Ser Phe Thr His Ile Ile Gly Tyr Ala Met Val Lys Ala Val Met
        195                 200                 205
Ala His Pro Asp Met Asn Asn Ser Tyr Asp Val Ile Asp Gly Lys Pro
    210                 215                 220
Thr Leu Ile Val Pro Glu His Ile Asn Leu Gly Leu Ala Ile Asp Leu
225                 230                 235                 240
Pro Gln Lys Asp Gly Ser Arg Ala Leu Val Val Ala Ala Ile Lys Glu
                245                 250                 255
Thr Glu Lys Met Asn Phe Ser Glu Phe Leu Ala Ala Tyr Glu Asp Ile
            260                 265                 270
Val Thr Arg Ser Arg Lys Gly Lys Leu Thr Met Asp Asp Tyr Gln Gly
        275                 280                 285
Val Thr Val Ser Leu Thr Asn Pro Gly Gly Ile Gly Thr Arg His Ser
    290                 295                 300
Val Pro Arg Leu Thr Lys Gly Gln Gly Thr Ile Ile Gly Val Gly Ser
305                 310                 315                 320
Met Asp Tyr Pro Ala Glu Phe Gln Gly Ala Ser Glu Asp Arg Leu Ala

                325                 330                 335
Glu Leu Gly Val Gly Lys Leu Val Thr Ile Thr Ser Thr Tyr Asp His

            340                 345                 350
Arg Val Ile Gln Gly Ala Val Ser Gly Glu Phe Leu Arg Thr Met Ser
        355                 360                 365
Arg Leu Leu Thr Asp Asp Ser Phe Trp Asp Glu Ile Phe Asp Ala Met
    370                 375                 380
Asn Val Pro Tyr Thr Pro Met Arg Trp Ala Gln Asp Val Pro Asn Thr
385                 390                 395                 400
Gly Val Asp Lys Asn Thr Arg Val Met Gln Leu Ile Glu Ala Tyr Arg
                405                 410                 415
Ser Arg Gly His Leu Ile Ala Asp Thr Asn Pro Leu Ser Trp Val Gln
            420                 425                 430
Pro Gly Met Pro Val Pro Asp His Arg Asp Leu Asp Ile Glu Thr His
        435                 440                 445
Ser Leu Thr Ile Trp Asp Leu Asp Arg Thr Phe Ser Val Gly Gly Phe
    450                 455                 460
Gly Gly Lys Glu Thr Met Thr Leu Arg Glu Val Leu Ser Arg Leu Arg
465                 470                 475                 480
Ala Ala Tyr Thr Leu Lys Val Gly Ser Glu Tyr Thr His Ile Leu Asp
                485                 490                 495
Arg Asp Glu Arg Thr Trp Leu Gln Asp Arg Leu Glu Ala Gly Met Pro
            500                 505                 510
Lys Pro Thr Gln Ala Glu Gln Lys Tyr Ile Leu Gln Lys Leu Asn Ala
        515                 520                 525
Ala Glu Ala Phe Glu Asn Phe Leu Gln Thr Lys Tyr Val Gly Gln Lys
    530                 535                 540
Arg Phe Ser Leu Glu Gly Ala Glu Ala Leu Ile Pro Leu Met Asp Ser
545                 550                 555                 560
Ala Ile Asp Thr Ala Ala Gly Gln Gly Leu Asp Glu Val Val Ile Gly
                565                 570                 575
Met Pro His Arg Gly Arg Leu Asn Val Leu Phe Asn Ile Val Gly Lys
            580                 585                 590
Pro Leu Ala Ser Ile Phe Asn Glu Phe Glu Gly Gln Met Glu Gln Gly
        595                 600                 605
Gln Ile Gly Gly Ser Gly Asp Val Lys Tyr His Leu Gly Ser Glu Gly
    610                 615                 620
Gln His Leu Gln Met Phe Gly Asp Gly Glu Ile Lys Val Ser Leu Thr
625                 630                 635                 640
Ala Asn Pro Ser His Leu Glu Ala Val Asn Pro Val Met Glu Gly Ile
                645                 650                 655
Val Arg Ala Lys Gln Asp Tyr Leu Asp Lys Gly Val Asp Gly Lys Thr
            660                 665                 670
Val Val Pro Leu Leu Leu His Gly Asp Ala Ala Phe Ala Gly Leu Gly
        675                 680                 685
Ile Val Pro Glu Thr Ile Asn Leu Ala Ile Ser Cys Val Asp Val Gly
    690                 695                 700
Gly Thr Ile His Ile Val Val Asn Asn Gln Ile Gly Phe Thr Thr Thr
705                 710                 715                 720
Pro Asp Ser Ser Arg Ser Met His Tyr Ala Thr Asp Tyr Ala Lys Ala
                725                 730                 735
Phe Gly Cys Pro Val Phe His Val Asn Gly Asp Asp Pro Glu Ala Val
            740                 745                 750
Val Trp Val Gly Gln Leu Ala Thr Glu Tyr Arg Arg Arg Phe Gly Lys
        755                 760                 765
Asp Val Phe Ile Asp Leu Val Cys Tyr Arg Leu Arg Gly His Asn Glu
    770                 775                 780
Ala Asp Asp Pro Ser Met Thr Gln Pro Lys Met Tyr Glu Leu Ile Thr
785                 790                 795                 800
Gly Arg Glu Thr Val Arg Ala Gln Tyr Thr Glu Asp Leu Leu Gly Arg
                805                 810                 815
Gly Asp Leu Ser Asn Glu Asp Ala Glu Ala Val Val Arg Asp Phe His
            820                 825                 830
Asp Gln Met Glu Ser Val Phe Asn Glu Val Lys Glu Gly Gly Lys Lys
        835                 840                 845

Gln Ala Glu Ala Gln Thr Gly Ile Thr Gly Ser Gln Lys Leu Pro His
    850                 855                 860
Gly Leu Glu Thr Asn Ile Ser Arg Glu Glu Leu Leu Glu Leu Gly Gln
865                 870                 875                 880
Ala Phe Ala Asn Thr Pro Glu Gly Phe Asn Tyr His Pro Arg Val Ala
                885                 890                 895
Pro Val Ala Lys Lys Arg Val Ser Ser Val Thr Glu Gly Gly Ile Asp
            900                 905                 910
Trp Ala Trp Gly Glu Leu Leu Ala Phe Gly Ser Leu Ala Asn Ser Gly
        915                 920                 925
Arg Leu Val Arg Leu Ala Gly Glu Asp Ser Arg Arg Gly Thr Phe Thr
    930                 935                 940
Gln Arg His Ala Val Ala Ile Asp Pro Ala Thr Ala Glu Glu Phe Asn
945                 950                 955                 960
Pro Leu His Glu Leu Ala Gln Ser Lys Gly Asn Asn Gly Lys Phe Leu
                965                 970                 975
Val Tyr Asn Ser Ala Leu Thr Glu Tyr Ala Gly Met Gly Phe Glu Tyr
            980                 985                 990
Gly Tyr Ser Val Gly Asn Glu Asp Ser Val Val Ala Trp Glu Ala Gln
        995                 1000                1005
Phe Gly Asp Phe Ala Asn Gly Ala Gln Thr Ile Ile Asp Glu Tyr
    1010                1015                1020
Val Ser Ser Gly Glu Ala Lys Trp Gly Gln Thr Ser Lys Leu Ile
    1025                1030                1035
Leu Leu Leu Pro His Gly Tyr Glu Gly Gln Gly Pro Asp His Ser
    1040                1045                1050
Ser Ala Arg Ile Glu Arg Phe Leu Gln Leu Cys Ala Glu Gly Ser
    1055                1060                1065
Met Thr Val Ala Gln Pro Ser Thr Pro Ala Asn His Phe His Leu
    1070                1075                1080
Leu Arg Arg His Ala Leu Ser Asp Leu Lys Arg Pro Leu Val Ile
    1085                1090                1095
Phe Thr Pro Lys Ser Met Leu Arg Asn Lys Ala Ala Ala Ser Ala
    1100                1105                1110
Pro Glu Asp Phe Thr Glu Val Thr Lys Phe Gln Ser Val Ile Asp
    1115                1120                1125
Asp Pro Asn Val Ala Asp Ala Ala Lys Val Lys Lys Val Met Leu
    1130                1135                1140
Val Ser Gly Lys Leu Tyr Tyr Glu Leu Ala Lys Arg Lys Glu Lys
    1145                1150                1155
Asp Gly Arg Asp Asp Ile Ala Ile Val Arg Ile Glu Met Leu His
    1160                1165                1170
Pro Ile Pro Phe Asn Arg Ile Ser Glu Ala Leu Ala Gly Tyr Pro
    1175                1180                1185
Asn Ala Glu Glu Val Leu Phe Val Gln Asp Glu Pro Ala Asn Gln
    1190                1195                1200
Gly Pro Trp Pro Phe Tyr Gln Glu His Leu Pro Glu Leu Ile Pro
    1205                1210                1215
Asn Met Pro Lys Met Arg Arg Val Ser Arg Arg Ala Gln Ser Ser
    1220                1225                1230
Thr Ala Thr Gly Val Ala Lys Val His Gln Leu Glu Glu Lys Gln
    1235                1240                1245
Leu Ile Asp Glu Ala Phe Glu Ala
    1250                1255

<210> 53
<211> 40
<212> DNA
<213> Artificial sequence

<220>
<223> primer

<400>  53
gccgggatcc ctgtgtgatt cacactgcat aaggccctct                              40



<210>  54
<211>  40
<212>  DNA
<213>  Artificial sequence

<220>
<223>  primer

<400>  54
gccgggatcc ccatcgccgc catccctgat ggtttcaatc                              40



<210>  55
<211>  28
<212>  DNA
<213>  Artificial sequence

<220>
<223>  sucA8primer

<400>  55
ctgcgtcgac gtcggaggca ccatccac                                           28



<210>  56
<211>  30
<212>  DNA
<213>  Artificial sequence

<220>
<223>  sucA801primer

<400>  56
ctgcgtctcg acgtcggagg caccatccac                                         30



<210>  57
<211>  36
<212>  DNA
<213>  Artificial sequence

<220>
<223>  sucA805primer

<400>  57
gctaaaagct gcgtcgacgt cggaggcacc atccac                                  36



<210>  58
<211>  36
<212>  DNA
<213>  Artificial sequence

<220>
<223>  sucA77

<400>  58
gctataagct gcgtcgacgt cggaggcacc atccac                                  36

<210> 59
<211> 33
<212> DNA
<213> Artificial sequence

<220>
<223> yggB primer-N

<400> 59
ggggatcctg ccgatttcgg caggatcaat gtc 33


<210> 60
<211> 31
<212> DNA
<213> Artificial sequence

<220>
<223> yggB primer-C

<400> 60
ttccttgctt gcggggatcc catcggggat g 31


<210> 61
<211> 2432
<212> DNA
<213> Corynebacterium glutamicum

<220>
<221> CDS
<222> (507)..(2093)

<400> 61
aggggcggcg gatcgaccac ggcttgcaac cgtggcggga gtgggctgtt gagaagctgc 60
cacattcacg actttctggc tcctttacta aataaggatt ttcacaggac ccgtccaagc 120
caagccgatt tcaactcagc ctaaagacaa agccctcatt taaaattgtt ccgacgcgga 180
tgcgtgtgca cgcagtgcga cagatgtctg ttgcaaagtt ggctacttgg gtcataacca 240
acaagaaagc cctcgttcca acactgtggt gagtgttgtc gagggcgctt gacgagacga 300
cttggaaggc cgttacggca ggcgccgcgc ggttactact acaagtcgaa taatggtcat 360
ggtgtgtcat gctacacaca tcgagtttcc aattccacaa cgcacgaaaa ttcccacccc 420
caaaactccc ccacttcggt taaggaatca ggattctcac aaagttcagg caggctcccg 480
ctacttttca gcgctaatct tggctc atg att tta ggc gta ccc att caa tat 533
Met Ile Leu Gly Val Pro Ile Gln Tyr
1               5
ttg ctc tat tca ttg tgg aat tgg att gtc gat acc ggt ttt gat gta 581
Leu Leu Tyr Ser Leu Trp Asn Trp Ile Val Asp Thr Gly Phe Asp Val
10                  15                  20                  25
gca att atc ctg gtc ttg gcg ttt ttg att cca cgt atc ggc cga ctg 629
Ala Ile Ile Leu Val Leu Ala Phe Leu Ile Pro Arg Ile Gly Arg Leu
                30                  35                  40
gcc atg cgt att atc aag cgg cga gtg gag tct gca gcc gat gcg gac 677
Ala Met Arg Ile Ile Lys Arg Arg Val Glu Ser Ala Ala Asp Ala Asp
            45                  50                  55
acc act aag aac cag ctc gcg ttc gct ggc gtt ggc gtt tat atc gcg 725
Thr Thr Lys Asn Gln Leu Ala Phe Ala Gly Val Gly Val Tyr Ile Ala
        60                  65                  70
caa att gtg gcg ttt ttc atg ctt gcc gtc tcc gcg atg cag gct ttt 773
Gln Ile Val Ala Phe Phe Met Leu Ala Val Ser Ala Met Gln Ala Phe
    75                  80                  85
ggt ttc tct ctc gcg ggc gct gcg att ccg gca acc att gcg tca gct 821
Gly Phe Ser Leu Ala Gly Ala Ala Ile Pro Ala Thr Ile Ala Ser Ala

90                  95                  100                 105
gcc att ggc ctt ggt gcg cag tcg att gtt gcg gac ttc ttg gcc gga      869
Ala Ile Gly Leu Gly Ala Gln Ser Ile Val Ala Asp Phe Leu Ala Gly
                110                 115                 120
ttt ttc atc ctg acg gaa aag caa ttc ggc gtg ggt gac tgg gtg cgt      917
Phe Phe Ile Leu Thr Glu Lys Gln Phe Gly Val Gly Asp Trp Val Arg
            125                 130                 135
ttt gag ggc aac ggc atc gtt gtc gaa ggc acc gtc att gag atc acc      965
Phe Glu Gly Asn Gly Ile Val Val Glu Gly Thr Val Ile Glu Ile Thr
        140                 145                 150
atg cgc gcg acc aaa att cgc acg att gca caa gag acc gtg atc atc     1013
Met Arg Ala Thr Lys Ile Arg Thr Ile Ala Gln Glu Thr Val Ile Ile
    155                 160                 165
ccc aac tcc acg gcg aaa gtg tgc atc aac aat tct aat aac tgg tcg     1061
Pro Asn Ser Thr Ala Lys Val Cys Ile Asn Asn Ser Asn Asn Trp Ser
170                 175                 180                 185
cgt gcg gtt gtc gtt att ccg atc ccc atg ttg ggt tct gaa aac atc     1109
Arg Ala Val Val Val Ile Pro Ile Pro Met Leu Gly Ser Glu Asn Ile
                190                 195                 200
aca gat gtc atc gcg cgc tct gaa gct gcg act cgt cgc gca ctt ggc     1157
Thr Asp Val Ile Ala Arg Ser Glu Ala Ala Thr Arg Arg Ala Leu Gly
            205                 210                 215
cag gag aaa atc gca ccg gaa atc ctc ggt gaa ctc gat gtg cac cca     1205
Gln Glu Lys Ile Ala Pro Glu Ile Leu Gly Glu Leu Asp Val His Pro
        220                 225                 230
gcc acg gaa gtc aca ccg cca acg gtg gtc ggc atg ccg tgg atg gtc     1253
Ala Thr Glu Val Thr Pro Pro Thr Val Val Gly Met Pro Trp Met Val
    235                 240                 245
acc atg cgt ttc ctc gtg caa gtc acc gcc ggc aat caa tgg ctg gtc     1301
Thr Met Arg Phe Leu Val Gln Val Thr Ala Gly Asn Gln Trp Leu Val
250                 255                 260                 265
gaa cgc gcc atc cgc aca gaa atc atc aac gaa ttc tgg gaa gaa tac     1349
Glu Arg Ala Ile Arg Thr Glu Ile Ile Asn Glu Phe Trp Glu Glu Tyr
                270                 275                 280
ggc agc gca acc act aca tcg gga acc ctc att gat tcc tta cac gtt     1397
Gly Ser Ala Thr Thr Thr Ser Gly Thr Leu Ile Asp Ser Leu His Val
            285                 290                 295
gag cat gaa gag cca aag acc tcg ctt atc gac gcc tcc ccc cag gct     1445
Glu His Glu Glu Pro Lys Thr Ser Leu Ile Asp Ala Ser Pro Gln Ala
        300                 305                 310
ctt aag gaa ccg aag ccg gag gct gcg gcg acg gtt gca tcg cta gct     1493
Leu Lys Glu Pro Lys Pro Glu Ala Ala Ala Thr Val Ala Ser Leu Ala
    315                 320                 325
gca tcg tct aac gac gat gca gac aat gca gac gcc tcg gcg atc aat     1541
Ala Ser Ser Asn Asp Asp Ala Asp Asn Ala Asp Ala Ser Ala Ile Asn
330                 335                 340                 345
gca ggc aat cca gag aag gaa ctt gat tcc gat gtg ctg gaa caa gaa     1589
Ala Gly Asn Pro Glu Lys Glu Leu Asp Ser Asp Val Leu Glu Gln Glu
                350                 355                 360
ctc tcc agc gaa gaa ccg gaa gaa aca gca aaa cca gat cac tct ctc     1637
Leu Ser Ser Glu Glu Pro Glu Glu Thr Ala Lys Pro Asp His Ser Leu
            365                 370                 375
cga ggc ttc ttc cgc act gat tac tac cca aat cgg tgg cag aag atc     1685
Arg Gly Phe Phe Arg Thr Asp Tyr Tyr Pro Asn Arg Trp Gln Lys Ile
        380                 385                 390
ctg tcg ttt ggc gga cgt gtc cgc atg agc act tcc ctg ttg ttg ggt     1733
Leu Ser Phe Gly Gly Arg Val Arg Met Ser Thr Ser Leu Leu Leu Gly
    395                 400                 405
gcg ctg ctc ttg ctg tca cta ttt aag gtc atg act gtg gaa cca agt     1781
Ala Leu Leu Leu Leu Ser Leu Phe Lys Val Met Thr Val Glu Pro Ser
410                 415                 420                 425
gag aat tgg caa aac tcc agt gga tgg ctg tca cca agc act gcc acc     1829
Glu Asn Trp Gln Asn Ser Ser Gly Trp Leu Ser Pro Ser Thr Ala Thr

                430                 435                 440
tca act gcg gtg acc acc tcc gaa act tcc gcg cca gta agc act tct 1877
Ser Thr Ala Val Thr Thr Ser Glu Thr Ser Ala Pro Val Ser Thr Ser
            445                 450                 455
tcg atg aca gtg ccc act acg gtg gag gag acc cca acg atg gaa tct 1925
Ser Met Thr Val Pro Thr Thr Val Glu Glu Thr Pro Thr Met Glu Ser
        460                 465                 470
agc gtc gaa acg cag cag gaa acc tca acc cct gca acc gca acg ccc 1973
Ser Val Glu Thr Gln Gln Glu Thr Ser Thr Pro Ala Thr Ala Thr Pro
    475                 480                 485
cag cga gcc gac acc atc gaa ccg acc gag gaa gcc acg tcg cag gag 2021
Gln Arg Ala Asp Thr Ile Glu Pro Thr Glu Glu Ala Thr Ser Gln Glu
490                 495                 500                 505
gaa acg act gcg tcg cag acg cag tct cca gca acc gcg gtt caa gag 2069
Glu Thr Thr Ala Ser Gln Thr Gln Ser Pro Ala Thr Ala Val Gln Glu
                510                 515                 520
aca gtt gcg ccg acg tcc acc cct taggacgctg attacagacg tgtcccattt 2123
Thr Val Ala Pro Thr Ser Thr Pro
            525
ctttactact attggaaatt atgagttcag acgcagaaaa ggcatccgtg gagctttccg 2183
aaaaatttca cccagaacgc acccatattt tgggcgccgt tgtttttggc ctgatctcat 2243
tattagtcat cggcgcagcc cctcagtacc tgttttggct gctcgcgctc cctgtcatct 2303
tcggttactg ggttctaaaa tcatccacga tcgttgatga acagggcatc accgcaaact 2363
acgccttcaa gggcaaaaag gttgtggcct gggaagacct cgcaggaatc ggattcaagg 2423
gtgcccgca 2432

<210> 62
<211> 529
<212> PRT
<213> Corynebacterium glutamicum

<400> 62
Met Ile Leu Gly Val Pro Ile Gln Tyr Leu Leu Tyr Ser Leu Trp Asn
1               5                   10                  15
Trp Ile Val Asp Thr Gly Phe Asp Val Ala Ile Ile Leu Val Leu Ala
            20                  25                  30
Phe Leu Ile Pro Arg Ile Gly Arg Leu Ala Met Arg Ile Ile Lys Arg
        35                  40                  45
Arg Val Glu Ser Ala Ala Asp Ala Asp Thr Thr Lys Asn Gln Leu Ala
    50                  55                  60
Phe Ala Gly Val Gly Val Tyr Ile Ala Gln Ile Val Ala Phe Phe Met
65                  70                  75                  80
Leu Ala Val Ser Ala Met Gln Ala Phe Gly Phe Ser Leu Ala Gly Ala
                85                  90                  95
Ala Ile Pro Ala Thr Ile Ala Ser Ala Ala Ile Gly Leu Gly Ala Gln
            100                 105                 110
Ser Ile Val Ala Asp Phe Leu Ala Gly Phe Phe Ile Leu Thr Glu Lys
        115                 120                 125
Gln Phe Gly Val Gly Asp Trp Val Arg Phe Glu Gly Asn Gly Ile Val
    130                 135                 140
Val Glu Gly Thr Val Ile Glu Ile Thr Met Arg Ala Thr Lys Ile Arg
145                 150                 155                 160
Thr Ile Ala Gln Glu Thr Val Ile Ile Pro Asn Ser Thr Ala Lys Val
                165                 170                 175
Cys Ile Asn Asn Ser Asn Asn Trp Ser Arg Ala Val Val Val Ile Pro
            180                 185                 190
Ile Pro Met Leu Gly Ser Glu Asn Ile Thr Asp Val Ile Ala Arg Ser
        195                 200                 205
Glu Ala Ala Thr Arg Arg Ala Leu Gly Gln Glu Lys Ile Ala Pro Glu
    210                 215                 220
Ile Leu Gly Glu Leu Asp Val His Pro Ala Thr Glu Val Thr Pro Pro
225                 230                 235                 240

Thr Val Val Gly Met Pro Trp Met Val Thr Met Arg Phe Leu Val Gln
                245                 250                 255
Val Thr Ala Gly Asn Gln Trp Leu Val Glu Arg Ala Ile Arg Thr Glu
            260                 265                 270
Ile Ile Asn Glu Phe Trp Glu Glu Tyr Gly Ser Ala Thr Thr Thr Ser
        275                 280                 285
Gly Thr Leu Ile Asp Ser Leu His Val Glu His Glu Glu Pro Lys Thr
    290                 295                 300
Ser Leu Ile Asp Ala Ser Pro Gln Ala Leu Lys Glu Pro Lys Pro Glu
305                 310                 315                 320
Ala Ala Ala Thr Val Ala Ser Leu Ala Ala Ser Ser Asn Asp Asp Ala
                325                 330                 335
Asp Asn Ala Asp Ala Ser Ala Ile Asn Ala Gly Asn Pro Glu Lys Glu
            340                 345                 350
Leu Asp Ser Asp Val Leu Glu Gln Glu Leu Ser Ser Glu Glu Pro Glu
        355                 360                 365
Glu Thr Ala Lys Pro Asp His Ser Leu Arg Gly Phe Phe Arg Thr Asp
    370                 375                 380
Tyr Tyr Pro Asn Arg Trp Gln Lys Ile Leu Ser Phe Gly Gly Arg Val
385                 390                 395                 400
Arg Met Ser Thr Ser Leu Leu Leu Gly Ala Leu Leu Leu Leu Ser Leu
                405                 410                 415
Phe Lys Val Met Thr Val Glu Pro Ser Glu Asn Trp Gln Asn Ser Ser
            420                 425                 430
Gly Trp Leu Ser Pro Ser Thr Ala Thr Ser Thr Ala Val Thr Thr Ser
        435                 440                 445
Glu Thr Ser Ala Pro Val Ser Thr Ser Ser Met Thr Val Pro Thr Thr
    450                 455                 460
Val Glu Glu Thr Pro Thr Met Glu Ser Ser Val Glu Thr Gln Gln Glu
465                 470                 475                 480
Thr Ser Thr Pro Ala Thr Ala Thr Pro Gln Arg Ala Asp Thr Ile Glu
                485                 490                 495
Pro Thr Glu Glu Ala Thr Ser Gln Glu Glu Thr Thr Ala Ser Gln Thr
            500                 505                 510
Gln Ser Pro Ala Thr Ala Val Gln Glu Thr Val Ala Pro Thr Ser Thr
        515                 520                 525
Pro

<210>  63
<211>  2432
<212>  DNA
<213>  Corynebacterium glutamicum

<220>
<221>  CDS
<222>  (507)..(2093)

<400>  63
aggggcggcg gatcgaccac ggcttgcaac cgtggcggga gtgggctgtt gagaagctgc      60
cacattcacg actttctggc tcctttacta aataaggatt ttcacaggac ccgtccaagc     120
caagccgatt tcaactcagc ctaaagacaa agccctcatt taaaattgtt ccgacgcgga     180
tgcgtgtgca cgcagtgcga cagatgtctg ttgcaaagtt ggctacttgg gtcataacca     240
acaagaaagc cctcgttcca acactgtggt gagtgttgtc gagggcgctt gacgagacga     300
cttggaaggc cgttacggca ggcgccgcgc ggttactact acaagtcgaa taatggtcat     360
ggtgtgtcat gctacacaca tcgagtttcc aattccacaa cgcacgaaaa ttcccacccc     420
caaaactccc ccacttcggt taaggaatca ggattctcac aaagttcagg caggctcccg     480
ctacttttca gcgctaatct tggctc atg att tta ggc gta ccc att caa tat      533
                             Met Ile Leu Gly Val Pro Ile Gln Tyr
                             1               5
ttg ctc tat tca ttg tgg aat tgg att gtc gat acc ggt ttt gat gta      581
Leu Leu Tyr Ser Leu Trp Asn Trp Ile Val Asp Thr Gly Phe Asp Val
10                  15                  20                  25

gca att atc ctg gtc ttg gcg ttt ttg att cca cgt atc ggc cga ctg 629
Ala Ile Ile Leu Val Leu Ala Phe Leu Ile Pro Arg Ile Gly Arg Leu
                30                  35                  40
gcc atg cgt att atc aag cgg cga gtg gag tct gca gcc gat gcg gac 677
Ala Met Arg Ile Ile Lys Arg Arg Val Glu Ser Ala Ala Asp Ala Asp
            45                  50                  55
acc act aag aac cag ctc gcg ttc gct ggc gtt ggc gtt tat atc gcg 725
Thr Thr Lys Asn Gln Leu Ala Phe Ala Gly Val Gly Val Tyr Ile Ala
        60                  65                  70
caa att gtg gcg ttt ttc atg ctt gcc gtc tcc gcg atg cag gct ttt 773
Gln Ile Val Ala Phe Phe Met Leu Ala Val Ser Ala Met Gln Ala Phe
    75                  80                  85
ggt ttc tct ctc gcg ggc gct gcg att ccg gca acc att gcg tca gct 821
Gly Phe Ser Leu Ala Gly Ala Ala Ile Pro Ala Thr Ile Ala Ser Ala
90                  95                  100                 105
gcc att ggc ctt ggt acg cag tcg att gtt gcg gac ttc ttg gcc gga 869
Ala Ile Gly Leu Gly Thr Gln Ser Ile Val Ala Asp Phe Leu Ala Gly
                110                 115                 120
ttt ttc atc ctg acg gaa aag caa ttc ggc gtg ggt gac tgg gtg cgt 917
Phe Phe Ile Leu Thr Glu Lys Gln Phe Gly Val Gly Asp Trp Val Arg
            125                 130                 135
ttt gag ggc aac ggc atc gtt gtc gaa ggc acc gtc att gag atc acc 965
Phe Glu Gly Asn Gly Ile Val Val Glu Gly Thr Val Ile Glu Ile Thr
        140                 145                 150
atg cgc gcg acc aaa att cgc acg att gca caa gag acc gtg atc atc 1013
Met Arg Ala Thr Lys Ile Arg Thr Ile Ala Gln Glu Thr Val Ile Ile
    155                 160                 165
ccc aac tcc acg gcg aaa gtg tgc atc aac aat tct aat aac tgg tcg 1061
Pro Asn Ser Thr Ala Lys Val Cys Ile Asn Asn Ser Asn Asn Trp Ser
170                 175                 180                 185
cgt gcg gtt gtc gtt att ccg atc ccc atg ttg ggt tct gaa aac atc 1109
Arg Ala Val Val Val Ile Pro Ile Pro Met Leu Gly Ser Glu Asn Ile
                190                 195                 200
aca gat gtc atc gcg cgc tct gaa gct gcg act cgt cgc gca ctt ggc 1157
Thr Asp Val Ile Ala Arg Ser Glu Ala Ala Thr Arg Arg Ala Leu Gly
            205                 210                 215
cag gag aaa atc gca ccg gaa atc ctc ggt gaa ctc gat gtg cac cca 1205
Gln Glu Lys Ile Ala Pro Glu Ile Leu Gly Glu Leu Asp Val His Pro
        220                 225                 230
gcc acg gaa gtc aca ccg cca acg gtg gtc ggc atg ccg tgg atg gtc 1253
Ala Thr Glu Val Thr Pro Pro Thr Val Val Gly Met Pro Trp Met Val
    235                 240                 245
acc atg cgt ttc ctc gtg caa gtc acc gcc ggc aat caa tgg ctg gtc 1301
Thr Met Arg Phe Leu Val Gln Val Thr Ala Gly Asn Gln Trp Leu Val
250                 255                 260                 265
gaa cgc gcc atc cgc aca gaa atc atc aac gaa ttc tgg gaa gaa tac 1349
Glu Arg Ala Ile Arg Thr Glu Ile Ile Asn Glu Phe Trp Glu Glu Tyr
                270                 275                 280
ggc agc gca acc act aca tcg gga acc ctc att gat tcc tta cac gtt 1397
Gly Ser Ala Thr Thr Thr Ser Gly Thr Leu Ile Asp Ser Leu His Val
            285                 290                 295
gag cat gaa gag cca aag acc tcg ctt atc gac gcc tcc ccc cag gct 1445
Glu His Glu Glu Pro Lys Thr Ser Leu Ile Asp Ala Ser Pro Gln Ala
        300                 305                 310
ctt aag gaa ccg aag ccg gag gct gcg gcg acg gtt gca tcg cta gct 1493
Leu Lys Glu Pro Lys Pro Glu Ala Ala Ala Thr Val Ala Ser Leu Ala
    315                 320                 325
gca tcg tct aac gac gat gca gac aat gca gac gcc tcg gcg atc aat 1541
Ala Ser Ser Asn Asp Asp Ala Asp Asn Ala Asp Ala Ser Ala Ile Asn
330                 335                 340                 345
gca ggc aat cca gag aag gaa ctt gat tcc gat gtg ctg gaa caa gaa 1589
Ala Gly Asn Pro Glu Lys Glu Leu Asp Ser Asp Val Leu Glu Gln Glu
                350                 355                 360

ctc tcc agc gaa gaa ccg gaa gaa aca gca aaa cca gat cac tct ctc 1637
Leu Ser Ser Glu Glu Pro Glu Glu Thr Ala Lys Pro Asp His Ser Leu
            365                 370                 375
cga ggc ttc ttc cgc act gat tac tac cca aat cgg tgg cag aag atc 1685
Arg Gly Phe Phe Arg Thr Asp Tyr Tyr Pro Asn Arg Trp Gln Lys Ile
        380                 385                 390
ctg tcg ttt ggc gga cgt gtc cgc atg agc act tcc ctg ttg ttg ggt 1733
Leu Ser Phe Gly Gly Arg Val Arg Met Ser Thr Ser Leu Leu Leu Gly
    395                 400                 405
gcg ctg ctc ttg ctg tca cta ttt aag gtc atg act gtg gaa cca agt 1781
Ala Leu Leu Leu Leu Ser Leu Phe Lys Val Met Thr Val Glu Pro Ser
410                 415                 420                 425
gag aat tgg caa aac tcc agt gga tgg ctg tca cca agc act gcc acc 1829
Glu Asn Trp Gln Asn Ser Ser Gly Trp Leu Ser Pro Ser Thr Ala Thr
                430                 435                 440
tca act gcg gtg acc acc tcc gaa act tcc gcg cca gta agc act tct 1877
Ser Thr Ala Val Thr Thr Ser Glu Thr Ser Ala Pro Val Ser Thr Ser
            445                 450                 455
tcg atg aca gtg ccc act acg gtg gag gag acc cca acg atg gaa tct 1925
Ser Met Thr Val Pro Thr Thr Val Glu Glu Thr Pro Thr Met Glu Ser
        460                 465                 470
agc gtc gaa acg cag cag gaa acc tca acc cct gca acc gca acg ccc 1973
Ser Val Glu Thr Gln Gln Glu Thr Ser Thr Pro Ala Thr Ala Thr Pro
    475                 480                 485
cag cga gcc gac acc atc gaa ccg acc gag gaa gcc acg tcg cag gag 2021
Gln Arg Ala Asp Thr Ile Glu Pro Thr Glu Glu Ala Thr Ser Gln Glu
490                 495                 500                 505
gaa acg act gcg tcg cag acg cag tct cca gca acc gcg gtt caa gag 2069
Glu Thr Thr Ala Ser Gln Thr Gln Ser Pro Ala Thr Ala Val Gln Glu
                510                 515                 520
aca gtt gcg ccg acg tcc acc cct taggacgctg attacagacg tgtcccattt 2123
Thr Val Ala Pro Thr Ser Thr Pro
            525
ctttactact attggaaatt atgagttcag acgcagaaaa ggcatccgtg gagctttccg 2183
aaaaatttca cccagaacgc acccatattt tgggcgccgt tgtttttggc ctgatctcat 2243
tattagtcat cggcgcagcc cctcagtacc tgttttggct gctcgcgctc cctgtcatct 2303
tcggttactg ggttctaaaa tcatccacga tcgttgatga acagggcatc accgcaaact 2363
acgccttcaa gggcaaaaag gttgtggcct gggaagacct cgcaggaatc ggattcaagg 2423
gtgcccgca 2432

<210> 64
<211> 529
<212> PRT
<213> Corynebacterium glutamicum

<400> 64
Met Ile Leu Gly Val Pro Ile Gln Tyr Leu Leu Tyr Ser Leu Trp Asn
1               5                   10                  15
Trp Ile Val Asp Thr Gly Phe Asp Val Ala Ile Ile Leu Val Leu Ala
            20                  25                  30
Phe Leu Ile Pro Arg Ile Gly Arg Leu Ala Met Arg Ile Ile Lys Arg
        35                  40                  45
Arg Val Glu Ser Ala Ala Asp Ala Asp Thr Thr Lys Asn Gln Leu Ala
    50                  55                  60
Phe Ala Gly Val Gly Val Tyr Ile Ala Gln Ile Val Ala Phe Phe Met
65                  70                  75                  80
Leu Ala Val Ser Ala Met Gln Ala Phe Gly Phe Ser Leu Ala Gly Ala
                85                  90                  95
Ala Ile Pro Ala Thr Ile Ala Ser Ala Ala Ile Gly Leu Gly Thr Gln
            100                 105                 110
Ser Ile Val Ala Asp Phe Leu Ala Gly Phe Phe Ile Leu Thr Glu Lys
        115                 120                 125

Gln Phe Gly Val Gly Asp Trp Val Arg Phe Glu Gly Asn Gly Ile Val
    130                 135                 140
Val Glu Gly Thr Val Ile Glu Ile Thr Met Arg Ala Thr Lys Ile Arg
145                 150                 155                 160
Thr Ile Ala Gln Glu Thr Val Ile Ile Pro Asn Ser Thr Ala Lys Val
                165                 170                 175
Cys Ile Asn Asn Ser Asn Asn Trp Ser Arg Ala Val Val Val Ile Pro
            180                 185                 190
Ile Pro Met Leu Gly Ser Glu Asn Ile Thr Asp Val Ile Ala Arg Ser
        195                 200                 205
Glu Ala Ala Thr Arg Arg Ala Leu Gly Gln Glu Lys Ile Ala Pro Glu
    210                 215                 220
Ile Leu Gly Glu Leu Asp Val His Pro Ala Thr Glu Val Thr Pro Pro
225                 230                 235                 240
Thr Val Val Gly Met Pro Trp Met Val Thr Met Arg Phe Leu Val Gln
                245                 250                 255
Val Thr Ala Gly Asn Gln Trp Leu Val Glu Arg Ala Ile Arg Thr Glu
            260                 265                 270
Ile Ile Asn Glu Phe Trp Glu Glu Tyr Gly Ser Ala Thr Thr Thr Ser
        275                 280                 285
Gly Thr Leu Ile Asp Ser Leu His Val Glu His Glu Glu Pro Lys Thr
    290                 295                 300
Ser Leu Ile Asp Ala Ser Pro Gln Ala Leu Lys Glu Pro Lys Pro Glu
305                 310                 315                 320
Ala Ala Ala Thr Val Ala Ser Leu Ala Ala Ser Ser Asn Asp Asp Ala
                325                 330                 335
Asp Asn Ala Asp Ala Ser Ala Ile Asn Ala Gly Asn Pro Glu Lys Glu
            340                 345                 350
Leu Asp Ser Asp Val Leu Glu Gln Glu Leu Ser Ser Glu Glu Pro Glu
        355                 360                 365
Glu Thr Ala Lys Pro Asp His Ser Leu Arg Gly Phe Phe Arg Thr Asp
    370                 375                 380
Tyr Tyr Pro Asn Arg Trp Gln Lys Ile Leu Ser Phe Gly Gly Arg Val
385                 390                 395                 400
Arg Met Ser Thr Ser Leu Leu Leu Gly Ala Leu Leu Leu Leu Ser Leu
                405                 410                 415
Phe Lys Val Met Thr Val Glu Pro Ser Glu Asn Trp Gln Asn Ser Ser
            420                 425                 430
Gly Trp Leu Ser Pro Ser Thr Ala Thr Ser Thr Ala Val Thr Thr Ser
        435                 440                 445
Glu Thr Ser Ala Pro Val Ser Thr Ser Ser Met Thr Val Pro Thr Thr
    450                 455                 460
Val Glu Glu Thr Pro Thr Met Glu Ser Ser Val Glu Thr Gln Gln Glu
465                 470                 475                 480
Thr Ser Thr Pro Ala Thr Ala Thr Pro Gln Arg Ala Asp Thr Ile Glu
                485                 490                 495
Pro Thr Glu Glu Ala Thr Ser Gln Glu Glu Thr Thr Ala Ser Gln Thr
            500                 505                 510
Gln Ser Pro Ala Thr Ala Val Gln Glu Thr Val Ala Pro Thr Ser Thr
        515                 520                 525
Pro

<210>  65
<211>  29
<212>  DNA
<213>  Artificial sequence

<220>
<223>  5'-primer for 8-type mutation

<400>  65
tggccttggt acgcagtcga ttgttgcgg                                    29

<210> 66
<211> 29
<212> DNA
<213> Artificial sequence

<220>
<223> 3'-primer for 8-type mutation

<400> 66
ccgcaacaat cgactgcgta ccaaggcca 29

<210> 67
<211> 2131
<212> DNA
<213> Corynebacterium melassecola

<220>
<221> CDS
<222> (403)..(2001)

<400> 67
caggacccgt ccaagccaag ccgatttcaa ctcagcctaa agacaaagcc ctcatttaaa 60
attgttccga cgcggatgcg tgtgcacgca gtgcgacaga tgtctgttgc aaagttggct 120
acttgggtca taaccaacaa gaaagccctc gttccaacac tgtggtgagt gttgtcgagg 180
gcgcttgacg agacgacttg gaaggccgtt acggcaggcg ccgcgcggtt actactacaa 240
gtcgaataat ggtcatgatg tgtcatgcta cacacatcga gtttccaatt ccacaacgca 300
cgaaaattcc cacccccaaa actcccccac ttcggttaag gaatcaggat tctcacaaag 360
ttcaggcagg ctcccgctac ttttcagcgc taatcttggc tc atg att tta ggc 414
Met Ile Leu Gly
1
gta ccc att caa tat ttg ctc tat tca ttg tgg aat tgg att gtc gat 462
Val Pro Ile Gln Tyr Leu Leu Tyr Ser Leu Trp Asn Trp Ile Val Asp
5                   10                  15                  20
acc ggt ttt gat gta gca att atc ctg gtc ttg gcg ttt ttg att cca 510
Thr Gly Phe Asp Val Ala Ile Ile Leu Val Leu Ala Phe Leu Ile Pro
                25                  30                  35
cgt atc ggc cga ctg gcc atg cgt att atc aag cgc cga gtg gag tct 558
Arg Ile Gly Arg Leu Ala Met Arg Ile Ile Lys Arg Arg Val Glu Ser
            40                  45                  50
gca gcc gat gcg gac acc act aag aac cag ctc gcg ttc gct ggc gtt 606
Ala Ala Asp Ala Asp Thr Thr Lys Asn Gln Leu Ala Phe Ala Gly Val
        55                  60                  65
ggc gtt tat atc gcg caa att gtg gcg ttt ttc atg ctt gcc gtc tcc 654
Gly Val Tyr Ile Ala Gln Ile Val Ala Phe Phe Met Leu Ala Val Ser
    70                  75                  80
gcg atg cag gct ttt ggt ttc tct ctc gcg ggc gct gcg att ccg gca 702
Ala Met Gln Ala Phe Gly Phe Ser Leu Ala Gly Ala Ala Ile Pro Ala
85                  90                  95                  100
acc att gcg tca gct gcc att ggt ctt ggt gcg cag tcg att gtt gcg 750
Thr Ile Ala Ser Ala Ala Ile Gly Leu Gly Ala Gln Ser Ile Val Ala
                105                 110                 115
gac ttc ttg gcc gga ttt ttc atc ctg acg gaa aag caa ttc ggc gtg 798
Asp Phe Leu Ala Gly Phe Phe Ile Leu Thr Glu Lys Gln Phe Gly Val
            120                 125                 130
ggt gac tgg gtg cgt ttt gag ggc aac ggc atc gtt gtt gaa ggc acc 846
Gly Asp Trp Val Arg Phe Glu Gly Asn Gly Ile Val Val Glu Gly Thr
        135                 140                 145
gtc att gag atc acc atg cgc gcg acc aaa att cgc acg att gca caa 894
Val Ile Glu Ile Thr Met Arg Ala Thr Lys Ile Arg Thr Ile Ala Gln
    150                 155                 160
gag acc gtg atc atc ccc aac tcc acg gcg aaa gtg tgc atc aac aat 942

Glu Thr Val Ile Ile Pro Asn Ser Thr Ala Lys Val Cys Ile Asn Asn
165                 170                 175                 180
tct aat aac tgg tcg cgt gcg gtt gtc gtt att ccg atc ccc atg ttg      990
Ser Asn Asn Trp Ser Arg Ala Val Val Val Ile Pro Ile Pro Met Leu
                185                 190                 195
ggt tct gaa aac atc aca gat gtc atc gcg cgc tct gaa gct gcg act      1038
Gly Ser Glu Asn Ile Thr Asp Val Ile Ala Arg Ser Glu Ala Ala Thr
            200                 205                 210
cgt cgc gca ctt ggc cag gag aaa atc gca ccg gaa atc ctc ggt gaa      1086
Arg Arg Ala Leu Gly Gln Glu Lys Ile Ala Pro Glu Ile Leu Gly Glu
        215                 220                 225
ctc gat gtg cac cca gcc acg gaa gtc aca ccg cca acg gtg gtc ggc      1134
Leu Asp Val His Pro Ala Thr Glu Val Thr Pro Pro Thr Val Val Gly
    230                 235                 240
atg ccg tgg atg gtc acc atg cgt ttc ctc gtg caa gtc acc gcc ggc      1182
Met Pro Trp Met Val Thr Met Arg Phe Leu Val Gln Val Thr Ala Gly
245                 250                 255                 260
aat caa tgg ctg gtc gaa cgc gcc atc cgc aca gaa atc atc aac gaa      1230
Asn Gln Trp Leu Val Glu Arg Ala Ile Arg Thr Glu Ile Ile Asn Glu
                265                 270                 275
ttc tgg gaa gaa tac ggc agc gca acc act aca tcg gga acc ctc att      1278
Phe Trp Glu Glu Tyr Gly Ser Ala Thr Thr Thr Ser Gly Thr Leu Ile
            280                 285                 290
gat tcc tta cac gtt gcg cat gaa gag cca aag acc tcg ctt atc gac      1326
Asp Ser Leu His Val Ala His Glu Glu Pro Lys Thr Ser Leu Ile Asp
        295                 300                 305
gcc tcc ccc cag gct ctt aag gaa ccg aag ccg gag gct gcg gcg acg      1374
Ala Ser Pro Gln Ala Leu Lys Glu Pro Lys Pro Glu Ala Ala Ala Thr
    310                 315                 320
gtt gca tcg cta gct gca tcg tct aac gac gat gca gac aat gca gac      1422
Val Ala Ser Leu Ala Ala Ser Ser Asn Asp Asp Ala Asp Asn Ala Asp
325                 330                 335                 340
gcc tcg gcg atc aat gca ggc aat cca gag aag gaa ctt gat tcc gat      1470
Ala Ser Ala Ile Asn Ala Gly Asn Pro Glu Lys Glu Leu Asp Ser Asp
                345                 350                 355
gtg ctg gaa caa gaa ctc tcc agc gaa gaa ccg gaa gaa aca gca aaa      1518
Val Leu Glu Gln Glu Leu Ser Ser Glu Glu Pro Glu Glu Thr Ala Lys
            360                 365                 370
cca gat cac tct ctc cga ggc ttc ttc cgc act gat tac tac cca aat      1566
Pro Asp His Ser Leu Arg Gly Phe Phe Arg Thr Asp Tyr Tyr Pro Asn
        375                 380                 385
cgg tgg cag aag atc ctg tcg atc ggc gga cgt gtc cgc atg agc acg      1614
Arg Trp Gln Lys Ile Leu Ser Ile Gly Gly Arg Val Arg Met Ser Thr
    390                 395                 400
tcc ctg ctg ttg ggt gcg ctc cta ttg ctg tca ctg ttt aag gtc atg      1662
Ser Leu Leu Leu Gly Ala Leu Leu Leu Leu Ser Leu Phe Lys Val Met
405                 410                 415                 420
act gtg gaa cca agt gag aat tgg caa aac tcc agt gga tgg ctg tca      1710
Thr Val Glu Pro Ser Glu Asn Trp Gln Asn Ser Ser Gly Trp Leu Ser
                425                 430                 435
cca ggc act gcc acc tca act gcg gcg acc acc tcc gaa act tcc gcg      1758
Pro Gly Thr Ala Thr Ser Thr Ala Ala Thr Thr Ser Glu Thr Ser Ala
            440                 445                 450
cca gta agc acg cct tcg atg aca gtg ccc act acg gtg gag gag acc      1806
Pro Val Ser Thr Pro Ser Met Thr Val Pro Thr Thr Val Glu Glu Thr
        455                 460                 465
cca acg atg gaa tct agc gtc gaa acg cag cag gaa acc tca acc cct      1854
Pro Thr Met Glu Ser Ser Val Glu Thr Gln Gln Glu Thr Ser Thr Pro
    470                 475                 480
gca acc gca acg ccg cag cga gcc gac acc atc gaa ccg acc gag gaa      1902
Ala Thr Ala Thr Pro Gln Arg Ala Asp Thr Ile Glu Pro Thr Glu Glu
485                 490                 495                 500
gcc acg tcg cag gag gaa acg act gcg tcg cag acg cag tct cca gca      1950

Ala Thr Ser Gln Glu Glu Thr Thr Ala Ser Gln Thr Gln Ser Pro Ala
                505                 510                 515
gtg gaa gca cca acc gcg gtc caa gag aca gtt gcg ccg acg tcc acc      1998
Val Glu Ala Pro Thr Ala Val Gln Glu Thr Val Ala Pro Thr Ser Thr
            520                 525                 530
cct taggacgctg attacagacg tgtcccattt ctttactact attggaaatt           2051
Pro

atgagttcag acgcagaaaa ggcatccgtg gagctttccg aaaaatttca cccagaacgc    2111
acccatattt tgggcgccgt                                                2131


<210>  68
<211>  533
<212>  PRT
<213>  Corynebacterium melassecola

<400>  68
Met Ile Leu Gly Val Pro Ile Gln Tyr Leu Leu Tyr Ser Leu Trp Asn
1               5                   10                  15
Trp Ile Val Asp Thr Gly Phe Asp Val Ala Ile Ile Leu Val Leu Ala
            20                  25                  30
Phe Leu Ile Pro Arg Ile Gly Arg Leu Ala Met Arg Ile Ile Lys Arg
        35                  40                  45
Arg Val Glu Ser Ala Ala Asp Ala Asp Thr Thr Lys Asn Gln Leu Ala
    50                  55                  60
Phe Ala Gly Val Gly Val Tyr Ile Ala Gln Ile Val Ala Phe Phe Met
65                  70                  75                  80
Leu Ala Val Ser Ala Met Gln Ala Phe Gly Phe Ser Leu Ala Gly Ala
                85                  90                  95
Ala Ile Pro Ala Thr Ile Ala Ser Ala Ala Ile Gly Leu Gly Ala Gln
            100                 105                 110
Ser Ile Val Ala Asp Phe Leu Ala Gly Phe Phe Ile Leu Thr Glu Lys
        115                 120                 125
Gln Phe Gly Val Gly Asp Trp Val Arg Phe Glu Gly Asn Gly Ile Val
    130                 135                 140
Val Glu Gly Thr Val Ile Glu Ile Thr Met Arg Ala Thr Lys Ile Arg
145                 150                 155                 160
Thr Ile Ala Gln Glu Thr Val Ile Ile Pro Asn Ser Thr Ala Lys Val
                165                 170                 175
Cys Ile Asn Asn Ser Asn Asn Trp Ser Arg Ala Val Val Val Ile Pro
            180                 185                 190
Ile Pro Met Leu Gly Ser Glu Asn Ile Thr Asp Val Ile Ala Arg Ser
        195                 200                 205
Glu Ala Ala Thr Arg Arg Ala Leu Gly Gln Glu Lys Ile Ala Pro Glu
    210                 215                 220
Ile Leu Gly Glu Leu Asp Val His Pro Ala Thr Glu Val Thr Pro Pro
225                 230                 235                 240
Thr Val Val Gly Met Pro Trp Met Val Thr Met Arg Phe Leu Val Gln
                245                 250                 255
Val Thr Ala Gly Asn Gln Trp Leu Val Glu Arg Ala Ile Arg Thr Glu
            260                 265                 270
Ile Ile Asn Glu Phe Trp Glu Glu Tyr Gly Ser Ala Thr Thr Thr Ser
        275                 280                 285
Gly Thr Leu Ile Asp Ser Leu His Val Ala His Glu Glu Pro Lys Thr
    290                 295                 300
Ser Leu Ile Asp Ala Ser Pro Gln Ala Leu Lys Glu Pro Lys Pro Glu
305                 310                 315                 320
Ala Ala Ala Thr Val Ala Ser Leu Ala Ala Ser Ser Asn Asp Asp Ala
                325                 330                 335
Asp Asn Ala Asp Ala Ser Ala Ile Asn Ala Gly Asn Pro Glu Lys Glu
            340                 345                 350
Leu Asp Ser Asp Val Leu Glu Gln Glu Leu Ser Ser Glu Glu Pro Glu

355                 360                 365
Glu Thr Ala Lys Pro Asp His Ser Leu Arg Gly Phe Phe Arg Thr Asp
    370                 375                 380
Tyr Tyr Pro Asn Arg Trp Gln Lys Ile Leu Ser Ile Gly Gly Arg Val
385                 390                 395                 400
Arg Met Ser Thr Ser Leu Leu Leu Gly Ala Leu Leu Leu Leu Ser Leu
                405                 410                 415
Phe Lys Val Met Thr Val Glu Pro Ser Glu Asn Trp Gln Asn Ser Ser
            420                 425                 430
Gly Trp Leu Ser Pro Gly Thr Ala Thr Ser Thr Ala Ala Thr Thr Ser
        435                 440                 445
Glu Thr Ser Ala Pro Val Ser Thr Pro Ser Met Thr Val Pro Thr Thr
    450                 455                 460
Val Glu Glu Thr Pro Thr Met Glu Ser Ser Val Glu Thr Gln Gln Glu
465                 470                 475                 480
Thr Ser Thr Pro Ala Thr Ala Thr Pro Gln Arg Ala Asp Thr Ile Glu
                485                 490                 495
Pro Thr Glu Glu Ala Thr Ser Gln Glu Glu Thr Thr Ala Ser Gln Thr
            500                 505                 510
Gln Ser Pro Ala Val Glu Ala Pro Thr Ala Val Gln Glu Thr Val Ala
        515                 520                 525
Pro Thr Ser Thr Pro
    530

<210>  69
<211>  2131
<212>  DNA
<213>  Corynebacterium melassecola

<220>
<221>  CDS
<222>  (403)..(2001)

<400>  69
caggacccgt ccaagccaag ccgatttcaa ctcagcctaa agacaaagcc ctcatttaaa      60
attgttccga cgcggatgcg tgtgcacgca gtgcgacaga tgtctgttgc aaagttggct     120
acttgggtca taaccaacaa gaaagccctc gttccaacac tgtggtgagt gttgtcgagg     180
gcgcttgacg agacgacttg gaaggccgtt acggcaggcg ccgcgcggtt actactacaa     240
gtcgaataat ggtcatgatg tgtcatgcta cacacatcga gtttccaatt ccacaacgca     300
cgaaaattcc cacccccaaa actcccccac ttcggttaag gaatcaggat tctcacaaag     360
ttcaggcagg ctcccgctac ttttcagcgc taatcttggc tc atg att tta ggc        414
                                               Met Ile Leu Gly
                                               1
gta ccc att caa tat ttg ctc tat tca ttg tgg aat tgg att gtc gat       462
Val Pro Ile Gln Tyr Leu Leu Tyr Ser Leu Trp Asn Trp Ile Val Asp
5                   10                  15                  20
acc ggt ttt gat gta gca att atc ctg gtc ttg gcg ttt ttg att cca       510
Thr Gly Phe Asp Val Ala Ile Ile Leu Val Leu Ala Phe Leu Ile Pro
                25                  30                  35
cgt atc ggc cga ctg gcc atg cgt att atc aag cgc cga gtg gag tct       558
Arg Ile Gly Arg Leu Ala Met Arg Ile Ile Lys Arg Arg Val Glu Ser
            40                  45                  50
gca gcc gat gcg gac acc act aag aac cag ctc gcg ttc gct ggc gtt       606
Ala Ala Asp Ala Asp Thr Thr Lys Asn Gln Leu Ala Phe Ala Gly Val
        55                  60                  65
ggc gtt tat atc gcg caa att gtg gcg ttt ttc atg ctt gcc gtc tcc       654
Gly Val Tyr Ile Ala Gln Ile Val Ala Phe Phe Met Leu Ala Val Ser
    70                  75                  80
gcg atg cag gct ttt ggt ttc tct ctc gcg ggc gct gcg att ccg gca       702
Ala Met Gln Ala Phe Gly Phe Ser Leu Ala Gly Ala Ala Ile Pro Ala
85                  90                  95                  100
acc att gcg tca gct gcc att ggt ctt ggt gcg cag tcg att gtt gcg       750

Thr Ile Ala Ser Ala Ala Ile Gly Leu Gly Ala Gln Ser Ile Val Ala
                105                 110                 115
gac ttc ttg gcc gga ttt ttc atc ctg acg gaa aag caa ttc ggc gtg 798
Asp Phe Leu Ala Gly Phe Phe Ile Leu Thr Glu Lys Gln Phe Gly Val
            120                 125                 130
ggt gac tgg gtg cgt ttt gag ggc aac ggc atc gtt gtt gaa ggc acc 846
Gly Asp Trp Val Arg Phe Glu Gly Asn Gly Ile Val Val Glu Gly Thr
        135                 140                 145
gtc att gag atc acc atg cgc gcg acc aaa att cgc acg att gca caa 894
Val Ile Glu Ile Thr Met Arg Ala Thr Lys Ile Arg Thr Ile Ala Gln
    150                 155                 160
gag acc gtg atc atc ccc aac tcc acg gcg aaa gtg tgc atc aac aat 942
Glu Thr Val Ile Ile Pro Asn Ser Thr Ala Lys Val Cys Ile Asn Asn
165                 170                 175                 180
tct aat aac tgg tcg cgt gcg gtt gtc gtt att ccg atc ccc atg ttg 990
Ser Asn Asn Trp Ser Arg Ala Val Val Val Ile Pro Ile Pro Met Leu
                185                 190                 195
ggt tct gaa aac atc aca gat gtc atc gcg cgc tct gaa gct gcg act 1038
Gly Ser Glu Asn Ile Thr Asp Val Ile Ala Arg Ser Glu Ala Ala Thr
            200                 205                 210
cgt cgc gca ctt ggc cag gag aaa atc gca ccg gaa atc ctc ggt gaa 1086
Arg Arg Ala Leu Gly Gln Glu Lys Ile Ala Pro Glu Ile Leu Gly Glu
        215                 220                 225
ctc gat gtg cac cca gcc acg gaa gtc aca ccg cca acg gtg gtc ggc 1134
Leu Asp Val His Pro Ala Thr Glu Val Thr Pro Pro Thr Val Val Gly
    230                 235                 240
atg ccg tgg atg gtc acc atg cgt ttc ctc gtg caa gtc acc gcc ggc 1182
Met Pro Trp Met Val Thr Met Arg Phe Leu Val Gln Val Thr Ala Gly
245                 250                 255                 260
aat caa tgg ctg gtc gaa cgc gcc atc cgc aca gaa atc atc aac gaa 1230
Asn Gln Trp Leu Val Glu Arg Ala Ile Arg Thr Glu Ile Ile Asn Glu
                265                 270                 275
ttc tgg gaa gaa tac ggc agc gca acc act aca tcg gga acc ctc att 1278
Phe Trp Glu Glu Tyr Gly Ser Ala Thr Thr Thr Ser Gly Thr Leu Ile
            280                 285                 290
gat tcc tta cac gtt gcg cat gaa gag cca aag acc tcg ctt atc gac 1326
Asp Ser Leu His Val Ala His Glu Glu Pro Lys Thr Ser Leu Ile Asp
        295                 300                 305
gcc tcc ccc cag gct ctt aag gaa ccg aag ccg gag gct gcg gcg acg 1374
Ala Ser Pro Gln Ala Leu Lys Glu Pro Lys Pro Glu Ala Ala Ala Thr
    310                 315                 320
gtt gca tcg cta gct gca tcg tct aac gac gat gca gac aat gca gac 1422
Val Ala Ser Leu Ala Ala Ser Ser Asn Asp Asp Ala Asp Asn Ala Asp
325                 330                 335                 340
gcc tcg gcg atc aat gca ggc aat cca gag aag gaa ctt gat tcc gat 1470
Ala Ser Ala Ile Asn Ala Gly Asn Pro Glu Lys Glu Leu Asp Ser Asp
                345                 350                 355
gtg ctg gaa caa gaa ctc tcc agc gaa gaa ccg gaa gaa aca gca aaa 1518
Val Leu Glu Gln Glu Leu Ser Ser Glu Glu Pro Glu Glu Thr Ala Lys
            360                 365                 370
cca gat cac tct ctc cga ggc ttc ttc cgc act gat tac tac cca aat 1566
Pro Asp His Ser Leu Arg Gly Phe Phe Arg Thr Asp Tyr Tyr Pro Asn
        375                 380                 385
cgg tgg cag aag atc ctg tcg atc ggc gga cgt gtc cgc atg agc acg 1614
Arg Trp Gln Lys Ile Leu Ser Ile Gly Gly Arg Val Arg Met Ser Thr
    390                 395                 400
tcc ctg ctg ttg ggt gcg ctc cta ttg ctg tca ctg ttt aag gtc atg 1662
Ser Leu Leu Leu Gly Ala Leu Leu Leu Leu Ser Leu Phe Lys Val Met
405                 410                 415                 420
act gtg gaa cta agt gag aat tgg caa aac tcc agt gga tgg ctg tca 1710
Thr Val Glu Leu Ser Glu Asn Trp Gln Asn Ser Ser Gly Trp Leu Ser
                425                 430                 435

cca ggc act gcc acc tca act gcg gcg acc acc tcc gaa act tcc gcg     1758
Pro Gly Thr Ala Thr Ser Thr Ala Ala Thr Thr Ser Glu Thr Ser Ala
            440                 445                 450
cca gta agc acg cct tcg atg aca gtg ccc act acg gtg gag gag acc     1806
Pro Val Ser Thr Pro Ser Met Thr Val Pro Thr Thr Val Glu Glu Thr
        455                 460                 465
cca acg atg gaa tct agc gtc gaa acg cag cag gaa acc tca acc cct     1854
Pro Thr Met Glu Ser Ser Val Glu Thr Gln Gln Glu Thr Ser Thr Pro
    470                 475                 480
gca acc gca acg ccg cag cga gcc gac acc atc gaa ccg acc gag gaa     1902
Ala Thr Ala Thr Pro Gln Arg Ala Asp Thr Ile Glu Pro Thr Glu Glu
485                 490                 495                 500
gcc acg tcg cag gag gaa acg act gcg tcg cag acg cag tct cca gca     1950
Ala Thr Ser Gln Glu Glu Thr Thr Ala Ser Gln Thr Gln Ser Pro Ala
                505                 510                 515
gtg gaa gca cca acc gcg gtc caa gag aca gtt gcg ccg acg tcc acc     1998
Val Glu Ala Pro Thr Ala Val Gln Glu Thr Val Ala Pro Thr Ser Thr
            520                 525                 530
cct taggacgctg attacagacg tgtcccattt ctttactact attggaaatt          2051
Pro

atgagttcag acgcagaaaa ggcatccgtg gagctttccg aaaaatttca cccagaacgc   2111
acccatattt tgggcgccgt                                               2131


<210>  70
<211>  533
<212>  PRT
<213>  Corynebacterium melassecola

<400>  70
Met Ile Leu Gly Val Pro Ile Gln Tyr Leu Leu Tyr Ser Leu Trp Asn
1               5                   10                  15
Trp Ile Val Asp Thr Gly Phe Asp Val Ala Ile Ile Leu Val Leu Ala
            20                  25                  30
Phe Leu Ile Pro Arg Ile Gly Arg Leu Ala Met Arg Ile Ile Lys Arg
        35                  40                  45
Arg Val Glu Ser Ala Ala Asp Ala Asp Thr Thr Lys Asn Gln Leu Ala
    50                  55                  60
Phe Ala Gly Val Gly Val Tyr Ile Ala Gln Ile Val Ala Phe Phe Met
65                  70                  75                  80
Leu Ala Val Ser Ala Met Gln Ala Phe Gly Phe Ser Leu Ala Gly Ala
                85                  90                  95
Ala Ile Pro Ala Thr Ile Ala Ser Ala Ala Ile Gly Leu Gly Ala Gln
            100                 105                 110
Ser Ile Val Ala Asp Phe Leu Ala Gly Phe Phe Ile Leu Thr Glu Lys
        115                 120                 125
Gln Phe Gly Val Gly Asp Trp Val Arg Phe Glu Gly Asn Gly Ile Val
    130                 135                 140
Val Glu Gly Thr Val Ile Glu Ile Thr Met Arg Ala Thr Lys Ile Arg
145                 150                 155                 160
Thr Ile Ala Gln Glu Thr Val Ile Ile Pro Asn Ser Thr Ala Lys Val
                165                 170                 175
Cys Ile Asn Asn Ser Asn Asn Trp Ser Arg Ala Val Val Val Ile Pro
            180                 185                 190
Ile Pro Met Leu Gly Ser Glu Asn Ile Thr Asp Val Ile Ala Arg Ser
        195                 200                 205
Glu Ala Ala Thr Arg Arg Ala Leu Gly Gln Glu Lys Ile Ala Pro Glu
    210                 215                 220
Ile Leu Gly Glu Leu Asp Val His Pro Ala Thr Glu Val Thr Pro Pro
225                 230                 235                 240
Thr Val Val Gly Met Pro Trp Met Val Thr Met Arg Phe Leu Val Gln
                245                 250                 255

Val Thr Ala Gly Asn Gln Trp Leu Val Glu Arg Ala Ile Arg Thr Glu
            260                 265                 270
Ile Ile Asn Glu Phe Trp Glu Glu Tyr Gly Ser Ala Thr Thr Thr Ser
        275                 280                 285
Gly Thr Leu Ile Asp Ser Leu His Val Ala His Glu Glu Pro Lys Thr
    290                 295                 300
Ser Leu Ile Asp Ala Ser Pro Gln Ala Leu Lys Glu Pro Lys Pro Glu
305                 310                 315                 320
Ala Ala Ala Thr Val Ala Ser Leu Ala Ala Ser Ser Asn Asp Asp Ala
                325                 330                 335
Asp Asn Ala Asp Ala Ser Ala Ile Asn Ala Gly Asn Pro Glu Lys Glu
            340                 345                 350
Leu Asp Ser Asp Val Leu Glu Gln Glu Leu Ser Ser Glu Glu Pro Glu
        355                 360                 365
Glu Thr Ala Lys Pro Asp His Ser Leu Arg Gly Phe Phe Arg Thr Asp
    370                 375                 380
Tyr Tyr Pro Asn Arg Trp Gln Lys Ile Leu Ser Ile Gly Gly Arg Val
385                 390                 395                 400
Arg Met Ser Thr Ser Leu Leu Leu Gly Ala Leu Leu Leu Leu Ser Leu
                405                 410                 415

Phe Lys Val Met Thr Val Glu Leu Ser Glu Asn Trp Gln Asn Ser Ser
            420                 425                 430
Gly Trp Leu Ser Pro Gly Thr Ala Thr Ser Thr Ala Ala Thr Thr Ser
        435                 440                 445
Glu Thr Ser Ala Pro Val Ser Thr Pro Ser Met Thr Val Pro Thr Thr
    450                 455                 460
Val Glu Glu Thr Pro Thr Met Glu Ser Ser Val Glu Thr Gln Gln Glu
465                 470                 475                 480
Thr Ser Thr Pro Ala Thr Ala Thr Pro Gln Arg Ala Asp Thr Ile Glu
                485                 490                 495
Pro Thr Glu Glu Ala Thr Ser Gln Glu Glu Thr Thr Ala Ser Gln Thr
            500                 505                 510
Gln Ser Pro Ala Val Glu Ala Pro Thr Ala Val Gln Glu Thr Val Ala
        515                 520                 525
Pro Thr Ser Thr Pro
    530

<210>  71
<211>  20
<212>  DNA
<213>  Artificial sequence

<220>
<223>  5'-primer for 66-type mutation

<400>  71
gactgtggaa ctaagtgaga                                                20


<210>  72
<211>  20
<212>  DNA
<213>  Artificial sequence

<220>
<223>  3'-primer for 66-type mutation

<400>  72
tctcacttag ttccacagtc                                                20


<210>  73

<211>  2592
<212>  DNA
<213>  Corynebacterium glutamicum

<220>
<221>  CDS
<222>  (548)..(2146)

<400>  73
tcgagcgatc gaacaattgc ctgtgcacct gtcatccgct caggggcggc ggatcgacca      60
cggcttgcaa ccgtggcggg agtgggctgt tgagaagctg ccacattcac gactttctgg     120
ctcctttact aaataaggat tttcacagga cccgtccaag ccaagccgat ttcaactcag     180
cctaaagaca aagccctcat ttaaaattgt tccgacgcgg atgcgtgtgc acgcagtgcg     240
acagatgtct gttgcaaagt tggctacttg ggtcataacc aacaagaaag ccctcgttcc     300
aacactgtgg tgagtgttgt cgagggcgct tgacgagacg acttggaagg ccgttacggc     360
aggcgccgcg cggttactac tacaagtcga ataatggtca tggtgtgtca tgctacacac     420
atcgagtttc caattccaca acgcacgaaa attcccaccc ccaaaactcc cccacttcgg     480
ttaaggaatc aggattctca caaagttcag gcaggctccc gctacttttc agcgctaatc     540
ttggctc atg att tta ggc gta ccc att caa tat ttg ctc tat tca ttg      589
        Met Ile Leu Gly Val Pro Ile Gln Tyr Leu Leu Tyr Ser Leu
        1               5                   10
tgg aat tgg att gtc gat acc ggt ttt gat gta gca att atc ctg gtc      637
Trp Asn Trp Ile Val Asp Thr Gly Phe Asp Val Ala Ile Ile Leu Val
15                  20                  25                  30
ttg gcg ttt ttg att cca cgt atc ggc cga ctg gcc atg cgt att atc      685
Leu Ala Phe Leu Ile Pro Arg Ile Gly Arg Leu Ala Met Arg Ile Ile
                35                  40                  45
aag cag cga gtg gag tct gca gcc gat gcg gac acc act aag aac cag      733
Lys Gln Arg Val Glu Ser Ala Ala Asp Ala Asp Thr Thr Lys Asn Gln
            50                  55                  60
ctc gcg ttc gct ggc gtt ggc gtt tat atc gcg caa att gtg gcg ttt      781
Leu Ala Phe Ala Gly Val Gly Val Tyr Ile Ala Gln Ile Val Ala Phe
        65                  70                  75
ttc atg ctt gcc gtc tcc gcg atg cag gct ttt ggt ttc tct ctc gcg      829
Phe Met Leu Ala Val Ser Ala Met Gln Ala Phe Gly Phe Ser Leu Ala
    80                  85                  90
ggc gct gcg att ccg gca acc att gcg tca gct gcc att ggt ctt ggt      877
Gly Ala Ala Ile Pro Ala Thr Ile Ala Ser Ala Ala Ile Gly Leu Gly
95                  100                 105                 110
gcg cag tcg att gtt gcg gac ttc ttg gcc gga ttt ttc atc ctg acg      925
Ala Gln Ser Ile Val Ala Asp Phe Leu Ala Gly Phe Phe Ile Leu Thr
                115                 120                 125
gaa aag caa ttc ggc gtg ggt gac tgg gtg cgc ttt gag ggc aac ggc      973
Glu Lys Gln Phe Gly Val Gly Asp Trp Val Arg Phe Glu Gly Asn Gly
            130                 135                 140
atc gtt gtt gaa ggc acc gtc att gag atc acc atg cgc gcg acc aaa     1021
Ile Val Val Glu Gly Thr Val Ile Glu Ile Thr Met Arg Ala Thr Lys
        145                 150                 155
att cgc acg att gca caa gag acc gtg atc atc ccg aac tcc acg gcg     1069
Ile Arg Thr Ile Ala Gln Glu Thr Val Ile Ile Pro Asn Ser Thr Ala
    160                 165                 170
aaa gtg tgc atc aac aat tct aat aac tgg tcg cgt gcg gtt gtc gtt     1117
Lys Val Cys Ile Asn Asn Ser Asn Asn Trp Ser Arg Ala Val Val Val
175                 180                 185                 190
att ccg atc ccc atg ttg ggt tct gaa aac atc aca gat gtc atc gcg     1165
Ile Pro Ile Pro Met Leu Gly Ser Glu Asn Ile Thr Asp Val Ile Ala
                195                 200                 205
cgc tct gaa gct gcg act cgt cgc gca ctt ggc cag gag aaa atc gca     1213
Arg Ser Glu Ala Ala Thr Arg Arg Ala Leu Gly Gln Glu Lys Ile Ala
            210                 215                 220
ccg gaa atc ctc ggt gaa ctc gat gtg cac cca gcc acg gaa gtc aca     1261
Pro Glu Ile Leu Gly Glu Leu Asp Val His Pro Ala Thr Glu Val Thr

225                 230                 235
ccg cca acg gtg gtc ggc atg ccg tgg atg gtc acc atg cgt ttc ctc      1309
Pro Pro Thr Val Val Gly Met Pro Trp Met Val Thr Met Arg Phe Leu
    240                 245                 250
gtg caa gtc acc gcc ggc aat caa tgg ctg gtc gaa cgc gcc atc cgc      1357
Val Gln Val Thr Ala Gly Asn Gln Trp Leu Val Glu Arg Ala Ile Arg
255                 260                 265                 270
aca gaa atc atc aac gaa ttc tgg gaa gaa tac ggc agc gca acc act      1405
Thr Glu Ile Ile Asn Glu Phe Trp Glu Glu Tyr Gly Ser Ala Thr Thr
                275                 280                 285
aca tcg gga acc ctc att gat tcc tta cac gtt gag cat gaa gag cca      1453
Thr Ser Gly Thr Leu Ile Asp Ser Leu His Val Glu His Glu Glu Pro
            290                 295                 300
aag acc tcg ctt atc gac gcc tcc ccc cag gct ctt aag gaa ccg aag      1501
Lys Thr Ser Leu Ile Asp Ala Ser Pro Gln Ala Leu Lys Glu Pro Lys
        305                 310                 315
ccg gag gct gcg gcg acg gtt gca tcg cta gct gca tcg tct aac gac      1549
Pro Glu Ala Ala Ala Thr Val Ala Ser Leu Ala Ala Ser Ser Asn Asp
    320                 325                 330
gat gca gac aat gca gac gcc tcg gcg atc aat gca ggc aat cca gag      1597
Asp Ala Asp Asn Ala Asp Ala Ser Ala Ile Asn Ala Gly Asn Pro Glu
335                 340                 345                 350
aag gaa ctt gat tcc gat gtg ctg gaa caa gaa ctc tcc agc gaa gaa      1645
Lys Glu Leu Asp Ser Asp Val Leu Glu Gln Glu Leu Ser Ser Glu Glu
                355                 360                 365
ccg gaa gaa aca gca aaa cca gat cac tct ctc cga ggc ttc ttc cgc      1693
Pro Glu Glu Thr Ala Lys Pro Asp His Ser Leu Arg Gly Phe Phe Arg
            370                 375                 380
act gat tac tac cca aat cgg tgg cag aag atc ctg tcg ttt ggc gga      1741
Thr Asp Tyr Tyr Pro Asn Arg Trp Gln Lys Ile Leu Ser Phe Gly Gly
        385                 390                 395
cgt gtc cgc atg agc act tcc ctg ttg ttg ggt gcg ctg ctc ttg ctg      1789
Arg Val Arg Met Ser Thr Ser Leu Leu Leu Gly Ala Leu Leu Leu Leu
    400                 405                 410
tca cta ttt aag gtc atg act gtg gaa cca agt gag aat tgg caa aac      1837
Ser Leu Phe Lys Val Met Thr Val Glu Pro Ser Glu Asn Trp Gln Asn
415                 420                 425                 430
tcc agt gga tgg ctg tca tca agc act gcc acc tca act gcg gtg acc      1885
Ser Ser Gly Trp Leu Ser Ser Ser Thr Ala Thr Ser Thr Ala Val Thr
                435                 440                 445
acc tcc gaa act tcc gcg cca gca agc acg cct tcg atg aca gtg ccc      1933
Thr Ser Glu Thr Ser Ala Pro Ala Ser Thr Pro Ser Met Thr Val Pro
            450                 455                 460
act acg gtg gag gag acc cca acg atg gaa tct agc gtc gaa acg cag      1981
Thr Thr Val Glu Glu Thr Pro Thr Met Glu Ser Ser Val Glu Thr Gln
        465                 470                 475
cag gaa acc tca acc cct gca acc gca acg ccc cag cga gcc gac acc      2029
Gln Glu Thr Ser Thr Pro Ala Thr Ala Thr Pro Gln Arg Ala Asp Thr
    480                 485                 490
atc gaa ccg acc gag gaa gcc acg tcg cag gag gaa acg act gca tcg      2077
Ile Glu Pro Thr Glu Glu Ala Thr Ser Gln Glu Glu Thr Thr Ala Ser
495                 500                 505                 510
cag acg cag tct cca gca gtg gaa gca cca acc gcg gtc caa gaa aca      2125
Gln Thr Gln Ser Pro Ala Val Glu Ala Pro Thr Ala Val Gln Glu Thr
                515                 520                 525
gtt gcg ccg acg tcc acc cct taggacgctg attacagacg tgtctcattt      2176
Val Ala Pro Thr Ser Thr Pro
            530
ctttactact attggaaatt atgagttcag acgcagaaaa ggcatccgtg gagctttccg      2236
aaaaatttca cccagaacgc acccatattt tgggcgccgt tgtttttggc ctgatctcat      2296
tattagtcat cggcgcagcc cctcagtacc tgttttggct gctcgcgctc cctgtcatct      2356
tcggttactg ggttctaaaa tcatccacga tcgttgatga acagggcatc accgcaaact      2416
acgccttcaa gggcaaaaag gttgtggcct gggaagacct cgcaggaatc ggattcaagg      2476

gtgcccgcac tttcgctcgc accacctccg atgcagaagt caccctcccc ggcgtcacct    2536
tcaactccct tccccgcctt gaagctgctt cccacggccg catccccgat gcgatc        2592

<210>  74
<211>  533
<212>  PRT
<213>  Corynebacterium glutamicum

<400>  74
Met Ile Leu Gly Val Pro Ile Gln Tyr Leu Leu Tyr Ser Leu Trp Asn
1               5                   10                  15
Trp Ile Val Asp Thr Gly Phe Asp Val Ala Ile Ile Leu Val Leu Ala
            20                  25                  30
Phe Leu Ile Pro Arg Ile Gly Arg Leu Ala Met Arg Ile Ile Lys Gln
        35                  40                  45
Arg Val Glu Ser Ala Ala Asp Ala Asp Thr Thr Lys Asn Gln Leu Ala
    50                  55                  60
Phe Ala Gly Val Gly Val Tyr Ile Ala Gln Ile Val Ala Phe Phe Met
65                  70                  75                  80
Leu Ala Val Ser Ala Met Gln Ala Phe Gly Phe Ser Leu Ala Gly Ala
                85                  90                  95
Ala Ile Pro Ala Thr Ile Ala Ser Ala Ala Ile Gly Leu Gly Ala Gln
            100                 105                 110
Ser Ile Val Ala Asp Phe Leu Ala Gly Phe Phe Ile Leu Thr Glu Lys
        115                 120                 125
Gln Phe Gly Val Gly Asp Trp Val Arg Phe Glu Gly Asn Gly Ile Val
    130                 135                 140
Val Glu Gly Thr Val Ile Glu Ile Thr Met Arg Ala Thr Lys Ile Arg
145                 150                 155                 160
Thr Ile Ala Gln Glu Thr Val Ile Ile Pro Asn Ser Thr Ala Lys Val
                165                 170                 175
Cys Ile Asn Asn Ser Asn Asn Trp Ser Arg Ala Val Val Val Ile Pro
            180                 185                 190
Ile Pro Met Leu Gly Ser Glu Asn Ile Thr Asp Val Ile Ala Arg Ser
        195                 200                 205
Glu Ala Ala Thr Arg Arg Ala Leu Gly Gln Glu Lys Ile Ala Pro Glu
    210                 215                 220
Ile Leu Gly Glu Leu Asp Val His Pro Ala Thr Glu Val Thr Pro Pro
225                 230                 235                 240
Thr Val Val Gly Met Pro Trp Met Val Thr Met Arg Phe Leu Val Gln
                245                 250                 255
Val Thr Ala Gly Asn Gln Trp Leu Val Glu Arg Ala Ile Arg Thr Glu
            260                 265                 270
Ile Ile Asn Glu Phe Trp Glu Glu Tyr Gly Ser Ala Thr Thr Thr Ser
        275                 280                 285
Gly Thr Leu Ile Asp Ser Leu His Val Glu His Glu Glu Pro Lys Thr
    290                 295                 300
Ser Leu Ile Asp Ala Ser Pro Gln Ala Leu Lys Glu Pro Lys Pro Glu
305                 310                 315                 320
Ala Ala Ala Thr Val Ala Ser Leu Ala Ala Ser Ser Asn Asp Asp Ala
                325                 330                 335
Asp Asn Ala Asp Ala Ser Ala Ile Asn Ala Gly Asn Pro Glu Lys Glu
            340                 345                 350
Leu Asp Ser Asp Val Leu Glu Gln Glu Leu Ser Ser Glu Glu Pro Glu
        355                 360                 365
Glu Thr Ala Lys Pro Asp His Ser Leu Arg Gly Phe Phe Arg Thr Asp
    370                 375                 380
Tyr Tyr Pro Asn Arg Trp Gln Lys Ile Leu Ser Phe Gly Gly Arg Val
385                 390                 395                 400
Arg Met Ser Thr Ser Leu Leu Leu Gly Ala Leu Leu Leu Leu Ser Leu
                405                 410                 415
Phe Lys Val Met Thr Val Glu Pro Ser Glu Asn Trp Gln Asn Ser Ser

420                 425                 430
Gly Trp Leu Ser Ser Ser Thr Ala Thr Ser Thr Ala Val Thr Thr Ser
        435                 440                 445
Glu Thr Ser Ala Pro Ala Ser Thr Pro Ser Met Thr Val Pro Thr Thr
    450                 455                 460
Val Glu Glu Thr Pro Thr Met Glu Ser Ser Val Glu Thr Gln Gln Glu
465                 470                 475                 480
Thr Ser Thr Pro Ala Thr Ala Thr Pro Gln Arg Ala Asp Thr Ile Glu
                485                 490                 495
Pro Thr Glu Glu Ala Thr Ser Gln Glu Glu Thr Thr Ala Ser Gln Thr
            500                 505                 510
Gln Ser Pro Ala Val Glu Ala Pro Thr Ala Val Gln Glu Thr Val Ala
        515                 520                 525
Pro Thr Ser Thr Pro
    530

<210>  75
<211>  22
<212>  DNA
<213>  Artificial sequence

<220>
<223>  5'-primer

<400>  75
caattcggcg tgggtgactg gg                                                22

<210>  76
<211>  25
<212>  DNA
<213>  Artificial sequence

<220>
<223>  3'-primer

<400>  76
gtgcggatgg cgcgttcgac cagcc                                             25

<210>  77
<211>  20
<212>  DNA
<213>  Artificial sequence

<220>
<223>  5'-primer for N-terminal fragment of odhA gene

<400>  77
ccaggcactc gtcctcggtt                                                   20

<210>  78
<211>  48
<212>  DNA
<213>  Artificial sequence

<220>
<223>  3'-primer for N-terminal fragment of odhA gene

<400>  78
aggctagtgc aggactataa agaccagttc tcctaaaaat aacgtgtc                    48

<210>  79
<211>  48
<212>  DNA
<213>  Artificial sequence

<220>
<223>  5'-primer for C-terminal fragment of odhA gene

<400>  79
gacacgttat ttttaggaga actggtcttt atagtcctgc actagcct                48


<210>  80
<211>  20
<212>  DNA
<213>  Artificial sequence

<220>
<223>  C'-primer for C-terminal fragment of odhA gene

<400>  80
tccatcgtgg ccaccgatcc                                               20


<210>  81
<211>  25
<212>  DNA
<213>  Artificial sequence

<220>
<223>  5'-primer for odhA disruption

<400>  81
cgggatcccc accggcgtac tcgtg                                         25


<210>  82
<211>  28
<212>  DNA
<213>  Artificial sequence

<220>
<223>  3'-primer for odhA disruption

<400>  82
ccacggatcc ttccaatgct attggttg                                      28


<210>  83
<211>  2102
<212>  DNA
<213>  Corynebacterium glutamicum

<220>
<221>  CDS
<222>  (501)..(2099)

<400>  83
ggcggatcga ccacggcttg caaccgtggc gggagtgggc tgttgagaag ctgccacatt      60
cacgactttc tggctccttt actaaataag gattttcaca ggacccgtcc aagccaagcc     120
gatttcaact cagcctaaag acaaagccct catttaaaat tgttccgacg cggatgcgtg     180

tgcacgcagt gcgacagatg tctgttgcaa agttggctac ttgggtcata accaacaaga 240
aagccctcgt tccaacactg tggtgagtgt tgtcgagggc gcttgacgag acgacttgga 300
aggccgttac ggcaggcgcc gcgcggttac tactacaagt cgaataatgg tcatggtgtg 360
tcatgctaca cacatcgagt ttccaattcc acaacgcacg aaaattccca cccccaaaac 420
tcccccactt cggttaagga atcaggattc tcacaaagtt caggcaggct cccgctactt 480
ttcagcgcta atcttggctc atg att tta ggc gta ccc att caa tat ttg ctc 533
Met Ile Leu Gly Val Pro Ile Gln Tyr Leu Leu
1               5                   10
tat tca ttg tgg aat tgg att gtc gat acc ggt ttt gat gta gca att 581
Tyr Ser Leu Trp Asn Trp Ile Val Asp Thr Gly Phe Asp Val Ala Ile
            15                  20                  25
atc ctg gtc ttg gcg ttt ttg att cca cgt atc ggc cga ctg gcc atg 629
Ile Leu Val Leu Ala Phe Leu Ile Pro Arg Ile Gly Arg Leu Ala Met
        30                  35                  40
cgt att atc aag cgc cga gtg gag tct gca gcc gat gcg gac acc act 677
Arg Ile Ile Lys Arg Arg Val Glu Ser Ala Ala Asp Ala Asp Thr Thr
    45                  50                  55
aag aac cag ctc gcg ttc gcc ggc gtt ggc gtt tat atc gcg caa att 725
Lys Asn Gln Leu Ala Phe Ala Gly Val Gly Val Tyr Ile Ala Gln Ile
60                  65                  70                  75
gtg gcg ttt ttc atg ctt gcc gtc tcc gcg atg cag gct ttt ggt ttc 773
Val Ala Phe Phe Met Leu Ala Val Ser Ala Met Gln Ala Phe Gly Phe
                80                  85                  90
tct ctc gcg ggc gct gcg att ccg gca acc att gcg tca gct gcc att 821
Ser Leu Ala Gly Ala Ala Ile Pro Ala Thr Ile Ala Ser Ala Ala Ile
            95                  100                 105
ggc ctt ggt gcg cag tcg att gtt gcg gac ttc ttg gcc gga ttt ttc 869
Gly Leu Gly Ala Gln Ser Ile Val Ala Asp Phe Leu Ala Gly Phe Phe
        110                 115                 120
atc ctg acg gaa aag caa ttc ggc gtg ggt gac tgg gtg cgt ttt gag 917
Ile Leu Thr Glu Lys Gln Phe Gly Val Gly Asp Trp Val Arg Phe Glu
    125                 130                 135
ggc aac ggc atc gtt gtc gaa ggc acc gtc att gag atc acc atg cgc 965
Gly Asn Gly Ile Val Val Glu Gly Thr Val Ile Glu Ile Thr Met Arg
140                 145                 150                 155
gcg acc aaa att cgc acg att gca caa gag acc gtg atc atc ccc aac 1013
Ala Thr Lys Ile Arg Thr Ile Ala Gln Glu Thr Val Ile Ile Pro Asn
                160                 165                 170
tcc acg gcg aaa gtg tgc atc aac aat tct aat aac tgg tcg cgt gcg 1061
Ser Thr Ala Lys Val Cys Ile Asn Asn Ser Asn Asn Trp Ser Arg Ala
            175                 180                 185
gtt gtc gtt att ccg atc ccc atg ttg ggt tct gaa aac atc aca gat 1109
Val Val Val Ile Pro Ile Pro Met Leu Gly Ser Glu Asn Ile Thr Asp
        190                 195                 200
gtc atc gcg cgc tct gaa gct gcg act cgt cgc gca ctt ggc cag gag 1157
Val Ile Ala Arg Ser Glu Ala Ala Thr Arg Arg Ala Leu Gly Gln Glu
    205                 210                 215
aaa atc gca ccg gaa atc ctc ggt gaa ctc gat gtg cac cca gcc acg 1205
Lys Ile Ala Pro Glu Ile Leu Gly Glu Leu Asp Val His Pro Ala Thr
220                 225                 230                 235
gaa gtc acg ccg cca acg gtg gtc ggc atg ccg tgg atg gtc acc atg 1253
Glu Val Thr Pro Pro Thr Val Val Gly Met Pro Trp Met Val Thr Met
                240                 245                 250
cgt ttc ctc gtg caa gtc acc gcc ggc aat caa tgg ctg gtc gaa cgc 1301
Arg Phe Leu Val Gln Val Thr Ala Gly Asn Gln Trp Leu Val Glu Arg
            255                 260                 265
gcc atc cgc aca gaa atc atc agc gaa ttc tgg gaa gaa tac ggc agc 1349
Ala Ile Arg Thr Glu Ile Ile Ser Glu Phe Trp Glu Glu Tyr Gly Ser
        270                 275                 280
gca acc act aca tcg gga acc ctc att gat tcc tta cac gtt gag cat 1397
Ala Thr Thr Thr Ser Gly Thr Leu Ile Asp Ser Leu His Val Glu His
    285                 290                 295
gaa gag cca aag acc tcg ctt atc gac gcc tcc ccc cag gct ctt aag 1445

Glu Glu Pro Lys Thr Ser Leu Ile Asp Ala Ser Pro Gln Ala Leu Lys
300                 305                 310                 315
gaa ccg aag ccg gag gct gcg gcg acg gtt gca tcg cta gct gca tcc      1493
Glu Pro Lys Pro Glu Ala Ala Ala Thr Val Ala Ser Leu Ala Ala Ser
                320                 325                 330
tct aac gac gat gca gac aat gca gac gcc tcg gtg atc aat gca ggc      1541
Ser Asn Asp Asp Ala Asp Asn Ala Asp Ala Ser Val Ile Asn Ala Gly
            335                 340                 345
aat cca gag aag gaa ctt gat tcc gat gtg ctg gaa caa gaa ctc tcc      1589
Asn Pro Glu Lys Glu Leu Asp Ser Asp Val Leu Glu Gln Glu Leu Ser
        350                 355                 360
agc gaa gaa ccg gaa gaa aca gca aaa cca gat cac tct ctc cga ggc      1637
Ser Glu Glu Pro Glu Glu Thr Ala Lys Pro Asp His Ser Leu Arg Gly
    365                 370                 375
ttc ttc cgc act gat tac tac cca aat cgg tgg cag aag atc ctg tcg      1685
Phe Phe Arg Thr Asp Tyr Tyr Pro Asn Arg Trp Gln Lys Ile Leu Ser
380                 385                 390                 395
ttt ggc gga cgt gtc cgc atg agc acg tcc ctg ttg ttg ggt gcg ctg      1733
Phe Gly Gly Arg Val Arg Met Ser Thr Ser Leu Leu Leu Gly Ala Leu
                400                 405                 410
ctc ttg ctg tca cta ttt aag gtc atg act gtg gaa cca agt gag aat      1781
Leu Leu Leu Ser Leu Phe Lys Val Met Thr Val Glu Pro Ser Glu Asn
            415                 420                 425
tgg caa aac tcc agt gga tgg ctg tca cca agc act gcc acc tca act      1829
Trp Gln Asn Ser Ser Gly Trp Leu Ser Pro Ser Thr Ala Thr Ser Thr
        430                 435                 440
gcg gtg acc acc tcc gaa act tcc gcg cca gta agc acg cct tcg atg      1877
Ala Val Thr Thr Ser Glu Thr Ser Ala Pro Val Ser Thr Pro Ser Met
    445                 450                 455
aca gtg ccc act acg gtg gag gag acc cca acg atg gaa tct aac gtc      1925
Thr Val Pro Thr Thr Val Glu Glu Thr Pro Thr Met Glu Ser Asn Val
460                 465                 470                 475
gaa acg cag cag gaa acc tca acc cct gca acc gca acg ccc cag cga      1973

Glu Thr Gln Gln Glu Thr Ser Thr Pro Ala Thr Ala Thr Pro Gln Arg
                480                 485                 490
gcc gac acc atc gaa ccg acc gag gaa gcc acg tcg cag gag gaa acg      2021
Ala Asp Thr Ile Glu Pro Thr Glu Glu Ala Thr Ser Gln Glu Glu Thr
            495                 500                 505
act gcg tcg cag acg cag tct cca gca gtg gaa gca cca acc gcg gtc      2069
Thr Ala Ser Gln Thr Gln Ser Pro Ala Val Glu Ala Pro Thr Ala Val
        510                 515                 520
caa gag aca gtt gcg ccg acg tcc acc cct tag                          2102
Gln Glu Thr Val Ala Pro Thr Ser Thr Pro
    525                 530


<210>  84
<211>  533
<212>  PRT
<213>  Corynebacterium glutamicum

<400>  84
Met Ile Leu Gly Val Pro Ile Gln Tyr Leu Leu Tyr Ser Leu Trp Asn
1               5                   10                  15
Trp Ile Val Asp Thr Gly Phe Asp Val Ala Ile Ile Leu Val Leu Ala
            20                  25                  30
Phe Leu Ile Pro Arg Ile Gly Arg Leu Ala Met Arg Ile Ile Lys Arg
        35                  40                  45
Arg Val Glu Ser Ala Ala Asp Ala Asp Thr Thr Lys Asn Gln Leu Ala
    50                  55                  60
Phe Ala Gly Val Gly Val Tyr Ile Ala Gln Ile Val Ala Phe Phe Met
65                  70                  75                  80

Leu Ala Val Ser Ala Met Gln Ala Phe Gly Phe Ser Leu Ala Gly Ala
                85                  90                  95
Ala Ile Pro Ala Thr Ile Ala Ser Ala Ala Ile Gly Leu Gly Ala Gln
            100                 105                 110
Ser Ile Val Ala Asp Phe Leu Ala Gly Phe Phe Ile Leu Thr Glu Lys
        115                 120                 125
Gln Phe Gly Val Gly Asp Trp Val Arg Phe Glu Gly Asn Gly Ile Val
    130                 135                 140
Val Glu Gly Thr Val Ile Glu Ile Thr Met Arg Ala Thr Lys Ile Arg
145                 150                 155                 160
Thr Ile Ala Gln Glu Thr Val Ile Ile Pro Asn Ser Thr Ala Lys Val
                165                 170                 175
Cys Ile Asn Asn Ser Asn Asn Trp Ser Arg Ala Val Val Val Ile Pro
            180                 185                 190
Ile Pro Met Leu Gly Ser Glu Asn Ile Thr Asp Val Ile Ala Arg Ser
        195                 200                 205
Glu Ala Ala Thr Arg Arg Ala Leu Gly Gln Glu Lys Ile Ala Pro Glu
    210                 215                 220
Ile Leu Gly Glu Leu Asp Val His Pro Ala Thr Glu Val Thr Pro Pro
225                 230                 235                 240
Thr Val Val Gly Met Pro Trp Met Val Thr Met Arg Phe Leu Val Gln
                245                 250                 255
Val Thr Ala Gly Asn Gln Trp Leu Val Glu Arg Ala Ile Arg Thr Glu
            260                 265                 270
Ile Ile Ser Glu Phe Trp Glu Glu Tyr Gly Ser Ala Thr Thr Thr Ser
        275                 280                 285
Gly Thr Leu Ile Asp Ser Leu His Val Glu His Glu Glu Pro Lys Thr
    290                 295                 300
Ser Leu Ile Asp Ala Ser Pro Gln Ala Leu Lys Glu Pro Lys Pro Glu
305                 310                 315                 320
Ala Ala Ala Thr Val Ala Ser Leu Ala Ala Ser Ser Asn Asp Asp Ala
                325                 330                 335
Asp Asn Ala Asp Ala Ser Val Ile Asn Ala Gly Asn Pro Glu Lys Glu
            340                 345                 350
Leu Asp Ser Asp Val Leu Glu Gln Glu Leu Ser Ser Glu Glu Pro Glu
        355                 360                 365
Glu Thr Ala Lys Pro Asp His Ser Leu Arg Gly Phe Phe Arg Thr Asp
    370                 375                 380
Tyr Tyr Pro Asn Arg Trp Gln Lys Ile Leu Ser Phe Gly Gly Arg Val
385                 390                 395                 400
Arg Met Ser Thr Ser Leu Leu Leu Gly Ala Leu Leu Leu Leu Ser Leu
                405                 410                 415
Phe Lys Val Met Thr Val Glu Pro Ser Glu Asn Trp Gln Asn Ser Ser
            420                 425                 430
Gly Trp Leu Ser Pro Ser Thr Ala Thr Ser Thr Ala Val Thr Thr Ser
        435                 440                 445
Glu Thr Ser Ala Pro Val Ser Thr Pro Ser Met Thr Val Pro Thr Thr
    450                 455                 460
Val Glu Glu Thr Pro Thr Met Glu Ser Asn Val Glu Thr Gln Gln Glu
465                 470                 475                 480
Thr Ser Thr Pro Ala Thr Ala Thr Pro Gln Arg Ala Asp Thr Ile Glu
                485                 490                 495
Pro Thr Glu Glu Ala Thr Ser Gln Glu Glu Thr Thr Ala Ser Gln Thr
            500                 505                 510
Gln Ser Pro Ala Val Glu Ala Pro Thr Ala Val Gln Glu Thr Val Ala
        515                 520                 525
Pro Thr Ser Thr Pro
    530

<210>  85
<211>  533
<212>  PRT
<213>  Corynebacterium glutamicum

<220>
<221>  MISC_FEATURE
<222>  (48), (275), (298), (343), (396), (438), (445), (454), (457),  (474)
(517)..(520)
<223>  Xaa = any amino acid

<400>  85
Met Ile Leu Gly Val Pro Ile Gln Tyr Leu Leu Tyr Ser Leu Trp Asn
1               5                   10                  15
Trp Ile Val Asp Thr Gly Phe Asp Val Ala Ile Ile Leu Val Leu Ala
            20                  25                  30
Phe Leu Ile Pro Arg Ile Gly Arg Leu Ala Met Arg Ile Ile Lys Xaa
        35                  40                  45
Arg Val Glu Ser Ala Ala Asp Ala Asp Thr Thr Lys Asn Gln Leu Ala
    50                  55                  60
Phe Ala Gly Val Gly Val Tyr Ile Ala Gln Ile Val Ala Phe Phe Met
65                  70                  75                  80
Leu Ala Val Ser Ala Met Gln Ala Phe Gly Phe Ser Leu Ala Gly Ala
                85                  90                  95
Ala Ile Pro Ala Thr Ile Ala Ser Ala Ala Ile Gly Leu Gly Ala Gln
            100                 105                 110
Ser Ile Val Ala Asp Phe Leu Ala Gly Phe Phe Ile Leu Thr Glu Lys
        115                 120                 125
Gln Phe Gly Val Gly Asp Trp Val Arg Phe Glu Gly Asn Gly Ile Val
    130                 135                 140
Val Glu Gly Thr Val Ile Glu Ile Thr Met Arg Ala Thr Lys Ile Arg
145                 150                 155                 160
Thr Ile Ala Gln Glu Thr Val Ile Ile Pro Asn Ser Thr Ala Lys Val
                165                 170                 175
Cys Ile Asn Asn Ser Asn Asn Trp Ser Arg Ala Val Val Val Ile Pro
            180                 185                 190
Ile Pro Met Leu Gly Ser Glu Asn Ile Thr Asp Val Ile Ala Arg Ser
        195                 200                 205
Glu Ala Ala Thr Arg Arg Ala Leu Gly Gln Glu Lys Ile Ala Pro Glu
    210                 215                 220
Ile Leu Gly Glu Leu Asp Val His Pro Ala Thr Glu Val Thr Pro Pro
225                 230                 235                 240
Thr Val Val Gly Met Pro Trp Met Val Thr Met Arg Phe Leu Val Gln
                245                 250                 255
Val Thr Ala Gly Asn Gln Trp Leu Val Glu Arg Ala Ile Arg Thr Glu
            260                 265                 270
Ile Ile Xaa Glu Phe Trp Glu Glu Tyr Gly Ser Ala Thr Thr Thr Ser
        275                 280                 285
Gly Thr Leu Ile Asp Ser Leu His Val Xaa His Glu Glu Pro Lys Thr
    290                 295                 300
Ser Leu Ile Asp Ala Ser Pro Gln Ala Leu Lys Glu Pro Lys Pro Glu
305                 310                 315                 320
Ala Ala Ala Thr Val Ala Ser Leu Ala Ala Ser Ser Asn Asp Asp Ala
                325                 330                 335
Asp Asn Ala Asp Ala Ser Xaa Ile Asn Ala Gly Asn Pro Glu Lys Glu
            340                 345                 350
Leu Asp Ser Asp Val Leu Glu Gln Glu Leu Ser Ser Glu Glu Pro Glu
        355                 360                 365
Glu Thr Ala Lys Pro Asp His Ser Leu Arg Gly Phe Phe Arg Thr Asp
    370                 375                 380
Tyr Tyr Pro Asn Arg Trp Gln Lys Ile Leu Ser Xaa Gly Gly Arg Val
385                 390                 395                 400
Arg Met Ser Thr Ser Leu Leu Leu Gly Ala Leu Leu Leu Leu Ser Leu
                405                 410                 415
Phe Lys Val Met Thr Val Glu Pro Ser Glu Asn Trp Gln Asn Ser Ser
            420                 425                 430
Gly Trp Leu Ser Pro Xaa Thr Ala Thr Ser Thr Ala Xaa Thr Thr Ser

435                 440                 445
Glu Thr Ser Ala Pro Xaa Ser Thr Xaa Ser Met Thr Val Pro Thr Thr
    450                 455                 460
Val Glu Glu Thr Pro Thr Met Glu Ser Xaa Val Glu Thr Gln Gln Glu
465                 470                 475                 480
Thr Ser Thr Pro Ala Thr Ala Thr Pro Gln Arg Ala Asp Thr Ile Glu
                485                 490                 495
Pro Thr Glu Glu Ala Thr Ser Gln Glu Glu Thr Thr Ala Ser Gln Thr
            500                 505                 510
Gln Ser Pro Ala Xaa Xaa Xaa Xaa Thr Ala Val Gln Glu Thr Val Ala
        515                 520                 525
Pro Thr Ser Thr Pro
    530

<210>  86
<211>  1550
<212>  DNA
<213>  Corynebacterium glutamicum

<220>
<221>  CDS
<222>  (585)..(1121)

<400>  86
gcattgatgt tgaggtccac gacaatgcag atgcttacat ccgcaatggt ggcgaaactg      60
tcggcatgct ggtttctgaa ggtgcagaga agattgccag cggcagcggc gctgtcatcc     120
cggacgcatc cgcagagttt tccgcacgcg tgttgtctgc cgagtaccgc accaacactc     180
ttaccggcca gcgttttatc cacgcaacag ttgatggcct cttcgctttt gatgtgtgcc     240
ttcctgatgc accagaacta cctgcccgtg acagcgtgtt gtctggcaaa gtcatgctga     300
ctgctgccgt tatccccact gaggtcaccg gctgcggtgg ctccggtggc ggctgtggct     360
caggtagctg tggctgcggc ggacactaaa attctgcaca attttttaag aggaccccag     420
ctccggggtc cttttccatt ttttacccag tacttgaaac gtgatatatg tctattctga     480
aaatcatcgc atagaatact ggtgcaatcg tttacgttcg ctattaatgt gacacgtata     540
agccgacact tttaacgaag cgcagaagga gtgagagcaa gaaa atg gga gaa caa      596
                                                 Met Gly Glu Gln
                                                 1
ctt ccg ttt gct aat ggt tca cgc tcc aac aaa ctg ccg ctc atc gtc      644
Leu Pro Phe Ala Asn Gly Ser Arg Ser Asn Lys Leu Pro Leu Ile Val
5                   10                  15                  20
atc ggt ttg tgc tgc ata atg ctg atc ctg tgg ctt aaa ctt ccc ggc      692
Ile Gly Leu Cys Cys Ile Met Leu Ile Leu Trp Leu Lys Leu Pro Gly
                25                  30                  35
gta ctg ctt gcc acc atc att ggg gtt gcc acg gtg agt gtg atg cgg      740
Val Leu Leu Ala Thr Ile Ile Gly Val Ala Thr Val Ser Val Met Arg
            40                  45                  50
atg cgc acc tcc acc cca gaa act gcc tcg ctg gtt act tct att cgg      788
Met Arg Thr Ser Thr Pro Glu Thr Ala Ser Leu Val Thr Ser Ile Arg
        55                  60                  65
ctg tct gcg gaa gat att tcc gat gtg caa cat gag tgg cag cag ttt      836
Leu Ser Ala Glu Asp Ile Ser Asp Val Gln His Glu Trp Gln Gln Phe
    70                  75                  80
ttg acc tcc ccc gag gcc gat gcg ctg gct gat cgc acg ctt gtc cgt      884
Leu Thr Ser Pro Glu Ala Asp Ala Leu Ala Asp Arg Thr Leu Val Arg
85                  90                  95                  100
ccc gca ctg gcg gat cca gat tgt ggc gat aag gct atc gag aaa ttt      932
Pro Ala Leu Ala Asp Pro Asp Cys Gly Asp Lys Ala Ile Glu Lys Phe
                105                 110                 115
cat tat gaa atc agc aat gcc aat cgc ttc ttg ggc agg ttg gac gct      980
His Tyr Glu Ile Ser Asn Ala Asn Arg Phe Leu Gly Arg Leu Asp Ala
            120                 125                 130
cgt ctg caa caa aac ctc gtg gtc agt gag cta gaa aca ctt ctc aaa     1028
Arg Leu Gln Gln Asn Leu Val Val Ser Glu Leu Glu Thr Leu Leu Lys

135                 140                 145
gta acg gac gag cgc gca cta gag ctg cgg gaa acg tgg ctg gat gcg     1076
Val Thr Asp Glu Arg Ala Leu Glu Leu Arg Glu Thr Trp Leu Asp Ala
    150                 155                 160
cgt aaa gcg gcc cag aaa ctt ggg ccg aac tac aat cgc gaa tct         1121
Arg Lys Ala Ala Gln Lys Leu Gly Pro Asn Tyr Asn Arg Glu Ser
165                 170                 175
tagatctgca cctcaccgag tgcgatgatg gcgcttgggc cggtgagtgt ggagccgtcg   1181
tcaaatatct ggacttctac ttccccacct ggaacgcaca ctttaactgt gccttctccc   1241
aatccagcat cagctaaagc agcacacgct gcagcaacgg ttcccgtgcc acaggagcgg   1301
gtttcgccca ctccgcgttc ccacacgcgc atcgatactg cgtcatcttc taattctgtg   1361
acgatttcta cgttcacacc gtgggggaag aattcctgat caaacgtggg tgcgcgcagt   1421
tccatatcgg caagagccga cgcacttaag cccggcacaa cgcacgctag gtgtgggtta   1481
cccatatcaa cgccaaggcc agcgaatact tggccgttga tgtcgcaggt ggataatccc   1541
gtgacgtca                                                           1550

<210>  87
<211>  179
<212>  PRT
<213>  Corynebacterium glutamicum

<400>  87
Met Gly Glu Gln Leu Pro Phe Ala Asn Gly Ser Arg Ser Asn Lys Leu
1               5                   10                  15
Pro Leu Ile Val Ile Gly Leu Cys Cys Ile Met Leu Ile Leu Trp Leu
            20                  25                  30
Lys Leu Pro Gly Val Leu Leu Ala Thr Ile Ile Gly Val Ala Thr Val
        35                  40                  45
Ser Val Met Arg Met Arg Thr Ser Thr Pro Glu Thr Ala Ser Leu Val
    50                  55                  60
Thr Ser Ile Arg Leu Ser Ala Glu Asp Ile Ser Asp Val Gln His Glu
65                  70                  75                  80
Trp Gln Gln Phe Leu Thr Ser Pro Glu Ala Asp Ala Leu Ala Asp Arg
                85                  90                  95
Thr Leu Val Arg Pro Ala Leu Ala Asp Pro Asp Cys Gly Asp Lys Ala
            100                 105                 110
Ile Glu Lys Phe His Tyr Glu Ile Ser Asn Ala Asn Arg Phe Leu Gly
        115                 120                 125
Arg Leu Asp Ala Arg Leu Gln Gln Asn Leu Val Val Ser Glu Leu Glu
    130                 135                 140
Thr Leu Leu Lys Val Thr Asp Glu Arg Ala Leu Glu Leu Arg Glu Thr
145                 150                 155                 160
Trp Leu Asp Ala Arg Lys Ala Ala Gln Lys Leu Gly Pro Asn Tyr Asn
                165                 170                 175
Arg Glu Ser

<210>  88
<211>  28
<212>  DNA
<213>  Artificial sequence

<220>
<223>  5'-primer

<400>  88
ggagatctgc attgatgttg aggtccac                                        28

<210>  89
<211>  39

<212>  DNA
<213>  Artificial sequence

<220>
<223>  3'-primer

<400>  89
cggtgaggtg cagatctatt ctcccatttt cttgctctc                              39


<210>  90
<211>  39
<212>  DNA
<213>  Artificial sequence

<220>
<223>  5'-primer

<400>  90
gagagcaaga aaatgggaga atagatctgc acctcaccg                              39


<210>  91
<211>  28
<212>  DNA
<213>  Artificial sequence

<220>
<223>  3'-primer

<400>  91
ggagatcttg acgtcacggg attatcca                                          28

## Description

### BACKGROUND OF THE INVENTION

### Field of the Invention

The present invention relates to an L-glutamic acid-producing microorganism and a method for producing L-glutamic acid using the microorganism. L-glutamic acid is widely used in the food industry, for example, as a raw material in the production of seasonings.

### Brief Description of the Related Art

L-glutamic acid has been conventionally produced on an industrial scale by fermentation methods using coryneform bacteria which have L-glutamic acid-producing ability, such as bacteria belonging to the genus *Brevibacterium* or *Corynebacterium.* For this purpose, strains isolated from nature, or artificial mutants thereof, have been used.

Generally, wild-type strains of coryneform bacteria do not produce L-glutamic acid when excess biotin is present. Accordingly, L-glutamic acid production by coryneform bacteria is typically performed under biotin-limited conditions, or a surfactant or penicillin is added to the culture medium (Biosci. Biotech. Biochem., 1997,61(7), p1109-1112). On the other hand, mutant strains that can produce L-glutamic acid in the presence of excess biotin are used for L-glutamic acid production.These strains include a surfactant-temperature-sensitive strain (WO99/02692), a penicillin-sensitive strain (JP-A-55-0124492), and a lysozyme-sensitive strain (WO00/14241). However, such mutant strains may often show a decrease in fatty acid or cell wall synthesis, and there has been some difficulty in producing L-glutamic acid using these strains while maintaining sufficient growth of the strains.

Meanwhile, a genetically modified strain in which a gene encoding α-ketoglutaric acid dehydrogenase (α-KGDH) is disrupted has been used to produce L-glutamic acid in the presence of excess biotin (WO95/34672). However, this α-KGDH gene-deficient strain grows slowly because the TCA cycle is blocked midway, and it is difficult to obtain a sufficient amount of cells required for L-glutamic acid production.

The yggB gene of coryneform bacteria is a homolog of the yggB gene of *Escherichia coli* (FEMS Microbiol Lett. 2003, 218(2), p305-309, Mol Microbiol. 2004, 54(2), p420-438), and has been reported to be a kind of mechanosensitive channel (EMBO J. 1999, 18(7):1730-7.). However, its effect on L-glutamic acid production has not been previously reported.

### SUMMARY OF THE INVENTION

An object of the present invention is to provide a novel method to improve L-glutamic acid-producing ability of coryneform bacterium.

The inventors of the present invention have made extensive studies to achieve this object, and found that the L-glutamic acid-producing ability of coryneform bacterium can be improved by enhancing expression of the wild-type yggB gene. Furthermore, mutant-type yggB genes which improve L-glutamic acid-producing ability of coryneform bacterium in the presence of excess biotin as well as normal L-glutamic acid-producing conditions were obtained, and thereby accomplished the present invention.

It is an object of the present invention to provide a coryneform bacterium having L-glutamic acid-producing ability, wherein said coryneform bacterium is modified by using a yggB gene so that L-glutamic acid-producing ability of the strain is enhanced as compared to a non-modified strain.

It is an object of the present invention to provide the coryneform bacterium as described above, wherein said yggB gene is selected from the group consisting of:
(a) a DNA comprising nucleotides 1437 to 3035 of SEQ ID No: 5,
(b) a DNA that is able to hybridize with a nucleotide sequence complementary to the nucleotides 1437 to 3035 of SEQ ID No: 5 or a probe prepared from the nucleotides under stringent conditions, and wherein said DNA increases L-glutamic acid-producing ability of a coryneform bacterium when it is introduced into the coryneform bacterium,
(c) a DNA comprising nucleotides 507 to 2093 of SEQ ID No: 61,
(d) a DNA that is able to hybridize with a nucleotide sequence complementary to the nucleotides 507 to 2093 of SEQ ID No: 61 or a probe prepared from the nucleotides under stringent conditions, and wherein said DNA increases L-glutamic acid-producing ability of a coryneform bacterium when it is introduced into the coryneform bacterium,
(e) a DNA comprising nucleotides 403 to 2001 of SEQ ID No: 67,
(f) a DNA that is able to hybridize with a nucleotide sequence complementary to the nucleotides 403 to 2001 of SEQ ID No: 67 or a probe prepared from the nucleotides under stringent conditions, and wherein said DNA increases L-glutamic acid-producing ability of a coryneform bacterium when it is introduced into the coryneform bacterium,
(g) a DNA comprising nucleotides 501 to 2099 of SEQ ID No: 83, and
(h) a DNA that is able to hybridize with a nucleotide sequence complementary to the nucleotides 501 to 2099 of SEQ ID No: 83 or a probe prepared from the nucleotides under stringent conditions, and wherein said DNA increases L-glutamic acid-producing ability of a coryneform bacterium when it is introduced into the coryneform bacterium.

It is a further object to provide the coryneform bacterium as described above, wherein said yggB gene encodes a protein selected from the group consisting of:
(A) a protein comprising an amino acid sequence selected from the group consisting of SEQ ID NO: 6, 62, 68, 84 and 85, and
(B) a protein comprising an amino acid sequence selected from the group consisting of SEQ ID NO: 6, 62, 68, 84 and 85, whereby one or several amino acids in said protein are substituted, deleted, inserted, or added, and said yggB gene increases L-glutamic acid-producing ability of a coryneform bacterium when it is introduced into the coryneform bacterium.

It is a further object to provide the coryneform bacterium as described above, wherein said coryneform bacterium is modified so to enhance expression of the yggB gene as compared to a non-modified strain.

It is a further object to provide the coryneform bacterium as described above, wherein expression of the yggB gene is enhanced by increasing a copy number of the yggB gene or modifying an expression regulating sequence of the yggB gene.

It is a further object to provide the coryneform bacterium as described above, wherein said coryneform bacterium is modified by introducing a mutant-type yggB gene.

It is a further object to provide the coryneform bacterium as described above, wherein said mutant-type yggB gene has a mutation in a region encoding amino acids 419-533 of SEQ ID NO: 6, 68, 84 or 85, or amino acids 419-529 of SEQ ID NO: 62.

It is a further object to provide the coryneform bacterium as described above, wherein said mutation is deletion of said region.

It is a further object to provide the coryneform bacterium as described above, wherein said mutation is insertion of an insertion sequence or transposon into the region.

It is a further object to provide the coryneform bacterium as described above, wherein said mutant-type yggB gene has a mutation which results in replacement of the proline in said region with another amino acid.

It is a further object to provide the coryneform bacterium as described above, wherein said mutant-type yggB gene has a mutation which results in replacement of the proline at position 424 and/or the proline at position 437 in the amino acid sequence of SEQ ID NO: 6, 62, 68, 84 or 85 with another amino acid.

It is a further object to provide the coryneform bacterium as described above, wherein said mutant-type yggB gene has a mutation in the transmembrane-coding region of the yggB protein.

It is a further object to provide the coryneform bacterium as described above, wherein said transmembrane-coding region is selected from the group consisting of amino acids 1-23, amino acids 25-47, amino acids 62-84, amino acids 86-108, and amino acids 110-132 of SEQ ID NO: 6, 62, 68, 84 or 85.

It is a further object to provide the coryneform bacterium as described above, wherein said mutation is introduced without causing a frame-shift.

It is a further object to provide the coryneform bacterium as described above, wherein said mutant-type yggB gene has a mutation which results in replacement of the alanine at position 100 and/or the alanine at position 111 in the amino acid sequence shown in SEQ ID NO: 6, 62, 68, 84 or 85 with another amino acid.

It is a further object to provide the coryneform bacterium as described above, wherein said mutant-type yggB gene has a mutation which results in insertion of at least one amino acid between leucine at position 14 and tryptophan at position 15 in SEQ ID NO: 6, 62, 68, 84 or 85.

It is a further object to provide the coryneform bacterium as described above, wherein resistance to L-glutamic acid analogs of said strain is increased by introduction of the mutant-type yggB gene.

It is a further object to provide the coryneform bacterium as described above, wherein said coryneform bacterium is further modified to inactivate a gene which suppresses a function of said mutant-yggB gene.

It is a further object to provide the coryneform bacterium as described above, wherein said gene which suppresses a function of said mutant-yggB gene is a symA gene and wherein said symA gene is selected from the group consisting of:
(a) a DNA comprising nucleotides 585 to 1121 of SEQ ID No: 86,
(b) a DNA that is able to hybridize with a nucleotide sequence complementary to nucleotides 585 to 1121 of SEQ ID No: 86, or a probe prepared from said nucleotides under stringent conditions, and wherein said DNA suppresses a function of said mutant-type yggB gene in the coryneform bacterium.

It is a further object to provide the coryneform bacterium as described above, wherein said coryneform bacterium is further modified so to decrease α-ketoglutarate dehydrogenase activity.

It is a further object to provide the coryneform bacterium as described above, wherein said coryneform bacterium is a bacterium belonging to the genus Corynebacterium or the genus Brevibacterium.

It is an object of the present invention to provide a method for producing L-glutamic acid comprising culturing the coryneform bacterium as described in a medium so to cause accumulation of L-glutamic acid in the medium or cells, and collecting L-glutamic acid from the medium or the cells.

It is an object of the present invention to provide a mutant-type yggB gene selected from the group consisting of:
(a) a gene encoding amino acid sequence of SEQ ID NO: 8,
(b) a gene encoding a protein having a homology of not less than 80% to SEQ ID NO: 8, and having a function to increase L-glutamic acid-producing ability of coryneform bacterium under condition containing excess biotin when it is introduced into the coryneform bacterium,
(c) a gene encoding amino acid sequence of SEQ ID NO: 20,
(d) a gene encoding a protein having a homology of not less than 80% to SEQ ID NO: 20, and having a function to increase L-glutamic acid-producing ability of coryneform bacterium in the presence of excess biotin when said gene is introduced into the coryneform bacterium,
(e) a gene encoding amino acid sequence of SEQ ID NO: 22,
(f) a gene encoding a protein having a homology of not less than 80% to SEQ ID NO: 22, and having a function to increase L-glutamic acid-producing ability of coryneform bacterium in the presence of excess biotin when it said gene introduced into the coryneform bacterium,
(g) a gene encoding amino acid sequence of SEQ ID NO: 24,
(h) a gene encoding a protein having a homology of not less than 80% to SEQ ID NO: 24, and having a function to increase L-glutamic acid-producing ability of coryneform bacterium in the presence of excess biotin when said gene is introduced into the coryneform bacterium,
(i) a gene encoding amino acid sequence of SEQ ID NO: 64,
(j) a gene encoding a protein having a homology of not less than 80% to SEQ ID NO: 64, and having a function to increase L-glutamic acid-producing ability of coryneform bacterium in the presence of excess biotin when said gene is introduced into the coryneform bacterium,
(k) a gene encoding amino acid sequence of SEQ ID NO: 70,
(l) a gene encoding a protein having a homology of not less than 80% to SEQ ID NO: 70, and having a function to increase L-glutamic acid-producing ability of coryneform bacterium in the presence of excess biotin when it is introduced into the coryneform bacterium,
(m) a gene encoding amino acid sequence of SEQ ID NO: 74,
(n) a gene encoding a protein having a homology of not less than 80% to SEQ ID NO: 74, and having a function to increase L-glutamic acid-producing ability of coryneform bacterium in the presence of excess biotin when said gene is introduced into the coryneform bacterium.

It is an object of the present invention to provide a method for producing a coryneform bacterium having a mutant-type yggB gene, comprising inoculating a coryneform bacterium which is deficient in a gene encoding α-ketoglutarate dehydrogenase in a medium containing excess biotin, and selecting a strain that is capable of accumulating L-glutamic acid in the medium as a strain having a mutant-type yggB gene.

It is an object of the present invention to provide a method for producing a coryneform bacterium having a mutant-type yggB gene, comprising inoculating a coryneform bacterium introduced with a yggB gene in which mutation is introduced randomly *in vitro* in a medium containing excess biotin, and selecting a strain that is capable of accumulating L-glutamic acid in the medium as a strain having a mutant-type yggB gene.

It is an object of the present invention to provide a method for producing a coryneform bacterium having a mutant-type yggB gene, comprising inoculating a coryneform bacterium introduced with a transposable element randomly on a chromosome in a medium containing excess biotin, and selecting a strain that is capable of accumulating L-glutamic acid in the medium as a strain having a mutant-type yggB gene.

It is an object of the present invention to provide a method for producing a coryneform bacterium having a mutant-type yggB gene, comprising inoculating a coryneform bacterium into a medium containing L-glutamic acid analogs, and selecting a strain which grows in said medium.

It is an object of the present invention to provide the method for producing a coryneform bacterium as described above, wherein the strain having the mutant-type yggB gene is a coryneform bacterium as as described above.

It is an object of the present invention to provide the method of producing a coryneform bacterium as described above, wherein the medium containing excess biotin is a medium containing 30 µg/l or more of biotin.

### BRIEF DESCRIPTION OF THE DRAWINGS

Fig. 1 shows the procedure for constructing plasmid pBS3.
Fig. 2 shows the procedure for constructing plasmid pBS4S.
Fig. 3 shows the procedure for constructing plasmid pBS4sucAint.
Fig. 4 is a graph showing the accumulation of L-glutamic acid by the mutant-type yggB gene-introduced strain and the control strain.
Fig. 5 shows the 2A-1 type mutation in the yggB gene.
Fig. 6 is a photograph which shows the resistance of the mutant-type yggB gene-introduced strains to 4-fluoroglutamic acid on CM-Dex plate medium.
Fig. 7 shows growth of the control strain and the mutant-type yggB gene-introduced strains in a liquid medium containing 4-fluoroglutamic acid (4-FG).

### DETAILED DESCRIPTION OF EXEMPLARY EMBODIMENTS

Hereinafter, the present invention is explained in more detail.

### <1> Coryneform bacterium of the present invention

The coryneform bacterium of the present invention has L-glutamic acid-producing ability, and is modified using a yggB gene so that the L-glutamic acid-producing ability of the strain is enhanced as compared to a non-modified strain.

In the present invention, examples of coryneform bacterium include conventional coryneform bacteria, and also include bacteria that had been classified into the genus *Brevibacterium,* but are currently classified into the genus *Corynebacterium* (Int. J. Syst. Bacteriol., 41, 255(1991)), as well as the *Brevibacterium* bacteria that are very close to *Corynebacterium* bacteria. Examples of such coryneform bacterium include the following:
*Corynebacterium acetoacidophilum*
*Corynebacterium acetoglutamicum*
*Corynebacterium alkanolyticum*
*Corynebacterium callunae*
*Corynebacterium glutamicum*
*Corynebacterium lilium*
*Corynebacterium melassecola*
*Corynebacterium thermoaminogenes (Corynebacterium efficiens)*
*Corynebacterium herculis*
*Brevibacterium divaricatum*
*Brevibacterium flavum*
*Brevibacterium immariophilum*
*Brevibacterium lactofermentum (Corynebacterium glutamicum)*
*Brevibacterium roseum*
*Brevibacterium saccharolyticum*
*Brevibacterium thiogenitalis*
*Brevibacterium ammoniagenes*
*Brevibacterium album*
*Brevibacterium cerinum*
*Microbacterium ammoniaphilum*

Specific examples of the coryneform bacteria are as follows:
*Corynebacterium acetoacidophilum* ATCC13870
*Corynebacterium acetoglutamicum* ATCC15806
*Corynebacterium alkanolyticum* ATCC21511
*Corynebacterium callunae* ATCC15991
*Corynebacterium glutamicum* ATCC13020, ATCC13032, ATCC13060
*Corynebacterium lilium* ATCC15990
*Corynebacterium melassecola* ATCC17965
*Corynebacterium thermoaminogenes* AJ12340 (FERM BP-1539)
*Corynebacterium herculis* ATCC13868
*Brevibacterium divaricatum* ATCC14020
*Brevibacterium flavum* ATCC13826,
*Brevibacterium flavum (Corynebacterium glutamicum*) ATCC14067,
*Brevibacterium flavum* AJ12418 (FERM BP-2205)
*Brevibacterium immariophilum* ATCC14068
*Brevibacterium lactofermentum (Corynebacterium glutamicum*) ATCC13869
*Brevibacterium roseum* ATCC13825
*Brevibacterium saccharolyticum* ATCC14066
*Brevibacterium thiogenitalis* ATCC19240
*Brevibacterium ammoniagenes* ATCC6871, ATCC6872
*Brevibacterium album* ATCC15111
*Brevibacterium cerinum* ATCC 15112
*Microbacterium ammoniaphilum* ATCC15354

These strains are available from the American Type Culture Collection (ATCC, Address: P.O. Box 1549, Manassas, VA 20108, United States of America). That is, each strain is given a unique registration number which is listed in the catalogue of the ATCC. Strains can be ordered using this registration number. The AJ12340 strain was deposited at National Institute of Bioscience and Human Technology, Agency of Industrial Science and Technology, Ministry of International Trade and Industry (currently International Patent Organism Depositary, National Institute of Advanced Industrial Science and Technology at Tsukuba Central 6, 1-1, Higashi 1-chome, Tsukuba-shi, Ibaraki-ken 305-5466, Japan) on October 27, 1989 under the provisions of the Budapest Treaty and given an accession number of FERM BP-1539. The AJ12418 strain was deposited at National Institute of Bioscience and Human Technology, Agency of Industrial Science and Technology, Ministry of International Trade and Industry on January 5, 1989 under the provisions of the Budapest Treaty and given an accession number of FERM BP-2205.

The coryneform bacterium of the present invention has L-glutamic acid-producing ability. "L-glutamic acid-producing ability" means an ability to cause accumulation of a sufficient amount of L-glutamic acid in a medium when the coryneform bacterium of the present invention is cultured in the medium. L-glutamic acid-producing ability may be a property of a parent strain from which the coryneform bacterium of the present invention is bred, because most of the wild-type strains of coryneform bacterium produce L-glutamic acid under L-glutamic acid-producing conditions as described below. Nevertheless, L-glutamic acid-producing ability may be imparted or enhanced by a mutation, gene recombination technique, etc. as mentioned below. Furthermore, the L-glutamic acid-producing ability may be imparted by enhancing the expression of the yggB gene.

The phrase "L-glutamic acid-producing ability of coryneform bacterium is enhanced" means that the coryneform bacterium of the present invention has an enhanced ability to produce L-glutamic acid compared to a non-modified strain. Examples of non-modified strains include Corynebacterium glutamicum ATCC13032,13869,14067, and Corynebacterium melassecola ATCC 17965. A non-modified strain may also include one which expresses the wild-type yggB gene at the same level as the wild-type strains or one in which a mutation is not introduced into the coding region of a yggB gene.

An example of a method for imparting L-glutamic acid-producing ability includes enhancing expression of a gene encoding an L-glutamic acid biosynthetic enzyme. Examples of the enzymes involved in L-glutamic acid biosynthesis include glutamate dehydrogenase, glutamine synthetase, glutamate synthetase, isocitrate dehydrogenase, aconitate hydratase, citrate synthase, phosphoenolpyruvate carboxylase, pyruvate carboxylase, pyruvate dehydrogenase, pyruvate kinase, phosphoenolpyruvate synthase, enolase, phosphoglyceromutase, phosphoglycerate kinase, glyceraldehyde-3-phophate dehydrogenase, triose phosphate isomerase, fructose bisphosphate aldolase, phosphofructokinase, and glucose phosphate isomerase.

Enhancing the expression of these genes can be performed in the same way as enhancing the expression of the yggB gene described below.

Examples of microorganisms which have been modified so that expression of the citrate synthase gene, isocitrate dehydrogenase gene, pyruvate dehydrogenase gene, and/or glutamate dehydrogenase gene is/are enhanced include those microorganisms disclosed in WO00/18935 and JP2000-232890A (EP1010755A).

The modification for imparting L-glutamic acid-producing ability includes decreasing or eliminating an activity of an enzyme that catalyzes a reaction for synthesizing a compound other than L-glutamic acid, and branching from an L-glutamic acid biosynthesis pathway. Examples of such enzymes include isocitrate lyase, α-ketoglutarate dehydrogenase, acetyl phosphate transferase, acetate kinase, acetohydroxy acid synthase, acetolactate synthase, acetyl formate transferase, lactate dehydrogenase, and glutamate decarboxylase. Examples of strains in which α-ketoglutarate dehydrogenase activity is decreased include the following strains:
*Brevibacterium lactofermentum* ΔS strain (WO95/34672)
*Brevibacterium lactofermentum* AJ12821 strain (FERM BP-4172; FR9401748)
*Brevibacterium flavum* AJ12822 strain (FERM BP-4173; FR9401748)
*Brevibacterium glutamicum* AJ12823 strain (FERM BP-4174; FR9401748)

To decrease or eliminate the activity of the enzymes as described above, a mutation or deletion which causes a decrease or loss of the activity of the enzymes may be introduced into the genes of the enzymes on the chromosome. This may be achieved by, for example, disrupting the gene encoding the enzyme on the chromosome, or by modifying an expression control sequence such as a promoter and/or Shine Dargarno (SD) sequence of the gene. In addition, the activities of such enzymes may be decreased or eliminated by introducing a missense mutation which causes an amino acid substitution, a nonsense mutation which generates a stop codon, or a frame shift mutation which adds or deletes one or two nucleotides into a coding region, or by deleting a portion or the entire gene (Journal of biological Chemistry 272:8611-8617 (1997)). For example, activities of such enzymes may be decreased or eliminated by constructing a gene encoding a mutant enzyme in which its coding region is deleted and replacing a chromosomal gene with the resulting gene by homologous recombination, or by introducing a transposon or an IS factor into these genes.

For example, introduction of mutations to decrease or eliminate the activity of the above-described enzymes by gene recombination can be performed as follows. That is, a mutant-type gene is constructed by modifying a partial sequence of a target gene so that a normal enzyme is not produced, and the mutant-type gene is used to transform a coryneform bacterium to cause recombination with the target gene on a chromosome, and thereby, a target gene on a chromosome can be replaced with the mutant-type gene. Such gene disruption by gene substitution utilizing homologous recombination is already established, and includes a method that employs linear DNA or a method that employs a plasmid containing a temperature-sensitive replication origin (U.S. Patent 6,303,383, or JP-A-05-007491). Examples of temperature-sensitive plasmids for coryneform bacteria include p48K and pSFKT2 (USPatent 6303383), pHSC4 (France Patent Laid-open Publication No. 2667875,1992 and JP5-7491A), and so forth. In coryneform bacteria, these plasmids can autonomously replicate at least at a temperature of 25°C, but cannot autonomously replicate at a temperature of 37°C. The AJ12571 strain harboring pHSC4 was deposited at National Institute of Bioscience and Human Technology, Agency of Industrial Science and Technology, Ministry of International Trade and Industry (currently International Patent Organism Depositary, National Institute of Advanced Industrial Science and Technology at Tsukuba Central 6, 1-1, Higashi 1-chome, Tsukuba-shi, Ibaraki-ken 305-5466, Japan) on August 26, 1991 under the provisions of the Budapest Treaty and given an accession number of FERM BP-3524.

Gene disruption by gene substitution utilizing the above-mentioned homologous recombination can also be performed by using a plasmid which is not replicable in coryneform bacteria. A plasmid which is not replicable in coryneform bacteria and is replicable in *Escherichia* bacteria is preferably used. Examples of such a plasmid include pHSG299 (Takara Bio) and pHSG399 (Takara Bio).

A chromosomal gene encoding one of the above-mentioned enzymes can be replaced with a deletion-type gene, for example, by homologous recombination using sacB (Schafer, A. et al., Gene 145 (1994) 69-73). The sacB gene encodes levan sucrase and is used to efficiently select strains in which a chromosomal target gene is replaced by a mutant gene, and a vector portion is cured from a chromosome (WO2005/113745, and WO2005/113744).

At first, a recombinant plasmid is prepared by inserting a deletion-type (mutant) gene, a sacB gene, and a selection marker such as a chloramphenicol-resistant gene into a plasmid containing a temperature-sensitive replication origin. The plasmid is then introduced into a host strain of coryneform bacterium. When levan sucrase is expressed in coryneform bacterium, levan generated by the conversion of sucrose is lethal for the bacterium, and hence the bacterium cannot grow on sucrose-containing medium. Therefore, by culturing on a sucrose-containing plate, strains in which substitution occurs between the mutant gene in the plasmid and a chromosomal gene, and from which the other portions of the plasmid are cured from the cell, can be selected.

Examples of the sacB gene include the following:
*Bacillus subillus:* sacB GenBank Accession No. X02730 (SEQ ID NO: 11)
*Bacillus amyloliqufaciens* : sacB GenBank Accession Number X52988
*Zymomonas mobilis* : sacB GenBank Accession Number L33402
*Bacillus stearothermophilus :* surB GenBank Accession Number U34874
*Lactobacillus sanfranciscensis* : frfA GenBank Accession Number AJ508391
*Acetobacter xylinus* : 1sxA GenBank Accession Number AB034152
*Gluconacetobacter diazotrophicus :* 1sdA GenBank Accession No. L41732

The transformant strain is cultured at a temperature at which the temperature-sensitive replication origin functions (e.g. 25°C), to obtain a strain into which the plasmid has been introduced. Then, the transformant is cultured at a high temperature at which the temperature- sensitive replication origin does not function (e.g. 34 °C) to cure the temperature-sensitive plasmid, and spread on a plate medium containing an antibiotic drug such as kanamycin. Although strains from which the plasmid is cured cannot grow on a plate containing such an antibiotic drug, a few strains in which the chromosomal gene is replaced with the mutant gene can grow and appear as colonies.

In a strain in which the recombinant DNA containing the mutant gene is integrated into the chromosomal DNA, the recombinant DNA causes recombination with the gene that originally existed on the chromosome, and the fusion genes of the chromosomal gene and the mutant gene are inserted into the chromosome so that the other portions of the recombinant DNA (vector segment, temperature sensitive replication origin and antibiotic resistance marker) are present between the fusion genes. Then, in order to leave only the mutant gene on the chromosomal DNA, one copy of the gene is eliminated together with the vector segment (including the temperature-sensitive replication origin and the antibiotics resistance marker) from the chromosomal DNA. In this case, the native gene is left on the chromosomal DNA and the mutant gene is excised from the chromosomal DNA, or to the contrary, the mutant gene is left on the chromosomal DNA and the native gene is excised from the chromosome DNA. In both cases, the excised DNA is maintained in cells of coryneform bacterium when the coryneform bacterium is cultured at a temperature at which the temperature- sensitive replication origin can function. Then, a gene on the plasmid is cured from the cells along with the plasmid by culturing the coryneform bacterium at a temperature at which the temperature-sensitive replication origin cannot function. In the case of using a sacB gene, strains from which the plasmid is cured can be efficiently obtained by culturing the coryneform bacterium in a sucrose-containing medium. Strains in which a target gene is replaced with a mutant-type or deletion-type gene can be obtained by selecting strains in which a mutation is introduced into the target gene from the plasmid-cured strains.

The L-glutamic acid-producing ability may also be imparted by screening for a strain resistant to organic acid analogs, respiratory inhibitors, or superoxide generators, or by screening for a strain sensitive to inhibitors of cell wall synthesis. Examples of such methods include imparting resistance to monofluoroacetate (JP50-113209A), imparting resistance to adenine or thimine (JP57-065198A), imparting resistance to malonic acid (JP52-038088A), attenuating urease activity ((JP52-038088A), imparting resistance to benzopirone or naphtoquinone (JP56-1889A), imparting resistance to HOQNO (JP56-140895A), imparting resistance to α-ketomalonic acid (JP57-2689A), imparting resistance to guanidine (JP56-35981A), imparting resistance to daunomicin (JP58-158192A), and imparting sensitivity to penicillin (JP04-88994A).

Specific examples of such bacteria include the following strains:
*Brevibacterium flavum* AJ3949 (FERM BP-2632; JP50-113209A)
*Corynebacterium glutamicum* AJ11628 (FERM P-5736; JP57-065198A)
*Brevibacterium flavum* AJ11355 (FERM P-5007; JP56-1889A)
*Corynebacterium glutamicum* AJ11368 (FERM P-5020; JP56-1889A)
*Brevibacterium flavum* AJ11217 (FERM P-4318; JP57-2689A)
*Corynebacterium glutamicum* AJ11218 (FERM P-4319; JP57-2689A)
*Brevibacterium flavum* AJ11564 (FERM P-5472; JP56-140895A)
*Brevibacterium flavum* AJ11439 (FERM P-5136; JP56-35981A)
*Corynebacterium glutamicum* H7684 (FERM BP-3004; JP04-88994A)
*Brevibacterium lactofermentum* AJ11426 (FERM P5123 JP56-048890A)
*Corynebacterium glutamicum* AJ11440 (FERM P5137 JP56-048890A)
*Brevibacterium lactofermentum* AJ11796 (FERM P6402 JP58-158192A)

The coryneform bacterium of the present invention can be obtained by modifying the above-described coryneform bacterium having the L-glutamic acid-producing ability using a yggB gene so that L-glutamic acid-producing ability is further enhanced. Alternatively, modification using a yggB gene may be performed first, followed by additional modification to impart or enhance L-glutamic acid-producing ability.

Modification using a yggB gene includes, but is not limited to, enhancing the expression of a yggB gene in a coryneform bacterium and introducing a mutation into a yggB gene in a coryneform bacterium.

### <I> Enhancing expression of a yggB gene

A yggB gene encodes a kind of mechanosensitive channel, which is also referred to as "mscS" (FEMS Microbiol Lett. 2003 Jan 28;218(2):305-9).

Enhancing expression of a yggB gene in coryneform bacterium leads to improvement of the L-glutamic acid-producing ability of the coryneform bacterium as compared to a non-modified strain. That is, when a strain of coryneform bacterium which has been modified so that expression of the yggB gene is increased relative to a non-modified strain, such as a wild-type strain, the strain causes accumulation of more L-glutamic acid, or produces L-glutamic acid at a higher rate, than the non-modified strain. It is preferable that enhancing expression of a yggB gene leads to an increase in the amount of L-glutamic acid produced by more than 2% (yield per consumed sugar), more preferably more than 4%, and still more preferably more than 6%, as compared to a non-modified strain. Alternatively, the yield of L-glutamic acid per carbon (sugar), other than carbon used for generation of bacterial cells, may be increased by enhancing expression of a yggB gene.

Although the expression level of a yggB gene may be at any level as long as it is increased relative to a non-modified strain in which expression of the yggB gene is not enhanced, expression is preferably increased not less than 1.5-fold, more preferably not less than 2-fold, and still more preferably not less than 3-fold relative to a non-modified strain. The expression level of the yggB gene can be determined by measuring the amount of mRNA of yggB gene. Methods of determining the expression level include Northern hybridization and RT-PCR (Molecular cloning (Cold Spring Harbor Laboratory Press, Cold Spring Harbor (USA), 2001)). An example of a wild-type coryneform bacterium which can be used as a control includes *Corynebacterium glutamicum (Brevibacterium lactofermentum*) ATCC13869, ATCC13032, ATCC14067 and *C*. *melassecola* ATCC 17965 strain.

The L-glutamic acid-producing ability of the coryneform bacterium which was modified using a yggB gene may be enhanced as compared to a non-modified strain either under L-glutamic acid-producing conditions and/or in the presence of excess biotin. Herein, the "L-glutamic acid producing conditions" include when a substance that induces L-glutamic acid production is added to a conventional medium which contains a carbon source, a nitrogen source, inorganic salts, and a trace amount of organic nutrients, such as amino acids and vitamins, if necessary, and when the amount of a substance that inhibits the L-glutamic acid production is limited in such a conventional medium. The substances that induce L-glutamic acid production include penicillin and surfactants containing saturated fatty acid, such as Tween 40 (Trademark). The substance that inhibits the L-glutamic acid production includes biotin (Amino Acid Fermentation, Japan Scientific Societies Press 1986). The concentration of penicillin in the medium is preferably not less than 0.1 U/ml, more preferably not less than 0.2 U/ml, and still more preferably not less than 0.4 U/ml. The concentration of surfactants in the medium is preferably not less than 0.5 g/L, more preferably not less than 1 g/L, and still more preferably not less than 2 g/L. The concentration of biotin added to the medium under L-glutamic acid-producing conditions is preferably less than15 µg/L, more preferably less than 10 µg/L, and still more preferably less than5 µg/L. The L-glutamic acid-producing conditions may not contain biotin at all.

On the other hand, the excess biotin-containing conditions may be conditions containing not less than 30 µg/L biotin, more preferably not less than 40 µg/L, and still more preferably not less than 50 µg/L.

Examples of a yggB gene of coryneform bacteria include a DNA encoding the amino acid sequence of SEQ ID No: 6, a DNA encoding the amino acid sequence of SEQ ID No: 62, a DNA encoding the amino acid sequence of SEQ ID No: 68, and a DNA encoding the amino acid sequence of SEQ ID No: 84. Specific examples of a yggB gene of coryneform bacteria include nucleotides 1437-3035 of SEQ ID No: 5, nucleotides 507 to 2093 of SEQ ID No: 61, nucleotides 403 to 2001 of SEQ ID No: 67, and nucleotides 501 to 2099 of SEQ ID No: 83. The nucleotide sequence of nucleotides 501-2099 of SEQ ID No: 83 is the yggB gene of *Corynebacterium glutamicum* ATCC13032, and corresponds to nucleotide numbers 1336092-1337693 in the genome sequence of GenBank Accession No. NC_003450, and is registered as NCgl 1221 (NP_600492. Reports small-conductance mechanosensitive channel...[gi: 19552490]). The nucleotide sequence of nucleotides 1437-3035 of SEQ ID No: 5 is the yggB gene of *Corynebacterium glutamicum* ATCC13869. The nucleotide sequence of nucleotides 507-2093 of SEQ ID No: 61 is the yggB gene of *Corynebacterium glutamicum* (*Brevibacterium flavum)* ATCC14067. The nucleotide sequence of nucleotides 403-2001 of SEQ ID No: 67 is the yggB gene of *Corynebacterium melassecola* ATCC17965. Furthermore, since the nucleotide sequence of a yggB gene may differ depending on species and strains, the yggB gene used in the present invention may be a variant of the nucleotide sequence of nucleotides 1437-3035 of SEQ ID No: 5. A variant of the yggB gene may be searched for using the nucleotide sequence of nucleotides 1437-3035 of SEQ ID No: 5, for example, by BLAST (//blast.genome.jp/). A variant of the yggB gene includes a gene obtained by PCR using primers of SEQ ID NOS: 75 and 76. A yggB gene may be a gene derived from other microorganisms so long as it is able to increase the L-glutamic acid-producing ability of coryneform bacteria. A yggB gene may be a mutant-type yggB as described below.The yggB gene used in the present invention is not limited to a gene encoding a protein having the amino acid sequence shown in SEQ ID NO: 6, 62, 68, or 84, and may also include a gene encoding a protein having the amino acid sequence of SEQ ID NO: 6, 62, 68, or 84, whereby one or more amino acids are replaced, deleted, inserted, or added at one or more positions, while retaining the ability to enhance L-glutamic acid-producing ability of a coryneform bacterium when the gene is introduced into the coryneform bacterium. Although the number of "several" amino acid residues referred to herein may differ depending on positions in the three-dimensional structure or types of amino acid residues of the protein, it may be preferably 2 to 20, more preferably 2 to 10, particularly preferably 2 to 5. The yggB gene preferably encodes a protein which is not less than 80% homologous, more preferably not less than 90% homologous, even more preferably not less than 95% homologous, and particularly preferably not less than 97% homologous, to the amino acid sequence shown in SEQ ID NO: 6, 62, 68, 84 or 85, while maintaining the ability to enhance the L-glutamic acid-producing ability of coryneform bacterium. Homology between amino acid sequences as well as nucleotide sequences may be determined by algorithms including BLAST developed by Karlin and Altschul (Pro. Natl. Acad. Sci. USA, 90, 5873(1993)) and FASTA developed by Pearson (Methods Enzymol., 183,63 (1990)). Homology search programs including BLASTN and BLASTP have been developed based on the algorithm (available at //www.ncbi.nlm.nih.gov).

The above-mentioned substitution is preferably a conservative substitution. In the case of aromatic amino acids, conservative substitutions include substitutions of phe, trp, and tyr for each other. In the case of hydrophobic amino acids, conservative substitutions include substitutions of leu, ile, and val for each other. In the case of polar amino acids, conservative substitutions include substitutions of gln and asn for each other. In the case of basic amino acids, conservative substitutions include substitutions of arg, lys, and his for each other. In the case of acidic amino acids, conservative substitutions are substitutions of asp and glu for each other. In the case of hydroxyl group-containing amino acids, conservative substitutions are substitutions of ser and thr for each other. The conservative substitutions also include: substitution of Ser or Thr for Ala, substitution of Gln, His, or Lys for Arg, substitution of Glu, Gln, Lys, His, or Asp for Asn, substitution of Asn, Glu, or Gln for Asp, substitution of Ser or Ala for Cys, substitution of Asn, Glu, Lys, His, Asp, or Arg for Gln, substitution of Gly, Asn, Gln, Lys, or Asp for Glu, substitution of Pro for Gly, substitution of Asn, Lys, Gln, Arg, or Tyr for His, substitution of Leu, Met, Val, or Phe for Ile, substitution of Ile, Met, Val, or Phe for Leu, substitution of Asn, Glu, Gln, His, or Arg for Lys, substitution of Ile, Leu, Val, or Phe for Met, substitution of Trp, Tyr, Met, Ile, or Leu for Phe, substitution of Thr or Ala for Ser, substitution of Ser or Ala for Thr, substitution of Phe or Tyr for Trp, substitution of His, Phe, or Trp for Tyr and substitution of Met, Ile, or Leu for Val.

Especially, the following amino acids may be substituted or deleted in the amino acid sequence of SEQ ID NO: 6. The amino acid sequence of theYggB protein which is conserved among coryneform bacteria is shown in SEQ ID NO: 85,.i.e. a consensus seqeunce. The Xaa residue shown in SEQ ID NO: 85 may be substituted or deleted.
Glu at position 48 (preferably replaced by Arg)
Asp at position 275 (preferably replaced by Ser)
Glu at position 298 (preferably replaced by Ala)
Ala at position 343 (preferably replaced by Val)
Phe at position 396 (preferably replaced by Ile)
Ser at position 438 (preferably replaced by Gly)
Val at position 445 (preferably replaced by Ala)
Ala at position 454 (preferably replaced by Val)
Pro at position 457 (preferably replaced by Ser)
Ser at position 474 (preferably replaced by Asp)
Val at position 517 (preferably deleted)
Glu at position 518 (preferably deleted)
Ala at position 519 (preferably deleted)
Pro at position 520 (preferably deleted)

The above-described yggB gene homologue can be obtained by modifying the nucleotide sequence of nucleotides 1437-3035 of SEQ ID No: 5, nucleotides 507 to 2093 of SEQ ID No: 61, nucleotides 403 to 2001 of SEQ ID No: 67, or nucleotides 501 to 2099 of SEQ ID No: 83 by, for example, site-specific mutagenesis, so that substitution, deletion, insertion, or addition of an amino acid residue or residues occurs at a specific site in the encoded protein. Furthermore, such a gene can also be obtained by a conventionally known mutation treatment. Examples of the mutation treatment include treating a gene having the nucleotide sequence of nucleotides 1437-3035 of SEQ ID No: 5, nucleotides 507 to 2093 of SEQ ID No: 61, nucleotides 403 to 2001 of SEQ ID No: 67, or nucleotides 501 to 2099 of SEQ ID No: 83 in vitro with hydroxylamine, and treating a microorganism, for example, an *Escherichia* bacterium, harboring the gene with ultraviolet ray irradiation or a mutagenesis agent typically used in mutation treatments, such as N-methyl-N'-nitro-N-nitrosoguanidine (NTG) or EMS (ethyl methanesulfonate). The mutation for the substitution, deletion, insertion, addition, inversion, or the like of amino acid residues described above also includes a naturally occurring mutation arising from individual differences and differences in species of microorganisms harboring the yggB gene (mutant or variant). Whether these genes are able to enhance the L-glutamic acid-producing ability of coryneform bacterium can be confirmed by, for example, expressing the genes in a wild-type coryneform bacterium and determining if the L-glutamic acid-producing ability of the obtained coryneform bacterium is enhanced relative to a non-modified strain, such as a wild-type strain.

The yggB gene may also be a DNA which is able to hybridize with a DNA having the nucleotide sequence complementary to the nucleotides 1437-3035 of SEQ ID No: 5, nucleotides 507 to 2093 of SEQ ID No: 61, nucleotides 403 to 2001 of SEQ ID No: 67, or nucleotides 501 to 2099 of SEQ ID No: 83, or a probe that can be prepared from any of these nucleotide sequences under stringent conditions and is able to enhance L-glutamic acid-producing ability of coryneform bacterium when it is introduced into the coryneform bacterium.

"Stringent conditions" as used herein are conditions under which a so-called specific hybrid is formed, and a non-specific hybrid is not formed. Examples of stringent conditions include those under which highly homologous DNAs hybridize to each other, for example, DNAs not less than 70% homologous, preferably not less than 80% homologous, more preferably not less than 90% homologous, especially preferably not less than 95% homologous, hybridize to each other, and DNAs lower than 70% homologous do not hybridize to each other, and those under which DNAs hybridize to each other at a salt concentration with washing typical of Southern hybridization, i.e., washing once or preferably 2-3 times with 1 x SSC, 0.1% SDS at 60°C, preferably 0.1 x SSC, 0.1% SDS at 60°C, more preferably 0.1 x SSC, 0.1% SDS at 68°C.

A complementary partial sequence of the yggB gene can also be used as a probe. Such a probe can be prepared by PCR using oligonucleotides which are designed based on the nucleotide sequence of the yggB gene in a manner well known to those skilled in the art. When a DNA fragment having a length of about 300 bp is used as a probe, the washing conditions after hybridization can be exemplified as 2 x SSC, 0.1% SDS at 50°C. The oligonucleotides shown in SEQ ID No: 75 and 76 can be used to prepare said probe.

Furthermore, the yggB gene may be a mutant-type yggB gene as described in <II> to <IV> below.

Enhancing the expression of the above-described yggB gene can be attained by increasing the copy number of the yggB gene, modifying an expression regulatory sequence of the yggB gene, amplifying a gene encoding a regulatory factor that increases expression of the yggB gene, or disrupting or attenuating a gene encoding a regulatory factor that reduces expression of the yggB gene, by using transformation with a plasmid or homologous recombination, conjugation, transition, or the like.

For example, a recombinant DNA can be prepared by ligating a gene fragment containing the yggB gene to a vector, preferably a multi-copy vector, which can replicate in coryneform bacterium, and introducing the resulting vector into an L-glutamic acid-producing coryneform bacterium.

The copy number of the yggB gene may also be increased by integrating multiple copies of the yggB gene into a chromosomal DNA of a microorganism. In order to integrate multiple copies of the yggB gene into a chromosomal DNA of a microorganism, homologous recombination can be performed by targeting a sequence which exists in multiple copies on the chromosomal DNA. Repetitive DNA and inverted repeats at an end of a transposon can be used. Alternatively, as disclosed in JP2-109985A, it is also possible to incorporate the yggB gene into a transposon, and transfer it so that multiple copies of the gene are integrated into the chromosomal DNA. Integration of the yggB gene into the chromosome can be confirmed by Southern hybridization using a probe having a partial sequence of the yggB gene.

Hereinafter, an example of a method for constructing a coryneform bacterium which has been modified so that expression of the yggB gene is enhanced is shown. This method can be performed by conventional methods such as those described in Molecular cloning: Cold Spring Harbor Laboratory Press, Cold Spring Harbor (USA), 2001.

At first, the yggB gene is cloned from the chromosomal DNA of a coryneform bacterium. The chromosomal DNA can be prepared from a coryneform microorganism by, for example, the method of Saito and Miura (refer to H. Saito and K. Miura, Biochem. Biophys. Acta, 72, 619 (1963), Text for Bioengineering Experiments, Edited by the Society for Bioscience and Bioengineering, Japan, pp.97-98, Baifukan, 1992). For PCR, oligonucleotides such as those shown in SEQ ID NO: 75 and 76 can be used as primers.

Then, a recombinant DNA is prepared by ligating the amplified yggB gene to a vector DNA that can function in coryneform bacterium. Vectors which are autonomously replicable in *Escherichia coli* and coryneform bacterium are preferably used for plasmid construction. Examples of vectors which are autonomously replicable in coryneform bacteria include pAM330 (JP58-67699A), pHM1519 (JP58-77895A), pVK7 (US2003-0175912), and pSFK6 (JP2000-262288A), pCRY30 (JP3-210184A), pCRY21, pCRY2KE, pCRY2KX, pCRY31, pCRY3KE, pCRY3KX (JP2-72876A and US patent 5,185,262), pCRY2, and pCRY3 (JP1-191686), and pAJ655, pAJ611, pAJ1844 (JP58-192900A), pCG1 (JP57-134500A), pCG2 (JP58-35197), pCG4, pCG11 (S57-183799). Examples of vectors autonomously replicable in *Escherichia coli* include pUC19, pUC18, pHSG299, pHSG399, pHSG398, pACYC184, (pHSG and pACYC are available from Takara Bio), RSF1010, pBR322, pMW219 (pMW is available from Nippon Gene), pTrc99A (Amann et al., Gene 69:301-315(1988), and so forth.

In order to prepare a recombinant DNA by ligating the yggB gene to any of the vectors mentioned above, the vector and a fragment containing the yggB gene are digested with restriction enzymes and ligated to each other, usually by using a ligase such as a T4 DNA ligase.

The recombinant plasmid as prepared above is introduced into the host coryneform bacterium by a conventional transformation method. Examples of transformation methods include treating recipient cells with calcium chloride so to increase permeability of the DNA, which has been reported for Escherichia coli K-12 (Mandel, M. and Higa, A., J. Mol. Biol., 53,159 (1970)), preparing competent cells from cells which are at the growth phase, followed by transformation with DNA, which has been reported for Bacillus subtilis (Duncan, C.H., Wilson, G.A. and Young, F.E., Gene, 1, 153 (1977)), and so forth. In addition to these methods, introducing a recombinant DNA into protoplast- or spheroplast-like recipient cells, which have been reported to be applicable to Bacillus subtilis, actinomycetes, and yeasts (Chang, S. and Choen, S.N., Molec. Gen. Genet., 168, 111 (1979); Bibb, M.J., Ward, J.M. and Hopwood, O.A., Nature, 274,398 (1978); Hinnen, A., Hicks, J.B. and Fink, G.R., Proc. Natl. Sci., USA, 75, 1929 (1978)), can be employed. In addition, transformation of microorganisms can also be performed by the electric pulse method (JP2-207791A).

The copy number of a yggB gene can also be increased by integrating multiple copies of the gene into a chromosomal DNA of a coryneform bacterium. In order to integrate multiple copies of a yggB gene into a chromosomal DNA of a coryneform bacterium, homologous recombination can be carried out by targeting a sequence which exists in multiple copies on a chromosomal DNA. Repetitive DNA and inverted repeats at the end of a transposon can be used as a sequence which exists in multiple copies on a chromosomal DNA. Alternatively, as disclosed in EP0332488B and Vertes, A.A., Asai, Y., Inui, M., Kobayashi, M., Kurusu,Y. and Yukawa, H. :Mol.Gen.Genet.,245, 397-405 (1994), it is also possible to incorporate a yggB gene into a transposon, and transfer it so that multiple copies of the yggB gene are integrated into the chromosomal DNA.

Furthermore, a yggB gene can also be incorporated into a host chromosome by using Mu phage (EP0332488B) or conjugation method (Biotechnology (N Y). 1991 Jan;9(1):84 -7). Furthermore, the copy number of the yggG gene can also be increased by using the artificial transposon described below.

Furthermore, yggB gene may be amplified on a chromosome by inserting the yggB gene into a plasmid which has a replication origin not capable of replicating in a host coryneform bacterium or into a plasmid which has a replication origin not capable of replicating in a host coryneform bacterium and has an ability to transfer by conjugation. Examples of such a plasmid include pSUP301 (Simo et al., Bio/Technology 1, 784-791 (1983)), pK18mob and pK19mob (Schaefer et al., Gene 145, 69-73 (1994)), pGEM-T (Promega corporation, Madison, WI, USA), pCR2.1-TOPO (Shuman (1994) Journal of Biological Chemisty 269: 32678-84; US-A 5487993), pCR(R)Blunt (Invitrogen, Groningen, Netherlands; Bernard et al., Journal of Molecular Biology, 234: 534-541 (1993)), pEM1 (Schrumpf et al., 1991, Journal of Bacteriology 173: 4510-4516), and pBGS8 (Spratt et al., 1986, Gene, 41:337-342). A plasmid containing a yggB gene is transferred into coryneform bacterium by conjugation or transformation. Gene transfer by conjugation can be performed, for example, by a method described in Schaefer et al. (Applied and Environmental Microbiology 60, 756-759 (1994)). Gene transfer by transformation can be performed, for example, by a method described in Theirbach et al. (Applied Microbiology and Biotechnology 29, 356-362 (1988)), Dunican and Shivinan (Bio/Technology 7,1067-1070 (1989)), and Tauch et al. (FEMS Microbiological Letters 123, 343-347 (1994)).

Enhancing the expression of a yggB gene can also be attained by replacing an expression regulatory sequence, including a promoter of the yggB gene, on a chromosomal DNA or on a plasmid, with a stronger one, by modifying an element involved in regulating expression of the yggB gene such as an operator and/or a repressor, or by fusing a strong terminator downstream the stop codon of the yggB gene (Hamilton et al.; Journal of Bacterology 171:4617-4622). For example, the lac promoter, trp promoter, trc promoter, PL promoter, PS2 promoter (Appl Environ Microbiol. 2003 Jan;69(1):358-66; Mol Microbiol. 1993 Jul;9(1):97-109; WO93/03158), and so forth are known as strong promoters. A method for evaluating the strength of promoters and examples of strong promoters are disclosed in Goldstein et al. (Prokaryotic promoters in biotechnology. Biotechnol. Annul. Rev., 1995, 1, 105-128). Moreover, it is also possible to introduce several nucleotide substitutions into the promoter region for the yggB gene so that the promoter is stronger (WO00/18935). For example, the "-35 region" may be replaced with "TTGACA" or "TTGCCA", or the "-10 region" may be replaced with "TATAAT" or "TATAAC". Furthermore, it is known that a spacer sequence between the ribosome binding site (RBS) and translation initiation codon, especially, several nucleotides just upstream of the initiation codon, has a great influence on translation efficiency. Therefore, this sequence may be modified. "Expression regulatory sequence" of yggB gene means a region which influences the expression amount of yggB gene, and an example thereof includes an upstream region of yggB gene. The upstream region suitable for modification to enhance yggB gene expression includes a region upstream of the translation initiation codon of the yggB gene (for example, a region upstream of the nucleotide 1436 of SEQ ID NO: 5), and a preferable example thereof includes a region at least 500bp upstream of the translation initiation codon, and a more preferable example thereof includes a region at least 300bp upstream the translation initiation codon.

The substitution of an expression regulatory sequence can also be attained by, for example, using a temperature-sensitive plasmid as described above.

Modifying the expression regulatory sequence may be combined with increasing the copy number of the yggB gene.

### <II> Introduction of a mutant-type yggB gene

A modification using the yggB gene may be introducing a mutant-type yggB gene into a coryneform bacterium. Introduction of a mutant-type yggB gene includes introducing a mutation into a chromosomal yggB gene, introducing a plasmid comprising a mutant-type yggB gene, and replacement of a chromosomal yggB gene with a mutant-type yggB gene.

In the present invention, the "mutant-type yggB gene" means a yggB gene comprising a mutation in its coding region which allows the yggB gene to enhance L-glutamic acid-producing ability of a coryneform bacterium in the presence of excess biotin when it is introduced into the coryneform bacterium. A mutant-type yggB gene may be a gene which enhances L-glutamic acid-producing ability of coryneform bacterium under L-glutamic acid-producing conditions as well as in the presence of excess biotin, when it is introduced into the coryneform bacterium.

The phrase "L-glutamic acid-producing ability of coryneform bacterium in the presence of excess biotin is enhanced" means that, when the coryneform bacterium of the present invention is cultured in a medium containing biotin at a concentration which a non-modified strain of coryneform bacterium cannot substantially produce L-glutamic acid, for example, in a medium containing not less than 30 µg/L of biotin, the strain causes accumulation of more L-glutamic acid in the medium than that of a non-modified strain, or the strain produces L-glutamic acid at higher rates than that of the non-modified strain.

Hereinafter, examples of the method of obtaining the mutant-type yggB gene of the present invention and the method of introducing the mutant-type yggB gene are described. However, the method of obtaining the mutant-type yggB gene of the present invention and the method of introducing the mutant-type yggB gene are not limited to these examples.

### (II-1) Method of utilizing odhA gene-deficient strain

The inventors of the present invention have found that a mutant type yggB gene can be efficiently obtained by using an odhA (sucA) gene-disrupted strain in which a gene encoding the E1o subunit of α-ketoglutarate dehydrogenase is disrupted.

In the present invention, α-ketoglutarate dehydrogenase (α-KDGH) activity means an activity to catalyze the oxidative decarboxylating of α-ketoglutaric acid (2-oxoglutaric acid) to generate succinyl-CoA. The reaction is catalyzed by three kinds of enzymes, i.e., α-ketoglutarate dehydrogenase (E1o EC1.2.4.2), dihydrolipoamide-S-succinyltransferase (E2o), and dihydrolipoamide dehydrogenase (E3). α-ketoglutarate dehydrogenase is also called oxoglutarate dehydrogenase (succinyl-transferase) or 2-oxoglutarate dehydrogenase. The α-KGDH activity can be measured by the method of Shiio et al. (Isamu Shiio and Kyoko Ujigawa-Takeda, Agric. Biol. Chem., 44(8), 1897-1904, 1980).

The nucleotide sequence of the gene encoding the E1o subunit of α-ketoglutarate dehydrogenase (odhA) of coryneform bacterium has already been identified (Microbiology 142, 3347-3354, (1996), GenBank accession No. D84102). The nucleotide sequence of the odhA gene is shown in nucleotides 443-4213 of SEQ ID NO: 43 and the amino acid sequence of the odhA gene is shown in SEQ ID NO: 44.

The odhA gene-disrupted strain can be obtained, for example, by a method using the above-mentioned sacB gene. At first, the internal sequence of odhA gene is amplified by PCR using primers designed based on the nucleotide sequence of the odhA gene, such as those shown in SEQ ID NOS: 1 and 2, and a chromosomal DNA of coryneform bacterium such as ATCCC 13869 strain as a template. The internal fragment of the odhA gene is inserted into a plasmid to construct a plasmid for odhA gene-disruption. The plasmids used for gene disruption include temperature-sensitive plasmids for coryneform bacteria (JP-A-05-00791), and pBS3, which is a suicide vector that comprises a sacB gene as described in Example 1.

The obtained plasmid is introduced into a strain that cannot cause accumulation of L-glutamic acid in the presence of excess biotin, for example, a wild-type C. glutamicum ATCC13869, ATCC13032 strain, by the electric pulse method (JP-A-2-207791). A single cross-over recombinant is obtained in which homologous recombination between the mutant-type odhA gene on the plasmid and the chromosomal odhA gene has occurred. When the temperature-sensitive plasmid is used, a single cross-over recombinant is obtained at a temperature at which the plasmids cannot replicate. Whether the strain is a single cross-over recombinant or not can be confirmed, for example, by using oligonucleotides of SEQ ID NOS: 3 and 4.

The thus obtained odhA gene-disrupted strain is isolated in a medium containing sugar. In the process of isolation, a spontaneous mutation is introduced into the chromosomal yggB gene at a high frequency. Then, the ability of the isolated strain to produce L-glutamic acid is evaluated by culturing the strain in a medium containing excess biotin. For example, a candidate strain is inoculated in 20 ml of culture medium (30 g/l glucose, 15 g/l ammonium sulfate, 1 g/l KH₂PO₄, 0.4 g/l MgSO₄·7H₂O, 0.01 g/l FeSO₄·7H₂O, 0.01 g/l MnSO₄·4-5H₂O, 200 µg/l vitamin B1, 300 µg/l biotin, 0.48 g/l soybean hydrolysates (total nitrogen), adjusted to pH 8.0 with KOH: autoclaved at 115°C for 10 minutes), followed by addition of 1 g of heat-sterilized calcium carbonate, and the strain is cultured with shaking. After the sugar is completely consumed, the amount of accumulated L-glutamic acid is measured. Strains which produce L-glutamic acid, for example, those which produce not less than 50% (yield per sugar) of L-glutamic acid are selected, and the nucleotide sequence of the yggB gene of the selected strains are determined to obtain a strain in which a mutation is introduced into the yggB gene.

To construct a strain carrying only the mutant-type yggB gene, it is preferable to replace the odhA gene on the chromosome disrupted by the plasmid with a wild-type odhA gene. Strains deficient in the odhA gene grow considerably slower in a medium without sugar. However, when the odhA gene reverts to the wild-type gene, the strain can grow well in a medium without sugar, for example, CM2B medium (10 g/l polypeptone, 10 g/l yeast extract, 5 g/l NaCl, 10 µg/l biotin, 20 g/l agar, adjusted to pH 7.0 with KOH). Therefore, the odhA gene-disrupted strain is spread over a CM2B plate to select growth-improved strains. The thus appeared growth-improved strains are purified on the CM2B plate, and whether the strain has the wild-type odhA gene can be estimated by testing the sensitivity of the strain to antibiotics based on the absence of remaining vectors. Furthermore, the nucleotide sequence of the odhA gene may be determined.

Alternatively, the mutant-type yggB gene may be cloned from the odhA-yggB double mutant strain, and introduced into a wild-type strain. For example, the mutant-type yggB gene can be amplified by performing PCR using the chromosomal DNA of the double mutant strain as a template and the primers shown in SEQ ID NOS: 9 and 10. The amplified product is inserted in a temperature-sensitive plasmid for coryneform bacteria, such as pHSC4 (JP-A-05-007491), or pBS3, a suicide vector described in Example 1 to replace a wild-type yggB gene on a chromosome with the mutant-type yggB gene. Whether a mutation is introduced into the yggB on the chromosome can be confirmed by determining the nucleotide sequence of the yggB gene on the chromosome.

### (II-2) Method of utilizing a transposable element

The coryneform bacteria having a mutant-type yggB gene may also be screened for by using a transposable element in coryneform bacteria. The transposable element may include an insertion sequence (IS) and an artificial transposon. The mutant-yggB gene may be a gene having an IS and/or transposon accidentally-inserted into the coding region, or a gene obtained artificially by using the artificial transposon. A strain in which a transposable element is inserted can be selected, for example, based on a decrease in sensitivity to 1-glutamic acid analogs. As an L-glutamic acid analog, 4-fluoroglutamic acid can be used. Furthermore, a strain in which a transposable element is inserted can be selected by randomly selecting antibiotics-resistant trains with an artificial transposon containing an antibiotics-resistant gene, and confirming a length of a yggB gene of the antibiotics-resistant strains by PCR.

The method described in JP-A-09-070291 may be used to introduce the IS into coryneform bacteria. An artificial transposon which includes a structural gene of a transposase and a marker gene sandwiched between inverted repeats (IR) on both sides of the IS may be used. In this case, the structural gene of a transposase may be present together with the marker gene and IS in the same plasmid or may be on a separate plasmid. Alternatively, the function of the transposon that is present on the chromosome of the host coryneform bacterium may be utilized. Examples of genes encoding a transposase derived from coryneform bacterium are shown by GenBank Accession numbers.

| | |
|---|---|
| 1. NCgl0179 | Cgl0182; transposase |
| 2. NCgl0235 | Cgl0238; putative transposase |
| 3. NCgl0348 | Cgl0355; putative transposase |
| 4. NCgl0688 | Cgl0718; putative transposase |
| 5. NCgl0919 | Cgl0959; transposase |
| 6. NCgl0993 | Cgl1037; transposase |
| 7. NCgl1021 | Cgl1066; transposase |
| 8. NCgl1464 | Cgl1521; putative transposase |
| 9. NCgl1496 | Cgl1557; transposase |
| 10. NCgl1662 | Cgl1733; putative transposase |
| 11. NCgl1664 | Cgl1734; transposase |
| 12. NCgl2131 | Cgl2212; transposase |
| 13. NCgl2284 | Cgl2367; transposase |
| 14. NCgl2392 | Cgl2479; putative transposase |
| 15. NCgl2418 | Cgl2504; putative transposase |
| 16. NCgl2420 | Cgl2506; putative transposase |
| 17. NCgl2460 | Cgl2548; predicted transposase |
| 18. NCgl2542 | Cgl2631; predicted transposase |
| 19. NCgl2665 | Cgl2761; putative transposase |
| 20. NCgl2748 | Cgl2845; putative transposase |
| 21. NCgl2850 | Cgl2951; predicted transposase |

The IS or artificial transposon may be introduced into coryneform bacteria using a suitable vector, for example, a plasmid replicable in coryneform bacteria. Specific examples of the plasmid include pHM1519 (Agric. Biol. Chem., 48, 2901-2903 (1984)), pAM330 (Agric. Biol. Chem., 48,2901-2903 (1984)), and plasmids obtained by modifying these to carry a drug-resistant gene. Furthermore, to efficiently amplify the IS or artificial transposon on the chromosome, a plasmid having a temperature-sensitive replication origin as described in the above (1) is preferably used (see JP-A-5-7491). The parent strain used for this screening is preferably a strain that cannot cause accumulation of L-glutamic acid in the presence of excess biotin, for example, the ATCC13869 strain, which is a wild-type strain of C. *glutamicum.*

As the method of introducing the plasmid carrying the IS or artificial transposon into coryneform bacteria, conventionally used methods, such as the protoplast method (Gene, 39,281-286 (1985)), electroporation method (Bio/Technology, 7, 1067-1070 (1989)), and the like may be used.

Introduction of the IS or artificial transposon carried on the temperature-sensitive plasmid into coryneform bacteria can be performed by transforming the coryneform bacteria with the plasmid, culturing the transformants at 25°C at which the plasmids can replicate to amplify the IS or artificial transposon at several tens to several hundreds copies per cell to enable introduction of the IS or artificial transposon into the chromosome, and then culturing the cells at 34°C to remove the excess plasmids. Alternatively, a DNA fragment of only IS or artificial transposon or a plasmid vector that cannot replicate in coryneform bacteria (for example, plasmid vector replicable in *Escherichia coli*) may be used to introduce the IS or artificial transposon into the chromosome of the coryneform bacteria (JP-A-7-107976, Vertes, A.A., Asai, Y., Inui, M., Kobayashi, M., Kurusu, Y. and Yukawa, H.: Mol. Gen. Genet., 245,397-405 (1994)).

The strain which has the IS or artificial transposon on the chromosome is cultured in a medium containing excess biotin so to select a strain that causes accumulation of L-glutamic acid. By determining the nucleotide sequence of the yggB gene on the chromosome of this strain, a coryneform bacterium having a mutant-type yggB gene can be obtained.

### (II-3) Method of randomly introducing a mutation into the yggB gene in vitro

Furthermore, the mutant-type yggB gene can be obtained by randomly introducing a mutation into the yggB gene *in vitro,* introducing the mutated gene into coryneform bacterium, and screening for strains which produce L-glutamic acid in the presence of excess biotin as a result of the presence of the mutant-type yggB gene. The parent strain useful for screening is preferably a strain that cannot cause accumulation of L-glutamic acid in the presence of excess biotin, for example, *Corynebacterium glutamicum* ATCC13869 strain, ATCC13032 strain, ATCC14067 strain, and *Corynebacterium melassecola* ATCC 17965 strain.

To screen for a mutant-type yggB gene, a yggB-deficient strain is preferably used. The construction of the yggB gene-disrupted strain can be performed by a method similar to the above-mentioned method in which the sacB gene is used. For example, PCR is performed using primers shown in SEQ ID NOS: 39 and 40 and the chromosomal DNA of *C*. *glutamicum* ATCC 13869 as a template to prepare an N-terminal fragment of the yggB gene. Similarly, PCR is performed using synthetic DNAs of SEQ ID NOS: 41 and 42 as primers to prepare a C-terminal fragment. SEQ ID NOS: 40 and 41 are complementary to each other. Subsequently, PCR is performed using a mixture of equimolar amounts of the N-terminal fragment and the C-terminal fragment as a template and synthetic DNAs of SEQ ID NOS: 39 and 42 as primers to prepare a fragment in which an internal sequence of yggB gene is deleted.

The obtained PCR fragment is inserted into a plasmid for gene disruption, for example, pBS4S carrying the levan sucrase gene. The obtained plasmid is introduced into the chromosome of coryneform bacterium, for example, C. *glutamicum* ATCC13869 strain to construct a yggB gene-disrupted strain.

Then, for example, in vitro mutagenesis of the yggB gene can be performed as follows. First, yggB is cloned into a plasmid that can replicate in coryneform bacterium. About 10 µg of the obtained yggB gene-carrying plasmid is dissolved in a buffer containing mutagens, for example, 500 mM phosphate buffer containing 400 mM hydroxylamine and 1 mM EDTA (pH 6.0), and heated at 75°C for 60 to 90 minutes to introduce a mutation into the yggB gene. After mutagenesis, the plasmid is desalted with SUPREC-02 (Takara Bio INC.) or the like, and then introduced into ATCC13869 ΔyggB strain, and transformants are screened in a medium containing an antibiotic. As a control, yggB gene-carrying plasmid without mutagenesis is introduced into the ATCC13869 ΔyggB strain. The emerged transformants are inoculated into a medium containing excess biotin and cultured with shaking, and then the concentration of accumulated L-glutamic acid is determined. L-glutamic acid does not substantially accumulate in a medium in which the wild-type yggB gene-carrying plasmid-introduced strain is cultured, whereas a significant amount of L-glutamic acid accumulates in a medium in which the mutant-type yggB gene-carrying plasmid-introduced strain is cultured. Whether the strain carries a mutant-type yggB gene or not can be confirmed by extracting a plasmid from the strain and determining the nucleotide sequence of the yggB gene.

Alternatively, a mutant-type yggB gene can be obtained by artificially introducing mutations into the yggB gene by such methods as error prone PCR, DNA shuffling, and StEP-PCR (Firth AE, Patrick WM; Bioinformatics. 2005 Jun 2; Statistics of protein library construction).

The methods of introducing a mutation into the yggB gene on the chromosome include, besides the above-mentioned method, a method of treating a coryneform bacterium with irradiation of X-rays or ultraviolet rays or with a mutagen such as N-methyl-N'-nitro-N-nitrosoguanidine, and selecting a strain which produces L-glutamic acid in the presence of excess biotin. Whether the mutant-type yggB gene has been introduced or not can be confirmed by determining the nucleotide sequence of the yggB gene on the chromosome.

### (II-4) Method of screening L-glutamic acid analog-resistant strains

Mutant-type yggB genes may be obtained by culturing a coryneform bacterium having a wild-type yggB gene in a medium containing an L-glutamic acid analog, and selecting L-glutamic acid analog-resistant strains which can grow in the medium. A parent strain used in this method is preferably a wild-type strain of coryneform bacterium as described above, and may be any strain having a wild-type yggB gene, including a strain having a plasmid containing a wild-type yggB gene.

"L-glutamic acid analogs" as used herein include γ-methyl L-glutamate, α-methyl glutamic acid, β-hydroxyglutamic acid, methioninesulfoximine, glutamic acid-γ-monohydroxamate, 2-amino-4-phosphonobutyric acid, γ-monoethyl L-glutamate, dimethyl L-glutamate, di-t-butyl L-glutamate, monofluoroglutamic acid, diethyl L-glutamate, D-glutamic acid, and 4-fluoroglutamic acid, and among these, 4-fluoroglutamic acid is preferably used. For example, L-glutamic acid analog-resistant strains can be obtained as follows. That is, a coryneform bacterium is inoculated on a minimum medium containing an L-glutamic acid analog, and colonies that have appeared after 24-48 hours are collected. Concentration of the L-glutamic acid analog added to the medium is preferably a concentration at which a strain having a non-mutated yggB gene cannot grow and a strain having a mutated yggB gene can grow. Specifically, the concentration of 4-fluoroglutamic acid is 1.25 mM or more, preferably 2.5 mM or more, and more preferably 5 mM or more. For example, "L-glutamic acid analog-resistant strain" as used herein means that when the strain is cultured in a minimum medium containing 4-fluoroglutamic acid where the viable cell count (number of cells capable of forming colonies) of a wild-type strain is suppressed to not more than 1/100 that of when in the absence of 4-fluoroglutamic acid, the strain exhibits 1/10 or more growth of the strain cultured in the absence of 4-fluoroglutamic acid.

The obtained L-glutamic acid analog-resistant strains are inoculated into a liquid medium containing excess biotin and cultured with shaking, followed by measurement of the concentration of L-glutamic acid which has accumulated in the medium. Whereas a strain having a wild-type yggB gene accumulates little L-glutamic acid, some of the L-glutamic acid analog-resistant strains accumulate a significant amount of L-glutamic acid. The yggB gene is amplified from such a strain and the nucleotide sequence thereof is determined, and thereby, a novel mutant-type yggB gene can be obtained.

### (III) Mutant-type yggB genes

Hereinafter, specific examples of the mutant-type yggB gene are described. However, the mutant-type yggB gene of the present invention is not limited to these genes.

The mutant type yggB gene obtained by the above-mentioned method is not particularly limited so long as it has a function to enhance the L-glutamic acid-producing ability of a coryneform bacterium in the presence of excess biotin when it is introduced into the coryneform bacterium.

### (III-1) Mutation in the C-terminal region of the yggB gene

This mutation is introduced into the region encoding amino acids 419-533 of SEQ ID NO: 6, 68, 84 or 85, or amino acids 419-529 of SEQ ID NO: 62. For example, in SEQ ID NO: 5, this region corresponds to the region consisting of nucleotides 2692 to3035. This mutation may be of any type, so long as it is introduced into the region, and includes point mutations and insertion of an artificial sequence. Among these, mutations which introduce an insertion sequence (IS) or an artificial transposon are preferable. The mutation may cause amino acid substitution (mis-sense mutation), frame-shift, or stop codon (non-sense mutation) as a result of the point mutation, insertion of IS, or transposon.

### (III-1-1) The mutation by insertion of transposable element (2A-1 type mutation)

Examples of a mutation in the C-terminal region include a mutation which inserts an transposable element such as insertion sequence (IS) next to the "G" at position 2691 of SEQ ID NO: 5. The nucleotide sequence of the mutant-type yggB gene having this mutation is shown in SEQ ID NO: 7, and the amino acid sequence of the mutant type YggB protein encoded by the gene is shown in SEQ ID NO: 8. The IS inserted into the nucleotide sequence of SEQ ID NO: 7 has high homology to IS 1207 (GenBank accession No. X96962) and IS719 (GenBank accession No. E12759). In the amino acid sequence of SEQ ID NO: 8, the C-terminal region containing the Val at position 419 and thereafter of the Ygg protein (SEQ ID NO: 6) is replaced by a shorter IS-derived sequence. This type of mutation, including the mutations changing or deleting the C-terminal region in the amino acid sequence of SEQ ID NOS: 6, 62, 68, 84 and 85, is named a 2A-1-type mutation.

Furthermore, the 2A-1-type mutation also includes other mutations which introduce another IS or transposon into the same site as the 2A-1 type mutation. The position at which the IS or transposon is inserted may be any position so long as it is located within the above-described region. It is preferable that an IS is inserted into a position where the transposase can readily recognize it, or hot spots where an IS is easy to insert.

### (III-1-2) The mutation which results in replacement of the proline with another amino acid (66-type and 22-type mutations)

Furthermore, an example of a mutation in the C-terminal region also includes a mutation which results in replacement of the proline in C-terminal region with another amino acid. The prolines at the following positions may be substituted with another amino acid in the amino acid sequence of SEQ ID NO: 6.
Pro at position 424 (424 in SEQ ID NO: 62,68,84,85)
Pro at position 437 (437 in SEQ ID NO: 62,68,84,85)
Pro at position 453 (453 in SEQ ID NO: 62,68,84,85)
Pro at position 457 (457 in SEQ ID NO: 62,68,84,85)
Pro at position 462 (462 in SEQ ID NO: 62,68,84,85)
Pro at position 469 (469i n SEQ ID NO: 62,68,84,85)
Pro at position 484 (484 in SEQ ID NO: 62,68,84,85)
Pro at position 489 (489 in SEQ ID NO: 62,68,84,85)
Pro at position 497 (497 in SEQ ID NO: 62,68,84,85)
Pro at position 515 (515 in SEQ ID NO: 62,68,84,85)
Pro at position 529 (529 in SEQ ID NO: 68,84,85, 525 in SEQ ID NO:62)
Pro at position 533 (533 in SEQ ID NO: 68,84,85, 529 in SEQ ID NO:62)

It is considered that the proline residues in the C-terminal region of the YggB protein play an important role in maintainance of three-dimensional structure of the YggB protein (Protein Eng. 2002 Jan;15(1):29-33, J Biol Chem. 1991 Dec 25;266(36):24287-94.).

Especially, replacing the proline at position 424 and/or the proline at position 437 in SEQ ID NO: 6, 62, 68, 84 or 85 with another amino acid are preferable.

Examples of the other amino acid include Ala, Arg, Asp, Asn, Cys, Glu, Gln, Gly, His, Ile, Met, Leu, Lys, Phe, Ser, Trp, Tyr, Val, and Thr. As the other amino acid replacing the proline at position 424, hydrophobic amino acids such as Ala, Gly, Val, Leu, and Ile are preferable, and amino acids having branched chain such as Leu, Val, and Ile are more preferable. An example of a mutation which replaces Pro at position 424 with Leu includes a mutation which replaces "C" at position 1673 with "T" in SEQ ID NO: 67. The nucleotide sequence of the mutant type yggB gene having this mutation is shown in SEQ ID NO: 69 and the amino acid sequence of the mutant type YggB protein encoded by the gene is shown in SEQ ID NO: 70.

As the other amino acid replacing the proline at position 437, amino acids containing a hydroxyl-group such as Thr, Ser, Tyr are preferable, and amino acids having Ser are more preferable. An example of a mutation which replaces Pro at position 437 with Ser includes a mutation which replaces "C" at position 2745 with "T" in SEQ ID NO:5. Moreover, this mutation may be accompanied by the mutation which replaces the "C" at position 3060 with "T" in SEQ ID NO: 5. The nucleotide sequence of the mutant type yggB gene having this mutation is shown in SEQ ID NO: 73 and the amino acid sequence of the mutant type YggB protein encoded by the gene is shown in SEQ ID NO: 74.

### (III-2) Mutation in a transmembrane region of the yggB gene

The YggB protein encoded by the yggB gene is presumed to have five transmembrane regions. In the amino acid sequence of the wild-type YggB protein shown in SEQ ID NOs: 6, 62, 68, 84 and 85, the transmembrane regions correspond to amino acids 1 to 23 (first transmembrane region), amino acids 25 to 47 (second transmembrane region), amino acids 62 to 84 (third transmembrane region), amino acids 86 to 108 (fourth transmembrane region), and amino acids 110 to 132 (fifth transmembrane region). In SEQ ID NO: 5, nucleotides encoding these regions correspond to nucleotides 1437 to 1505, nucleotides 1509 to 1577, nucleotides 1620 to 1688, nucleotides 1692 to 1760, and nucleotides 1764 to 1832, respectively. This type of mutation is preferably introduced into these regions. This kind of mutation preferably introduces a substitution, deletion, addition, insertion, or inversion of one or more amino acids into these regions without causing a frame shift mutation and a translation termination. Among these, mis-sense mutations causing amino acid substitutions in the above-mentioned regions is preferable. The number of "several" amino acids to be substituted, deleted, added, inserted, or inverted means 2 to 20, preferably 2 to 10, more preferably 2 to 5, and still more preferably 2 or 3. The mutations causing insertion and deletion of one or several amino acids without a frame shift are also preferable, and, more preferably, insertion or deletion of 3, 6, 9, 12, 15, 18 or 21 nucleotides, still more preferably, deletion or insertion of 3, 6, or 9 nucleotides, and much more preferably, deletion or insertion of 3 nucleotides.

Specific examples of a mutation in the transmembrane regions include the following:

### (III-2-1) Mutation in the first transmembrane region (A1-type mutation)

This type of mutation includes one which introduces one or more amino acids between the leucine at position 14 and the tryptophan at position 15 in the amino acid sequence shown by SEQ ID NOs: 6, 62, 68, 84 and 85, and more specifically includes a mutation which introduces three amino acids, for example, Cys-Ser-Leu, between the Leu at position 14 and the tryptophan at position 15. This mutation includes insertion of TTCATTGTG next to the G at position 1480 in the wild-type yggB gene of SEQ ID NO: 5. The nucleotide sequence of the mutant-type yggB gene having this mutation is shown in SEQ ID NO: 19 and the amino acid sequence of the mutant type YggB protein encoded by the gene is shown in SEQ ID NO: 20.

### (III-2-2) Mutation in the 4th transmembrane region (19-type mutation)

This type of mutation includes replacing the Ala at position 100 with another amino acid in the amino acid sequence shown in SEQ ID NOs: 6, 62, 68, 84 and 85. Examples of the other amino acid include Arg, Asp, Asn, Cys, Glu, Gln, Gly, His, Ile, Met, Leu, Lys, Phe, Pro, Ser, Trp, Tyr, Val, and Thr. Of these, amino acids containing a hydroxyl-group such as Thr, Ser, and Tyr are preferable and, threonine is more preferable. A mutation which replaces the Ala at position 100 with Thr includes a mutation which replaces the "G" at position 1734 with "A" of SEQ ID NO: 5.

The nucleotide sequence of the mutant type yggB gene having this mutation is shown in SEQ ID NO: 21 and the amino acid sequence of the mutant type YggB protein encoded by the gene is shown in SEQ ID NO: 22.

### (III-2-3) Mutation in the 5th transmembrane region (L30-type mutation, 8-type mutation)

This type of mutation includes one which replaces the Ala at position 111 with another amino acid in the amino acid sequence shown in SEQ ID NOs: 6, 62, 68, 84 and 85. Examples of the other amino acid include Arg, Asp, Asn, Cys, Glu, Gln, Gly, His, Ile, Met, Leu, Lys, Phe, Pro, Ser, Trp, Tyr, Val, and Thr. Of these, amino acids containing branched chains such as Val, Ile, and Leu, and amino acids containing hydroxyl-group such as Thr, Ser, and Tyr are preferable, and Val or Thr is preferable. This type of mutation includes one which replaces the "C" at position 1768 with a "T" in the nucleotide sequence of SEQ ID NO: 5 (L30-type mutation), and a mutation which replaces the "G" at position 837 with an "A" in the nucleotide sequence of SEQ ID NO: 61 (8-type mutation). The nucleotide sequence of the mutant-type yggB gene having an L30-type mutation is shown in SEQ ID NO: 23 and the amino acid sequence of the mutant type YggB protein encoded by this gene is shown in SEQ ID NO: 24. The nucleotide sequence of the mutant-type yggB gene having 8-type mutation is shown in SEQ ID NO: 63 and the amino acid sequence of the mutant type YggB protein encoded by this gene is shown in SEQ ID NO: 64.

### (IV) Equivalents of the mutant-type yggB genes

The "mutant type yggB gene" used in the present invention may be a functionally equivalent gene that is substantially homologous to the above-mentioned "mutant type yggB genes", for example, a mutant type gene comprising a nucleotide sequence that is able to hybridize to a nucleotide sequence complementary to at least one of the nucleotide sequences selected from the group consisting of the nucleotides 1437 to 2705 of SEQ ID NO: 7, the nucleotides 1437 to 3044 of SEQ ID NO: 19, the nucleotides 1437 to 3035 of SEQ ID NO: 21, the nucleotides 507 to 2093 of SEQ ID NO: 63, the nucleotides 403 to 2001 of SEQ ID NO: 69, and the nucleotides 1437 to 3035 of SEQ ID NO: 23, the nucleotides 548 to 2146 of SEQ ID NO: 73 with a probe prepared from these nucleotide sequences under stringent conditions, so long as the gene has a function to enhance L-glutamic acid-producing ability of a coryneform bacterium in the presence of excess biotin.

"Stringent conditions" as used herein are conditions under which a so-called specific hybrid is formed, and a non-specific hybrid is not formed. Examples of stringent conditions include, those under which DNAs having high homology hybridize to each other, for example, DNAs having a homology of not less than 70%, preferably not less than 80%, more preferably not less than 90%, especially preferably not less than 95%, hybridize to each other, and DNAs having homology lower than 70% do not hybridize to each other, and those under which DNAs hybridize to each other at a salt concentration with washing typical of Southern hybridization, i.e., washing once or preferably 2-3 times under 1 x SSC, 0.1% SDS at 60°C, preferably 0.1 x SSC, 0.1% SDS at 60°C, more preferably 0.1 x SSC, 0.1% SDS at 68°C.

The mutant-type yggB gene used in the present invention includes a gene encoding a protein having the amino acid sequence of SEQ ID NOs: 8, 20, 22, 24, 64, 70, or 74 whereby one or more amino acids are replaced, deleted, inserted or added at one or more positions other than the specific amino acid as described above, while maintaining a function to enhance L-glutamic acid-producing ability of a coryneform bacterium in the presence of excess biotin. Although the number of "several" amino acid residues referred to herein may differ depending on positions in the three-dimensional structure or types of amino acid residues of the protein, it may be preferably 2 to 20, more preferably 2 to 10, particularly preferably 2 to 5.

The yggB gene preferably encodes a protein having the above-described specific amino acid substitution or deletion and having homology of not less than 70%, more preferably not less than 80%, further more preferably not less than 90%, particularly preferably not less than 95% to the amino acid sequence shown in SEQ ID NOs: 8, 20, 22 or 24, 64, 70, or 74 while maintaining a function to enhance L-glutamic acid-producing ability of a coryneform bacterium in the presence of excess biotin. The above-mentioned substitution is preferably a conservative substitution (neutral mutation). In the case of aromatic amino acids, conservative substitutions include substitutions of phe, trp, and tyr for each other. In the case of hydrophobic amino acids, conservative substitutions include substitutions of leu, ile, and val for each other. In the case of polar amino acids, conservative substitutions include substitutions of gln and asn for each other. In the case of basic amino acids, conservative substitutions include substitutions of arg, lys, and his for each other. In the case of acidic amino acids, conservative substitutions are substitutions of asp and glu for each other. In the case of hydroxyl group-containig amino acids, conservative substitutions include substitutions of ser and thr for each other. The conservative substitutions also include: substitution of ser or thr for Ala, substitution of Gln, His or Lys for Arg, substitution of Glu, Gln, Lys, His or Asp for Asn, substitution of Asn, Glu or Gln for Asp, substitution of Ser or Ala for Cys, substitution of Asn, Glu, Lys, His, Asp or Arg for Gln, substitution of Gly, Asn, Gln, Lys or Asp for Glu, substitution of Pro for Gly, substitution of Asn, Lys, Gln, Arg or Tyr for His, substitution of Leu, Met, Val or Phe for Ile, substitution of Ile, Met, Val or Phe for Leu, substitution of Asn, Glu, Gln, His or Arg for Lys, substitution of Ile, Leu, Val or Phe for Met, substitution of Trp, Tyr, Met, Ile or Leu for Phe, substitution of Thr or Ala for Ser, substitution of Ser or Ala for Thr, substitution of Phe or Tyr for Trp, substitution of His, Phe or Trp for Tyr and substitution of Met, Ile or Leu for Val. As mentioned above, the amino acids shown as Xaa may be substituted in the amino acid sequence of SEQ ID NO: 85.

Especially, the following amino acids may be substituted or deleted in the amino acid sequences of SEQ ID NO: 8, 20, 22, 24, 64, 70, or 74.
Glu at position 48 (preferably replaced by Arg)
Asp at position 275 (preferably replaced by Ser)
Glu at position 298 (preferably replaced by Ala)
Ala at position 343 (preferably replaced by Val)
Phe at position 396 (preferably replaced by Ile)
Ser at position 438 (preferably replaced by Gly)
Val at position 445 (preferably replaced by Ala)
Ala at position 454 (preferably replaced by Val)
Pro at position 457 (preferably replaced by Ser)
Ser at position 474 (preferably replaced by Asp)
Val at position 517 (preferably deleted)
Glu at position 518 (preferably deleted)
Ala at position 519 (preferably deleted)
Pro at position 520 (preferably deleted)

### (V) Methods of introducing the above-described mutant-type yggB genes into coryneform bacterium

The mutant-type yggB gene having the above-mentioned specific mutation can be obtained by conventional methods including a site-directed mutagenesis technique. The site-directed mutagenesis technique includes an overlap extension PCR method that amplifies a mutant gene using a PCR primer having a mutation (Urban, A., Neukirchen, S. and Jaeger, K. E., A rapid and efficient method for site-directed mutagenesis using one-step overlap extension PCR. Nucleic Acids Res, 25, 2227-8. (1997)).

The coryneform bacterium of the present invention having the above-mentioned mutant-type yggB gene can be obtained by introducing the above-mentioned mutant-type yggB gene into a coryneform bacterium. A wild-type yggB gene on a chromosome may be replaced with the mutant-type yggB gene. The mutant-type yggB gene may be introduced into a coryneform bacterium in which a wild-type yggB gene is disrupted. In addition, as in the case of single cross-over recombinants, the mutant type yggB gene may co-exist with a wild-type yggB gene in a coryneform bacterium. For example, the substitution of the yggB gene on the chromosome can be performed by using, for example, a temperature-sensitive plasmid containing a sacB gene encoding the above-mentioned levan sucrase. Furthermore, to introduce the mutant type yggB gene into coryneform bacterium, a vector such as a plasmid replicable in coryneform bacterium or transposon comprising the mutant type yggB gene may be used.

To introduce the mutant type yggB gene into the chromosomal DNA of the coryneform bacteria, it is also possible to perform homologous recombination by targeting a sequence that is present on the chromosomal DNA in multiple copies. Examples of such a sequence include a repetitive DNA and an inverted repeat that is present on the end of a transposable element. The mutant type yggB gene may exist in coryneform bacterium in a single copy or multiple copies. The introduction of the mutant type yggB gene into coryneform bacterium can be confirmed by PCR, Southern hybridization, or the like.

Furthermore, the mutant type yggB gene may be under the control of a potent promoter which is derived from other genes, as described in WO00/18935. For example, lac promoter, trp promoter, trc promoter, PS2 promoter, and so on are known as potent promoters. It is also possible to introduce substitution of nucleotides into the promoter region of the mutant-type yggB gene so that expression of mutant-type yggB gene is enhanced. The substitution of the expression regulating sequence can be performed by using, for example, a temperature-sensitive plasmid.

### (VI) L-glutamic acid analog resistance

Furthermore, the coryneform bacterium of the present invention may have increased resistance to L-glutamic acid analogs as a result of the introduction of the mutant-type yggB gene of the present invention. "L-glutamic acid analogs" as used herein include γ-methyl L-glutamate, α-methyl glutamic acid, β-hydroxyglutamic acid, methioninesulfoximine, glutamic acid-γ-monohydroxamate, 2-amino-4-phosphonobutyric acid, γ-monoethyl L-glutamate, dimethyl L-glutamate, di-t-butyl L-glutamate, monofluoroglutamic acid, diethyl L-glutamate, D-glutamic acid, and 4-fluoroglutamic acid. For example, an increase in resistance to L-glutamic acid analogs is confirmed by the fact that when the strain of the present invention is cultured in a minimum medium containing 4-fluoroglutamic acid at a concentration which the viable cell count (number of cells capable of forming colonies) of the parent strain can be suppressed to not more than 1/100, the strain exhibits 1/10 or more growth as compared to when cultured in the absence of 4-fluoroglutamic acid. Specifically, it is preferable that the strain has a resistance to 1.25 mM or more, preferably 2.5 mM or more, and more preferably 5 mM or more of 4-fluoroglutamic acid.

### (VII) Further modification to inactivate the gene which suppresses the function of mutant-type yggB gene

The coryneform bacterium of the present invention may be further modified so to inactivate a gene which suppresses the function of mutant-type yggB gene. The "gene which suppresses the function of mutant-type yggB gene" means that L-glutamic acid production by a mutant-type yggB gene-introduced strain is suppressed by amplifying the gene in the strain. An example of such a gene includes the symA gene (suppressor of yggB mutation). The symA gene is shown as nucleotide numbers 2051306-2051845 of the genome sequence (Genbank Accession No. NC_003450) of corynebacterium glutamicum ATCC13032 strain, and registered as NCgl 1867 (NP_601149. hypothetical prot...[gi:19553147]). The symA gene of corynebacterium glutamicum ATCC13869 strain is shown in nucleotides 585-1121 of SEQ ID NO: 86. The symA gene may be a DNA that is able to hybridize with a nucleotide sequence complementary to nucleotides 585 to 1121 of SEQ ID No: 86, or a probe prepared from said nucleotides under stringent conditions, as long as the DNA suppresses a function of said mutant-type yggB gene in the coryneform bacterium.

Gene inactivation can be performed by disrupting the gene, deleting the gene, or modifying it to decrease expression of the gene. Inactivation of the symA gene can be performed using a similar method as the above-described methods for decreasing enzymatic activity.

### (VIII) Further modification to decrease α-ketoglutarate dehydrogenase activity

In the present invention, a coryneform bacterium is preferably modified so that the activity of α-ketoglutarate dehydrogenase (hereinafter, referred to as "α-KGDH") is decreased in addition to the modification using a yggB gene. The "α-KGDH activity is decreased" means that the α-KGDH activity is decreased as compared with that of the wild-type strain or non-modified strains, such as the parent strain. The α-KGDH activity can be measured according to the method of Shiio et al. (Isamu Shiio and Kyoko Ujigawa-Takeda, Agric. Biol. Chem., 44(8), 1897-1904, 1980). Although it is sufficient that the α-KGDH activity is decreased as compared to a non-modified strains such as a wild-type strain or a parent strain, it is preferable that the α-KGDH activity is decreased to about 1/2 time or less, preferably about 1/4 time or less, and more preferably about 1/10 time or less with respect to a wild-type or non-modified strain. The coryneform bacterium of the present invention may not have a detectable activity of α-KGDH.

The coryneform bacterium in which the α-KGDH activity is decreased can be constructed in a similar way as described above.

For example, α-KGDH activity may be decreased by introducing a gene encoding the E1o subunit of the α-KGDH complex having a mutation in the thiamine pyrophosphate-binding region (the region encoded by nucleotides 2498 to 2584 of SEQ ID NO: 43 (686Gly-714Asp of SEQ ID NO: 44)).

Examples of the strain having a decreased activity of α-KGDH include *Brevibacterium lactofermentum* ΔS strain (WO95/34672) and *Brevibacterium lactofermentum* AJ12821 (FERM BP-4172) strain (JP-A-06-237779). When using a coryneform bacterium carrying the mutant-type yggB gene and having decreased α-KGDH activity, either decreasing α-KGDH activity or introducing the mutant type yggB gene may be performed first.

### <2> Method of producing L-glutamic acid

L-glutamic acid can be produced by culturing the coryneform bacterium of the present invention in a medium to cause accumulation of L-glutamic acid in the medium and/or in the bacterial cells, and collecting the L-glutamic acid from the medium and/or the bacterial cells. In the production method of the present invention, L-glutamic acid is produced preferably by culturing the coryneform bacterium of the present invention, for example, at 25 to 40°C for 8 to 120 hours.

The culture medium may be an ordinary medium that contains a carbon source, a nitrogen source, an inorganic salt, and optionally organic micronutrients such as amino acids and vitamins. Either a synthetic medium or a natural medium may be used. Any kind of carbon and nitrogen source may be used so long as they can be utilized by the strain being cultured.

Saccharides such as glucose, glycerol, fructose, sucrose, maltose, mannose, galactose, starch hydrolysate, and molasses may be used as the carbon source. In addition, organic acids such as acetic acid and citric acid, and alcohols such as ethanol may also be used alone or in combination as a carbon source. Ammonia, ammonium salts such as ammonium sulfate, ammonium carbonate, ammonium chloride, ammonium phosphate, and ammonium acetate, nitrates, and the like may be used as the nitrogen source. Amino acids, vitamins, fatty acids, nucleic acids, substances containing peptone, casamino acid, yeast extract, and soybean protein decomposition products may be used in a slight amount as the organic nutrients. When an auxotrophic mutant strain that requires an amino acid etc. for growth is used, such a required nutrient is preferably added. Phosphates, magnesium salts, calcium salts, iron salts, manganese salts, and the like can be used as inorganic salts.

Surfactants such as Tween40, penicillin, or biotin may be added in an appropriate amount depending on a strain to be cultured. For example, a strain having a mutant-type yggB gene may be cultured in the presence of excess biotin, although such a strain may also be cultured under L-glutamic conditions containing surfactants or penicillin, or when biotin is limited.

Preferably, aerobic culturing is performed by controlling the fermentation temperature and adjusting the pH of the culture medium to 3 to 9. When the pH decreases during the culture, the medium is neutralized by adding alkali such as calcium carbonate or ammonia gas. Culture for about 10 to about 120 hours results in accumulation of a considerable amount of L-glutamic acid in the medium.

Furthermore, the culture may be performed by using a liquid medium adjusted to conditions under which the produced L-glutamic acid crystallizes and precipitates. The conditions under which L-glutamic acid crystallizes include pH 5.0 to 4.0, preferably pH 4.5 to 4.0, more preferably pH 4.3 to 4.0, particularly preferably pH 4.0 (EP1233069, EP1233070).

Collection of L-glutamic acid from the medium after completion of the culture may be performed by conventional methods. L-glutamic acid may be collected, for example, by removing bacterial cells from the medium and concentrating L-glutamic acid or by using ion exchange chromatography. When the culture is performed under conditions under which L-glutamic acid crystallizes and precipitates, the crystallized L-glutamic acid can be collected, for example, by centrifugation or filtration. In this case, L-glutamic acid dissolved in the medium may also be collected after crystallization of the dissolved L-glutamic acid.

### EXAMPLES

Hereinafter, the present invention is specifically explained by referring to the following non-limiting examples.

### Example 1

### < Construction of a vector for gene disruption carrying the sacB gene>

### (1-1) Construction of pBS3

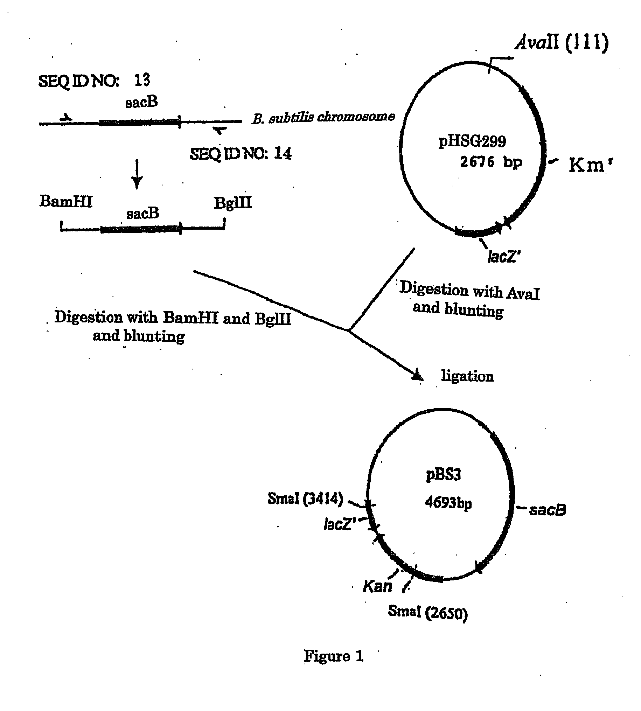

Construction of the gene disruption vector carrying the sacB gene was performed by using the method in WO2005/113745 and WO2005/113744. A sacB gene (SEQ ID NO: 11) was obtained by PCR using a chromosomal DNA of *Bacillus subtilis* as a template and oligonucleotides of SEQ ID NOS: 13 and 14 as primers. The PCR was performed using LA taq (manufactured by TaKaRa) as follows: one cycle of heat retention at 94°C for 5 minutes; and 25 cycles of denaturing at 94°C for 30 seconds, annealing at 49°C for 30 seconds, and elongation at 72°C for 2 minutes. The obtained PCR product was purified by a conventional method, and then digested with BglII and BamHI and blunt-ended. The fragment was inserted into pHSG299 which had been digested with AvaII and blunt-ended. The obtained DNA was used to transform competent cells of *Escherichia coli* JM109 (manufactured by TAKARA BIO INC.). Then, the transformed bacterial cells were spread on LB agar medium containing 25 µg/ml Kanamycin (hereinafter, abbreviated as "Km"), and incubated for one night. Thereafter, single colonies were isolated as transformants. Plasmids were extracted from the obtained transformants and the plasmid which had an insert of the object PCR product was named pBS3. Fig. 1 shows the procedure for construction of pBS3.

### (1-2) Construction of pBS4S

The SmaI recognition site in the kanamycin-resistant gene on pBS3 was modified by nucleotide substitution using cross-over PCR without causing amino acid substitution so that pBS3 is not cut by SmaI endnuclease. First, PCR was performed using pBS3 as a template and synthetic DNAs of SEQ ID NOS: 15 and 16 as primers, to thereby obtain an N-terminal fragment of the kanamycin-resistant gene. On the other hand, to obtain a C-terminal fragment of the kanamycin-resistant gene, PCR was performed using pBS3 as a template and synthetic DNAs of SEQ ID NOS: 17 and 18 as primers. PCR was performed using Pyrobest DNA Polymerase (manufactured by TAKARA BIO INC.) as follows: one cycle of heat retention at 98°C for 5 minutes; and 25 cycles of denaturing at 98°C for 10 seconds, annealing at 57°C for 30 seconds, and elongation at 72°C for 1 minute, to obtain the objective PCR product. SEQ ID NOS: 16 and 17 are partially complementary to each other and do not contain the SmaI recognition site. Then, to obtain a full-length fragment of the mutant kanamycin-resistant gene without the SmaI recognition site, the above-mentioned N-terminal and C-terminal gene products were mixed together in substantially equimolar amounts. PCR was performed using the gene products as a template and synthetic DNAs of SEQ ID NOS: 15 and 18 as primers to obtain a SmaI site-modified kanamycin-resistant gene fragment. The PCR was performed using Pyrobest DNA Polymerase (manufactured by TAKARA BIO INC.) as follows: one cycle of heat retention at 98°C for 5 minutes; and 25 cycles of denaturing at 98°C for 10 seconds, annealing at 57°C for 30 seconds, and elongation at 72°C for 1.5 minutes, to thereby obtain the object PCR product.

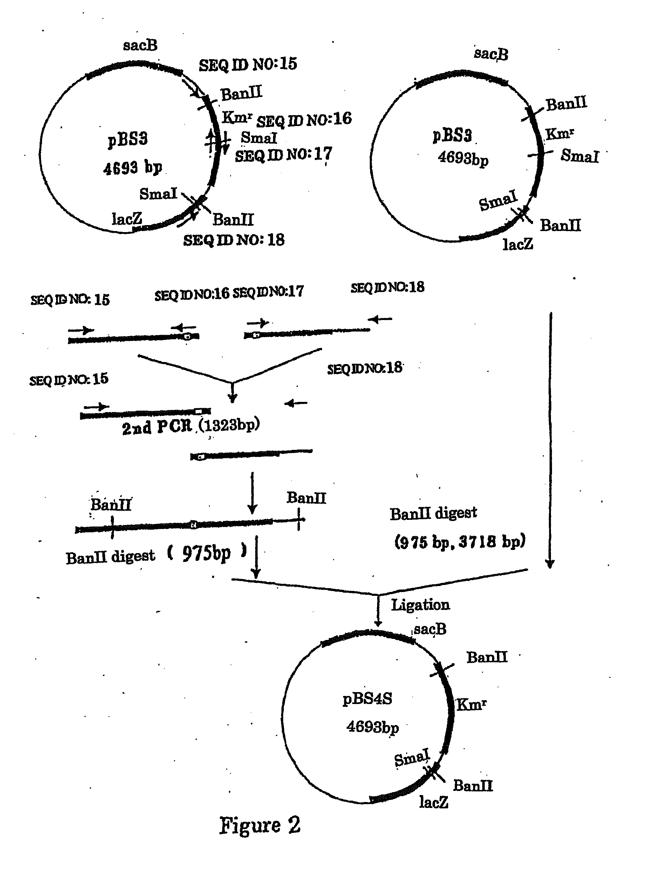

The PCR product was purified by a conventional method, and then digested with BanII and then inserted into the above-described BanII recognition site of pBS3. The resulting plasmid was used to transform competent cells of *Escherichia coli* JM109 (available from Takara Bio). That is, the transformed bacterial cells were spread on LB agar medium containing 25 µg/ml of kanamycin, and incubated for one night. Thereafter, colonies that appeared were selected as transformants. Plasmids were isolated from the obtained transformants and the plasmid having an insert of the object PCR product was named pBS4S. Fig. 2 shows the procedure for constructing pBS4S.

### Example 2

### <Construction of an odhA gene-disrupted strain from C. glutamicum ATCC13869 strain>

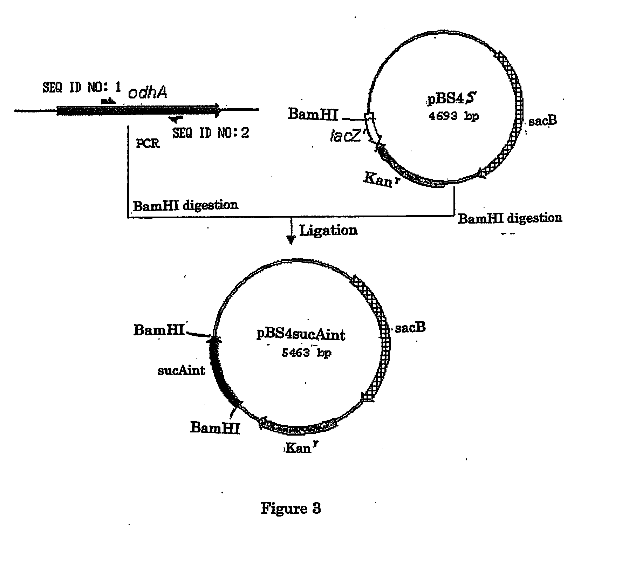

The nucleotide sequence of the odhA gene encoding the α-ketoglutarate dehydrogenase of coryneform bacterium has already been identified (Microbiology 142, 3347-3354, (1996), GenBank accession No. D84102). Based on the nucleotide sequence of the odhA gene, the primers described in SEQ ID NOS: 1 and 2 were designed, and PCR was performed using the primers and the chromosomal DNA of the ATCC13869 strain as a template to amplify the internal sequence of the odhA gene. The amplified PCR fragment was completely digested with BamHI and inserted to the BamHI site of pBS4S constructed in Example 1, thereby the plasmid pBS4SΔsucAint was obtained (Fig. 3).

pBS4SΔsucAint was introduced into *C*. *glutamicum* ATCC13869 strain by the electric pulse method (JP-A-02-207791) and the tranformed bacterial cells were spread over CM-Dex agar medium (5g/l glucose, 10g/l polypeptone, 10g/l yeast extract, 1 g/l KH₂PO₄, 0.4 g/l MgSO₄·7H₂O, 0.01 g/l FeSO₄·7H₂O, 0.01 g/l MnSO₄·4-5H₂O, 3 g/l urea, 1.2 g/l soybean protein hydrolysate, and 20 g/l agar, adjusted to pH 7.5 with NaOH: autoclaved at 120°C for 20 minutes) containing 25 µg/ml kanamycin. After culturing at 31.5°C, PCR was performed using each of the chromosome extracted from strains which appeared to confirm that these strains were single cross-over recombinants in which pBS4SΔsucAint was incorporated by homologous recombination into the chromosome. Primers each having a sequence (SEQ ID NO: 3) specific to pBS4S plasmid and a sequence (SEQ ID NO: 4) complementary to a chromosomal sequence was used for PCR. Since the sequence of pBS4S is absent in a non-recombinant strain, no fragment is amplified from the non-recombinant strain, whereas a single fragment is amplified from a single cross-over recombinant.

The single cross-over recombinant thus obtained was named 2A-1 strain. The wild-type 13869 strain and 2A-1 strain were inoculated in 20 ml of a flask medium (30g/l glucose, 15 g/l ammonium sulfate, 1 g/l KH₂PO₄, 0.4 g/l MgSO₄·7H₂O, 0.01 g/l FeSO₄·7H₂O, 0.01 g/l MnSO₄·4-5H₂O, 200 µg/l VB1 (vitamin B1), 300 µg/l Biotin, and 0.48 g/l soybean hydrolysates (T-N: total nitrogen), adjusted to pH 8.0 with KOH: autoclaved at 115°C for 10 minutes), followed by addition of 1 g of heat-sterilized calcium carbonate, and each of the strains was cultured with shaking at 31.5°C. After the sugar was completely consumed, the concentration of L-glutamic acid which had accumulated in the medium was determined. The results are shown in Table 1 (OD620 is turbidity at 620nm of culture solution diluted to 101 times, and indicates the cell amount, and Glu (g/L) indicates the amount of accumulated L-glutamic acid). It was found that the 2A-1 strain produced L-glutamic acid in the presence of an excess amount of biotin, whereas the parent strain ATTCC 13869 did not produce L-glutamic acid at all.

**<Table 1 Amount of L-glutamic acid produced by the control and the 2A-1 strain>**

| | OD620(x101) | Glu(g/L) |
|---|---|---|
| ATCC13869 | 0.658 | 0.2 |
| 2A-1 | 0.315 | 17.7 |
| Blank | 0.002 | 0.4 |

### Example 3

### <Construction of an odhA gene-revertant strain from the 2A-1 strain>

In the 2A-1 strain, the odhA gene on the chromosome was disrupted by pBS4SΔsucAint. By curing the plasmid from the chromosome of this strain, the odhA gene could be reverted to the wild-type one. Although the odhA gene-disrupted strain grows very slowly in a medium containing no sugar, the odhA gene-revertant strain in which the odhA gene-revered to the wild-type one grows well in a medium containing no sugar such as CM2B (10 g/l polypeptone, 10 g/l yeast extract, 5 g/l NaCl, 10 µg/l Biotin, 20 g/l agar, adjusted to pH 7.0 with KOH). To obtain such a revertant strain, the 2A-1 strain was spread over CM2B agar medium to select growth-improved strains. The growth-improved strain which appeared was named 2A-1R and isolated on the CM2B agar medium and the kanamycin-sensitivity of the 2A-1R strain was examined. As a result, it was found that all of the selected strains were kanamycin-sensitive and sucrose-resistant. Since the pBS4SΔsucAint contains a kanamycin-resistant gene and the sacB gene encoding levan sucrase, strains harboring pBS4SΔsucAint exhibit kanamycin-resistance and sucrose-sensitivity, while strains from which pBS4SΔsucAint was removed exhibit kanamycin-sensitivity and sucrose-resistance. Therefore, it was considered that the odhA gene reverted to the wild-type one in the 2A-1R strain. Determination of the nucleotide sequence of the odhA gene confirmed that the strain has the wild-type odhA gene.

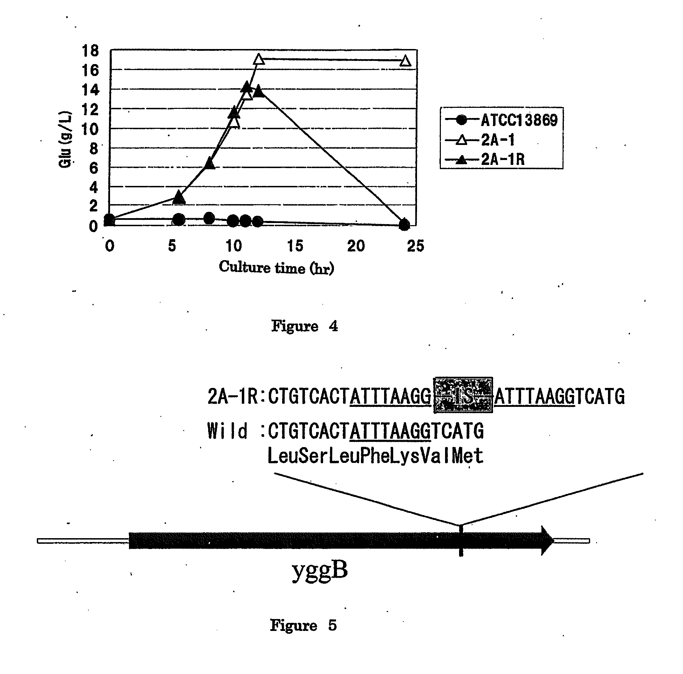

The ability of 2A-1R strain to produce L-glutamic acid in the presence of an excess amount of biotin was confirmed by the same method as in Example 2. The results are shown in Table 2 (OD620 is turbidity at 620nm of culture solution diluted to 101 times, and indicates the cell amount, and Glu (g/L) indicates the amount of accumulated L-glutamic acid). Although the accumulation of L-glutamic acid by the 2A-1R strain was slightly decreased as compared to the 2A-1 strain, the 2A-1R strain produced a much higher amount of L-glutamic acid in the presence of an excess amount of biotin than the wild-type ATCC13869 strain (Table 2). In addition, when the shaking culture was continued after the sugar was completely consumed, decomposition of L-glutamic acid was observed in the 2A-1R strain, which proved that the odhA gene had reverted to the wild-type in this strain (Fig. 4).

**<Table 2 L-glutamic acid production by the control strain, odhA gene-disrupted strain and odhA gene-revertant strain>**

| | OD620(x101) | Glu(g/L) |
|---|---|---|
| ATCC13869 | 0.696 | 0.5 |
| 2A-1 | 0.332 | 17.1 |
| 2A-1R | 0.327 | 14.3 |
| Blank | 0.002 | 0.6 |

### Example 4

### <Isolation of a gene that is involved in L-glutamic acid production by the 2A-1R strain>

On the CM2B agar medium, the 2A-1R strain could form colonies at substantially the same rate as that of the wild-type strain ATCC 13869. However, on the minimum plate medium (20 g/l glucose, 2.64 g/l ammonium sulfate, 0.5 g/l KH₂PO₄, 0.5 g/l K₂HPO₄, 0.25 g/l MgSO₄·7H₂O, 0.01 g/l FeSO₄·7H₂O, 0.01 g/l MnSO₄·7H₂O, 0.01 g/l CaCl₂, 0.02 mg/l CuSO₄, 40 g/l MOPS, 30 mg/l protocatechuic acid, 200 µg/l VB₁·Hu, 300 µg/l Biotin, 20 g/l agar, adjusted to pH 6.7 with NaOH), the 2A-1R strain showed a considerably decreased colony-forming rate as compared to the wild-type ATCC 13 869 strain. Accordingly, a gene that can recover the growth of the 2A-1R strain in the minimum medium was found.

The chromosomal DNA of the ATCC13869 strain was partially digested with Sau3AI and ligated to the shuttle vector pVK9 that had been digested with BamHI. The obtained plasmid was precipitated with ethanol and used to transform competent cell of *E. coli* DH5α (TAKARA BIO INC.) by an electric pulse method. pVK9 is a shuttle vector obtained by blunt-ending the AvaII site of pHSG299 (TAKARA BIO INC.) and inserting therein a fragment comprising a sequence automatically replicable in coryneform bacteria excised with BamHI and KpnI from pHK4 (JP-A-05-007491). The transformed cells were spread over an LB agar medium (10 g/l polypeptone, 5 g/l yeast extract, 5 g/l NaCl, 20 g/l agar, adjusted to pH 7.0 with NaOH) containing 25 µg/ml kanamycin, and cultured at 37°C for one night. On the next day, all of the colonies which appeared were collected from the plate with a platinum loop and plasmids were extracted to construct a plasmid library of the ATCC13869 strain. The plasmid library was transformed to the 2A-1R strain obtained in Example 3 by the electric pulse method, and the transformed cells were applied to a minimum agar medium containing 25 µg/ml kanamycin. The strains that showed an increased colony-forming rate were selected. By extracting a plasmid from the selected strains showing the increased colony-forming rate, it was found that the fragment having a nucleotide sequence shown in SEQ ID NO: 5 was inserted into the BamHI site of pVK9. The obtained plasmid was named pL5k.

Comparison of the nucleotide sequence inserted in the pL5k with the already published genome sequence of Corynebacterium glutamicum ATCC13032 (Acc. No. NC_003450) showed that pL5k contained only one ORF encoding the amino acid sequence shown in SEQ ID NO: 6.

The program "SOSUI" available on the Internet (sosui.proteome.bio.tuat.ac.jp/sosuiframe0E.html as of 2004/10/07) was used to predict whether the ORF encodes a membrane protein. Results of analysis of the ORF by using "SOSUI" suggested that five transmembrane regions are present in this amino acid sequence. In the amino acid sequence of SEQ ID NO: 6, the transmembrane regions correspond to the regions of amino acids 1 to 23, amino acids 25 to 47, amino acids 62 to 84, amino acids 86 to 108, and amino acids 110 to 132. DNA sequences encoding these regions correspond to the nucleotides 1437 to 1505, nucleotides 1509 to 1577, nucleotides 1620 to 1688, nucleotides 1692 to 1760, and nucleotides 1764 to 1832 of SEQ ID NO: 5. Each of the amino acid sequences of these regions is shown in SEQ ID NOS: 25 to 29 and
Table 3.

**<Table 3 Predicted transmembrane regions of the protein encoded by the inserted gene>**

| No. | N-terminal position | transmembrane region | C-terminal position | type | length | SEQ ID |
|---|---|---|---|---|---|---|
| 1 | 1 | MILGVPIQYLLYSLWNWI VDTGF | 23 | SECONDA RY | 23 | 25 |
| 2 | 25 | VAIILVLAFLIPRIGRLAMR IIK | 47 | PRIMARY | 23 | 26 |
| 3 | 62 | QLAFAGVGVYIAQIVAFF MLAVS | 84 | PRIMARY | 23 | 27 |
| 4 | 86 | MQAFGFSLAGAAIPATIAS AAIG | 108 | SECONDA RY | 23 | 28 |
| 5 | 110 | GAQSIVADFLAGFFILTEK QFGV | 132 | SECONDA RY | 23 | 29 |

A further search of the literature revealed that the ORF is named YggB (NCgl 1221) (FEMS Microbiology letters 218(2003)305-309).

### Example 5

### <Identification of the mutation introduced into the yggB gene of the 2A-1R strain>

The yggB gene could recover the growth of the 2A-1R strain in the minimum medium, which suggested the possibility that the yggB gene of the 2A-1R strain has some mutations. Accordingly, the nucleotide sequence of the yggB gene of the 2A-1R strain was determined. The results indicated that in the 2A-1R strain, an IS was inserted into the C-terminal region of the wild-type yggB gene (Fig. 5). The nucleotide sequence of the mutant type yggB gene derived from the 2A-1R strain is shown in SEQ ID NO: 7 and the corresponding amino acid sequence is shown in SEQ ID NO: 8.

This suggested the possibility that the ability of the 2A-1R strain to produce L-glutamic acid in the presence of an excess amount of biotin was due to the mutation in the yggB gene. It should be noted that this mutation was present not only in the 2A-1R strain, but also in the 2A-1 strain. This mutation is presumed to have occurred as a suppressor mutation to stably excrete L-glutamic acid from the cell when the odhA gene was disrupted. The mutation in which an IS was inserted was named the 2A-1 type mutation.

Example 6

### <Construction of a strain having the 2A-1 type mutant yggB gene and evaluation of L-glutamic acid-producing ability>

### (6-1) Introduction of the 2A-1 type mutation into a wild-type strain and evaluation of L-glutamic acid-producing ability (single cross-over recombinants)

PCR was performed using the chromosomal DNA of the 2A-1 strain as a template and synthetic DNAs shown in SEQ ID NOS: 9 and 10 as primers to amplify the fragment of yggB gene having the 2A-1 type mutation. The amplified product was treated with SacI and inserted into the SacI site of pBS3 obtained in Example 1 to thereby obtain a plasmid containing the 2A-1 type mutant yggB gene (pBS3yggB2A).

The obtained pBS3yggB2A was introduced into *C*. *glutamicum* ATCC13869 by the electric pulse method and the transformed cells were spread over CM-Dex agar medium containing 25 µg/ml kanamycin. The strains that appeared after culturing at 31.5°C were evaluated by PCR to confirm that they are single cross-over recombinants in which pBS3yggB2A was incorporated into the chromosome by homologous recombination. The obtained single cross-over recombinant was named 13869-2A. In this strain, both the wild-type yggB gene and mutant-type yggB gene exist and are expressed.

The ability of the obtained mutant-type yggB gene-introduced strain 13869-2A to produce L-glutamic acid in the presence of an excess amount of biotin was evaluated by the method described in Example 2. The results are shown in Table 4 (OD620 is turbidity at 620nm of culture solution diluted 101 times, and indicates the cell amount, and Glu (g/L) indicates the amount of accumulated L-glutamic acid). The 13869-2A strain produced L-glutamic acid in the presence of an excess amount of biotin when the wild-type ATCC13869 strain cannot produce L-glutamic acid. This indicated that the mutation in the yggB gene could enhance L-glutamic acid production in the presence of an excess amount of biotin.

**<Table 4 Amount of L-glutamic acid accumulated by the control strain, 2A-1R strain, and the mutant type yggB gene-introduced strain>**

| | OD620(x101) | Glu(g/L) |
|---|---|---|
| ATCC13869 | 0.625 | 0.3 |
| 2A-1R | 0.334 | 15.5 |
| 13869-2A | 0.582 | 3.6 |
| Blank | 0.002 | 0.6 |

### (6-2) Introduction of the 2A-1 type mutant yggB gene into the wild-type strain and evaluation of L-glutamic acid-producing ability (double cross-over recombinants)

To construct a strain having only the mutant-type yggB gene, the 13869-2A strain was cultured in the CM-Dex liquid medium for one night and the obtained culture was spread over the S10 agar medium (100 g/l sucrose, 10 g/l polypeptone, 10 g/l yeast extract, 1 g/l KH₂PO₄, 0.4 g/l MgSO₄·7H₂O, 0.01 g/l FeSO₄·7H₂O, 0.01 g/l MnSO₄·4-5H₂O, 3 g/l urea, 1.2 g/l soybean protein hydrolysates, 20 g/l agar, adjusted to pH 7.5 with NaOH: autoclaved at 120°C for 20 minutes) and cultured at 31.5°C. Among the colonies which appeared, the strain exhibiting sensitivity to kanamycin was isolated on CM2B agar medium. Chromosomal DNAs were prepared from the strains. Then, PCR was performed using synthetic DNAs shown in SEQ ID NOS: 9 and 10 as primers to confirm that the strain has only a mutant-type yggB gene. The strain containing the mutant-type yggB gene in which an IS-like sequence was inserted was named 13869-2A-7.

The ability of the obtained 13869-2A-7 strain to produce L-glutamic acid in the presence of an excess amount of biotin was evaluated by the method described in Example 2. The results are shown in Table 5 (OD620 is turbidity at 620nm of culture solution diluted to 101 times, and indicates the cell amount, and Glu (g/L) indicates the amount of accumulated L-glutamic acid). The 13869-2A-7 strain produced L-glutamic acid equivalent to or higher than the 2A-1R strain, which confirmed that L-glutamic acid production of the coryneform bacterium in the presence of an excess amount of biotin was caused by the mutation in the yggB gene.

**<Table 5 Amount of L-glutamic acid produced by the control strain, 2A-1R strain and the mutant-type yggB gene-introduced strain>**

| | OD620(x101) | Glu(g/L) |
|---|---|---|
| ATCC13869 | 0.648 | 0.4 |
| 2A-1R | 0.420 | 13.8 |
| 13869-2A-7 | 0.414 | 16.1 |
| Blank | 0.002 | 0.7 |

### Example 7

### <Construction of the A 1-type mutant yggB gene-introduced strain and evaluation of L-glutamic acid-producing ability >

As a result of the screening using the above-mentioned L-glutamic acid-producing odhA gene-disrupted strain (ΔsucA strain), five kinds of mutations were identified on the yggB gene besides the above-mentioned 2A-1 mutation. Hereinafter, these mutations were named A1-type mutation, 19-type mutation, L30-type mutation, 8-type mutation, and 66-type mutation. The mutant-type yggB genes having each of the A1-type mutation, 19-type mutation, and L30-type mutation were introduced into the chromosome of the ATCC 13869 strain, and the effect of each mutation was evaluated. The mutant-type yggB gene having the 8-type mutation was introduced into the chromosome of the ATCC14067 strain, and the effect of the mutation was evaluated. The mutant-type yggB gene having the 66-type mutation was introduced into the chromosome of the *C*. *melassecola* ATCC17965 strain, and the effect of the mutation was evaluated.

The A1 type mutation is a mutation which inserts "TTCATTGTG" next to the "G" at position 1480 of the wild-type yggB gene (the wild-type gene of *C*. *glutamicum* is shown in nucleotides 1437-3035 of SEQ ID NO: 5), and causes insertion of CysSerLeu between the Leu at position 14 and the Trp at position 15 in the amino acid sequence of SEQ ID NO: 6. The nucleotide sequence of the mutant type yggB gene in which this mutation was introduced is shown in SEQ ID NO: 19 and the amino acid sequence of the mutant type YggB encoded by this gene is shown in SEQ ID NO: 20.

The A1 type mutant gene can be obtained as follows. PCR is performed by using the synthetic DNAs shown in SEQ ID NOS: 30 and 31 as primers and the chromosomal DNA of ATCC13869 strain as a template to prepare an N-terminal fragment. Similarly, PCR is performed by using the synthetic DNAs shown in SEQ ID NOS: 32 and 33 as primers to prepare a C-terminal fragment. Subsequently, PCR is performed by using an equimolar mixture of the N-terminal fragment and the C-terminal fragment as a template and the synthetic DNAs shown in SEQ ID NOS: 9 and 34 as primers to amplify a partial fragment of the A1 type mutant yggB gene. The obtained mutant-type yggB gene fragment is treated with SacI and inserted into the SacI site of pBS4S to obtain a plasmid for introducing this type of mutation. The pBS4yggBA1 thus obtained is introduced into the chromosome of the ATCC13869 strain in the same manner as described in Example 6 and then only the plasmid portion was cured from the chromosome. The nucleotide sequence of the yggB gene of the obtained kanamycin-sensitive strain is determined and the strain having the A1 type mutant yggB gene is selected. The A1 type mutant yggB gene-introduced strain was named ATCC13869-A1 strain.

The ATCC13869-A1 strain and the control ATCC13869 strain were cultured in the same manner as in Example 2. After completion of the culture, the amount of L-glutamic acid which had accumulated in the culture medium was measured by the known method. It was found that the A1-type mutant yggB gene-introduced strain produced L-glutamic acid in the presence of an excess amount of biotin in a greater amount as compared to the control strain.

**<Table 6 Amount of L-glutamic acid produced by the control strain, and the mutant-type (A1-type) yggB gene-introduced strain>**

| strains | OD620(x101) | Glu(g/L) |
|---|---|---|
| ATCC13869 | 0.650 | 0.5 |
| ATCC13869-A1 | 0.548 | 8.6 |

### Example 8

### <Construction of the 19 type mutant yggB gene-introduced strain and evaluation of L-glutamic acid-producing ability>

The 19 type mutation is a mutation which replaces the "G" at position 1734 of the wild type yggB gene (the wild type gene of *C. glutamicum* is shown in nucleotides 1437-3035 of SEQ ID NO: 5) with an "A", and causes replacement of the Ala at position 100 with Thr in the amino acid sequence of SEQ ID NO: 6. The nucleotide sequence of the mutant type yggB gene having this type of mutation is shown in SEQ ID NO: 21 and the amino acid sequence of the mutant type YggB protein encoded by this gene is shown in SEQ ID NO: 22.

In the same manner as in Example 7, the 19 type mutant yggB gene-introduced strain is constructed. Specifically, PCR is performed by using the synthetic DNAs shown in SEQ ID NOS: 30 and 35 as primers and the chromosomal DNA of the ATCC 13869 strain as a template to prepare an N-terminal fragment. Similarly, PCR is performed by using the synthetic DNAs shown in SEQ ID NOS: 33 and 36 as primers to prepare a C-terminal fragment. Subsequently, PCR is performed by using an equimolar mixture of the N-terminal fragment and the C-terminal fragment as a template and the synthetic DNAs shown in SEQ ID NOS: 9 and 34 as primers, to amplify a partial fragment of the 19 type mutant yggB gene. The obtained yggB fragment is treated with SacI and inserted into the SacI site of pBS4S to obtain a plasmid for introducing this mutation. The thus obtained pBS4yggB19 is introduced into the chromosome of the ATCC13869 strain in the same manner as described in Example 6 and then the vector portion is cured from the chromosome. The nucleotide sequence of the yggB gene of the obtained kanamycin-sensitive strain is determined and the strain having the 19 type yggB gene is selected. The 19-type mutant strain was named ATCC13869-19 strain.

The ATCC13869-19 strain and the control ATCC13869 strain were cultured in the same manner as in Example 2. After completion of the culture, the amount of L-glutamic acid which had accumulated in the culture broth was measured by a conventional method. It was found that the 19-type mutant yggB gene-introduced strain produced L-glutamic acid in the presence of an excess amount of biotin in a greater amount as compared to the control strain.

**<Table 7 Amount of L-glutamic acid produced by the control strain, and the mutant-type (19-type) yggB gene-introduced strain>**

| strains | OD620(x101) | Glu(g/L) |
|---|---|---|
| ATCC13869 | 0.650 | 0.5 |
| ATCC13869-19 | 0.614 | 0.7 |

### Example 9

### <Construction of the L30 type mutant yggB-gene introduced strain and evaluation of L-glutamic acid-producing ability>

The L30 type mutation is a mutation which replaces the "C" at position 1768 of the wild type yggB gene (the wild type gene of *C. glutamicum* is shown in SEQ ID NO: 5) with "T", and causes replacement of the Ala at position 111 with Val in the amino acid sequence shown in SEQ ID NO: 6. The nucleotide sequence of the mutant type yggB gene having this type of mutation is shown in SEQ ID NO: 23 and the amino acid sequence of the mutant type YggB protein encoded by this gene is shown in SEQ ID NO: 24.

In the same manner as in Example 7, the L30 type mutant yggB gene-introduced strain was constructed. Specifically, PCR was performed by using the synthetic DNAs shown in SEQ ID NOS: 30 and 37 as primers and the chromosomal DNA of ATCC13869 strain as a template to prepare an N-terminal fragment. Similarly, PCR was performed by using the synthetic DNAs shown in SEQ ID NOS: 34 and 38 as primers to prepare a C-terminal fragment. Subsequently, PCR was performed by using an equimolar mixture of the N-terminal fragment and the C-terminal fragment as a template and the synthetic DNAs shown in SEQ ID NOS: 9 and 34 as primers to amplify a partial fragment of the L30 type mutant yggB gene. The obtained yggB fragment was treated with SacI and inserted into the SacI site of pBS4S to obtain a plasmid for introducing this type of mutation. The thus obtained pBS4yggB-L was introduced into the chromosome of ATCC13869 strain in the same manner as described in Example 6, and then the vector portion was cured from the chromosome. The nucleotide sequence of the yggB gene of the obtained kanamycin-sensitive strain was determined and the strain having L30 type mutant yggB gene was selected. The L30-type mutant yggB gene-introduced strain was named ATCC13869-L30 strain.

The ATCC13869-L30 strain and the control ATCC 13869 strain were cultured in the same manner as in Example 2. After completion of the culture, the amount of L-glutamic acid which had accumulated in the culture broth was measured by a conventional method. The results are shown in Table 8 (OD620 is turbidity at 620nm of culture solution diluted to 101 times, and indicates the cell amount, and Glu (g/L) indicates the amount of accumulated L-glutamic acid). ATCC13869-L30 strain having the L30 type mutant yggB gene caused accumulation of L-glutamic acid in a greater amount as compared to the parent ATCC13869 strain.

**<Table 8 Amount of L-glutamic acid produced by the control strain and the L30-type mutant yggB gene-introduced strain>**

| | OD620(x101) | Glu(g/L) |
|---|---|---|
| ATCC13869 | 0.650 | 0.5 |
| ATCC13869-L30 | 0.389 | 15.9 |

### Example 10

### Evaluation of the mutant-type yggB gene-introduced strains under L-glutamic acid-producing conditions

L-glutamic acid production of Coryneform bacterium is induced by addition of surfactants such as Tween40 or by limiting the biotin concentration. Therefore, the ATCC13869 strain and the ATCC13869-19 strain were cultured under a condition containing Tween40 and a condition containing a limited amount of biotin, respectively.

Each of the strains was inoculated into 20 ml of seed culture medium (80 g/l glucose, 30 g/l ammonium sulfate, 1 g/l KH₂PO₄, 0.4 g/l MgSO₄.7H₂O, 0.01 g/l FeSO₄.7H₂O, 0.01 g/l MnSO₄.4-5H₂O, 200 µg/l VB1, 60 µg/l biotin, 0.48 g/l soybean hydrolysate (T-N), adjusted to pH 8.0 with KOH: autoclaved at 115°C for 10 minutes), and then 1 g of sterilized calcium carbonate was added thereto, followed by a shaking culture at 31.5°C. The culture solution obtained after complete consumption of sugars was used as a seed culture solution in the following main culture.

For culturing with Tween40, 2 ml of the seed culture solution was inoculated into 20 ml of main culture medium (80 g/l glucose, 30 g/l ammonium sulfate, 1 g/l KH₂PO₄, 0.4 g/l MgSO₄.7H₂O, 0.01 g/l FeSO₄.7H₂O, 0.01 g/l MnSO₄.4-5H₂O, 200 µg/l VB1, 60 µg/l biotin, 0.48 g/l soybean hydrolysate (T-N), adjusted to pH 8.0 with KOH: autoclaved at 115°C for 10 minutes), and then 1 g of sterilized calcium carbonate was added thereto, followed by a shaking culture at 31.5°C. When OD620 of culture broth diluted 101-fold reached 0.2, Tween40 was added to a final concentration of 5g/L and the culture was continued.

For culturing with limited biotin, 1 ml of the seed culture solution was inoculated into 20 ml of main culture medium (80 g/l glucose, 30 g/l ammonium sulfate, 1 g/l KH₂PO₄, 0.4 g/l MgSO₄.7H₂O, 0.01 g/l FeSO₄.7H₂O, 0.01 g/l MnSO₄.4-5H₂O, 200 µg/l VB1, 0.48 g/l soybean hydrolysate (T-N), adjusted to pH 8.0 with KOH: autoclaved at 115°C for 10 minutes), and then 1 g of sterilized calcium carbonate was added thereto, followed by a shaking culture at 31.5°C. Under these culture conditions, a final concentration of biotin is calculated to be about 2.9µg/L.

After a 40 hour-culture, the amount of L-glutamic acid which had accumulated in the medium was measured for the Tween40-added culture and the biotin-limited culture. The result is shown in Table 9. It was found that the ATCC13869-19 strain produced L-glutamic acid in an amount greater than the control strain under L-glutamic acid-producing conditions.

**<Table 9 Amount of L-glutamic acid produced by the control strain and the 19-type mutant yggB gene-introduced strain under L-glutamic acid-producing conditions>**

| Strains | OD620 (X101) | Glu (g/L) |
|---|---|---|
| Tween40-added | | |
| ATCC13869 | 0.538 | 25.6 |
| ATCC13869-19 | 0.395 | 28.6 |
| biotin-limited | | |
| ATCC13869 | 0.462 | 36.0 |
| ATCC13869-19 | 0.431 | 40.0 |

The wild-type ATCC13869 strain, yggB mutant trains ATCC13869-19, ATCC13869-A1, ATCC13869-L30, and a strain having a plasmid containing a wild-type yggB gene (ATCC13869/pL5k-1), and a strain having a control plasmid (ATCC13869/pVK9) were cultured with Tween40. Each of these strains were cultured on a CM-Dex plate medium overnight, and cells collected from 1/6 area of the plate were inoculated in 20 ml of a flask medium (80g/l glucose, 30 g/l ammonium sulfate, 1 g/l KH₂PO₄, 0.4 g/l MgSO₄·7H₂O, 0.01 g/l FeSO₄·7H₂O, 0.01 g/l MnSO₄·4-5H₂O, 200 µg/l VB1, 60 µg/l biotin, and 0.48 g/l soybean hydrolysates (T-N: total nitrogen), adjusted to pH 8.0 with KOH: autoclaved at 115°C for 10 minutes), followed by addition of 1 g of heat-sterilized calcium carbonate, and each of the strains was cultured with shaking at 31.5°C. After a 5-hour culture, Tween40 was added to a final concentration of 1g/L and the culture was continued. Table 10 shows the amount of cells (OD620) and the amount of L-glutamic acid which had accumulated in the medium after 24 hours. It was found that the ATCC13869-19 strain, ATCC13869-A1 strain, ATCC13869-L30 strain, and the strain having a plasmid containing a wild-type yggB gene have an enhanced ability to produce L-glutamic acid under L-glutamic acid-producing conditions.

**<Table 10>**

| | OD620 (x101) | Glu(g/L) |
|---|---|---|
| ATCC13869 | 0.887 | 12.8 |
| ATCC13869 /pVK9 | 0.748 | 12.4 |
| ATCC13869/pL5k-1 | 0.711 | 19.2 |
| ATCC13869-19 | 0.786 | 21.0 |
| ATCC13869-A1 | 0.629 | 34.9 |
| ATCC13869-L30 | 0.649 | 28.3 |
| Blank | 0.001 | 0.5 |

### Example 11

### <Construction of the 8-type mutant yggB-gene introduced strain and evaluation of L-glutamic acid-producing ability>

The 8-type mutation is a mutation which replaces the "G" at position 837 of SEQ ID NO: 61 with an "A", and causes replacement of the Ala at position 111 with Thr in the amino acid sequence of SEQ ID NO: 62. The nucleotide sequence of the mutant-type yggB gene having this type of mutation is shown in nucleotides 507-2093 of SEQ ID NO: 63 and the amino acid sequence of the mutant-type YggB protein encoded by this gene is shown in SEQ ID NO: 64.

In the same manner as in Example 7, the 8-type mutant yggB gene-introduced strain is constructed. Specifically, PCR is performed by using the synthetic DNAs shown in SEQ ID NOS: 30 and 65 as primers and the chromosomal DNA of *Brevibacterium flavum* ATCC14067 strain as a template to prepare an N-terminal fragment. Similarly, PCR is performed by using the synthetic DNAs shown in SEQ ID NOS: 34 and 66 as primers to prepare a C-terminal fragment. Subsequently, PCR is performed by using an equimolar mixture of the N-terminal fragment and the C-terminal fragment as a template and the synthetic DNAs shown in SEQ ID NOS: 9 and 34 as primers to amplify a partial fragment of the 8-type mutant yggB gene. The obtained yggB gene fragment is treated with SacI and inserted into the SacI site of pBS4S to obtain a plasmid for introducing this type of mutation. The thus obtained pBS4yggB8 is introduced into the chromosome of ATCC14067 strain in the same manner as described in Example 6 and then the vector portion is cured from the chromosome. The nucleotide sequence of the yggB gene of the obtained kanamycin-sensitive strain is determined and the strain having the 8-type mutant yggB gene was selected. The 8-type mutant yggB gene-introduced strain is named ATCC14067-yggB8 strain.

### Example 12

### <Construction of the 66-type mutant yggB-gene introduced strain and evaluation of L-glutamic acid-producing ability>

The 66-type mutation is a mutation which replaces the "C" at position 1673 of SEQ ID NO: 67 with a "T", and causes replacement of the Pro at position 424 with Leu in the amino acid sequence of SEQ ID NO: 68. The nucleotide sequence of the mutant type-yggB gene having this type of mutation is shown in SEQ ID NO: 69 and the amino acid sequence of the mutant type YggB protein encoded by this gene is shown in SEQ ID NO: 70.

In the same manner as in Example 7, the 66-type mutant yggB gene-introduced strain is constructed. Specifically, PCR is performed by using the synthetic DNAs shown in SEQ ID NOS: 30 and 71 as primers and the chromosomal DNA of *C. melassecola* ATCC17965 strain as a template to prepare an N-terminal fragment. Similarly, PCR is performed by using the synthetic DNAs shown in SEQ ID NOS: 34 and 72 as primers to prepare a C-terminal fragment. Subsequently, PCR is performed by using an equimolar mixture of the N-terminal fragment and the C-terminal fragment as a template and the synthetic DNAs shown in SEQ ID NOS: 9 and 34 as primers to amplify a partial fragment of the 66-type mutant yggB gene. The obtained yggB fragment is treated with SacI and inserted into the SacI site of pBS4S to obtain a plasmid for introducing this type of mutation. The thus obtained pBS4yggB66 is introduced into the chromosome of ATCC17965 strain in the same manner as described in Example 6 and then the vector portion is cured from the chromosome. The nucleotide sequence of the yggB gene of the obtained kanamycin-sensitive strain is determined and the strain having the 66-type mutant yggB gene was selected. The 66-type mutant yggB gene-introduced strain is named ATCC17965-yggB66 strain.

### Example 13

### <Screening of the mutant-type yggB genes by in vitro mutation>

Mutant-type yggB genes may be obtained by introducing a random mutation into the wild-type yggB gene *in vitro,* transforming a coryneform bacterium with the mutation-introduced yggB gene, and selecting mutant strains capable of producing L-glutamic acid without addition of surfactants or penicillin in the presence of an excess amount of biotin.

### (13-1) Construction of a yggB gene-disrupted strain

To perform screening for mutant-type yggB genes, first a yggB gene-disrupted strain was constructed. PCR was performed by using the synthetic DNAs shown in SEQ ID NOS: 39 and 40 as primers and the chromosomal DNA of ATCC 13869 strain as a template to prepare an N-terminal fragment. Similarly, PCR was performed by using the synthetic DNAs shown in SEQ ID NOS: 41 and 42 as primers to prepare a C-terminal fragment. SEQ ID NO: 40 and SEQ ID NO: 41 are complementary to each other. Subsequently, PCR was performed by using an equimolar mixture of the N-terminal fragment and the C-terminal fragment as a template and the synthetic DNAs shown in SEQ ID NOS: 39 and 42 as primers to obtain a fragment containing a yggB gene in which the ORF is deleted.

The obtained PCR fragment was treated with SacI and inserted into the SacI site of pBS4S to obtain a plasmid useful for disrupting the yggB gene. The pBS4ΔyggB thus obtained was introduced into the chromosome of ATCC 13 869 strain in the same manner as described in Example 6 and the vector portion is cured from the chromosome. PCR was performed by using the chromosomal DNA of the obtained kanamycin-sensitive strain as a template and the synthetic DNAs of SEQ ID NOS: 39 and 42 as primers to confirm that the yggB gene was disrupted. The obtained yggB-disrupted strain was named ATCC13869ΔyggB strain.

### (13-2) in vitro screening of mutant-type yggB genes

Mutagenesis of the yggB gene was performed as follows. First, the above-described pL5k plasmid was treated with XhoI and SalI and self-ligated to remove the region other than the yggB gene, and thereby the plasmid pL5kXS was obtained. A SalI recognition site does not exist on the nucleotide sequence of SEQ ID NO: 5 but is present on the multi-cloning site of pBS3. About 10 µg of the obtained pL5kXS was dissolved in 500 mM phosphate buffer containing 400 mM hydroxylamine and 1 mM EDTA (pH 6.0), and heated at 75°C for 30 to 90 minutes to introduce a mutation. The plasmid after mutagenesis treatment was desalted using SUPREC-02 (manufactured by TAKARA BIO INC.) and then introduced into ATCC13869ΔyggB strain by the method described in Example 6. Transformed cells were screened on the CM2B medium containing 25 µg/ml of kanamycin. As a control, pL5kXS without mutagenesis treatment was introduced into the ATCC13869ΔyggB strain. The appeared transformants are inoculated into 2 m of a liquid CM2BGU2 medium (CM2B medium described in Example 3 further containing 10 g/l glucose and 15 g/l urea) and cultured at 31.5°C for 5 hours with shaking, followed by determination of the concentration of L-glutamic acid which had accumulated in the culture broth.

Table 11 shows the result of culturing of the strain obtained by transforming the ATCC13869ΔyggB strain with the mutated pL5kXS on the CM2BGU2 medium. Three strains which cause accumulation of more than 1g/L of L-glutamic acid were obtained among the transformants transformed with 60, or 90-minute mutated plasmids. The amount of L-glutamic acid contained in the starting medium is 0.16g/L, and the amount of L-glutamic acid which had accumulated by the control ATCC13869ΔyggB/pL5kXS (without mutagenesis treatment) strain was 0.31g/L.

Table 12 shows the results of culturing transformants obtained by transforming the ATCC13869ΔyggB strain with mutated pL5kXS on the CM2BGU medium, which has the same composition as the above-mentioned CM2BGU2 medium except that the concentration of urea is 1.5g/L. One clone which causes accumulation of more than 1g/L of L-glutamic acid was obtained among the transformants transformed with 90-minute mutated plasmids.

**<Table 11. Amount of L-glutamic acid production by strains transformed with mutated plasmids>**

| **Glu accumulation (g/L)** | **Number of clones** | | |
|---|---|---|---|
| | **Time of mutagenesis** | | |
| | 30min | 60min | 90min |
| Glu≦0.4 | 40 | 36 | 39 |
| 0.4≦Glu≦0.6 | 8 | 11 | 6 |
| 0.6<Glu≦0.8 | 0 | 0 | 1 |
| 0.8<Glu≦1 | 0 | 0 | 0 |
| 1<Glu | 0 | 1 | 2 |

**<Table 12. Amount of L-glutamic acid production by strains transformed with mutated plasmids>**

| **Glu accumulation** (g/L) | **Number of clones** | |
|---|---|---|
| | **Time of mutagenesis** | |
| | 60min | 90min |
| Glu≦0.7 | 45 | 41 |
| 0.7<Glu≦0.9 | 2 | 7 |
| 0.9<Glu | 1 | 0 |

A plasmid was extracted from the strain transformed with 60-minute mutated plasmids which produced more than 1g/L of L-glutamic acid shown in Table 11, and the obtained plasmid was named pL5kXSm-22. A plasmid was also extracted from the strain transformed with 60-minute mutated plasmids which produced more than 0.9g/L of L-glutamic acid shown in Table 12, and the obtained plasmid was named pL5kXSm-27. ATCC13869 strain was transformed with the plasmids and the obtained strains were named ATCC13869ΔyggB/pL5kXS and ATCC13869ΔyggB/pL5kXSm-27, ATCC13869ΔyggB/pL5kXSm-22, respectively. These strains were cultured under the conditions described in Example 2, and the L-glutamic acid accumulation after a 4-hour culture was analyzed. Table 13 shows the mean value of three independent experiments. It was found that the ATCC13869Δ1yggB/pL5kXSm-27 strain and the ATCC13869ΔyggB/pL5kXSm-22 strain produced significantly more L-glutamic acid than the non-mutated plasmid-introduced strain. These results demonstrate that the mutant-type yggB gene of the present invention can be obtained by *in vitro* random mutagenesis. The nucleotide sequence of the yggB genes contained in pL5kXSm-22 is shown in SEQ ID NOS: 73 and 75, respectively. The pL5kXSm-22 plasmid has a mutation which replaces "C" at position 2745 with "T" in SEQ ID NO: 5 and causes replacement of Pro at position 437 of SEQ ID NO: 6 with Ser. Moreover, this mutation was accompanied by the mutation which replaces "C" at position 3060 with T in SEQ ID NO: 5 (22 type mutation). The nucleotide sequence of the mutant type yggB gene having this mutation is shown in SEQ ID NO: 73 and the amino acid sequence of the mutant type YggB protein encoded by the gene is shown in SEQ ID NO: 74.

**<Table 13. Amount of L-glutamic acid production by strains transformed with plasmids comprising mutated yggB gene>**

| Strains | 0D620 (x101) | Glu (g/L) |
|---|---|---|
| ATCC13869*Δ*yggB/pL5kXS (no treatment) | 0.253 | 0.58 |
| ATCC13869*Δ*yggB/pL5kXSm22 | 0.232 | 1.10 |
| ATCC13869*Δ*yggB/pL5kXSm27 | 0.245 | 0.62 |

### (13-3) Introduction of the mutant-type yggB genes into coryneform bacteria and evaluation of L-glutamic acid production

The mutant-type yggB gene obtained in (13-2) is introduced into a coryneform bacterium. The method of introducing the gene is as follows.

Each of the mutant-type yggB genes is introduced into pBS4S by the method described in Example 6 and the obtained plasmid is used to obtain a strain in which a wild-type yggB gene on the chromosome is replaced with the mutant-type yggB gene. A strain in which the mutant-type yggB gene is introduced and the control ATCC 13869 strain were cultured in the same manner as in Example 2. After completion of the culture, the amount of L-glutamic acid which had accumulated in the culture medium is measured by a known method to confirm that accumulation of L-glutamic acid is increased by introduction of the mutant-type yggB gene. In this manner, a strain having a mutant-type yggB gene-introduced strain with increased ability to produce L-glutamic acid can be obtained.

### Example 14

### <Evaluation of the L-glutamic acid analog-resistance of the strains having the mutant-type yggB gene>

### (14-1) Evaluation of resistance to 4-fluoroglutamic acid on a solid medium

It was predicted that strains which have enhanced L-glutamic acid-producing ability due to introduction of the mutant-type yggB gene would have decreased sensitivity (increased resistance) to L-glutamic acid analogs. Therefore, sensitivity of the strains to 4-fluoroglutamic acid was analyzed as follows.

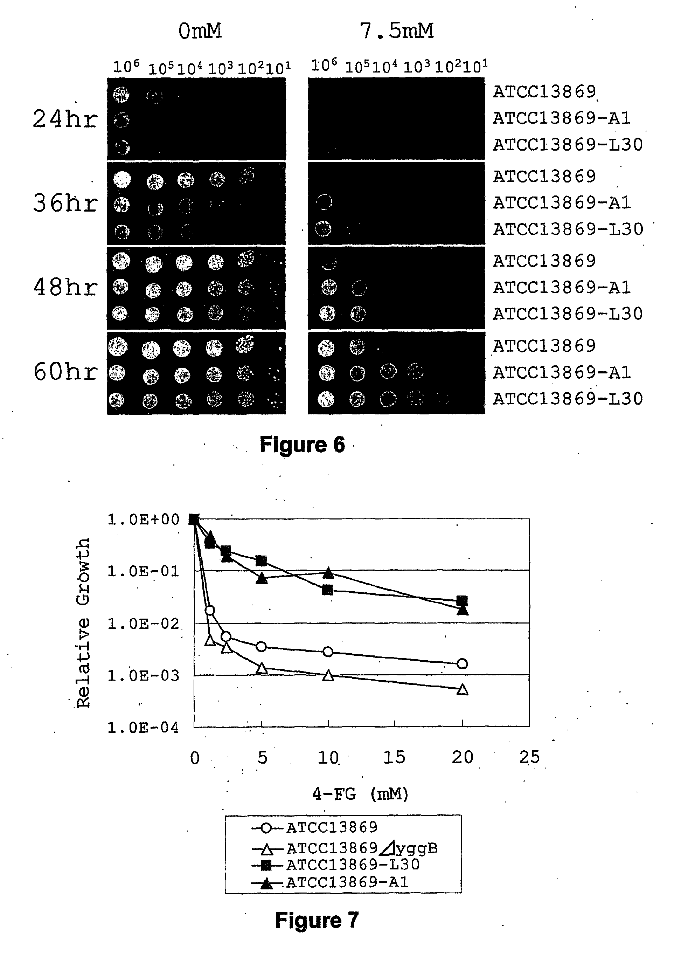

4-fluoroglutamic acid solution which was adjusted to pH6.7 with NaOH and sterilized with filtration was added to the minimum medium described in Example 4 so that the final concentration of 4-fluoroglutamic acid was 7.5mM. Each of the ATCC13869 strain, ATCC13869-L30 strain, and ATCC13869-A1 strain was spread over the CM-Dex plate and cultured overnight. Then, the cells were collected from the plate and washed with sterilized 0.85% NaCl solution, and diluted to the cell concentration described in Figure 6, spotted onto the plate containing 4-fluoroglutamic acid and a control plate which did not contain 4-fluoroglutamic acid, and cultured at 31.5°C. The time-course of growth of each of strain is shown in Figure 6. Although the wild-type ATCC13869 strain grows faster than the mutant-type yggB gene-introduced strains in the absence of 4-fluoroglutamic acid, the mutant-type yggB gene-introduced strain grow faster than wild-type ATCC13869 strain in the presence of 4-fluoroglutamic acid.

### (14-2) Evaluation of resistance to 4-fluoroglutamic acid in a liquid medium

4-fluoroglutamic acid was added into the minimum liquid medium having the same composition as described in Example 4 but not containing agar to the final concentration of 1.25mM, 2.5mM, 5mM, 10mM, and 20mM, respectively. Each of the ATCC13869 strain, ATCC13869ΔyggB strain, ATCC13869-L30 strain, and ATCC13869-A1 strain was spread over a CM-Dex plate and cultured at 31.5°C overnight. Then, cells were collected, and after washing with the sterilized 0.85% NaCl solution, inoculated into the liquid medium and cultured at 31.5 °C with shaking. When the OD660 of each strain cultured without 4-fluoroglutamic acid reached 1.0, the culture was terminated and the obtained culture solution was diluted appropriately and spread over the CM-Dex plate overnight. The number of colonies which appeared were calculated and designated as a viable cell number. Figure 7 shows the relative cell number at each concentration of 4-fluoroglutamic acid when the cell number of the culture without 4-fluoroglutamic acid was set to 1.

It was found that the ATCC13869-A1 strain and the ATCC13869-L30 strain have decreased sensitivity to 4-fluoroglutamic acid.

These results also showed that strains having a mutant-type yggB gene can be obtained by the screening using L-glutamic acid analogs such as 4-fluoroglutamic acid.

### Example 15

### <Construction of the yggB gene and odhA gene-double mutant strain>

The yggB gene and odhA gene-double mutant strain were prepared by introducing a mutant-type odhA gene into the above-mentioned ATCC13869-L30 strain.

First, each of the mutations shown in Table 14 was introduced into the odhA gene encoding E1o subunit of α-KGDH on the chromosome of the ATCC13869-L30 strain. In Table 14, nucleotide sequences of the region corresponding to nucleotides 2528 to 2562 of SEQ ID NO: 43 in each of the mutant-type odhA gene are shown. In Table 15, amino acid sequences of the region corresponding to amino acids 696 to 707 of SEQ ID NO: 44 in each of the amino acid sequences encoded by the mutant-type odhA genes are shown.

The L30sucA8 strain in which the odhA gene having the nucleotide sequence of SEQ ID NO: 45 is introduced can be obtained as follows. The mutant odhA gene fragment is prepared by PCR using primers of SEQ ID NOS: 53 and 54. The obtained fragment is digested with BamHI and ligated to the BamHI site of plasmid pKF 19m which is attached to Mutan-Super Express Km (Takara Bio). Then, PCR is performed using a primer of SEQ ID NO: 55 having a phosphorylated 5'-end and the selection primer of Mutan-Super Express Km, and the obtained PCR product is used to transform sup0-*E*. *coli* strain, such as MV1184 strain, to obtain a plasmid containing the mutant odhA fragment. This fragment is inserted into the pBS4S plasmid and the obtained plasmid is used to transform the ATCC13869-L30 strain to thereby obtain a strain in which the plasmid is integrated into its chromosome. Then, a strain which is resistant to sucrose and sensitive to kanamycin is selected from these strains. The nucleotide sequence of the odhA gene of the selected strains is determined and the strain in which function of α-KGDH is deficient by a frameshift mutation in the odhA gene is selected as ATCC13869-L30sucA8 (odhA8) strain.

The other odhA mutant strains can be obtained by the similar procedures using the ATCC13869-L30 strain.

The sucA801 strain in which a mutant odhA gene having a nucleotide sequence of SEQ ID NO: 47 is introduced can be obtained by a similar method as described above in which a primer of SEQ ID NO: 56 having a phosphorylated 5'-end is used instead of a primer of SEQ ID NO: 55.

The sucA805 strain in which a mutant odhA gene having a nucleotide sequence of SEQ ID NO: 49 is introduced can be obtained by a similar method as described above in which a primer of SEQ ID NO: 57 having a phosphorylated 5'-end is used instead of a primer of SEQ ID NO: 55.

The sucA77 strain in which a mutant odhA gene having a nucleotide sequence of SEQ ID NO: 51 is introduced can be obtained by a similar method as described above in which a primer of SEQ ID NO: 58 having a phosphorylated 5'-end is used instead of a primer of SEQ ID NO: 55.

The L30sucA8 strain does not have intracellular α-KGDH because the sucA8 mutation is a frame-shift mutation which causes an immature truncation of the α-KGDH protein. On the other hand, sucA801 strain, sucA805 strain, and sucA77 strain have decreased but some α-KGDH activity because these mutations are not frame-shift mutations and do not cause immature truncation of the α-KGDH protein.

**Table 14: partial nucleotide acid sequence of odhA mutant genes**

| **Strains** | **Nucleotide sequence of odhA gene** |
|---|---|
| ATCC13869-L30 | CTG GCT AAG CTG CGT GGC TAC GAC GTC GGA GGC ACC |
| L30sucA8 | CTG GCT AAG CTG CGT C GAC GTC GGA GGC ACC |
| L30sucA801 | CTG GCT AAG CTG CGT CTC GAC GTC GGA GGC ACC |
| L30sucA805 | CTG GCT AAA AGC TGC GTC GAC GTC GGA GGC ACC |
| L30sucA77 | CTG GCT ATA AGC TGC GTC GAC GTC GGA GGC ACC |

**Table 15: amino acid sequence of odhA mutants**

| **Strains** | **Amino acid sequence of E1o subunit** |
|---|---|
| Wild | Leu Ala Lys Leu Arg Gly Tyr Asp Val Gly Gly Thr |
| L30sucA8 (ΔsucA) | Leu Ala Lys Leu Arg Arg Arg Arg Arg His |
| L30sucA801 | Leu Ala Lys Leu Arg --- Leu Asp Val Gly Gly Thr |
| L30sucA805 | Leu Ala Lys Ser Cys --- Val Asp Val Gly Gly Thr |
| L30sucA77 | Leu Ala Ile Ser Cys --- Val Asp Val Gly Gly Thr |

### <L-glutamic acid production using the yggB gene and the odhA gene-double mutant strain>

L-glutamic acid productivity of the obtained ygg gene and odhA gene-double mutant strain was evaluated by culturing these strains in a Sakaguchi flask. Each of the strains listed in Table 14 was cultured at 31.5°C overnight on CM-Dex agar medium, and then 1/6 of the culture was transferred to 20 ml of a medium containing 60 g/l glucose, 22.5 g/l (NH₄)₂SO₄, g/l KH₂PO₄, 0.4 g/l MgSO₄·7H₂O, 0.01 g/l FeSO₄·7H₂O, 0.01 g/l MnSO₄·4-5H₂O, 200 µg/l vitamin B1, 0.48 g/l soybean protein hydrolysate, and 300 µg/l biotin (adjusted to pH 8.0 with KOH), added with CaCO₃ and cultured with stirring at 115rpm at 31.5°C. The amount of accumulated L-glutamic acid after 19 hours of culture is shown in Table 16. The sucA801, sucA805, and sucA77 strains exhibited higher L-glutamic acid productivity than the ATCC13869-L30 strain carrying a wild-type odhA gene and the sucA8 strain carrying odhA gene with a frame-shift mutation. These results showed that L-glutamic acid is efficiently produced by regulating α-KGDH activity by introducing mutations into the proximate of thiamine pyrophosphate binding region of the odhA gene.

**Table 16: L-glutamic acid production by odhA mutant strains**

| Strain | L-glutamic acid (g/L) |
|---|---|
| ATCC13869-L30 | 4.9 |
| L30sucA8 | 19.8 |
| L30sucA801 | 22.1 |
| L30sucA805 | 23.8 |
| L30sucA77 | 21.6 |

### Example 16

### <Disruption of the odhA gene in the ATCC14067yggB8 strain>

The odhA gene was disrupted in the ATCC14067strain and the ATCC14067yggB8 strain, respectively and the obtained strains were cultured. First, a plasmid for disrupting the odhA gene was constructed. PCR was performed using the synthetic oligonucleotides shown in SEQ ID NOS: 77 and 78 as primers and chromosomal DNA of ATCC14067 strain as a template to amplify a fragment covering the N-terminal region of the odhA gene. Another PCR was performed using the synthetic oligonucleotides shown in SEQ ID NOS: 79 and 80 as primers and chromosomal DNA of ATCC14067 strain as a template to amplify a fragment covering C-terminal region of the odhA gene. Subsequently, PCR was performed using a mixture of equimolar amounts of the N-terminal fragment and the C-terminal fragment as a template and synthetic DNAs of SEQ ID NOS: 81 and 82 as primers to prepare a fragment in which an internal sequence of the odhA gene is deleted. The obtained PCR product was digested with BamHI and inserted into the pBS4S constructed in Example 1 to obtain a plasmid pBSΔsucA47.

ATCC14067 strain and ATCC14067yggB8 of Example 11 were transformed with the pBSΔsucA47 in the same way as described in Example 6 to introduce the deletion-type odhA gene into the chromosome and remove only the vector portion. Strains in which the odhA gene was disrupted were selected from kanamycin-sensitive strains by PCR using the primers of SEQ ID NOS: 77 and 80. The thus obtained strains were named ATCC14067ΔodhA strain and ATCC14067ΔodhA yggB8 strain, respectively.

Table 17 shows the results of cultivation of the ATCC14067ΔodhA strain and the ATCC14067ΔodhA yggB8 strain according to the method described in Example 3. It was found that introduction of the 8-type mutant yggB gene enhanced L-glutamic acid producing ability of the odhA gene-disrupted strain.

**<Table 17. L-glutamic acid production of the odhA gene-disrupted strain and the odhA-disrupted and 8-type mutant yggB gene-introduced strain >**

| | OD620 (x101) | Glu (g/L) |
|---|---|---|
| ATCC14067*Δ*odhA | 0.270 | 5.8 |
| ATCC14067*Δ*odhA yggB8 | 0.242 | 22.0 |

### Example 17

### <Disruption of the symA gene in the yggB-mutant strain>

The symA gene was disrupted in the 2A-1R strain constructed in Example 3 and having an IS inserted into the yggB gene, and the obtained strain was cultured in comparison with the 2A-1R strain. The nucleotide sequence of the symA gene from the ATCC13869 strain is shown in SEQ ID NO: 86, and the amino acid sequence is shown in SEQ ID NO: 87. First, a plasmid for the purpose of disrupting the symA gene was constructed. PCR was performed using the synthetic oligonucleotides shown in SEQ ID NOS: 88 and 89 as primers and chromosomal DNA of ATCC 13869 strain as a template to amplify a fragment covering N-terminal region of the symA gene. Another PCR was performed using the synthetic oligonucleotides shown in SEQ ID NOS: 90 and 91 as primers and chromosomal DNA of ATCC 13869 strain as a template to amplify a fragment covering the C-terminal region of the symA gene. Subsequently, PCR was performed using a mixture of equimolar amounts of the N-terminal fragment and the C-terminal fragment as a template and synthetic DNAs of SEQ ID NOS: 88 and 91 as primers to prepare a fragment in which an internal sequence of the SymA gene is deleted. The obtained PCR product was digested with BamHI and inserted into the pBS4S constructed in Example 1 to obtain a plasmid pBSΔ1867.

The 2A-IR strain was transformed with the pBSΔ1867 in the same way as described in Example 6 to introduce the deletion-type SymA gene into the chromosome and remove only the vector portion. Strains in which the SymA gene was disrupted were selected from kanamycin-sensitive strains by PCR using the primers of SEQ ID NOS: 88 and 91. The thus obtained strains were named 2A-1RΔSymA strain.

Table 18 shows the results of cultivation of the 2A-1RΔSymA strain and the 2A-1R strain according to the method described in Example 3. It was found that deletion of the SymA gene further enhanced L-glutamic acid producing ability of the mutant-type yggB gene-introduced strain.

**<Table 18. L-glutamic acid production of the 2A-1R strain and the symA gene-disrupted 2A-1R strain>**

| | OD620(×51) | Glu (g/L) |
|---|---|---|
| 2A-1R | 0.846 | 12.4 |
| 2A-IRΔsymA | 0.709 | 15.8 |

### Example 18

### <Construction of a wild-type yggB gene-amplified strain>

pL5k containing a wild-type yggB gene of a coryneform bacterium was used to introduce the wild-type yggB gene into coryneform bacterium. A plasmid having a similar structure as pL5k can also be constructed by performing PCR using primers of SEQ ID Nos: 59 and 60 and chromosomal DNA of ATGC 13869 strain as a template, digesting the amplified product with BamHI, and inserting the resulting fragment into the BamHI site of pVK9. pVK9 is a shuttle vector which was obtained by blunt-ending the AvaII site of pHSG299 (Takara Bio) and inserting therein a fragment involved in autonomous replication in coryneform bacteria excised from pHK4 (JP-A-05-007491) with BamHI and KpnI.

A *Corynebacterium glutamicum* ATCC 13869 strain was transformed with pL5k by the electric pulse method (JP-A-2-207791). The transformed cells were spread over a CM2B plate medium (10 g/l polypeptone, 10 g/l yeast extract, 5 g/l NaCl, 20 g/l agar, adjusted to pH 7.0 with KOH) and cultured at 31.5°C for one night. On the next day, colonies which appeared were purified on a CM2B plate medium containing 25 µg/ml kanamycin to obtain a wild-type yggB-gene amplified strain. Plasmids were extracted from the transformants by a conventional method to confirm that the target plasmid was introduced. The wild-type yggB-gene amplified strain thus obtained was named ATCC13869/pL5k. As a control strain, ATCC13869/pVK9 introduced with pVK9 was constructed in the same manner as described above.

### Example 19

### <Evaluation of a wild-type yggB gene-amplified strain>

### (2-1) Evaluation under biotin-limited culture conditions

Three clones were isolated from each of the wild-type yggB gene-amplified strain and control strain constructed in Example 17, and each clone was inoculated into 20 ml of a seed culture medium (80 g/l glucose, 30 g/l ammonium sulfate, 1 g/l KH₂PO₄, 0.4 g/l MgSO₄.7H₂O, 0.01 g/l FeSO₄.7H₂O, 0.01 g/l MnSO₄.4-5H₂O, 200 µg/l VB1,60 µg/l biotin, 0.48 g/l soybean hydrolysate (T-N), adjusted to pH 8.0 with KOH: autoclaved at 115°C for 10 minutes), and then 1 g of sterilized calcium carbonate was added thereto, followed by shaking culture at 31.5°C. After complete consumption of sugars, 2 ml of the culture was inoculated into 20 ml of main culture medium containing no biotin (80 g/l glucose, 30 g/l ammonium sulfate, 1 g/l KH₂PO₄, 0.4 g/l MgSO₄.7H₂O, 0.01 g/l FeSO₄.7H₂O, 0.01. g/l MnSO₄.4-5H₂O, 200 µg/l VB1, 0.48 g/l soybean hydrolysate (T-N), adjusted to pH 8.0 with KOH: autoclaved at 115°C for 10 minutes), and then 1 g of sterilized calcium carbonate was added thereto, followed by a shaking culture at 31.5°C. After the complete consumption of sugars, the concentration of L-glutamic acid in the medium was determined. As a result, it was found that the wild-type yggB gene-amplified strain (ATCC13869/pL5k) caused accumulation of more L-glutamic acid than the vector-introduced strain. (In Table 19, OD620 is turbidity at 620nm of culture solution diluted to 101 times, and indicates the cell amount and GH (g/L) indicates the amount of L-glutamic acid which hadaccumulated.)

**Table 19. Amount of L-glutamic acid which had accumulated under biotin limited conditions**

| | OD620 | GH(g/L) |
|---|---|---|
| ATCC13869/pVK9-1 | 51.4 | 43.6 |
| ATCC13869/pVK9-2 | 51.3 | 43.8 |
| ATCC13869/pVK9-3 | 52.6 | 43.0 |
| ATCC13869/pL5k-1 | 44.7 | 46.0 |
| ATCC13869/pL5k-2 | 47.2 | 45.2 |
| ATCC13869/pL5k-3 | 43.9 | 45.8 |
| Blank | 0 | 0.4 |

### (2-2) Evaluation under surfactant-added conditions

The same clones used in the above (2-1) were inoculated into 20 ml of a seed culture medium (80 g/l glucose, 30 g/l ammonium sulfate, 1 g/l KH₂PO₄, 0.4 g/l MgSO₄.7H₂O, 0.01 g/l FeSO₄.7H₂O, 0.01 g/l MnSO₄.4-5H₂O, 200 µg/l VB1, 60 µg/l biotin, 0.48 g/l soybean hydrolysate (T-N), adjusted to pH 8.0 with KOH: autoclaved at 115°C for 10 minutes), and then 1 g of sterilized calcium carbonate was added thereto, followed by a shaking culture at 31.5°C. After the complete consumption of sugars, 2 ml of the culture was inoculated into 20 ml of main culture medium (80 g/l glucose, 30 g/l ammonium sulfate, 1 g/l KH₂PO₄, 0.4 g/l MgSO₄.7H₂0, 0.01 g/l FeSO₄.7H₂O, 0.01 g/l MnSO₄.4-5H₂O, 200 µg/l VB 1, 60 µg/l biotin, 0.48 g/l soybean hydrolysate (T-N), adjusted to pH 8.0 with KOH: autoclaved at 115°C for 10 minutes), and then 1 g of sterilized calcium carbonate was added thereto, followed by a shaking culture at 31.5°C. After 2 hours from the beginning of the culture, Tween 40 was added to a final concentration of 5 g/L, and the culture was continued. After the complete consumption of sugars, the concentration of L-glutamic acid in the medium was determined. As a result, it was found that the wild-type yggB gene-amplified strain (ATCC13869/pL5k) caused accumulation of more L-glutamic acid than the vector-introduced strain.

**Table 20 Amount of L-glutamic acid which had accumulated under surfactant-added conditions**

| | OD620 | GH(g/L) |
|---|---|---|
| ATCC13869/pVK9-1 | 50.7 | 40.0 |
| ATCC13869/pVK9-2 | 50.7 | 39.3 |
| ATCC13869/pVK9-3 | 51.7 | 38.7 |
| ATCC13869/pL5k-1 | 38.6 | 42.1 |
| ATCC13869/pL5k-2 | 40.6 | 46.0 |
| ATCC13869/pL5k-3 | 39.1 | 45.5 |
| Blank | 0 | 0.4 |

(OD620 is turbidity at 620nm of culture solution diluted 101 times, and indicates the cell amount and GH indicates the amount of L-glutamic acid which had accumulated.)

### (2-3) Evaluation under penicillin G-added conditions

The same clones used in the above (2-1) were inoculated into 20 ml of a seed culture medium (80 g/l glucose, 30 g/l ammonium sulfate, g/l KH₂PO₄, 0.4 g/l MgSO₄.7H₂O, 0.01 g/l FeSO₄.7H₂O, 0.01 g/l MnSO₄.4-5H₂O, 200 µg/l VB1, 60 µg/l biotin, 0.48 g/l soybean hydrolysate (T-N), adjusted to pH 8.0 with KOH: autoclaved at 115°C for 10 minutes), and then 1 g of sterilized calcium carbonate was added thereto, followed by a shaking culture at 31.5°C. After the complete consumption of sugars, 2 ml of the culture was inoculated into 20 ml of main culture medium (80 g/l glucose, 30 g/l ammonium sulfate, 1 g/l KH₂PO₄, 0.4 g/l MgSO₄.7H₂O, 0.01 g/l FeSO₄.7H₂O, 0.01 g/l MnSO₄.4-5H₂O, 200 µg/l VB1,60 µg/l biotin, 0.48 g/l soybean hydrolysate (T-N), adjusted to pH 8.0 with KOH: autoclaved at 115°C for 10 minutes), and then 1 g of sterilized calcium carbonate was added thereto, followed by a shaking culture at 31.5°C. After 2 hours of the culture, penicillin G was added to a final concentration of 0.5 U/ml and the culture was continued. After the complete consumption of sugars, the concentration of L-glutamic acid in the medium was determined. As a result, it was found that the wild-type yggB gene-amplified strain (ATCC13869/pL5k) caused accumulation of more L-glutamic acid than the vector-introduced strain. (Table 21)

**Table 21 Amount of L-glutamic acid accumulated under penicillin G-added conditions**

| | OD620 | GH(g/L) |
|---|---|---|
| ATCC13869/pVK9-1 | 66.4 | 24.4 |
| ATCC 13869/pVK9-2 | 65.5 | 24.0 |
| ATCC13869/pVK9-3 | 66.9 | 24.4 |
| ATCC13869/pL5k-1 | 59.5 | 30.8 |
| ATCC13869/pL5k-2 | 59.6 | 29.8 |
| ATCC13869/pL5k-3 | 60.1 | 29.4 |
| Blank | 0 | 0.4 |

(OD620 is turbidity at 620nm of culture solution diluted 101 times, and indicates the cell amount and GH indicates the amount of L-glutamic acid which had accumulated.)

### INDUSTRIAL APPLICABILITY

According to the present invention, L-glutamic acid is efficiently produced by using a strain modified by using yggB genes.

While the invention has been described in detail with reference to exemplary embodiments thereof, it will be apparent to one skilled in the art that various changes can be made, and equivalents employed, without departing from the scope of the invention. Each of the aforementioned documents is incorporated by reference herein in its entirety.

### The application discloses the following embodiments:

1. A coryneform bacterium having L-glutamic acid-producing ability, wherein said coryneform bacterium is modified by using a yggB gene so that L-glutamic acid-producing ability of the strain is enhanced as compared to a non-modified strain.
2. The coryneform bacterium according to item 1, wherein said yggB gene is selected from the group consisting of:
   (a) a DNA comprising nucleotides 1437 to 3035 of SEQ ID No: 5,
   (b) a DNA that is able to hybridize with a nucleotide sequence complementary to nucleotides 1437 to 3035 of SEQ ID No: 5, or a probe prepared from said nucleotides under stringent conditions, and wherein said DNA increases L-glutamic acid-producing ability of a coryneform bacterium when it is introduced into the coryneform bacterium,
   (c) a DNA comprising nucleotides 507 to 2093 of SEQ ID No: 61,
   (d) a DNA that is able to hybridize with a nucleotide sequence complementary to nucleotides 507 to 2093 of SEQ ID No: 61, or a probe prepared from said nucleotides under stringent conditions, and wherein said DNA increases L-glutamic acid-producing ability of a coryneform bacterium when it is introduced into the coryneform bacterium,
   (e) a DNA comprising nucleotides 403 to 2001 of SEQ ID No: 67,
   (f) a DNA that is able to hybridize with a nucleotide sequence complementary to nucleotides 403 to 2001 of SEQ ID No: 67, or a probe prepared from said nucleotides under stringent conditions, and wherein said DNA increases L-glutamic acid-producing ability of a coryneform bacterium when it is introduced into the coryneform bacterium,
   (g) a DNA comprising nucleotides 501 to 2099 of SEQ ID No: 83, and
   (h) a DNA that is able to hybridize with a nucleotide sequence complementary to nucleotides 501 to 2099 of SEQ ID No: 83, or a probe prepared from said nucleotides under stringent conditions, and wherein said DNA increases L-glutamic acid-producing ability of a coryneform bacterium when it is introduced into the coryneform bacterium.
3. The coryneform bacterium according to item 1, wherein said yggB gene encodes a protein selected from the group consisting of:
   (A) a protein comprising an amino acid sequence selected from the group consisting of SEQ ID NO: 6, 62, 68, 84, and 85, and
   (B) a protein comprising an amino acid sequence selected from the group consisting of SEQ ID NO: 6, 62, 68, 84, and 85, whereby one or several amino acids in said protein are substituted, deleted, inserted, or added, and said yggB gene increases L-glutamic acid-producing ability of a coryneform bacterium when it is introduced into the coryneform bacterium.
4. The coryneform bacterium according to any one of items 1 to 3, wherein said coryneform bacterium is modified so to enhance expression of the yggB gene as compared to a non-modified strain.
5. The coryneform bacterium according to item 4, wherein expression of the yggB gene is enhanced by increasing a copy number of the yggB gene or modifying an expression regulating sequence of the yggB gene.
6. The coryneform bacterium according to item 1, wherein said coryneform bacterium is modified by introducing a mutant-type yggB gene.
7. The coryneform bacterium according to item 6, wherein said mutant-type yggB gene has a mutation in the region encoding amino acids 419-533 of SEQ ID NO: 6, 68, 84 or 85, or amino acids 419-529 of SEQ ID NO: 62.
8. The coryneform bacterium according to item 7, wherein said mutation is deletion of said region.
9. The coryneform bacterium according to item 7, wherein said mutation is insertion of an insertion sequence or transposon into said region.
10. The coryneform bacterium according to item 7, wherein said mutant-type yggB gene has a mutation which results in replacement of the proline in said region with another amino acid.
11. The coryneform bacterium according to item 7, wherein said mutant-type yggB gene has a mutation which results in replacement of the proline at position 424 and/or the proline at position 437 in SEQ ID NO: 6,62,68, 84 or 85 with another amino acid.
12. The coryneform bacterium according to item 6, wherein said mutant-type yggB gene has a mutation in the transmembrane-coding region of the yggB protein.
13. The coryneform bacterium according to item 12, wherein said transmembrane-coding region is selected from the group consisting of amino acids 1-23, amino acids 25-47, amino acids 62-84, amino acids 86-108, and amino acids 110-132 of SEQ ID NO: 6, 62, 68, 84 or 85.
14. The coryneform bacterium according to item 12, wherein said mutation is introduced without causing a frame-shift.
15. The coryneform bacterium according to item 12, wherein said mutant-type yggB gene has a mutation which results in replacement of the alanine at position 100 and/or the alanine at position 111 in SEQ ID NO: 6, 62, 68, 84 or 85 with another amino acid.
16. The coryneform bacterium according to item 12, wherein said mutant-type yggB gene has a mutation which results in insertion of at least one amino acid between leucine at position 14 and tryptophan at position 15 in SEQ ID NO: 6, 62, 68, 84 or 85.
17. The coryneform bacterium according to any one of items 6 to 16, wherein resistance to L-glutamic acid analogs of said strain is increased by introduction of the mutant-type yggB gene.
18. The coryneform bacterium according to any one of items 6 to 16, wherein said coryneform bacterium is further modified to inactivate a gene which suppresses a function of said mutant-yggB gene.
19. The coryneform bacterium according to item 18, wherein said gene which suppresses a function of said mutant-yggB gene is a symA gene and wherein said symA gene is selected from the group consisting of:
   (a) a DNA comprising nucleotides 585 to 1121 of SEQ ID No: 86, and
   (b) a DNA that is able to hybridize with a nucleotide sequence complementary to nucleotides 585 to 1121 of SEQ ID No: 86, or a probe prepared from said nucleotides under stringent conditions, and wherein said DNA suppresses a function of said mutant-type yggB gene in the coryneform bacterium.
20. The coryneform bacterium according to any one of items 1 to 19, wherein said coryneform bacterium is further modified so to decrease α-ketoglutarate dehydrogenase activity.
21. The coryneform bacterium according to any one of items 1 to 20, wherein said coryneform bacterium belongs to the genus Corynebacterium or the genus Brevibacterium.
22. A method for producing L-glutamic acid comprising
   culturing the coryneform bacterium according to any one of items 1 to 21 in a medium so to cause accumulation of L-glutamic acid in the medium or the bacterium, and
   collecting L-glutamic acid from the medium or the bacterium.
23. A mutant-type yggB gene selected from the group consisting of:
   (a) a gene encoding the amino acid sequence of SEQ ID NO: 8,
   (b) a gene encoding a protein having a homology of not less than 80% to SEQ ID NO: 8, and having a function to increase L-glutamic acid-producing ability of coryneform bacterium in the presence of excess biotin when said gene is introduced into the coryneform bacterium,
   (c) a gene encoding amino acid sequence of SEQ ID NO: 20,
   (d) a gene encoding a protein having a homology of not less than 80% to SEQ ID NO: 20, and having a function to increase L-glutamic acid-producing ability of coryneform bacterium in the presence of excess biotin when said gene is introduced into the coryneform bacterium,
   (e) a gene encoding amino acid sequence of SEQ ID NO: 22,
   (f) a gene encoding a protein having a homology of not less than 80% to SEQ ID NO: 22, and having a function to increase L-glutamic acid-producing ability of coryneform bacterium in the presence of excess biotin when said gene is introduced into the coryneform bacterium,
   (g) a gene encoding amino acid sequence of SEQ ID NO: 24,
   (h) a gene encoding a protein having a homology of not less than 80% to SEQ ID NO: 24, and having a function to increase L-glutamic acid-producing ability of coryneform bacterium in the presence of excess biotin when said gene is introduced into the coryneform bacterium,
   (i) a gene encoding amino acid sequence of SEQ ID NO: 64,
   (j) a gene encoding a protein having a homology of not less than 80% to SEQ ID NO: 64, and having a function to increase L-glutamic acid-producing ability of coryneform bacterium in the presence of excess biotin when said gene is introduced into the coryneform bacterium,
   (k) a gene encoding amino acid sequence of SEQ ID NO: 70,
   (l) a gene encoding a protein having a homology of not less than 80% to SEQ ID NO: 70, and having a function to increase L-glutamic acid-producing ability of coryneform bacterium in the presence of excess biotin when it is introduced into the coryneform bacterium.
   (m) a gene encoding amino acid sequence of SEQ ID NO: 74,
   (n) a gene encoding a protein having a homology of not less than 80% to SEQ ID NO: 74, and having a function to increase L-glutamic acid-producing ability of coryneform bacterium in the presence of excess biotin when said gene is introduced into the coryneform bacterium.
24. A method for producing a coryneform bacterium having a mutant-type yggB gene comprising:
   a) inoculating a coryneform bacterium which is deficient in a gene encoding α-ketoglutarate dehydrogenase into a medium containing excess biotin, and
   b) selecting a strain that is capable of causing accumulation of L-glutamic acid in the medium.
25. A method for producing a coryneform bacterium having a mutant-type yggB gene comprising:
   a) inoculating a coryneform bacterium having a yggB gene which contains a randomly introduced *in vitro* mutation into a medium containing excess biotin, and
   b) selecting a strain that is capable of causing accumulation of L-glutamic acid in the medium.
26. A method for producing a coryneform bacterium having a mutant-type yggB gene comprising:
   a) inoculating a coryneform bacterium having a transposable element randomly introduced onto a chromosome into a medium containing excess biotin, and
   b) selecting a strain that is capable of causing accumulation of L-glutamic acid in the medium.
27. A method for producing a coryneform bacterium having a mutant-type yggB gene comprising:
   a) inoculating a coryneform bacterium into a medium containing L-glutamic acid analogs, and
   b) selecting a strain which grows on the medium containing L-glutamic acid analogs.
28. A method for producing a coryneform bacterium comprising
   a) inoculating a coryneform bacterium which is deficient in a gene encoding α-ketoglutarate dehydrogenase into a medium containing excess biotin, and
   b) selecting a strain that is capable of causing accumulation of L-glutamic acid in the medium,
   wherein said strain is the coryneform bacterium of any one of items 6 to 16.
29. The method of producing a coryneform bacterium according to item 28, wherein said medium contains not less than 30 µg/l of biotin.

## Claims

1. Mutant yggB gene that has the ability of enhancing L-glutamic acid production when introduced into Corynebacterium glutamicum ATCC 13869, in the presence of at least 30 µg/l biotin, as compared to Corynebacterium glutamicum ATCC 13869 wherein said mutant yggB gene has not been introduced.

2. The mutant type yggB gene according to claim 1, encoding an amino acid sequence obtainable by replacing, deleting, inserting or adding 1 to 20 amino acid residues in an amino acid sequence selected from Seq Id No.: 6, 62, 68 or 84.

3. The mutant type yggB gene according to claim 1 or 2, encoding an amino acid sequence having a mutation in any one selected from the group consisting of amino acids 1-23, amino acids 25-47, amino acids 62-84, amino acids 86-108, and amino acids 110-132 of SEQ ID No: 6, 62, 68, 84 or 85.

4. The mutant type yggB gene according to claim 3, wherein said mutation is not a frame shift mutation.

5. The mutant type yggB gene according to claim 3 or 4, encoding an amino acid sequence obtainable by substituting one or more amino acids in an amino acid sequence selected from Seq Id No.: 6, 62, 68 or 84, by missense mutation.

6. The mutant type yggB gene according to any one of claims 1 to 5, encoding an amino acid sequence having a replacement of the alanine at position 100 and/or the alanine at position 111 in SEQ ID NO: 6, 62, 68, 84 or 85 with another amino acid.

7. The mutant type yggB gene according to any one of claims 1 to 6, encoding
a) an amino acid sequence having a homology of not less than 80% to SEQ ID NO: 22, or
b) an amino acid sequence according to Seq Id No. : 22 having 1 to 20 amino acids replaced, deleted, inserted or added, or
c) an amino acid sequence having a homology of not less than 80% to SEQ ID NO: 24, or
d) an amino acid sequence according to Seq Id No.: 24 having 1 to 20 amino acids replaced, deleted, inserted or added, or
e) an amino acid sequence having a homology of not less than 80% to SEQ ID NO: 64, or
f) an amino acid sequence according to Seq Id No.: 64 having 1 to 20 amino acids replaced, deleted, inserted or added.

8. The mutant type yggB gene according to claim 1 or 2, encoding an amino acid sequence having a mutation in amino acids 419-533 of SEQ ID NO: 6, 68, 84 or 85, or amino acids 419-529 of SEQ ID NO: 62.

9. The mutant type yggB gene according to claim 8, encoding an amino acid sequence having a deletion of amino acids 419-533 in an amino acid sequence selected from Seq Id No.: 6, 68, 84 or 85, or a deletion of amino acids 419-529 of Seq Id No.: 62.

10. The mutant type yggB gene according to claim 8, encoding an amino acid sequence having an insertion of an insertion sequence or transposon into amino acids 419-533 in an amino acid sequence selected from Seq Id No.: 6, 68, 84 or 85, or amino acids 419-529 of Seq Id No.: 62.

11. The mutant type yggB gene according to claim 1, 2, 8 or 10, encoding
a) an amino acid sequence according to Seq Id No.: 8, or
b) an amino acid sequence having a homology of not less than 80% to Seq Id No.: 8, or
c) an amino acid sequence according to Seq Id No.: 8 having 1 to 20 amino acids replaced, deleted, inserted or added.

12. The mutant type yggB gene according to claim 8, encoding an amino acid sequence having a replacement of proline with another amino acid at
position 424 in SEQ ID NO: 6,62,68,84 or 85;
position 437 in SEQ ID NO: 6,62,68,84 or 85;
position 453 in SEQ ID NO: 6,62,68,84 or 85;
position 457 in SEQ ID NO: 6,62,68,84 or 85;
position 462 in SEQ ID NO: 6,62,68,84 or 85;
position 469 in SEQ ID NO: 6,62,68,84 or 85;
position 484 in SEQ ID NO: 6,62,68,84 or 85;
position 489 in SEQ ID NO: 6,62,68,84 or 85;
position 497 in SEQ ID NO: 6,62,68,84 or 85;
position 515 in SEQ ID NO: 6,62,68,84 or 85;
position 529 in SEQ ID NO: 6,68,84 or 85;
position 525 in SEQ ID NO:62;
position 533 in SEQ ID NO 68,84 or 85;
position 529 in SEQ ID NO:62.

13. The mutant type yggB gene according to claim 12, encoding an amino acid sequence having a replacement of the proline at position 424 and/or the proline at position 437 in SEQ ID NO: 6, 62, 68, 84 or 85 with another amino acid.

14. The mutant type yggB gene according to claim 12 or 13, encoding
a) an amino acid sequence according to Seq Id No.: 70 or 74, or
b) an amino acid sequence having a homology of not less than 80% to Seq Id No.: 70 or 74, or
c) an amino acid sequence according to Seq Id No.: 70 or 74 having 1 to 20 amino acids replaced, deleted, inserted or added.

15. The mutant type yggB gene according to any one of claims 1 to 3, encoding an amino acid sequence having an insertion of at least one amino acid between leucine at position 14 and tryptophan at position 15 in SEQ ID NO: 6, 62, 68, 84 or 85.

16. The mutant type yggB gene according to claim 15, encoding
a) an amino acid sequence according to Seq Id No.: 20, or
b) an amino acid sequence having a homology of not less than 80% to Seq Id No.: 20, or
c) an amino acid sequence according to Seq Id No.: 20 having 1 to 20 amino acids replaced, deleted, inserted or added.

17. Coryneform bacterium having L-glutamic acid-producing ability, comprising a mutant yggB gene according to any one of claims 1 to 16.

18. The coryneform bacterium according to any one of claims 1 to 17, wherein said coryneform bacterium is further modified to inactivate a symA gene comprising nucleotides 585 to 1121 of SEQ ID No: 86.

19. The coryneform bacterium according to any one of claims 1 to 18, wherein said coryneform bacterium is further modified to decrease α-ketoglutarate dehydrogenase activity.

20. The coryneform bacterium according to any one of claims 1 to 19, wherein said coryneform bacterium belongs to the genus Corynebacterium or the genus Brevibacterium.

21. Method for producing L-glutamic acid comprising culturing a coryneform bacterium having L-glutamic acid-producing ability according to any one of claims 17 to 20 in a medium so as to cause accumulation of L-glutamic acid in the medium or the bacterium, and collecting L-glutamic acid from the medium or the bacterium.

22. A method for producing a seasoning, comprising producing L-glutamic acid according to the method of claim 21.
